(19) Europäisches Patentamt
European Patent Office
Office européen des brevets

(11) **EP 4 545 531 A1**

(12) **EUROPEAN PATENT APPLICATION**
published in accordance with Art. 153(4) EPC

(43) Date of publication:
**30.04.2025 Bulletin 2025/18**

(21) Application number: **23830201.2**

(22) Date of filing: **26.06.2023**

(51) International Patent Classification (IPC):
***C07D 487/06*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 487/06**

(86) International application number:
**PCT/CN2023/102500**

(87) International publication number:
**WO 2024/002024 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.06.2022 CN 202210734328**
**15.09.2022 CN 202211120467**
**20.02.2023 CN 202310135765**

(71) Applicant: **CSPC Zhongqi Pharmaceutical Technology (Shijiazhuang) Co., Ltd.**
**Shijiazhuang, Hebei 050035 (CN)**

(72) Inventors:
- **WANG, Kuanglei**
  **Shijiazhuang, Hebei 050035 (CN)**
- **GUO, Jianqiao**
  **Shijiazhuang, Hebei 050035 (CN)**
- **ZHAO, Chuanwu**
  **Shijiazhuang, Hebei 050035 (CN)**
- **CHU, Wenhao**
  **Shijiazhuang, Hebei 050035 (CN)**
- **ZHANG, Xiaolin**
  **Shijiazhuang, Hebei 050035 (CN)**
- **ZHANG, Xuejiao**
  **Shijiazhuang, Hebei 050035 (CN)**
- **GUO, Wenmin**
  **Shijiazhuang, Hebei 050035 (CN)**
- **GENG, Jia**
  **Shijiazhuang, Hebei 050035 (CN)**
- **LIU, Yongmei**
  **Shijiazhuang, Hebei 050035 (CN)**
- **WANG, Kezhu**
  **Shijiazhuang, Hebei 050035 (CN)**
- **CHENG, Qianyi**
  **Shijiazhuang, Hebei 050035 (CN)**

(74) Representative: **Mewburn Ellis LLP**
**Aurora Building**
**Counterslip**
**Bristol BS1 6BX (GB)**

# (54) TRICYCLIC COMPOUNDS AND USES THEREOF

(57) The present invention provides a class of compounds represented by formula (I), or tautomers, stereoisomers, or pharmaceutically acceptable salts thereof. The compounds of the present invention have a strong MAT2A inhibitory effect and have medical use for treating and/or preventing diseases, symptoms, and conditions mediated by MAT2A, such as tumors.

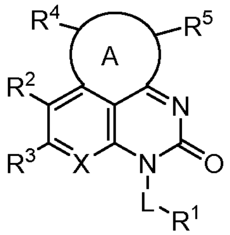

(I)

EP 4 545 531 A1

## Description

The present application claims the priorities to the Chinese Patent Application No. 202210734328.0 filed with the China National Intellectual Property Administration on June 27, 2022, the Chinese Patent Application No. 202211120467.0 filed with the China National Intellectual Property Administration on September 15, 2022, and the Chinese Patent Application No. 202310135765.5 filed with the China National Intellectual Property Administration on February 20, 2023, the contents of which are incorporated herein by reference in their entireties.

## TECHNICAL FIELD

The present application relates to the field of medical technology, and specifically to a class of novel compounds having MAT2A inhibitory effects, and their uses for treating and preventing diseases, disorders and conditions mediated by MAT2A, such as tumors.

## BACKGROUND

MAT2A, whose full name is Methionine adenosyltransferase 2A, is also known as S-Adenosylmethionine Synthase Isoform Type.

MAT2A is expressed in all tissues, including erythrocytes, brain, fetal liver, kidney, and pancreatic tissues, but is less abundant in adult liver tissue. Higher expression of MAT2A and MAT2B results in growth, migration, and invasion of cancer cells. Overall, lower expression of MAT2A and MAT2B leads to increased apoptosis and decreased growth, migration, and metastasis of cells.

MTAP is methionine transferase that can catalyze the transfer of adenosine and plays an important role in the salvage synthesis of ATP. MTAP deficiency accounts for approximately 15% of all solid tumors. MTAP is deficient to varying degrees in many types of tumors. MTAP deficiency may lead to accumulation of a substrate of said enzyme, methylthioadenosine (MTA). Increased concentration of MTA partially inhibits the activity of PRMT5, while other methyltransferases are relatively unaffected. Inhibition of MAT2A will result in a decrease in the methyl donor, S-adenosylmethionine (SAM), which is a substrate of PRMT5, thereby further inhibiting PRMT5, affecting tumor cell mRNA splicing and causing DNA damage. Therefore, MAT2A inhibitors can benefit tumors with MTAP deficiency.

There are several patents disclosing MAT2A inhibitors, such as WO2018039972, WO2019191470, WO2020139991, WO2020139992, WO2020243376, and WO2020123395.

## DETAILED DESCRIPTION OF THE INVENTION

The present application provides a type of MAT2A inhibitor compounds having novel structures, or a tautomer, a stereoisomer, or a pharmaceutically acceptable salt thereof, and also provides a use of such compounds, or the tautomer, a stereoisomer, or the pharmaceutically acceptable salt thereof for treating and/or preventing a disease, disorder or condition mediated by MAT2A.

Specifically, **in a first aspect,** the present application provides a compound represented by formula (I), or a tautomer, a stereoisomer, or a pharmaceutically acceptable salt thereof, which has the following structures:

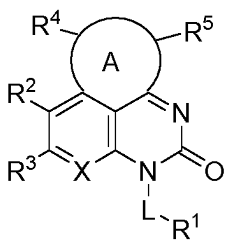

(I),

wherein,

ring A is selected from the group consisting of $C_{5-10}$ carbocyclyl, $C_{6-14}$ aryl, 5-12 membered heteroaryl, and 5-12 membered heterocyclyl;

$R^1$ is selected from the group consisting of optionally substituted $C_{3-12}$ carbocyclyl, optionally substituted $C_{6-14}$ aryl, optionally substituted 5-14 membered heteroaryl, and optionally substituted 5-14 membered heterocyclyl; wherein

each of the carbocyclyl, the aryl, the heteroaryl, and the heterocyclyl is optionally substituted by one or more $R^a$;

L is selected from the group consisting of bond, -O-, -S-, -$C_{1-4}$ alkylene-, -O$C_{1-4}$ alkylene-, -C(O)-, -C(O)O-, -OC(O)-, -N($R^{a1}$)C(O)-, -C(O)N($R^{a1}$)- and -N($R^{a1}$)-; wherein the alkylene is optionally substituted by one or more $R^{a1}$;

$R^a$ is, at each occurrence, independently selected from the group consisting of deuterium, halogen, oxo, -CN, -OH, -SH, -$NO_2$, -$NH_2$, -W-O$R^{a1}$, -W-S$R^{a1}$, -W-C(O)$R^{a4}$, -W-C(O)O$R^{a1}$, -W-OC(O)$R^{a1}$, -W-OC(O)O$R^{a1}$, -W-C(O)N$R^{a2}R^{a3}$, -W-C(O)N$R^{a2}$O$R^{a1}$, -W-OC(O)N$R^{a2}R^{a3}$, -W-N$R^{a2}R^{a3}$, -W-N$R^{a2}$C(O)$R^{a4}$, -W-N$R^{a2}$C(O)O$R^{a1}$, -W-N$R^{a2}$-C(O)N$R^{a2}R^{a3}$, -W-S(O)$R^{a4}$, -W-S$(O)_2R^{a4}$, -W-$SO_2$N$R^{a2}R^{a3}$, -W-N$R^{a2}$S$(O)_2R^{a4}$, -W-OS$(O)_2R^{a4}$, -W-N$R^{a2}$S$(O)_2$N-$R^{a2}R^{a3}$, -W-OS$(O)_2$N$R^{a2}R^{a3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, $C_{6-14}$ aryl, 3-12 membered heterocyclyl, and 5-16 membered heteroaryl; wherein each of the alkyl, the alkenyl, the alkynyl, the alkoxy, the alkylthiol, the cycloalkyl, the cycloalkenyl, the aryl, the heterocyclyl, and the heteroaryl is optionally substituted by one or more substituents selected from the group consisting of deuterium, halogen, oxo, oxime, -CN, -OH, - $NO_2$, -$NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

$R^{a1}$ is, at each occurrence, independently hydrogen, deuterium, $C_{1-6}$ alkyl, $C_{1-6}$ alkoxy, $C_{3-10}$ cycloalkyl, $C_{6-14}$ aryl, 5-12 membered heteroaryl, or 3-20 membered heterocyclyl, wherein each of the alkyl, the alkoxy, the cycloalkyl, the aryl, the heteroaryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^{a2}$ and $R^{a3}$ are, at each occurrence, independently hydrogen, deuterium, $C_{1-6}$ alkyl, or $C_{1-6}$ alkoxy, wherein each of the alkyl and the alkoxy is optionally substituted by one or more substituents selected from the group consisting of halogen, deuterium, cyano, hydroxyl, amino, carboxyl, $C_{1-6}$ alkyl, $C_{1-6}$ alkoxy, $C_{1-6}$ alkyl, $C_{3-8}$ cycloalkyl, phenyl, $C_{1-6}$ haloalkyl, $C_{1-6}$ haloalkoxy, and halophenyl; or when $R^{a2}$ and $R^{a3}$ are attached to the same nitrogen atom, $R^{a2}$ and $R^{a3}$, together with the nitrogen atom to which they are attached, form 3-10 membered heterocycloalkyl or 5-12 membered heteroaryl, each of which is optionally substituted by one or more of halogen, hydroxyl, amino, nitro, sulfhydryl, cyano, oxo, $C_{1-6}$ alkyl, $C_{1-6}$ alkoxy, $C_{3-10}$ cycloalkyl, 3-10 membered heterocycloalkyl, $C_{6-14}$ aryl, and 5-12 membered heteroaryl;

$R^{a4}$ is, at each occurrence, independently hydrogen, deuterium, $C_{1-6}$ alkyl, $C_{1-6}$ alkoxy, $C_{3-8}$ cycloalkyl, $C_{6-14}$ aryl, 5-12 membered heteroaryl, or 3-20 membered heterocyclyl, wherein each of the alkyl, the alkoxy, the cycloalkyl, the aryl, the heteroaryl, and the heterocyclyl is optionally substituted by one or more substituents selected from the group consisting of halogen, deuterium, cyano, hydroxyl, amino, $C_{1-6}$ alkyl, $C_{1-6}$ alkoxy, phenyl, $C_{1-6}$ haloalkyl, $C_{1-6}$ haloalkoxy, and halophenyl;

X is N or C$R^6$;

$R^2$, $R^3$ and $R^6$ are each independently selected from the group consisting of hydrogen, deuterium, halogen, -CN, -OH, -SH, -$NO_2$, -$NH_2$, -W-O$R^{b1}$-, -W-S$R^{b1}$, -W-C(O)$R^{b4}$, -W-C(O)O$R^{b1}$, -W-OC(O)$R^{b1}$, -W-OC(O)O$R^{b1}$, -W-C(O)N$R^{b2}R^{b3}$, -W-C(O)N$R^{b2}$O$R^{b1}$, -W-OC(O)N$R^{b2}R^{b3}$, -W-N$R^{b2}R^{b3}$, -W-N$R^{b2}$C(O)$R^{b4}$, -W-N$R^{b2}$C(O)O$R^{b1}$, -W-N$R^{b2}$C(O)N$R^{b2}R^{b3}$, -W-S(O)$R^{b4}$, -W-S$(O)_2R^{b4}$, -W-$SO_2$N$R^{b2}R^{b3}$, -W-N$R^{b2}$S$(O)_2R^{b4}$, -W-OS$(O)_2R^{b4}$, -W-N$R^{b2}$S$(O)_2$N$R^{b2}R^{b3}$, -W-OS$(O)_2$N$R^{b2}R^{b3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, $C_{6-14}$ aryl, 3-12 membered heterocyclyl, and 5-16 membered heteroaryl; wherein each of the alkyl, the alkenyl, the alkynyl, the alkoxy, the alkylthiol, the cycloalkyl, the cycloalkenyl, the aryl, the heterocyclyl, and the heteroaryl is optionally substituted by one or more substituents selected from the group consisting of deuterium, halogen, oxo, oxime, -CN, -OH, - $NO_2$, -$NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

$R^{b1}$ is, at each occurrence, independently hydrogen, deuterium, $C_{1-6}$ alkyl, $C_{1-6}$ alkoxy, $C_{3-10}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^{b2}$ and $R^{b3}$ are, at each occurrence, independently hydrogen, deuterium, $C_{1-6}$ alkyl, or $C_{1-6}$ alkoxy, wherein each of the alkyl and the alkoxy is optionally substituted by one or more substituents selected from the group consisting of halogen, deuterium, cyano, hydroxyl, amino, carboxyl, $C_{1-6}$ alkyl, $C_{1-6}$ alkoxy, $C_{1-6}$ alkyl, $C_{3-8}$ cycloalkyl, phenyl, $C_{1-6}$ haloalkyl, $C_{1-6}$ haloalkoxy, and halophenyl; or when $R^{b2}$ and $R^{b3}$ are attached to the same nitrogen atom, $R^{b2}$ and $R^{b3}$, together with the nitrogen atom to which they are attached, form 3-10 membered heterocycloalkyl or 5-12 membered heteroaryl, each of which is optionally substituted by one or more of halogen, hydroxyl, amino, nitro, sulfhydryl, cyano, oxo, $C_{1-6}$ alkyl, $C_{1-6}$ alkoxy, $C_{3-10}$ cycloalkyl, 3-10 membered heterocycloalkyl, $C_{6-14}$ aryl, and 5-12 membered heteroaryl;

$R^{b4}$ is, at each occurrence, independently hydrogen, deuterium, $C_{1-6}$ alkyl, $C_{1-6}$ alkoxy, $C_{3-8}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^4$ and $R^5$ are each independently selected from the group consisting of hydrogen, deuterium, halogen, oxime, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, $C_{6-14}$ aryl, 3-12 membered heterocyclyl, and 5-16 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, the aryl, and the heteroaryl is optionally substituted by one or more $R^c$; or $R^4$ and $R^5$ are attached to the same ring atom, to the adjacent ring atoms, or to the ring atoms separated by one atom, and $R^4$ and $R^5$, together with the carbon atom(s) and/or nitrogen atom(s) to which they are attached, form optionally substituted $C_{3-10}$ cycloalkyl, optionally substituted $C_{6-14}$ aryl, optionally substituted 5-12 membered heteroaryl, or optionally substituted 5-12 membered heterocyclyl; and the term "optionally substituted" means that the hydrogen on the substituted group is not substituted or one or more substitutable sites of the substituted group are independently substituted by substituents selected from $R^c$;

$R^c$ is independently selected from the group consisting of deuterium, halogen, oxime, - CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, 3-12 membered heterocyclyl, and 5-16 membered heteroaryl; wherein each of the alkyl, the alkenyl, the alkynyl, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

$R^{c1}$ is, at each occurrence, independently hydrogen, deuterium, $C_{1-6}$ alkyl, $C_{1-6}$ alkoxy, $C_{3-10}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^{c2}$ and $R^{c3}$ are, at each occurrence, independently hydrogen, deuterium, $C_{1-6}$ alkyl, or $C_{1-6}$ alkoxy, wherein each of the alkyl and the alkoxy is optionally substituted by one or more substituents selected from the group consisting of halogen, deuterium, cyano, hydroxyl, amino, carboxyl, $C_{1-6}$ alkyl, $C_{1-6}$ alkoxy, $C_{1-6}$ alkyl, $C_{3-8}$ cycloalkyl, phenyl, $C_{1-6}$ haloalkyl, $C_{1-6}$ haloalkoxy, and halophenyl; or when $R^{c2}$ and $R^{c3}$ are attached to the same nitrogen atom, $R^{c2}$ and $R^{c3}$, together with the nitrogen atom to which they are attached, form 3-10 membered heterocycloalkyl or 5-12 membered heteroaryl, each of which is optionally substituted by one or more of halogen, hydroxyl, amino, nitro, sulfhydryl, cyano, oxo, $C_{1-6}$ alkyl, $C_{1-6}$ alkoxy, $C_{3-10}$ cycloalkyl, 3-10 membered heterocycloalkyl, $C_{6-14}$ aryl, and 5-12 membered heteroaryl;

$R^{c4}$ is, at each occurrence, independently hydrogen, deuterium, $C_{1-6}$ alkyl, $C_{1-6}$ alkoxy, $C_{3-8}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

W is selected from the group consisting of bond, $C_{1-3}$ alkylene, $-OC_{1-3}$ alkylene, and $-SC_{1-3}$ alkylene, and the alkylene is optionally substituted by one or more substituents selected from the group consisting of deuterium, halogen, oxo, -CN, -OH, and $-NH_2$;

unless otherwise stated, the heteroatom(s) in the heterocyclyl and the heteroaryl is(are) independently selected from the group consisting of O, N and S, and the number of the heteroatoms is 1, 2 or 3.

In a further preferred embodiment, wherein ring A is selected from the group consisting of $C_{5-10}$ cycloalkyl, $C_{6-10}$ aryl, 5-10 membered heteroaryl, and 5-10 membered heterocyclyl.

In a further preferred embodiment, wherein ring A is selected from the group consisting of $C_{5-10}$ cycloalkyl, $C_6$ aryl, 5-8 membered heteroaryl, and 5-10 membered heterocyclyl.

In a further preferred embodiment, wherein ring A is selected from the group consisting of $C_{5-10}$ cycloalkyl and 5-10 membered heterocyclyl.

In a further preferred embodiment, wherein ring A is selected from the group consisting of 5-10 membered heterocyclyl.

In a further preferred embodiment, wherein ring A is selected from the group consisting of 5-8 membered heterocyclyl.

In a further preferred embodiment, wherein ring A is selected from the group consisting of 5-7 membered heterocyclyl, the heteroatom(s) is(are) independently selected from the group consisting of O and N, and the number of the heteroatoms is 1 or 2.

In a further preferred embodiment, wherein ring A is selected from the group consisting of 5-6 membered heterocyclyl, the heteroatom(s) is(are) independently selected from the group consisting of O and N, and the number of the heteroatoms is 1 or 2.

In a further preferred embodiment, wherein ring A is selected from the group consisting of 5-6 membered heterocyclyl, the heteroatom(s) is(are) independently selected from N, and the number of the heteroatoms is 1 or 2.

In a further preferred embodiment, wherein ring A is selected from the group consisting of 5 membered heterocyclyl, the heteroatom(s) is(are) independently selected from the group consisting of O and N, and the number of the heteroatoms is 1 or 2.

In a further preferred embodiment, wherein ring A is selected from the group consisting of 5 membered heterocyclyl, the heteroatom(s) is(are) independently selected from N, and the number of the heteroatoms is 1 or 2.

In a further preferred embodiment, wherein ring A is selected from the group consisting

of 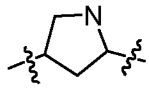, 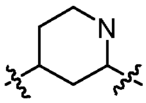, 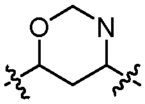, 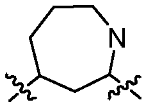, 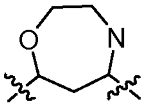 and 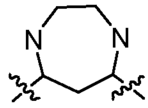.

In a further preferred embodiment, wherein ring A is selected from the group consisting

of 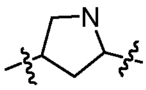, 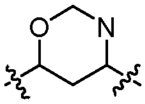, 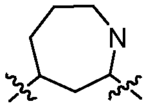 and 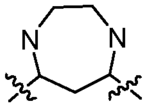.

In a further preferred embodiment, wherein ring A is selected from the group consisting

of 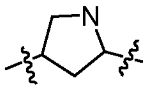, 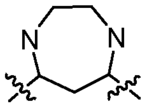 and 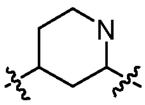.

In a further preferred embodiment, wherein ring A is selected from the group consisting

of 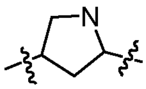.

In a further preferred embodiment, wherein X is selected from the group consisting of N and CH.

In a further preferred embodiment, wherein X is selected from the group consisting of N.

In a further preferred embodiment, wherein X is selected from the group consisting of CH.

In a further preferred embodiment, wherein $R^1$ is selected from the group consisting of the following optionally substituted groups: $C_{3-10}$ cycloalkyl, $C_{5-10}$ bridged cycloalkyl, $C_{5-10}$ fused cycloalkyl, $C_{5-10}$ spirocycloalkyl, $C_{6-10}$ aryl, 5-10 membered heteroaryl, 5-10 membered monocyclic heterocyclyl, 5-10 membered bridged heterocyclyl, 5-10 membered fused heterocyclyl, and 5-10 membered spirocyclic heterocyclyl; wherein each of the cycloalkyl, the bridged cycloalkyl, the fused cycloalkyl, the spirocycloalkyl, the aryl, the heteroaryl, the monocyclic heterocyclyl, the bridged heterocyclyl, the fused heterocyclyl and the spirocyclic heterocyclyl is optionally substituted by one or more $R^a$.

In a further preferred embodiment, wherein $R^1$ is selected from the group consisting of the following optionally substituted groups: $C_{3-6}$ cycloalkyl, $C_{5-10}$ fused cycloalkyl, $C_{5-10}$ spirocycloalkyl, $C_{6-10}$ aryl, 5-10 membered heteroaryl, 5-10 membered monocyclic heterocyclyl, 5-10 membered bridged heterocyclyl, 5-10 membered fused heterocyclyl, and 5-10 membered spirocyclic heterocyclyl; wherein each of the cycloalkyl, the fused cycloalkyl, the spirocycloalkyl, the aryl, the heteroaryl, the monocyclic heterocyclyl, the bridged heterocyclyl, the fused heterocyclyl and the spirocyclic heterocyclyl is optionally substituted by one or more $R^a$.

In a further preferred embodiment, wherein $R^1$ is selected from the group consisting of the following optionally substituted groups: $C_{6-10}$ aryl, 5-10 membered heteroaryl, 5-10 membered monocyclic heterocyclyl, 5-10 membered bridged heterocyclyl, 5-10 membered fused heterocyclyl, and 5-10 membered spirocyclic heterocyclyl; wherein each of the aryl, the heteroaryl, the monocyclic heterocyclyl, the bridged heterocyclyl, the fused heterocyclyl and the spirocyclic heterocyclyl is optionally substituted by one or more $R^a$.

In a further preferred embodiment, wherein $R^1$ is selected from the group consisting of the following optionally

substituted groups: $C_{6-10}$ aryl, 5-8 membered heteroaryl, 5-8 membered monocyclic heterocyclyl, 5-8 membered bridged heterocyclyl, 5-8 membered fused heterocyclyl, and 5-8 membered spirocyclic heterocyclyl; wherein each of the aryl, the heteroaryl, the monocyclic heterocyclyl, the bridged heterocyclyl, the fused heterocyclyl and the spirocyclic heterocyclyl is optionally substituted by one or more $R^a$.

In a further preferred embodiment, wherein $R^1$ is selected from the group consisting of the following optionally substituted groups: $C_6$ aryl, 5-8 membered heteroaryl, and 5-8 membered monocyclic heterocyclyl; wherein each of the aryl, the heteroaryl, and the monocyclic heterocyclyl is optionally substituted by one or more $R^a$.

In a further preferred embodiment, wherein $R^1$ is selected from the group consisting of the following optionally substituted groups: $C_6$ aryl, 5-6 membered heteroaryl, and 5-6 membered monocyclic heterocyclyl; wherein each of the aryl, the heteroaryl, and the monocyclic heterocyclyl is optionally substituted by one or more $R^a$, the heteroatom(s) is(are) independently selected from the group consisting of O and N, and the number of the heteroatoms is 1 or 2.

In a further preferred embodiment, wherein $R^1$ is selected from the group consisting of the following optionally substituted groups: $C_6$ aryl and 5-6 membered heteroaryl; wherein each of the aryl and the heteroaryl is optionally substituted by one or more $R^a$, the heteroatom(s) is(are) independently selected from N, and the number of the heteroatoms is 1 or 2.

In a further preferred embodiment, wherein $R^1$ is selected from the group consisting of phenyl, pyridinyl and imidazolyl, each of which is optionally substituted by one or more $R^a$.

In a further preferred embodiment, wherein $R^1$ is selected from the group consisting of pyridinyl optionally substituted by one or more $R^a$.

In a further preferred embodiment, wherein $R^1$ is selected from the group consisting of

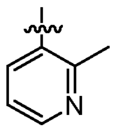 , 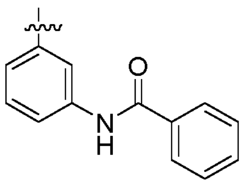 , 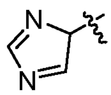 , 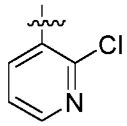 , 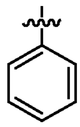 and 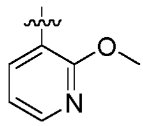 .

In a further preferred embodiment, wherein $R^1$ is selected from the group consisting of

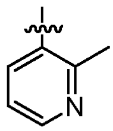 , 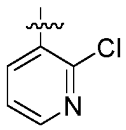 , and 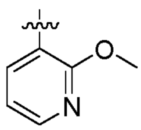 .

In a further preferred embodiment, wherein L is selected from the group consisting of bond, -O-, -S-, -$C_{1-4}$ alkylene- and -$OC_{1-4}$ alkylene-; wherein the alkylene is optionally substituted by one or more substituents selected from the group consisting of deuterium, halogen, oxo, -CN, -OH, and -$NH_2$.

In a further preferred embodiment, wherein L is selected from the group consisting of bond, -O-, -S-, -$C_{1-2}$ alkylene- and -$OC_{1-2}$ alkylene-; wherein the alkylene is optionally substituted by one or more substituents selected from the group consisting of deuterium, halogen, oxo, -CN, -OH, and -$NH_2$.

In a further preferred embodiment, wherein L is selected from the group consisting of bond, -O-, -S-, methylene and ethylene; wherein the methylene or the ethylene is optionally substituted by one or more substituents selected from the group consisting of deuterium, halogen, oxo, -CN, -OH, and -$NH_2$.

In a further preferred embodiment, wherein L is selected from the group consisting of bond, -O-, methylene and ethylene.

In a further preferred embodiment, wherein L is selected from the group consisting of bond and methylene.

In a further preferred embodiment, wherein L is selected from the group consisting of bond.

In a further preferred embodiment, wherein $R^a$ is, at each occurrence, independently selected from the group consisting of deuterium, halogen,-CN, -OH, -SH, -$NO_2$, -$NH_2$, -W-$OR^{a1}$, -W-$SR^{a1}$, -W-C(O)$R^{a4}$, -W-C(O)$OR^{a1}$, -W-OC(O)$R^{a1}$, -W-C(O)$NR^{a2}R^{a3}$, -W-OC(O)$NR^{a2}R^{a3}$, -W-$NR^{a2}R^{a3}$, -W-$NR^{a2}$C(O)$R^{a4}$, -W-S(O)$R^{a4}$, -W-$S(O)_2R^{a4}$, -W-$SO_2N$-$R^{a2}R^{a3}$, -W-$NR^{a2}S(O)_2R^{a4}$, -W-$OS(O)_2R^{a4}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, 3-12 membered heterocyclyl, and 5-16 membered heteroaryl; wherein each of the alkyl, the alkenyl, the alkynyl, the alkoxy, the alkylthiol, the cycloalkyl, the heterocyclyl, and the heteroaryl is optionally substituted by one or more substituents selected from the group consisting of deuterium, halogen, oxo, oxime, -CN, -OH, -$NO_2$, -$NH_2$, $C_{1-6}$ alkyl, phenyl, $C_{1-6}$ alkoxy, $C_{1-6}$ haloalkyl, and $C_{1-6}$ haloalkoxy, halophenyl.

In a further preferred embodiment, wherein $R^a$ is, at each occurrence, independently selected from the group

consisting of deuterium, halogen, -CN, -OH, -SH, $-NO_2$, $-NH_2$, $-W-OR^{a1}$, $-W-SR^{a1}$, $-W-C(O)R^{a4}$, $-W-C(O)OR^{a1}$, $-W-OC(O)R^{a1}$, $-W-C(O)NR^{a2}R^{a3}$, $-W-NR^{a2}R^{a3}$, $-W-NR^{a2}C(O)R^{a4}$, $-W-S(O)R^{a4}$, $-W-S(O)_2R^{a4}$, $-W-SO_2NR^{a2}R^{a3}$, $-W-NR^{a2}-S(O)_2R^{a4}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, 5-10 membered heterocyclyl, and 5-10 membered heteroaryl; wherein each of the alkyl, the alkenyl, the alkynyl, the alkoxy, the alkylthiol, the cycloalkyl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy.

In a further preferred embodiment, wherein $R^a$ is, at each occurrence, independently selected from the group consisting of deuterium, halogen, -CN, -OH, -SH, $-NO_2$, $-NH_2$, $-W-OR^{a1}$, $-W-SR^{a1}$, $-W-C(O)R^{a4}$, $-W-C(O)OR^{a1}$, $-W-OC(O)R^{a1}$, $-W-C(O)NR^{a2}R^{a3}$, $-W-NR^{a2}R^{a3}$, $-W-NR^{a2}C(O)R^{a4}$, $-W-S(O)2R^{a4}$, $-W-SO_2NR^{a2}R^{a3}$, $-W-NR^{a2}S(O)_2R^{a4}$, $C_{1-6}$ alkyl, $C_{2-6}$ alkenyl, $C_{2-6}$ alkynyl, and $C_{1-6}$ alkoxy; wherein each of the alkyl, the alkenyl, the alkynyl, and the alkoxy is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy.

In a further preferred embodiment, wherein $R^a$ is, at each occurrence, independently selected from the group consisting of deuterium, halogen, -CN, -OH, $-NH_2$, $-W-OR^{a1}$, $-W-SR^{a1}$, $- W-C(O)R^{a4}$, $-W-C(O)OR^{a1}$, $-W-OC(O)R^{a1}$, $-W-NR^{a2}C(O)R^{a4}$, $-C_{1-4}$ alkyl, and $C_{1-4}$ alkoxy; wherein the alkyl or the alkoxy is optionally substituted by one or more substituents selected from the group consisting of deuterium, halogen, oxo, -CN, -OH, $-NO_2$, $-NH_2$, $C_{1-4}$ alkyl, and $C_{1-4}$ alkoxy.

In a further preferred embodiment, wherein $R^a$ is, at each occurrence, independently selected from the group consisting of deuterium, halogen, -OH, $-NH_2$, $-W-NR^{a2}C(O)R^{a4}$, $C_{1-4}$ alkyl, and $C_{1-4}$ alkoxy; wherein the alkyl or the alkoxy is optionally substituted by one or more substituents selected from the group consisting of deuterium, halogen, oxo, -OH, $-NH_2$, $C_{1-4}$ alkyl, $C_{1-4}$ alkoxy, $C_{1-4}$ haloalkyl, and $C_{1-4}$ haloalkoxy.

In a further preferred embodiment, wherein $R^a$ is, at each occurrence, independently selected from the group consisting of deuterium, halogen, $C_{1-4}$ alkyl, $-W-NR^{a2}C(O)R^{a4}$, and $C_{1-4}$ haloalkyl; wherein the alkyl or the haloalkyl is optionally substituted by one or more deuterium.

In a further preferred embodiment, wherein $R^a$ is, at each occurrence, independently selected from the group consisting of deuterium, halogen, $C_{1-4}$ alkyl, $C_{1-4}$ alkoxy, and $-W-NR^{a2}C(O)R^{a4}$; wherein the alkyl or the haloalkyl is optionally substituted by one or more deuterium.

In a further preferred embodiment, wherein $R^a$ is, at each occurrence, independently selected from the group consisting of deuterium, halogen, $C_{1-4}$ alkyl, $C_{1-4}$ alkoxy, and $-NHC(O)R^{a4}$; wherein the alkyl is optionally substituted by one or more deuterium.

In a further preferred embodiment, wherein $R^a$ is, at each occurrence, independently selected from the group consisting of halogen, $C_{1-4}$ alkyl, and $C_{1-4}$ alkoxy.

In a further preferred embodiment, wherein $R^a$ is, at each occurrence, independently selected from the group consisting of deuterium, F, Cl, Br, methyl, ethyl, propyl, methoxy, ethoxy, trifluoromethyl, trifluoroethyl and -NHC(O)-phenyl.

In a further preferred embodiment, wherein $R^a$ is, at each occurrence, independently selected from the group consisting of Cl, methyl, methoxy, and -NHC(O)-phenyl.

In a further preferred embodiment, wherein $R^a$ is, at each occurrence, independently selected from the group consisting of Cl, methyl, and -NHC(O)-phenyl.

In a further preferred embodiment, wherein $R^{a1}$ is, at each occurrence, independently hydrogen, deuterium, $C_{1-6}$ alkyl, $C_{1-6}$ alkoxy, $C_{3-6}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{1-6}$ haloalkyl, and $C_{1-6}$ haloalkoxy; the heteroatom(s) is(are) independently selected from the group consisting of O, N and S, and the number of the heteroatoms is 1, 2 or 3.

In a further preferred embodiment, wherein $R^{a1}$ is, at each occurrence, independently hydrogen, deuterium, $C_{1-4}$ alkyl, $C_{1-4}$ alkoxy, $C_{3-6}$ cycloalkyl, $C_{6-10}$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{a1}$ is, at each occurrence, independently hydrogen, deuterium, $C_{1-4}$ alkyl, $C_{1-4}$ alkoxy, $C_{3-6}$ cycloalkyl, $C_6$ aryl, or 5-6 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{a1}$ is, at each occurrence, independently hydrogen, deuterium,

methyl, ethyl, propyl, methoxy, ethoxy or propoxy, wherein each of the methyl, the ethyl, the propyl, the methoxy, the ethoxy and the propoxy is optionally substituted by one or more substituents selected from the group consisting of halogen, deuterium, hydroxyl, amino, and $C_{1-4}$ alkyl.

In a further preferred embodiment, wherein $R^{a1}$ is, at each occurrence, independently hydrogen, methyl, ethyl, or propyl, wherein each of the methyl, the ethyl, and the propyl is optionally substituted by one or more substituents selected from the group consisting of halogen, deuterium, hydroxyl, amino, and $C_{1-4}$ alkyl.

In a further preferred embodiment, wherein $R^{a2}$ and $R^{a3}$ are, at each occurrence, independently hydrogen, deuterium, $C_{1-4}$ alkyl, or $C_{1-4}$ alkoxy, wherein the alkyl or the alkoxy is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy.

In a further preferred embodiment, wherein $R^{a2}$ and $R^{a3}$ are, at each occurrence, independently hydrogen, deuterium, $C_{1-4}$ alkyl, or $C_{1-4}$ alkoxy, wherein the alkyl or the alkoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^{a2}$ and $R^{a3}$ are, at each occurrence, independently hydrogen, deuterium, methyl, ethyl, propyl, methoxy, ethoxy, or propoxy, wherein each of the methyl, the ethyl, the propyl, the methoxy, the ethoxy, and the propoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^{a2}$ and $R^{a3}$ are, at each occurrence, independently hydrogen, methyl, ethyl, or propyl, wherein each of the methyl, the ethyl, and the propyl is optionally substituted by one or more substituents selected from the group consisting of halogen, deuterium, hydroxyl, amino, and $C_{1-4}$ alkyl.

In a further preferred embodiment, wherein $R^{a4}$ is, at each occurrence, independently hydrogen, deuterium, $C_{1-4}$ alkyl, $C_{1-4}$ alkoxy, $C_{3-8}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-6}$ alkyl, $C_{1-6}$ alkoxy, phenyl, $C_{1-6}$ haloalkyl, $C_{1-6}$ haloalkoxy, and halophenyl; the heteroatom(s) is(are) independently selected from the group consisting of O, N and S, and the number of the heteroatoms is 1, 2 or 3.

In a further preferred embodiment, wherein $R^{a4}$ is, at each occurrence, independently hydrogen, deuterium, $C_{1-4}$ alkyl, $C_{1-4}$ alkoxy, $C_{3-6}$ cycloalkyl, $C_6$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{a4}$ is, at each occurrence, independently hydrogen, deuterium, $C_{1-4}$ alkyl, $C_{1-4}$ alkoxy, $C_{3-6}$ cycloalkyl, or $C_6$ aryl, wherein each of the alkyl, the alkoxy, the cycloalkyl, and the aryl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^{a4}$ is, at each occurrence, independently hydrogen, deuterium, methyl, ethyl, propyl, methoxy, ethoxy, propoxy, cyclopropanyl, cyclobutanyl or phenyl; wherein each of the methyl, the ethyl, the propyl, the methoxy, the ethoxy, the propoxy, the cyclopropanyl, the cyclobutanyl and the phenyl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^{a4}$ is, at each occurrence, independently hydrogen, deuterium, methyl, ethyl, propyl, methoxy, ethoxy, propoxy or phenyl; wherein each of the methyl, the ethyl, the propyl, the methoxy, the ethoxy, the propoxy and the phenyl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^2$ is selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$, - $W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-6}$ cycloalkyl, $C_{6-12}$ aryl, 3-6 membered heterocyclyl, and 5-12 membered heteroaryl; wherein each of the alkyl, the alkenyl, the alkynyl, the haloalkyl, the haloalkoxy, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy.

In a further preferred embodiment, wherein $R^2$ is selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$,$-W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-6}$ alkyl, $C_{1-6}$ haloalkyl, $C_{1-6}$ haloalkoxy, $C_{1-6}$ alkoxy,

$C_{1-6}$ alkylthiol, $C_{3-6}$ cycloalkyl, $C_{6-12}$ aryl, 5-6 membered heterocyclyl, and 5-6 membered heteroaryl; wherein each of the alkyl, the alkenyl, the alkynyl, the haloalkyl, the haloalkoxy, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy; the heteroatom(s) is(are) independently selected from the group consisting of O and N, and the number of the heteroatoms is 1 or 2.

In a further preferred embodiment, wherein $R^2$ is selected from the group consisting of hydrogen, deuterium, halogen, -CN, -OH, -SH, $-NO_2$, $-NH_2$, $-W-OR^{b1}$, $-W-SO_2NR^{b2}R^{b3}$, $-W-NR^{b2}S(O)_2R^{b4}$, $C_{1-4}$ alkyl, $C_{1-4}$ alkoxy, $C_{3-6}$ cycloalkyl, and $C_6$ aryl; wherein each of the alkyl, the alkoxy, the cycloalkyl, and the aryl is optionally substituted by one or more substituents selected from the group consisting of deuterium, halogen, oxo, -CN, -OH, $-NO_2$, $-NH_2$, $C_{1-4}$ alkyl, $C_{1-4}$ alkoxy, $C_{1-4}$ haloalkyl, and $C_{1-4}$ haloalkoxy.

In a further preferred embodiment, wherein $R^2$ is selected from the group consisting of hydrogen, deuterium, halogen, -CN, -OH, -SH, $-NO_2$, $-NH_2$, methyl, ethyl, isopropyl, methoxy, ethoxy, propoxy, $-W-SO_2NR^{b2}R^{b3}$ and $-W-NR^{b2}S(O)_2R^{b4}$; wherein each of the methyl, the ethyl, the isopropyl, the methoxy, the ethoxy, and the propoxy is optionally substituted by one or more substituents selected from the group consisting of deuterium, halogen, oxo, -CN, -OH, $-NO_2$, $-NH_2$, $C_{1-4}$ alkyl, $C_{1-4}$ alkoxy, $C_{1-4}$ haloalkyl, and $C_{1-4}$ haloalkoxy.

In a further preferred embodiment, wherein $R^2$ is selected from the group consisting of hydrogen, deuterium, halogen, -CN, -OH, $-NH_2$, methyl, ethyl, isopropyl, $-SO_2NH_2$, and - $NHSO_2H$.

In a further preferred embodiment, wherein $R^2$ is selected from the group consisting of hydrogen.

In a further preferred embodiment, wherein $R^6$ is selected from the group consisting of hydrogen.

In a further preferred embodiment, wherein $R^3$ is selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$,$-W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-6}$ cycloalkyl, $C_{6-12}$ aryl, 3-6 membered heterocyclyl, and 5-10 membered heteroaryl; wherein each of the alkyl, the alkenyl, the alkynyl, the haloalkyl, the haloalkoxy, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy; the heteroatom(s) is(are) independently selected from the group consisting of O and N, and the number of the heteroatoms is 1 or 2.

In a further preferred embodiment, wherein $R^3$ is selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$, - $W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-4}$ alkyl, $C_{2-4}$ alkenyl, $C_{2-4}$ alkynyl, $C_{1-4}$ alkoxy, $C_{1-4}$ alkylthiol, $C_{3-6}$ cycloalkyl, $C_{6-}$ aryl, 3-6 membered heterocyclyl, and 5-6 membered heteroaryl; wherein each of the alkyl, the alkenyl, the alkynyl, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy; the heteroatom(s) is(are) independently selected from the group consisting of O and N, and the number of the heteroatoms is 1 or 2.

In a further preferred embodiment, wherein $R^3$ is selected from the group consisting of hydrogen, deuterium, halogen, -CN, -OH, $-NH_2$, $-W-OR^{b1}$, $-W-SR^{b1}$, $-W-C(O)R^{b4}$, $-W-C(O)OR^{b1}$, $-W-OC(O)R^{b1}$, $-W-C(O)NR^{b2}R^{b3}$, $-W-NR^{b2}R^{b3}$, $-W-NR^{b2}C(O)R^{b4}$, $-W-NR^{b2}C(O)NR^{b2}R^{b3}$, $-W-S(O)R^{b4}$, $-W-S(O)_2R^{b4}$, $C_{1-4}$ alkyl, $C_{1-4}$ alkoxy, $C_{3-6}$ cycloalkyl, $C_{6-}$ aryl, 3-6 membered heterocyclyl, and 5-6 membered heteroaryl; wherein each of the alkyl, the alkoxy, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy; the heteroatom(s) is(are) independently selected from the group consisting of O and N, and the number of the heteroatoms is 1 or 2.

In a further preferred embodiment, wherein $R^3$ is selected from the group consisting of hydrogen, deuterium, halogen, -OH, $-NH_2$, $C_{1-4}$ alkyl, $C_{1-4}$ alkoxy, $C_{3-6}$ cycloalkyl, and 5-6 membered heterocyclyl; wherein each of the alkyl, the alkoxy, the cycloalkyl, and the heterocyclyl is optionally substituted by one or more substituents selected from the group consisting of deuterium, halogen, oxo, -CN, -OH, $-NO_2$, $-NH_2$, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^3$ is selected from the group consisting of F, Cl, Br, $-CF_3$, $-CHF_2-CH_2$, $-CH_2FCH_3$, $-OCF_3$ and cyclopropanyl.

In a further preferred embodiment, wherein $R^3$ is selected from the group consisting of Cl and $-CF_3$.

In a further preferred embodiment, wherein $R^3$ is selected from the group consisting of halogen, and $C_{1-4}$ alkyl optionally substituted by one or more halogen.

In a further preferred embodiment, wherein $R^3$ is selected from the group consisting of halogen.

In a further preferred embodiment, wherein $R^3$ is selected from the group consisting of Cl.

In a further preferred embodiment, wherein $R^{b1}$ is, at each occurrence, independently hydrogen, deuterium, $C_{1-6}$ alkyl, $C_{1-6}$ alkoxy, $C_{3-6}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{1-6}$ haloalkyl, and $C_{1-6}$ haloalkoxy.

In a further preferred embodiment, wherein $R^{b1}$ is, at each occurrence, independently hydrogen, deuterium, $C_{1-4}$ alkyl, $C_{1-4}$ alkoxy, $C_{3-6}$ cycloalkyl, $C_{6-10}$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-4}$ alkyl, $C_{1-4}$ alkoxy, $C_{1-4}$ haloalkyl, and $C_{1-4}$ haloalkoxy.

In a further preferred embodiment, wherein $R^{b1}$ is, at each occurrence, independently hydrogen, deuterium, $C_{1-4}$ alkyl, $C_{1-4}$ alkoxy, $C_{3-6}$ cycloalkyl, $C_6$ aryl, or 5-6 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{b1}$ is, at each occurrence, independently hydrogen, deuterium, methyl, ethyl, propyl, methoxy, ethoxy or propoxy, wherein each of the methyl, the ethyl, the propyl, the methoxy, the ethoxy and the propoxy is optionally substituted by one or more substituents selected from the group consisting of halogen, deuterium, hydroxyl, amino, and $C_{1-4}$ alkyl.

In a further preferred embodiment, wherein $R^{b1}$ is, at each occurrence, independently hydrogen, methyl, ethyl, or propyl, wherein each of the methyl, the ethyl, and the propyl is optionally substituted by one or more substituents selected from the group consisting of halogen, deuterium, hydroxyl, amino, and $C_{1-4}$ alkyl.

In a further preferred embodiment, wherein $R^{b2}$ and $R^{b3}$ are, at each occurrence, independently hydrogen, deuterium, $C_{1-4}$ alkyl, or $C_{1-4}$ alkoxy, wherein the alkyl or the alkoxy is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy.

In a further preferred embodiment, wherein $R^{b2}$ and $R^{b3}$ are, at each occurrence, independently hydrogen, deuterium, $C_{1-4}$ alkyl, or $C_{1-4}$ alkoxy, wherein the alkyl or the alkoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^{b2}$ and $R^{b3}$ are, at each occurrence, independently hydrogen, deuterium, methyl, ethyl, propyl, methoxy, ethoxy, or propoxy, wherein each of the methyl, the ethyl, the propyl, the methoxy, the ethoxy, and the propoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^{b2}$ and $R^{b3}$ are, at each occurrence, independently hydrogen, methyl, ethyl, or propyl, wherein each of the methyl, the ethyl, and the propyl is optionally substituted by one or more substituents selected from the group consisting of halogen, deuterium, hydroxyl, amino, and $C_{1-4}$ alkyl.

In a further preferred embodiment, wherein $R^{b4}$ is, at each occurrence, independently hydrogen, deuterium, $C_{1-4}$ alkyl, $C_{1-4}$ alkoxy, $C_{3-8}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-6}$ alkyl, $C_{1-6}$ alkoxy, phenyl, $C_{1-6}$ haloalkyl, $C_{1-6}$ haloalkoxy, and halophenyl; the heteroatom(s) is(are) independently selected from the group consisting of O, N and S, and the number of the heteroatoms is 1, 2 or 3.

In a further preferred embodiment, wherein $R^{b4}$ is, at each occurrence, independently hydrogen, deuterium, $C_{1-4}$ alkyl, $C_{1-4}$ alkoxy, $C_{3-6}$ cycloalkyl, $C_6$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{b4}$ is, at each occurrence, independently hydrogen, deuterium, $C_{1-4}$ alkyl, $C_{1-4}$ alkoxy, $C_{3-6}$ cycloalkyl, or $C_6$ aryl, wherein each of the alkyl, the alkoxy, the cycloalkyl, and the aryl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^{b4}$ is, at each occurrence, independently hydrogen, deuterium, methyl, ethyl, propyl, methoxy, ethoxy, propoxy, cyclopropanyl, cyclobutanyl or phenyl; wherein each of the methyl, the ethyl, the propyl, the methoxy, the ethoxy, the propoxy, the cyclopropanyl, the cyclobutanyl and the phenyl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^{b4}$ is, at each occurrence, independently hydrogen, deuterium, methyl, ethyl, propyl, methoxy, ethoxy, or propoxy; wherein each of the methyl, the ethyl, the propyl, the methoxy, the ethoxy, and the propoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^4$ and $R^5$ are each independently selected from the group consisting of hydrogen, deuterium, halogen, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $C_{1-6}$ alkyl, $C_{1-6}$ alkoxy, $C_{1-6}$ alkylthiol, $C_{3-8}$ cycloalkyl, $C_{3-8}$ cycloalkenyl, $C_{6-12}$ aryl, 4-12 membered heterocyclyl, and 5-12 membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; or $R^4$ and $R^5$ are attached to the same ring atom, to the adjacent ring atoms, or to the ring atoms separated by one atom, and $R^4$ and $R^5$, together with the carbon atom(s) and/or nitrogen atom(s) to which they are attached, form optionally substituted $C_{3-10}$ cycloalkyl, optionally substituted $C_{6-12}$ aryl, optionally substituted 5-12 membered heteroaryl, or optionally substituted 5-12 membered heterocyclyl; and the term "optionally substituted" means that the hydrogen on the substituted group is not substituted or one or more substitutable sites of the substituted group are independently substituted by substituents selected from $R^c$.

In a further preferred embodiment, wherein $R^4$ and $R^5$ are each independently selected from the group consisting of hydrogen, deuterium, halogen, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $C_{1-4}$ alkyl, $C_{1-4}$ alkoxy, $C_{1-4}$ alkylthiol, $C_{3-6}$ cycloalkyl, $C_{4-6}$ cycloalkenyl, $C_{6-12}$ aryl, 4-10 membered heterocyclyl, and 5-12 membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; or $R^4$ and $R^5$ are attached to the same ring atom, to the adjacent ring atoms, or to the ring atoms separated by one atom, and $R^4$ and $R^5$, together with the carbon atom(s) and/or nitrogen atom(s) to which they are attached, form optionally substituted $C_{3-10}$ cycloalkyl, optionally substituted $C_{6-12}$ aryl, optionally substituted 5-12 membered heteroaryl, or optionally substituted 5-12 membered heterocyclyl; and the term "optionally substituted" means that the hydrogen on the substituted group is not substituted or one or more substitutable sites of the substituted group are independently substituted by substituents selected from $R^c$; the heteroatom(s) is(are) independently selected from the group consisting of O, N and S, and the number of the heteroatoms is 1, 2 or 3.

In a further preferred embodiment, wherein $R^4$ and $R^5$ are each independently selected from the group consisting of hydrogen, deuterium, halogen, -CN, -OH, $-NH_2$, $-W-OR^{c1}$, $-W-SR^{c1}$, $-W-C(O)R^{c4}$, $-W-C(O)OR^{c1}$, $-W-OC(O)R^{c1}$, $-W-C(O)NR^{c2}R^{c3}$, $-W-OC(O)NR^{c2}R^{c3}$, $-W-NR^{c2}R^{c3}$, $- W-NR^{c2}C(O)R^{c4}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $C_{1-4}$ alkyl, $C_{1-4}$ alkoxy, $C_{1-4}$ alkylthiol, $C_{3-6}$ cycloalkyl, $C_{4-6}$ cycloalkenyl, $C_6$ aryl, 4-10 membered heterocyclyl, and 5-12 membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; the heteroatom(s) is(are) independently selected from the group consisting of O, N and S, and the number of the heteroatoms is 1, 2 or 3.

In a further preferred embodiment, wherein $R^4$ and $R^5$ are each independently selected from the group consisting of hydrogen, deuterium, halogen, -CN, -OH, $-NH_2$, $-W-OR^{c1}$, $-W-SR^{c1}$, $-W-C(O)R^{c4}$, $-W-C(O)OR^{c1}$, $-W-OC(O)R^{c1}$, $-W-C(O)NR^{c2}R^{c3}$, $-W-OC(O)NR^{c2}R^{c3}$, $-W-NR^{c2}R^{c3}$, $- W-NR^{c2}C(O)R^{c4}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $C_{1-4}$ alkyl, $C_{1-4}$ alkoxy, $C_{1-4}$ alkylthiol, $C_{3-6}$ cycloalkyl, $C_{4-6}$ cycloalkenyl, $C_6$ aryl, 4-10 membered heterocyclyl, and 5-6 membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; the heteroatom(s) is(are) independently selected from the group consisting of O, N and S, and the number of the heteroatoms is 1, 2 or 3.

In a further preferred embodiment, wherein $R^4$ and $R^5$ are each independently selected from the group consisting of hydrogen, deuterium, halogen, -OH, $-W-C(O)NR^{c2}R^{c3}$, $-W-NR^{c2}R^{c3}$, $-W-SO_2NR^{c2}R^{c3}$, $-W-C(O)R^{c4}$, $-W-S(O)_2R^{c4}$, $C_{1-4}$ alkyl, $C_{3-6}$ cycloalkyl, 4-10 membered heterocyclyl, $C_{6-12}$ aryl and 5-10 membered heteroaryl; wherein each of the alkyl, the cycloalkyl, the heterocyclyl, the phenyl and the heteroaryl is optionally substituted by one or more $R^c$; or $R^4$ and $R^5$, together with the carbon atom(s) and/or nitrogen atom(s) to which they are attached, form optionally substituted $C_{3-10}$ cycloalkyl, and optionally substituted 5-12 membered heterocyclyl; and the term "optionally substituted" means that the hydrogen on the substituted group is not substituted or one or more substitutable sites of the substituted group are independently substituted by substituents selected from $R^c$; the heteroatom(s) is(are) independently selected from the group consisting of O, N and S, and the number of the heteroatoms is 1, 2 or 3.

In a further preferred embodiment, wherein $R^4$ and $R^5$ are each independently selected from the group consisting of hydrogen, deuterium, halogen, -OH, $-W-C(O)NR^{c2}R^{c3}$, $-W-NR^{c2}R^{c3}$, $-W-SO_2NR^{c2}R^{c3}$, $-W-C(O)R^{c4}$, $-W-S(O)_2R^{c4}$, $C_{1-4}$ alkyl, $C_{3-6}$ cycloalkyl, 4-10 membered heterocyclyl, $C_{6-12}$ aryl and 5-6 membered heteroaryl; wherein each of the alkyl, the cycloalkyl, the heterocyclyl, the phenyl and the heteroaryl is optionally substituted by one or more $R^c$; or $R^4$ and

$R^5$, together with the carbon atom(s) and/or nitrogen atom(s) to which they are attached, form optionally substituted $C_{3-10}$ cycloalkyl, and optionally substituted 5-12 membered heterocyclyl; and the term "optionally substituted" means that the hydrogen on the substituted group is not substituted or one or more substitutable sites of the substituted group are independently substituted by substituents selected from $R^c$; the heteroatom(s) is(are) independently selected from the group consisting of O, N and S, and the number of the heteroatoms is 1, 2 or 3.

In a further preferred embodiment, wherein $R^4$ is hydrogen, deuterium, halogen, -OH, $C_{1-4}$ alkyl, $C_{1-4}$ alkoxy, or 4-6 membered heterocyclyl; wherein each of the alkyl, the alkoxy and the heterocyclyl is optionally substituted by $R^c$; and $R^5$ is selected from the group consisting of hydrogen, deuterium, halogen, -OH, -W-C(O)$NR^{c2}R^{c3}$, -W-$NR^{c2}R^{c3}$, -W-$SO_2NR^{c2}R^{c3}$, -W-C(O)$R^{c4}$, -W-$S(O)_2R^{c4}$, $C_{1-4}$ alkyl, $C_{3-6}$ cycloalkyl, 4-10 membered heterocyclyl, $C_{6-10}$ aryl and 5-10 membered heteroaryl; wherein each of the alkyl, the cycloalkyl, the heterocyclyl, the aryl and the heteroaryl is optionally substituted by one or more $R^c$; the heteroatom(s) is(are) independently selected from the group consisting of O and N, and the number of the heteroatoms is 1 or 2.

In a further preferred embodiment, wherein $R^4$ is hydrogen, deuterium, halogen, -OH, $C_{1-4}$ alkyl, $C_{1-4}$ alkoxy, or 4-6 membered heterocyclyl; wherein each of the alkyl, the alkoxy and the heterocyclyl is optionally substituted by $R^c$; and $R^5$ is selected from the group consisting of hydrogen, deuterium, halogen, -OH, -W-C(O)$NR^{c2}R^{c3}$, -W-$NR^{c2}R^{c3}$, -W-$SO_2NR^{c2}R^{c3}$, -W-C(O)$R^{c4}$, -W-$S(O)_2R^{c4}$, $C_{1-4}$ alkyl, $C_{3-6}$ cycloalkyl, 4-10 membered heterocyclyl, $C_{6-10}$ aryl and 5-6 membered heteroaryl; wherein each of the alkyl, the cycloalkyl, the heterocyclyl, the aryl and the heteroaryl is optionally substituted by one or more $R^c$; the heteroatom(s) is(are) independently selected from the group consisting of O and N, and the number of the heteroatoms is 1 or 2.

In a further preferred embodiment, wherein $R^4$ is hydrogen, deuterium, halogen, -OH, $C_{1-4}$ alkyl, $C_{1-4}$ alkoxy, or 4-6 membered heterocycloalkyl; wherein each of the alkyl, the alkoxy and the heterocycloalkyl is optionally substituted by one or more $R^c$; and $R^5$ is selected from the group consisting of hydrogen, deuterium, halogen, -OH, -W-C(O)$NR^{c2}R^{c3}$, -W-$NR^{c2}R^{c3}$, -W-$SO_2NR^{c2}R^{c3}$, -W-C(O)$R^{c4}$, -W-$S(O)_2R^{c4}$, $C_{1-4}$ alkyl, $C_{3-6}$ cycloalkyl, 4-6 membered heterocycloalkyl, phenyl and 9-10 membered heteroaryl; wherein each of the alkyl, the cycloalkyl, the heterocycloalkyl, the phenyl and the heteroaryl is optionally substituted by one or more $R^c$.

In a further preferred embodiment, wherein $R^4$ is hydrogen, deuterium, halogen, -OH, $C_{1-4}$ alkyl, $C_{1-4}$ alkoxy, or 4-6 membered heterocycloalkyl; wherein each of the alkyl, the alkoxy and the heterocycloalkyl is optionally substituted by one or more $R^c$; and $R^5$ is selected from the group consisting of hydrogen, deuterium, halogen, -OH, -W-C(O)$NR^{c2}R^{c3}$, -W-$NR^{c2}R^{c3}$, -W-$SO_2NR^{c2}R^{c3}$, -W-C(O)$R^{c4}$, -W-$S(O)_2R^{c4}$, $C_{1-4}$ alkyl, $C_{3-6}$ cycloalkyl, 4-6 membered heterocycloalkyl, phenyl and 5-6 membered heteroaryl; wherein each of the alkyl, the cycloalkyl, the heterocycloalkyl, the phenyl and the heteroaryl is optionally substituted by one or more $R^c$.

In a further preferred embodiment, wherein $R^4$ is hydrogen, deuterium, halogen, -OH, methyl, ethyl, propyl, methoxy, ethoxy, propoxy, azetidinyl or pyrrolidinyl; wherein each of the methyl, the ethyl, the propyl, the methoxy, the ethoxy, the propoxy, the azetidinyl and the pyrrolidinyl is optionally substituted by one or more $R^c$; and $R^5$ is selected from the group consisting of hydrogen, deuterium, halogen, -OH, -W-C(O)$NR^{c2}R^{c3}$, -W-$NR^{c2}R^{c3}$, -W-$SO_2NR^{c2}R^{c3}$, -W-C(O)$R^{c4}$, -W-$S(O)_2R^{c4}$, methyl, ethyl, propyl, methoxy, ethoxy, propoxy, cyclopropyl, cyclobutyl, cyclopentyl, cyclohexyl, azetidinyl, pyrrolidinyl, phenyl, imidazolyl, pyrazolyl, pyridinyl, pyrimidyl, and benzopyrazolyl; wherein each of the methyl, the ethyl, the propyl, the methoxy, the ethoxy, the propoxy, the cyclopropyl, the cyclobutyl, the cyclopentyl, the cyclohexyl, the azetidinyl, the pyrrolidinyl, the phenyl, the imidazolyl, the pyrazolyl, the pyridinyl, the pyrimidyl, and the benzopyrazolyl is optionally substituted by one or more $R^c$.

In a further preferred embodiment, wherein $R^4$ is hydrogen, deuterium, halogen, -OH, methyl, ethyl, propyl, methoxy, ethoxy, propoxy, azetidinyl or pyrrolidinyl; wherein each of the methyl, the ethyl, the propyl, the methoxy, the ethoxy, the propoxy, the azetidinyl and the pyrrolidinyl is optionally substituted by one or more $R^c$; and $R^5$ is selected from the group consisting of hydrogen, deuterium, halogen, -OH, -W-C(O)$NR^{c2}R^{c3}$, -W-$NR^{c2}R^{c3}$, -W-$SO_2NR^{c2}R^{c3}$, -W-C(O)$R^{c4}$, -W-$S(O)_2R^{c4}$, methyl, ethyl, propyl, methoxy, ethoxy, propoxy, cyclopropyl, cyclobutyl, cyclopentyl, cyclohexyl, azetidinyl, pyrrolidinyl, phenyl, imidazolyl, pyrazolyl, pyridinyl, and pyrimidyl; wherein each of the methyl, the ethyl, the propyl, the methoxy, the ethoxy, the propoxy, the cyclopropyl, the cyclobutyl, the cyclopentyl, the cyclohexyl, the azetidinyl, the pyrrolidinyl, the phenyl, the imidazolyl, the pyrazolyl, the pyridinyl, and the pyrimidyl is optionally substituted by one or more $R^c$.

In a further preferred embodiment, wherein $R^4$ is hydrogen, halogen, -OH, methyl, ethyl, propyl, methoxy, ethoxy, azetidinyl or pyrrolidinyl; wherein each of the methyl, the ethyl, the propyl, the methoxy, the ethoxy, the azetidinyl and the pyrrolidinyl is optionally substituted by one or more $R^c$; and $R^5$ is selected from the group consisting of hydrogen, halogen, -OH, -$C_{1-3}$ alkylene-C(O)$NR^{c2}R^{c3}$, -$C_{1-3}$ alkylene-$NR^{c2}R^{c3}$, -$C_{1-3}$ alkylene-$SO_2NR^{c2}R^{c3}$, -C(O)$R^{c4}$, -$S(O)_2R^{c4}$, methyl, ethyl, phenyl, cyclopropyl, cyclobutyl, phenyl, pyridinyl, and benzopyrazolyl; wherein each of the methyl, the ethyl, the phenyl, the cyclopropyl, the cyclobutyl, the phenyl, the pyridinyl, and the benzopyrazolyl is optionally substituted by one or more $R^c$.

In a further preferred embodiment, wherein $R^4$ is hydrogen, deuterium, halogen, -OH, methyl,

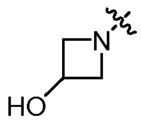, 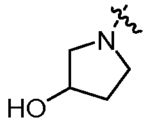 or 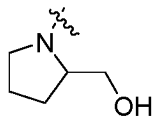;

and $R^5$ is selected from the group consisting of hydrogen, methyl, phenyl, benzoyl, $-SO_2CH_3$,

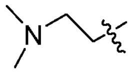, 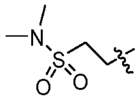, 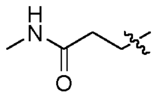, 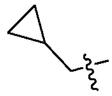,

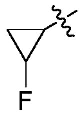, 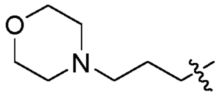, 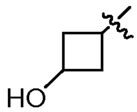, 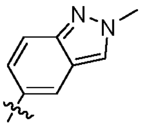 and 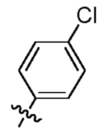.

In a further preferred embodiment, wherein $R^4$ is hydrogen, halogen, -OH or methyl; and $R^5$ is selected from the group consisting of hydrogen, methyl, phenyl, benzoyl, $-SO_2CH_3$,

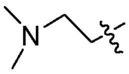, 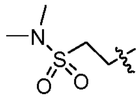, 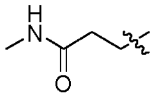, 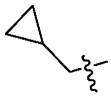, 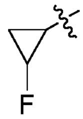, 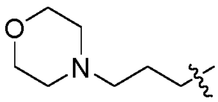, 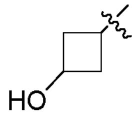, 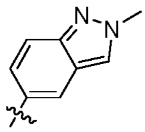

and

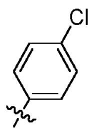.

In a further preferred embodiment, wherein $R^4$ is hydrogen, deuterium, halogen, -OH, methyl,

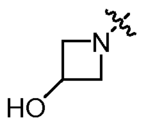, 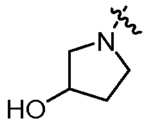 or 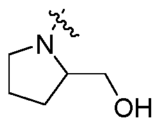;

and $R^5$ is selected from the group consisting of hydrogen, methyl, phenyl, benzoyl, $-SO_2CH_3$,

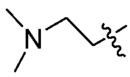, 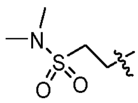, 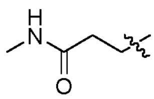, 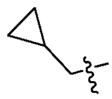,

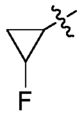, 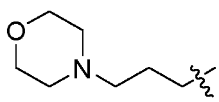 and 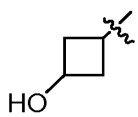.

In a further preferred embodiment, wherein $R^4$ is hydrogen, deuterium, halogen, -OH or methyl; and $R^5$ is selected from the group consisting of hydrogen, methyl, phenyl, benzoyl, $-SO_2CH_3$,

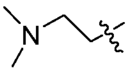, 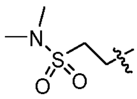, 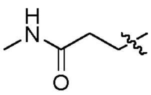, 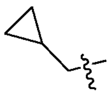, 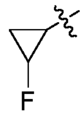, 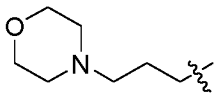 and 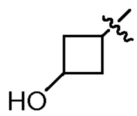.

In a further preferred embodiment, wherein $R^4$ is hydrogen; and $R^5$ is selected from the group consisting of hydrogen, $C_{1-6}$ alkyl, phenyl and 9-10 membered bicyclic heteroaryl, wherein the phenyl and heteroaryl are optionally substituted by one or more $R^c$.

In a further preferred embodiment, wherein $R^4$ is hydrogen; and $R^5$ is selected from the group consisting of hydrogen, $C_{1-4}$ alkyl, phenyl and 9-10 membered bicyclic heteroaryl, wherein the phenyl and heteroaryl are optionally substituted by one or more of halogen, -OH, and $C_{1-4}$ alkyl.

In a further preferred embodiment, wherein $R^4$ is hydrogen; and $R^5$ is selected from the group consisting of hydrogen, $C_{1-4}$ alkyl, phenyl and 9-10 membered bicyclic heteroaryl, wherein the phenyl and heteroaryl are optionally substituted by one or more of -F, -Cl, -Br, -OH, methyl, ethyl, n-propyl, and isopropyl; the heteroatom(s) in the heteroaryl is(are) selected from N, and the number of the heteroatom is 1, 2, or 3.

In a further preferred embodiment, wherein $R^c$ is independently selected from the group consisting of deuterium, halogen, oxime, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-10}$ cycloalkyl, $C_{3-10}$ cycloalkenyl, $C_{6-12}$ aryl, 3-12 membered heterocyclyl, and 5-16 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl.

In a further preferred embodiment, wherein $R^c$ is independently selected from the group consisting of deuterium, halogen, oxime,-CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-10}$ cycloalkyl, $C_{3-10}$ cycloalkenyl, $C_{6-12}$ aryl, 3-12 membered heterocyclyl, and 5-16 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl.

In a further preferred embodiment, wherein $R^c$ is independently selected from the group consisting of deuterium, halogen, oxime,-CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-10}$ cycloalkyl, $C_{3-10}$ cycloalkenyl, 3-12 membered heterocyclyl, $C_{6-12}$ aryl, and 5-12 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl.

In a further preferred embodiment, wherein $R^c$ is independently selected from the group consisting of deuterium, halogen, -CN, -OH, -SH, $-NH_2$, $-W-OR^{c1}$, $-W-SR^{c1}$, $-W-C(O)R^{c4}$, $-W-C(O)OR^{c1}$, $-W-OC(O)R^{c1}$, $-W-C(O)NR^{c2}R^{c3}$, $-W-NR^{c2}R^{c3}$, $-W-NR^{c2}C(O)R^{c4}$, $-W-S(O)_2R^{c4}$, $-W-SO_2NR^{c2}R^{c3}$, $-W-NR^{c2}S(O)_2R^{c4}$, $C_{1-4}$ alkyl, $C_{1-4}$ alkoxy, $C_{3-8}$ cycloalkyl, 4-8 membered heterocyclyl, $C_{6-12}$ aryl, and 5-8 membered heteroaryl; wherein each of the alkyl, the alkenyl, the alkynyl, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-4}$ alkyl, $C_{6-12}$ aryl, $C_{1-4}$ alkoxy, $C_{1-4}$ haloalkyl, and $C_{1-4}$ haloalkoxy.

In a further preferred embodiment, wherein $R^c$ is independently selected from the group consisting of deuterium, halogen, -CN, -OH, -SH, $-NH_2$, $-W-OR^{c1}$, $-W-SR^{c1}$, $-W-C(O)R^{c4}$, $-W-C(O)OR^{c1}$, $-W-OC(O)R^{c1}$, $-W-C(O)NR^{c2}R^{c3}$, $-W-NR^{c2}R^{c3}$, $-W-NR^{c2}C(O)R^{c4}$, $-W-S(O)_2R^{c4}$, $-W-SO_2NR^{c2}R^{c3}$, $-W-NR^{c2}S(O)_2R^{c4}$, $C_{1-4}$ alkyl, $C_{1-4}$ alkoxy, $C_{3-8}$ cycloalkyl, 4-6 membered heterocyclyl, $C_6$ aryl, and 5-6 membered heteroaryl; wherein each of the alkyl, the alkenyl, the alkynyl, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-4}$ alkyl,

$C_{6-12}$ aryl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^c$ is independently selected from the group consisting of deuterium, halogen, -CN, -OH, -W-$OR^{c1}$, -W-C(O)$NR^{c2}R^{c3}$, -W-$NR^{c2}R^{c3}$, -W-$SO_2NR^{c2}R^{c3}$, $C_{1-4}$ alkyl, and 4-8 membered heterocyclyl; wherein the heterocyclyl is optionally substituted by one or more substituents selected from the group consisting of deuterium, halogen, -OH, $C_{1-4}$ alkyl, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^c$ is independently selected from the group consisting of deuterium, halogen, -CN, -OH, -W-$OR^{c1}$, -W-C(O)$NR^{c2}R^{c3}$ , -W-$NR^{c2}R^{c3}$, -W-$SO_2NR^{c2}R^{c3}$, and 4-8 membered heterocyclyl; wherein the heterocyclyl is optionally substituted by one or more substituents selected from the group consisting of deuterium, halogen, -OH, $C_{1-4}$ alkyl, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^c$ is independently selected from the group consisting of -F, -Cl, -Br, -$CH_2$-OH, -OH, -C(O)NH-$CH_3$, -N$(CH_3)_2$, -$SO_2CH_3$, -$SO_2N(CH_3)_2$, morpholinyl, methyl, ethyl, n-propyl, and isopropyl.

In a further preferred embodiment, wherein $R^c$ is independently selected from the group consisting of -F, -Cl, -Br, -$CH_2$-OH, -OH, -C(O)NH-$CH_3$, -N$(CH_3)_2$, -$SO_2CH_3$, -$SO_2N(CH_3)_2$, and morpholinyl.

In a further preferred embodiment, wherein $R^{c1}$ is, at each occurrence, independently hydrogen, deuterium, $C_{1-6}$ alkyl, $C_{1-6}$ alkoxy, $C_{3-6}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{1-6}$ haloalkyl, and $C_{1-6}$ haloalkoxy.

In a further preferred embodiment, wherein $R^{c1}$ is, at each occurrence, independently hydrogen, deuterium, $C_{1-4}$ alkyl, $C_{1-4}$ alkoxy, $C_{3-6}$ cycloalkyl, $C_{6-10}$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-4}$ alkyl, $C_{1-4}$ alkoxy, $C_{1-4}$ haloalkyl, and $C_{1-4}$ haloalkoxy.

In a further preferred embodiment, wherein $R^{c1}$ is, at each occurrence, independently hydrogen, deuterium, $C_{1-4}$ alkyl, $C_{1-4}$ alkoxy, $C_{3-6}$ cycloalkyl, $C_6$ aryl, or 5-6 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{c1}$ is, at each occurrence, independently hydrogen, deuterium, methyl, ethyl, propyl, methoxy, ethoxy or propoxy, wherein each of the methyl, the ethyl, the propyl, the methoxy, the ethoxy and the propoxy is optionally substituted by one or more substituents selected from the group consisting of halogen, deuterium, hydroxyl, amino, and $C_{1-4}$ alkyl.

In a further preferred embodiment, wherein $R^{c1}$ is, at each occurrence, independently hydrogen, methyl, ethyl, or propyl, wherein each of the methyl, the ethyl, and the propyl is optionally substituted by one or more substituents selected from the group consisting of halogen, deuterium, hydroxyl, amino, and $C_{1-4}$ alkyl.

In a further preferred embodiment, wherein $R^{c2}$ and $R^{c3}$ are, at each occurrence, independently hydrogen, deuterium, $C_{1-4}$ alkyl, or $C_{1-4}$ alkoxy, wherein the alkyl or the alkoxy is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy.

In a further preferred embodiment, wherein $R^{c2}$ and $R^{c3}$ are, at each occurrence, independently hydrogen, deuterium, $C_{1-4}$ alkyl, or $C_{1-4}$ alkoxy, wherein the alkyl or the alkoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^{c2}$ and $R^{c3}$ are, at each occurrence, independently hydrogen, deuterium, methyl, ethyl, propyl, methoxy, ethoxy, or propoxy, wherein each of the methyl, the ethyl, the propyl, the methoxy, the ethoxy, and the propoxy is optionally substituted by one or more substituents selected from the group consisting of halogen, deuterium, hydroxyl, $C_{1-4}$ alkyl, and $C_{1-4}$ alkoxy.

In a further preferred embodiment, wherein $R^{c2}$ and $R^{c3}$ are, at each occurrence, independently hydrogen, methyl, ethyl, or propyl, wherein each of the methyl, the ethyl, and the propyl is optionally substituted by one or more substituents selected from the group consisting of halogen, deuterium, hydroxyl, amino, and $C_{1-4}$ alkyl.

In a further preferred embodiment, wherein $R^{c4}$ is, at each occurrence, independently hydrogen, deuterium, $C_{1-4}$ alkyl, $C_{1-4}$ alkoxy, $C_{3-8}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-6}$ alkyl, $C_{1-6}$ alkoxy, phenyl, $C_{1-6}$ haloalkyl, $C_{1-6}$ haloalkoxy, and halophenyl.

In a further preferred embodiment, wherein $R^{c4}$ is, at each occurrence, independently hydrogen, deuterium, $C_{1-4}$ alkyl, $C_{1-4}$ alkoxy, $C_{3-6}$ cycloalkyl, $C_6$ aryl, or 3-8 membered heterocyclyl, wherein each of the alkyl, the alkoxy, the

cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; the heteroatom(s) is(are) independently selected from the group consisting of O and N, and the number of the heteroatoms is 1 or 2.

In a further preferred embodiment, wherein $R^{c4}$ is, at each occurrence, independently hydrogen, deuterium, $C_{1-4}$ alkyl, $C_{1-4}$ alkoxy, $C_{3-6}$ cycloalkyl, or $C_6$ aryl, wherein each of the alkyl, the alkoxy, the cycloalkyl, and the aryl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^{c4}$ is, at each occurrence, independently hydrogen, deuterium, methyl, ethyl, propyl, methoxy, ethoxy, propoxy, cyclopropanyl, cyclobutanyl or phenyl; wherein each of the methyl, the ethyl, the propyl, the methoxy, the ethoxy, the propoxy, the cyclopropanyl, the cyclobutanyl and the phenyl is optionally substituted by one or more substituents selected from the group consisting of halogen, deuterium, hydroxyl, amino, $C_{1-4}$ alkyl, and $C_{1-4}$ alkoxy.

In a further preferred embodiment, wherein $R^{c4}$ is, at each occurrence, independently hydrogen, deuterium, methyl, ethyl, propyl, methoxy, ethoxy, or propoxy; wherein each of the methyl, the ethyl, the propyl, the methoxy, the ethoxy, and the propoxy is optionally substituted by one or more substituents selected from the group consisting of halogen, deuterium, hydroxyl, $C_{1-4}$ alkyl, and $C_{1-4}$ alkoxy.

In a further preferred embodiment, wherein W is selected from the group consisting of bond and $C_{1-3}$ alkylene; wherein the alkylene is optionally substituted by one or more substituents selected from the group consisting of deuterium, halogen, oxo, -CN, -OH, and $-NH_2$.

In a further preferred embodiment, wherein W is selected from the group consisting of bond and $C_{1-3}$ alkylene; wherein the alkylene is optionally substituted by one or more substituents selected from the group consisting of deuterium, halogen, oxo, -CN, -OH, and $-NH_2$.

In a further preferred embodiment, wherein W is selected from the group consisting of bond, methylene and ethylene; wherein the methylene or the ethylene is optionally substituted by one or more substituents selected from the group consisting of deuterium, halogen, oxo, -CN, -OH, and $-NH_2$.

**In a second aspect,** the present application provides a compound represented by formula (II), or a tautomer, a stereoisomer, or a pharmaceutically acceptable salt thereof:

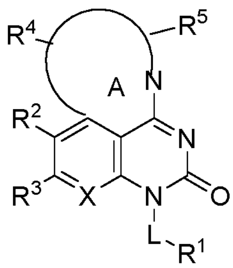 (II),

wherein ring A is a 5-12 membered heteroaromatic ring or a 5-12 membered heterocyclyl; and $R^1$, $R^2$, $R^3$, $R^4$, $R^5$, L and X are as defined in the compound of formula (I) of the present application.

In a further preferred embodiment, wherein L is bond.

In a further preferred embodiment, wherein the substitution site of $R^5$ is N atom on the ring.

In a further preferred embodiment, wherein ring A is selected from the group consisting

of 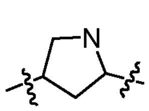, 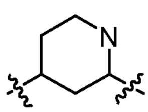, 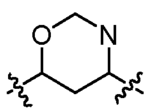, 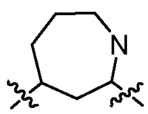, 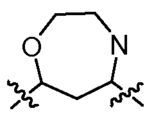 and 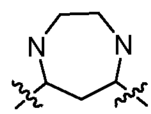.

In a further preferred embodiment, wherein ring A is selected from the group consisting

of 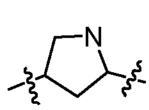, 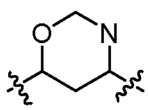, 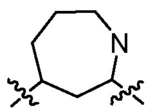 and 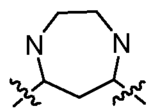.

In a further preferred embodiment, wherein ring A is selected from the group consisting

of 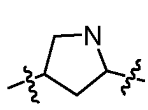, 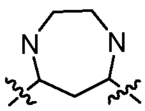 and 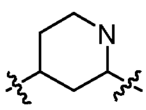.

In a further preferred embodiment, wherein ring A is selected from

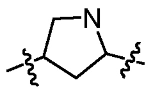.

**In a third aspect,** the present application provides a compound represented by formula (III), or a tautomer, a stereoisomer, or a pharmaceutically acceptable salt thereof, which has the following structures:

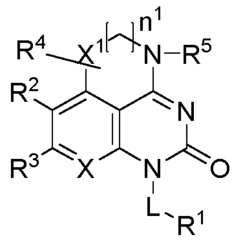

(III) ,

wherein $X^1$ is selected from the group consisting of C, N, O and S; $n^1$ is selected from the group consisting of 0, 1, 2 and 3; and $R^1$, $R^2$, $R^3$, $R^4$, $R^5$, L and X are as defined in the compound represented by formula (I) of the present application.

In a further preferred embodiment, wherein $n^1$ is selected from the group consisting of 0, 1 and 2.

In a further preferred embodiment, wherein L is selected from bond.

In a further preferred embodiment, wherein $X^1$ is selected from the group consisting of C, N and O.

In a further preferred embodiment, wherein

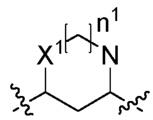

is selected from the group consisting of

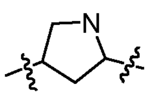, 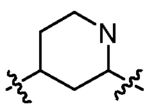, 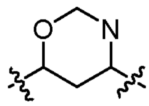, 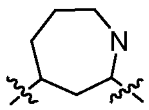 and 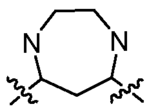.

In a further preferred embodiment, wherein ring A is selected from the group consisting

of 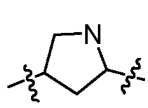, 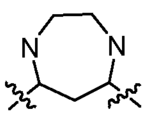 and 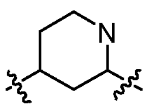.

**In a fourth aspect,** the present application provides a compound represented by formula (III-a), (III-b), (III-c), (III-d) or (III-e), or a tautomer, a stereoisomer, or a pharmaceutically acceptable salt thereof, which has the following structures:

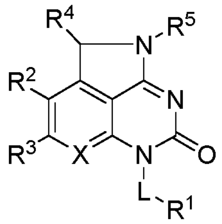
(III-a)

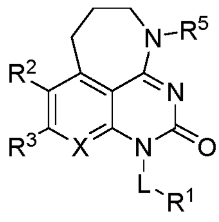
(III-b)

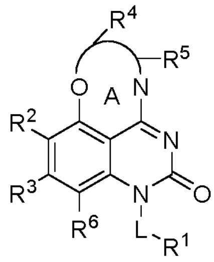
(III-c)

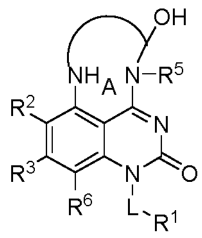
(III-d)

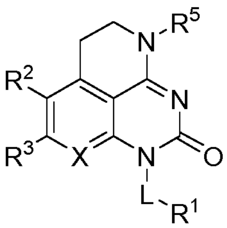
(III-e)

wherein $R^1$, $R^2$, $R^3$, $R^4$, $R^5$, $R^6$, L and X are as defined in the compound represented by formula (I).

In a further preferred embodiment, wherein L is selected from bond.

In a further preferred embodiment, wherein the substitution site of $R^5$ in the formula (III-c) is N atom on the ring.

In a further preferred embodiment, wherein ring A is selected from the group consisting of

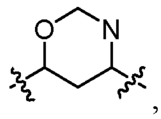
,
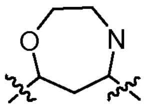
and
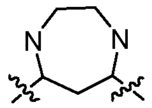
.

In a further preferred embodiment, wherein X is selected from the group consisting of N and CH.

In a further preferred embodiment, wherein L is bond.

In a further preferred embodiment, wherein $R^3$ is selected from the group consisting of - Cl and $-CF_3$.

In a further preferred embodiment, wherein $R^2$ is selected from hydrogen.

In a further preferred embodiment, wherein $R^6$ is selected from hydrogen.

In a further preferred embodiment, wherein $R^4$ is hydrogen; and $R^5$ is selected from the group consisting of hydrogen, methyl, phenyl,

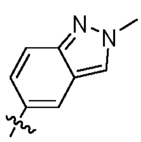
and
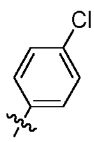
.

In a further preferred embodiment, wherein $R^1$ is selected from pyridinyl optionally substituted by one or more $R^a$.

In a further preferred embodiment, wherein $R^a$ is, at each occurrence, independently selected from the group consisting of Cl, methyl, and methoxy.

In a further preferred embodiment, wherein $R^1$ is selected from the group consisting of

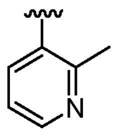
,
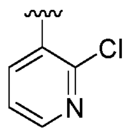
and
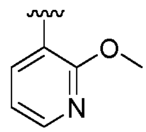
.

**In a fifth aspect,** the present application provides an atropisomer of a compound represented by formula (III-a), (III-b), (III-c), (III-d), or (III-e), or a pharmaceutically acceptable salt of the atropisomer thereof:
wherein $R^1$, $R^2$, $R^3$, $R^4$, $R^5$, $R^6$, L, and X are as defined in the compound represented by the formula (III-a), (III-b), (III-c), (III-d), or (III-e), wherein L is a bond, and $R^1$ is substituted with at least one $R^a$.

Atropisomers are stereoisomers resulting from hindered rotation of a single bond axis, where the rotational barrier is high enough to allow separation of the individual rotamers. (LaPlante et al., J. Med. Chem., 54: 7005(2011)). For the compounds of formula (III-a), (III-b), (III-c), (III-d) or (III-e), wherein $R^1$ is substituted with one or more $R^a$, a stereogenic axis is present at the bond between the quinazolinone ring and the group $R^1$. Due to the asymmetric nature of the substitution on the ring attached through this bond, and due to the restricted rotation of this bond caused by steric hindrance, such compounds can form rotamers. Accordingly, these compounds of formula (III-a), (III-b), (III-c), (III-d) or (III-e) may form two rotamers, which can be separated into individual atropisomers in some cases, such as by chromatography on a chiral stationary phase.

In a further preferred embodiment, the compounds of formula (III-a), (III-b), (III-c), (III-d) or (III-e) may be provided

as a mixture of two atropisomers or as a single atropisomer, which are separable and stable in solution at ambient and physiological temperatures. The absolute spatial structure of atropisomers can be determined by single crystal X-ray crystallography.

In a further preferred embodiment, the compounds of formula (III-a), (III-b), (III-c), (III-d) or (III-e) may be provided as individual atropisomers or as a mixture comprising two atropisomers of the compounds of formula (III-a), (III-b), (III-c), (III-d) or (III-e) in any ratio.

In a further preferred embodiment, for the compounds of formula (III-a), (III-b), (III-c), (III-d) or (III-e) or a salt thereof, only one atropisomer is provided, or only one atropisomer mixed with a small amount of other atropisomer(s) is provided. If an absolute configuration is not assigned, then the provided atropisomer may be defined by its elution order relative to another atropisomer during chromatography on a chiral stationary phase under specific conditions.

In a further preferred embodiment, for the atropisomers of the compounds of formula (III-a), (III-b), (III-c), (III-d) or (III-e), each of the atropisomers of the compounds of formula (III-a), (III-b), (III-c), (III-d) or (III-e) substantially free of its complementary atropisomer can be provided. As used herein, "substantially free" refers to providing the compound of formula (I) having an atropisomeric purity of at least 95% , preferably at least 99%, and more preferably at least 99.5%.

On the basis of the routine knowledge in the art, the above-mentioned preferred conditions can be arbitrarily combined to obtain preferred embodiments of the present application.

Preferably, the present application provides a compound, or a tautomer, a stereoisomer, an atropisomer, or a pharmaceutically acceptable salt thereof, wherein said compound has the following structure:

| Structural formulae and No. of compounds | Structural formulae and No. of compounds | Structural formulae and No. of compounds |
|---|---|---|
| 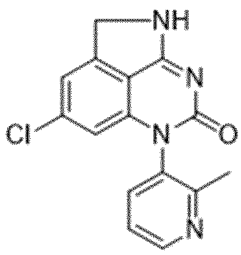 A1 | 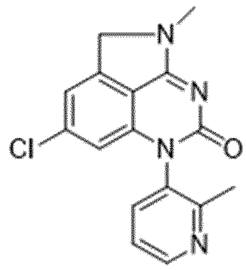 A2 | 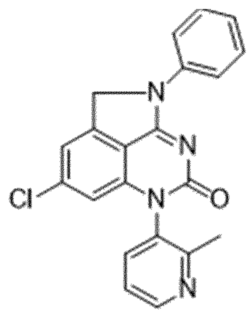 A3 |
| 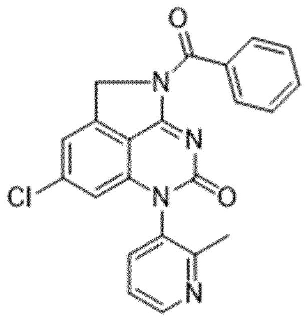 A4 | 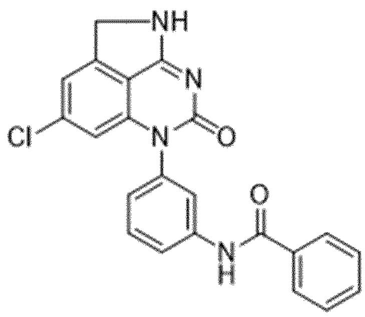 A5 | 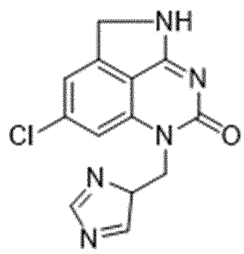 A6 |

(continued)

| Structural formulae and No. of compounds | Structural formulae and No. of compounds | Structural formulae and No. of compounds |
|---|---|---|
| 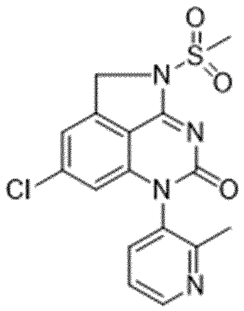 A7 | 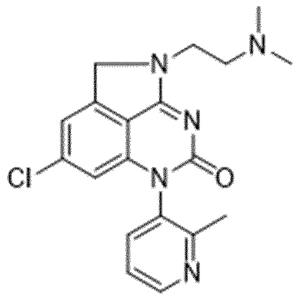 A8 | 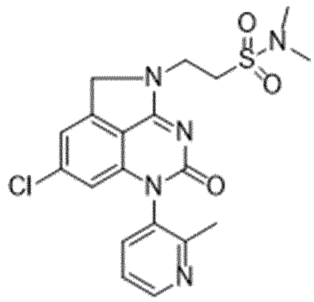 A9 |
| 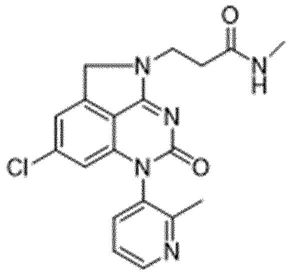 A10 | 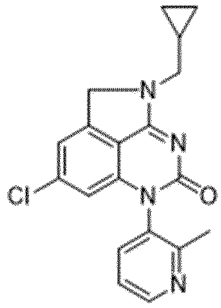 A11 | 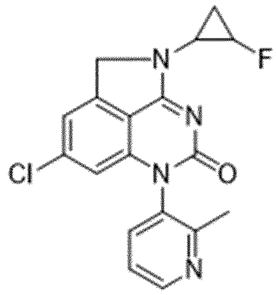 A12 |
| 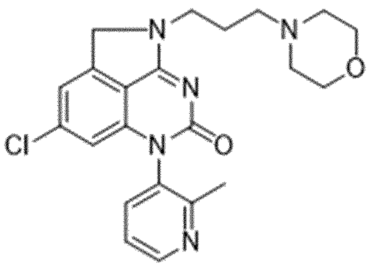 A13 | 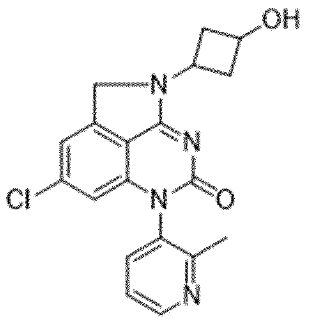 A14 | 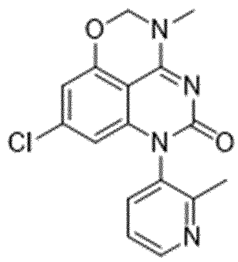 A15 |
| 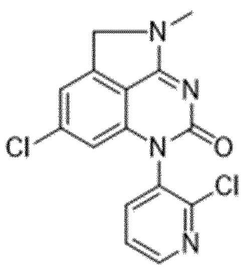 A16 | 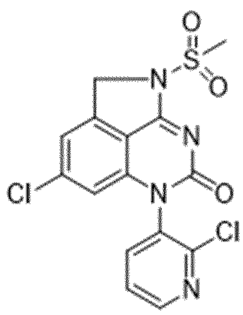 A17 | 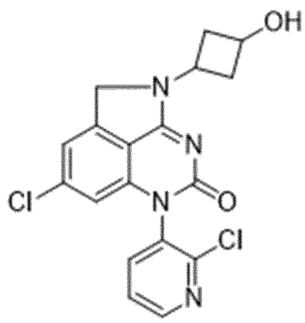 A18 |

(continued)

| Structural formulae and No. of compounds | Structural formulae and No. of compounds | Structural formulae and No. of compounds |
|---|---|---|
| 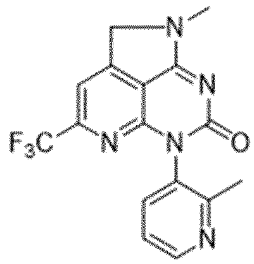 A19 | 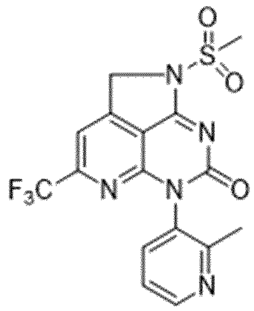 A20 | 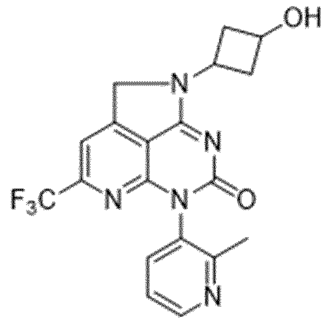 A21 |
| 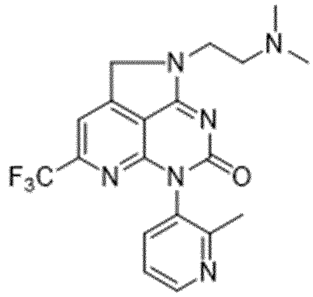 A22 | 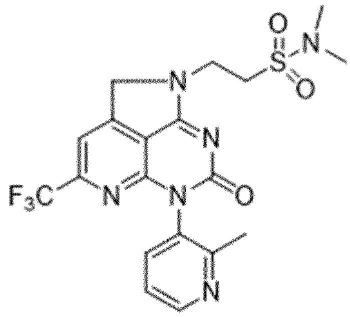 A23 | 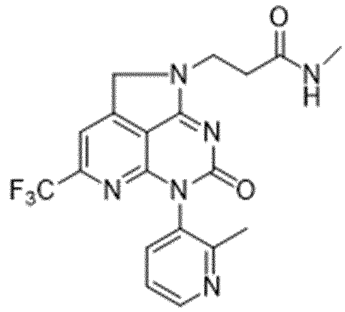 A24 |
| 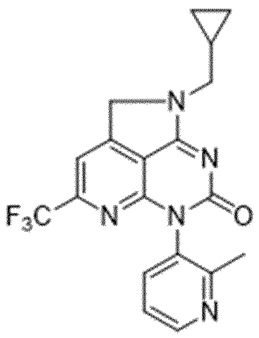 A25 | 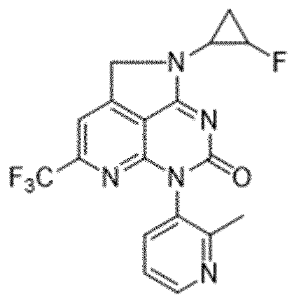 A26 | 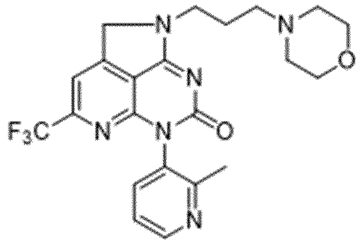 A27 |
| 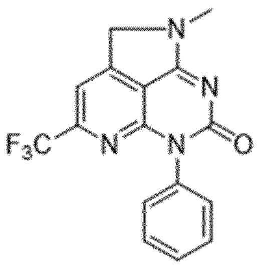 A28 | 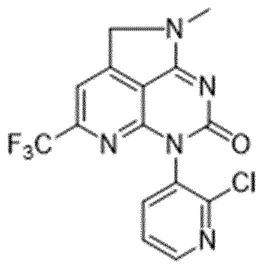 A29 | 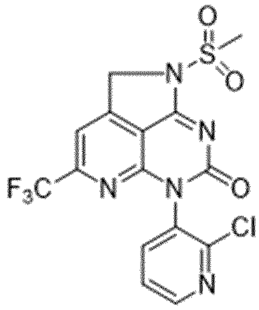 A30 |

(continued)

| Structural formulae and No. of compounds | Structural formulae and No. of compounds | Structural formulae and No. of compounds |
|---|---|---|
| 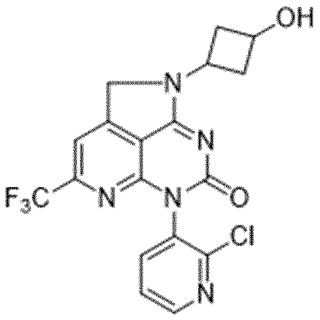 A31 | 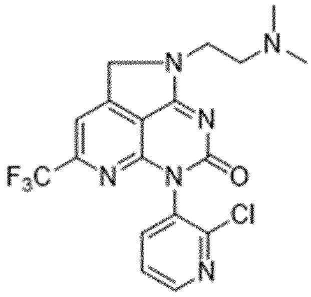 A32 | 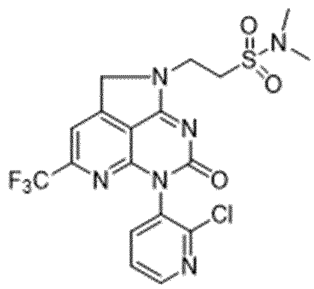 A33 |
| 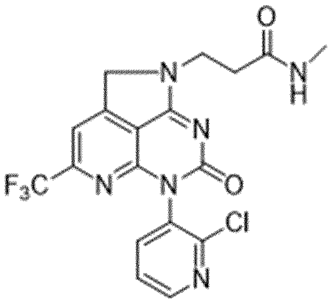 A34 | 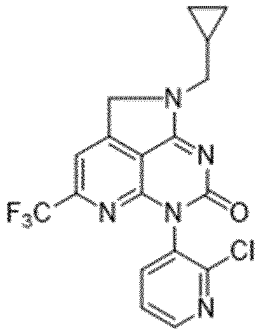 A35 | 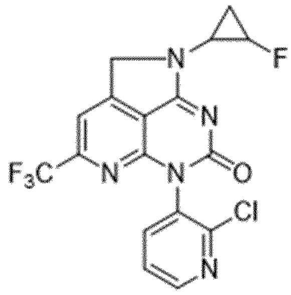 A36 |
| 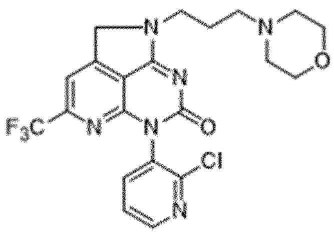 A37 | 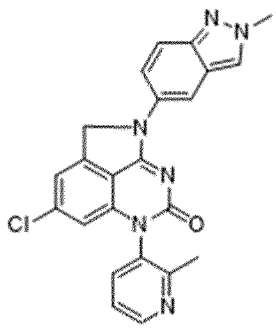 A38 | 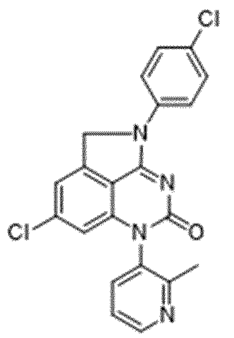 A39 |
| 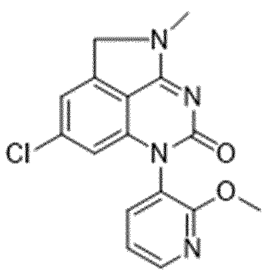 A40 | 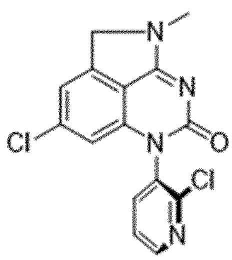 | 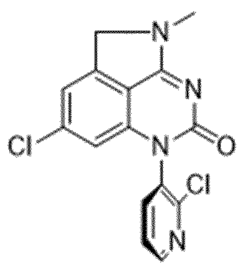 |

(continued)

| Structural formulae and No. of compounds | Structural formulae and No. of compounds | Structural formulae and No. of compounds |
|---|---|---|
| 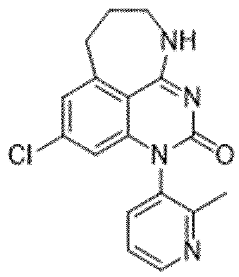 **B1** | 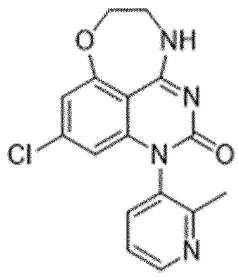 **B2** | 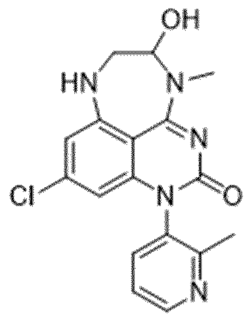 **B3** |
| 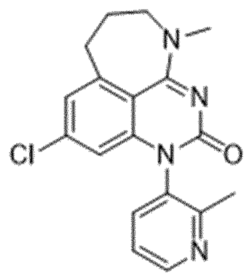 **B4** | 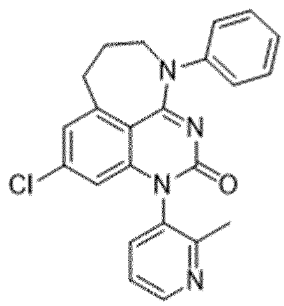 **B5** | 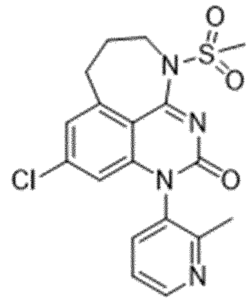 **B6** |
| 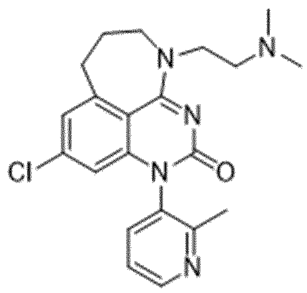 **B7** | 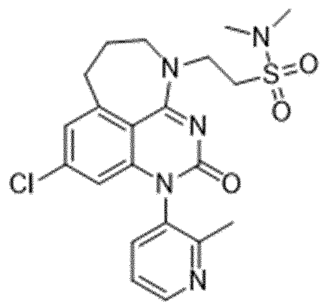 **B8** | 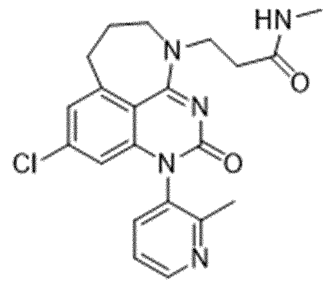 **B9** |
| 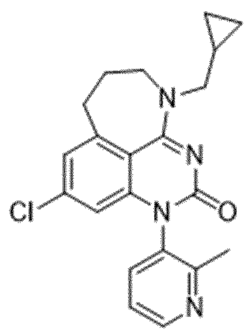 **B10** | 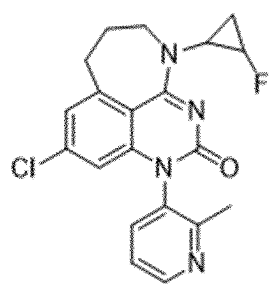 **B11** | 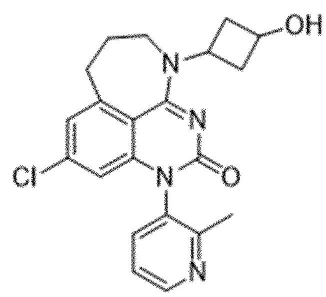 **B12** |

(continued)

| Structural formulae and No. of compounds | Structural formulae and No. of compounds | Structural formulae and No. of compounds |
|---|---|---|
| 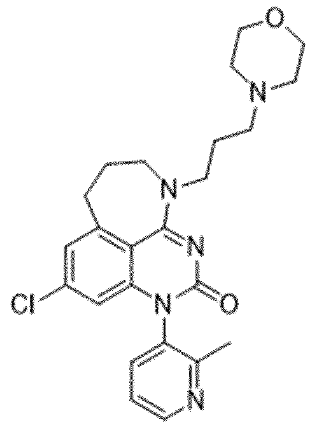 B13 | 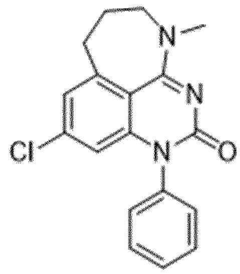 B14 | 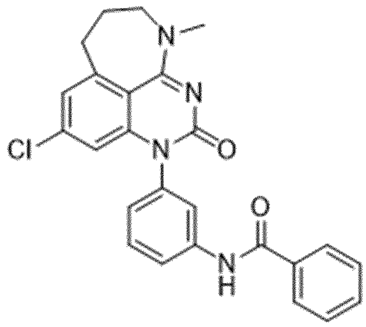 B15 |
| 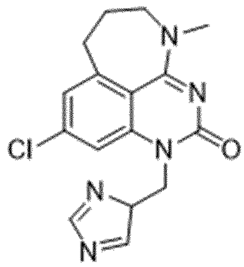 B16 | 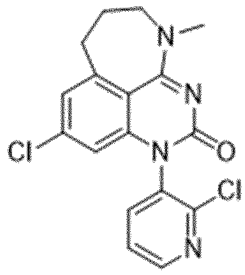 B17 | 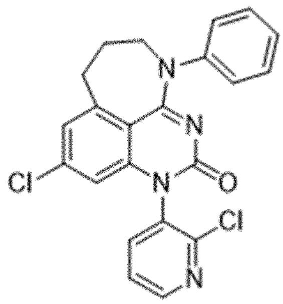 B18 |
| 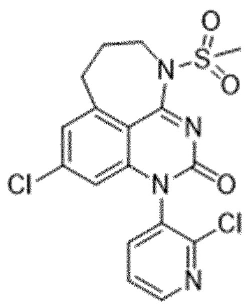 B19 | 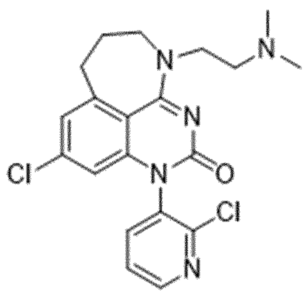 B20 | 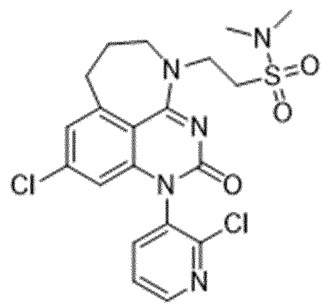 B21 |
| 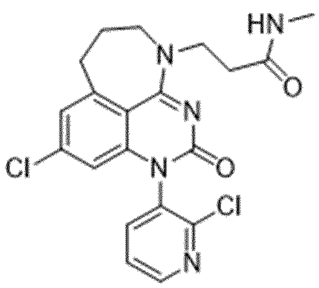 B22 | 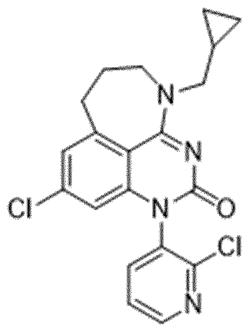 B23 | 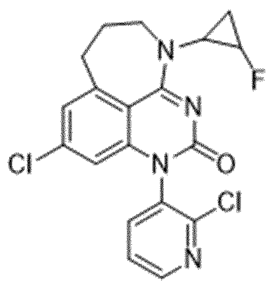 B24 |

(continued)

| Structural formulae and No. of compounds | Structural formulae and No. of compounds | Structural formulae and No. of compounds |
|---|---|---|
| 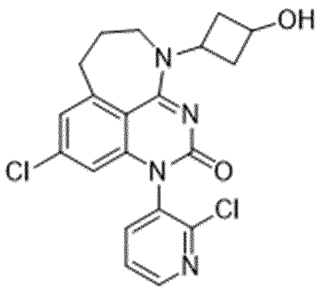 **B25** | 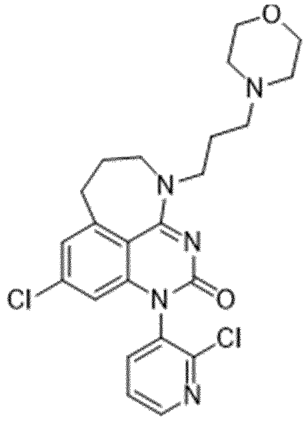 **B26** | 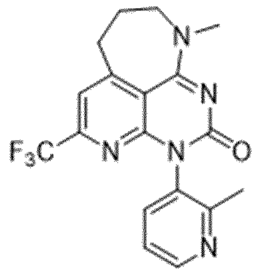 **B27** |
| 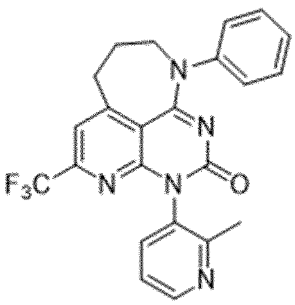 **B28** | 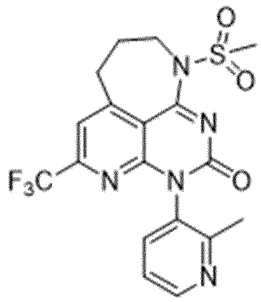 **B29** | 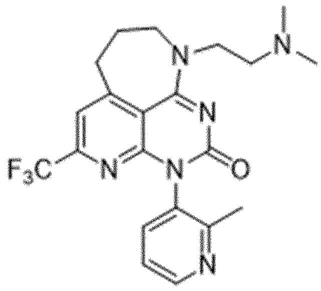 **B30** |
| 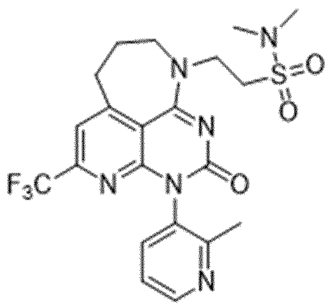 **B31** | 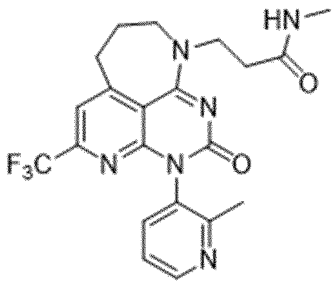 **B32** | 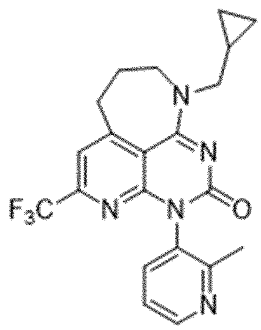 **B33** |

(continued)

| Structural formulae and No. of compounds | Structural formulae and No. of compounds | Structural formulae and No. of compounds |
|---|---|---|
| 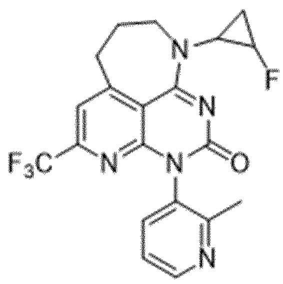 B34 | 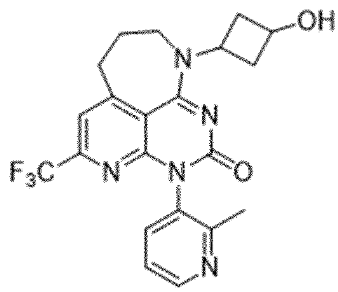 B35 | 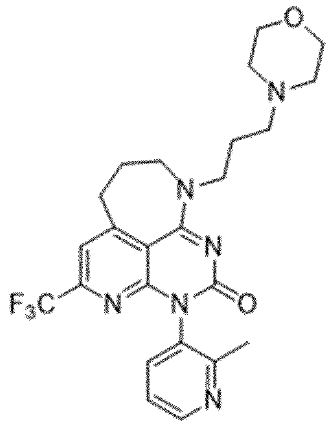 B36 |
| 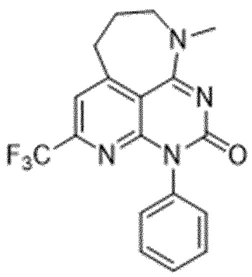 B37 | 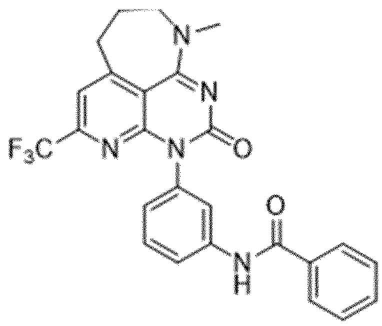 B38 | 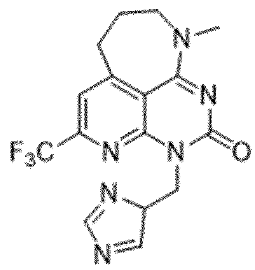 B39 |
| 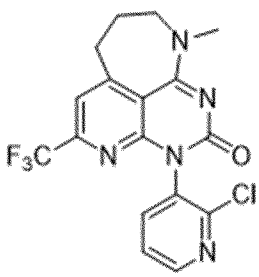 B40 | 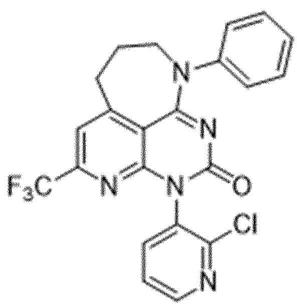 B41 | 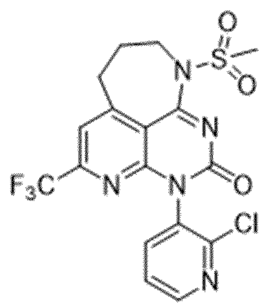 B42 |
| 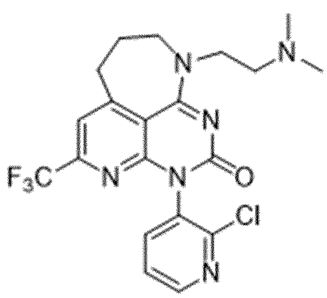 B43 | 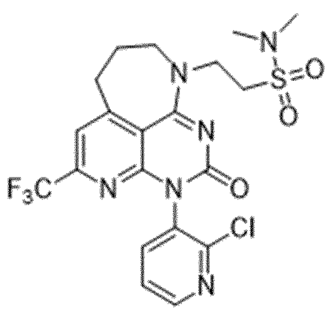 B44 | 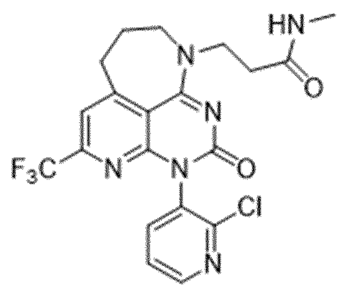 B45 |

(continued)

| Structural formulae and No. of compounds | Structural formulae and No. of compounds | Structural formulae and No. of compounds |
|---|---|---|
| 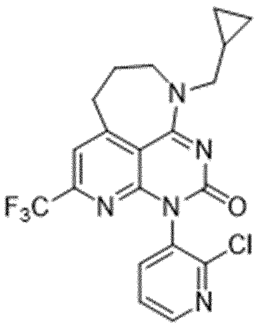 B46 | 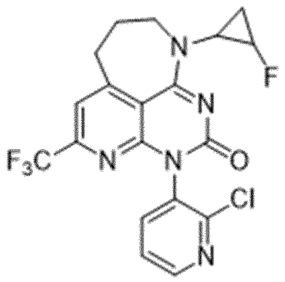 B47 | 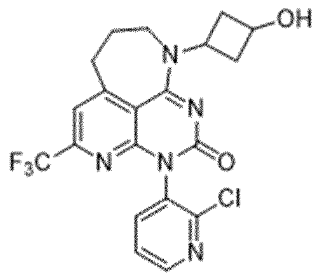 B48 |
| 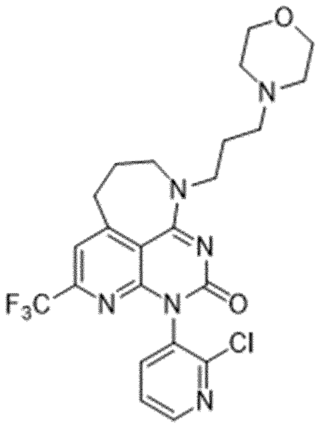 B49 | 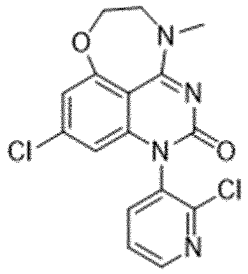 B50 | 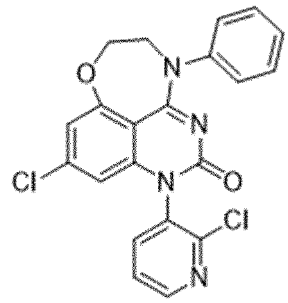 B51 |
| 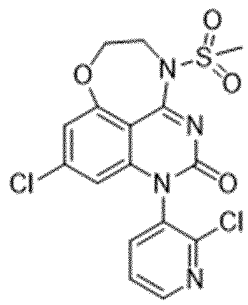 B52 | 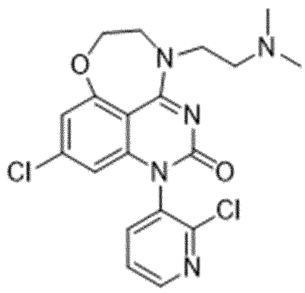 B53 | 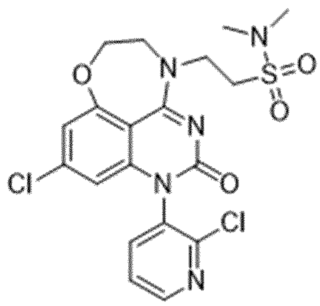 B54 |
| 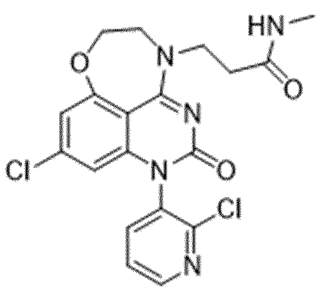 B55 | 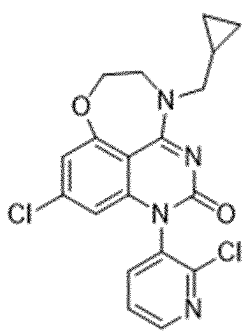 B56 | 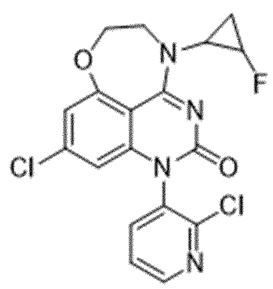 B57 |

(continued)

| Structural formulae and No. of compounds | Structural formulae and No. of compounds | Structural formulae and No. of compounds |
|---|---|---|
| 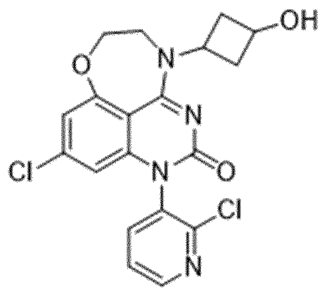 B58 | 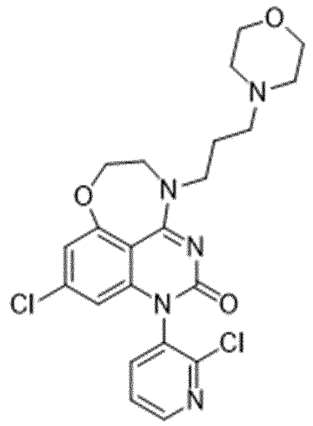 B59 | 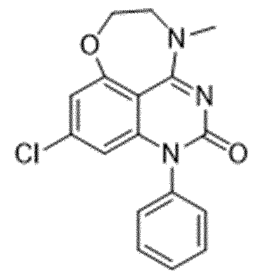 B60 |
| 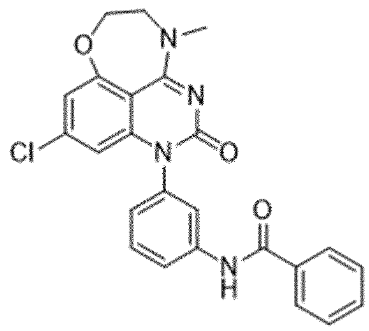 B61 | 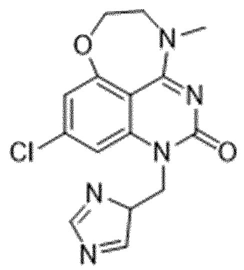 B62 | 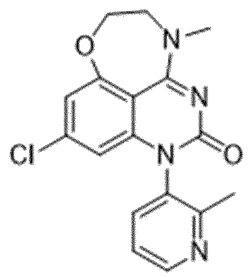 B63 |
| 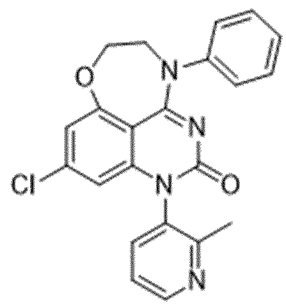 B64 | 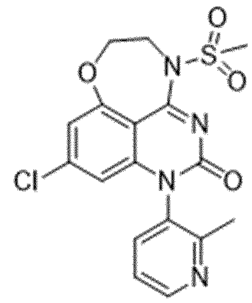 B65 | 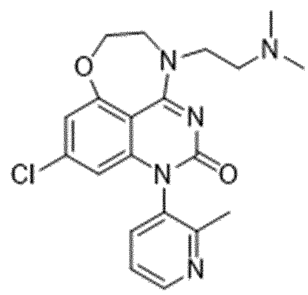 B66 |
| 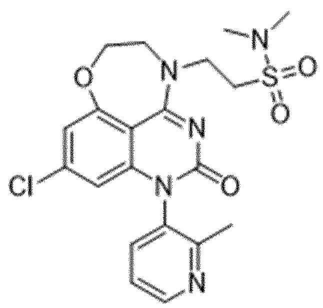 B67 | 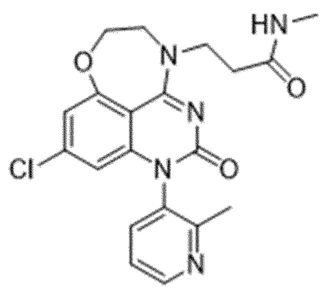 B68 | 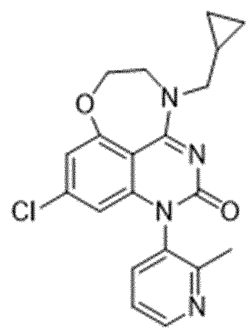 B69 |

(continued)

| Structural formulae and No. of compounds | Structural formulae and No. of compounds | Structural formulae and No. of compounds |
|---|---|---|
| 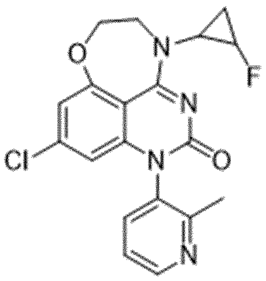 B70 | 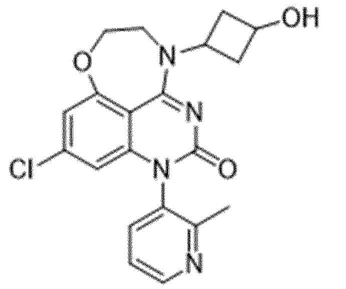 B71 | 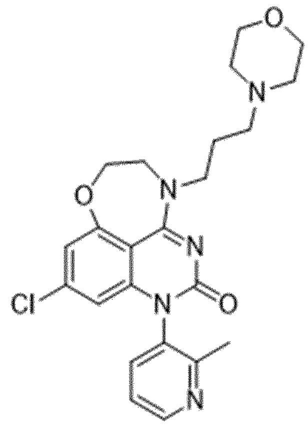 B72 |
| 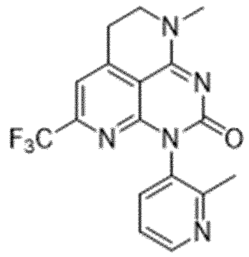 C1 | 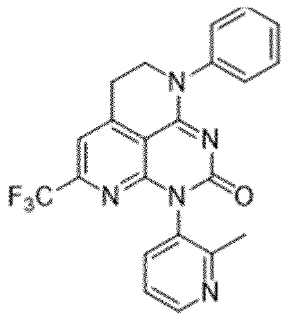 C2 | 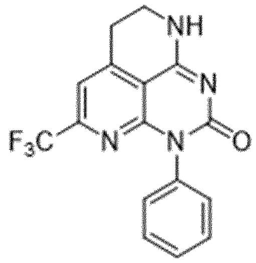 C3 |
| 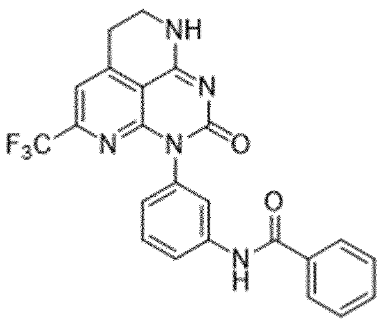 C4 | 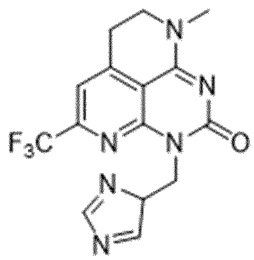 C5 | 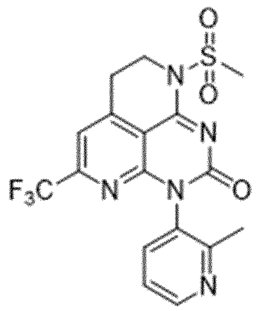 C6 |
| 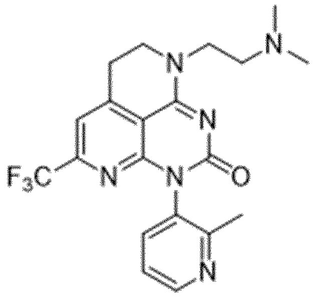 C7 | 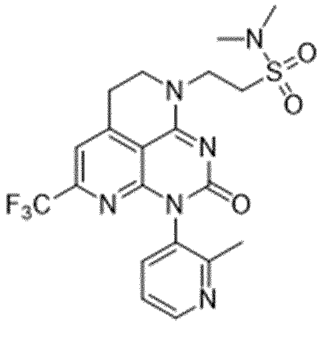 C8 | 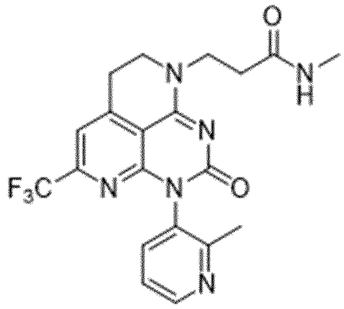 C9 |

(continued)

| Structural formulae and No. of compounds | Structural formulae and No. of compounds | Structural formulae and No. of compounds |
|---|---|---|
| 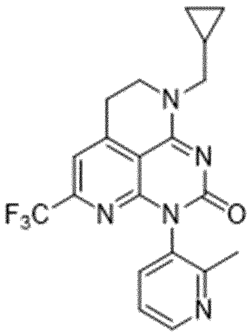 C10 | 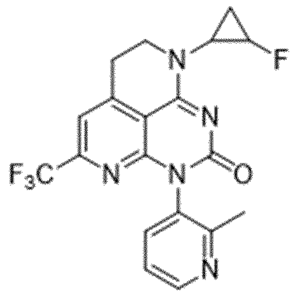 C11 | 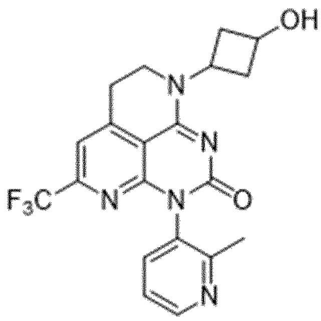 C12 |
| 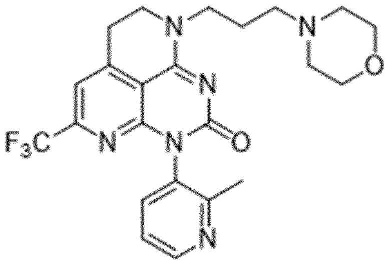 C13 | 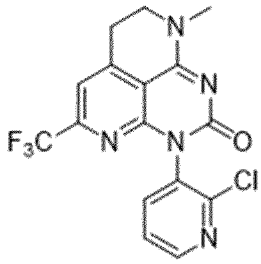 C14 | 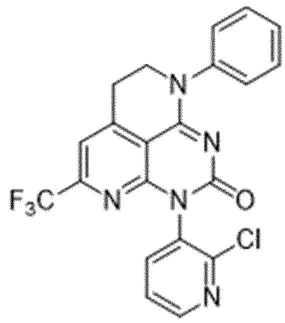 C15 |
| 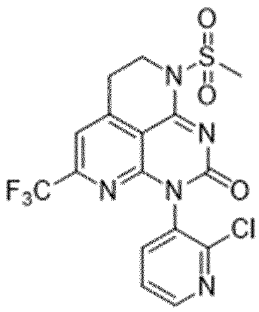 C16 | 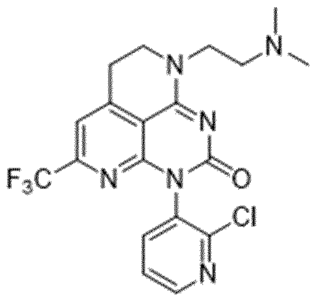 C17 | 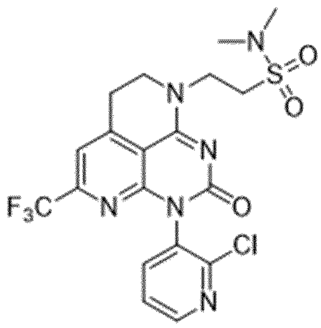 C18 |
| 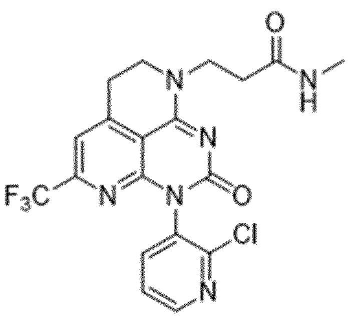 C19 | 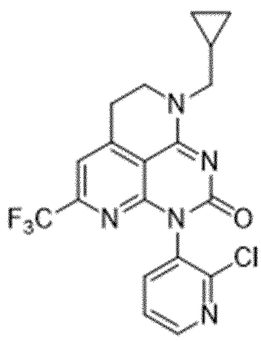 C20 | 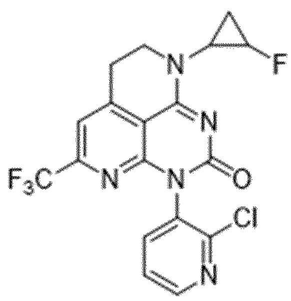 C21 |

(continued)

| Structural formulae and No. of compounds | Structural formulae and No. of compounds | Structural formulae and No. of compounds |
|---|---|---|
| 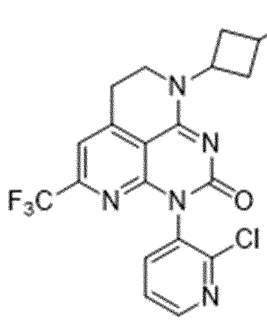 C22 | 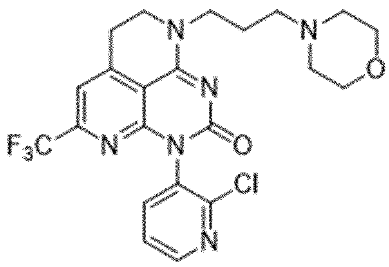 C23 | |

**In a sixth aspect,** the object of the present application also includes the provision of a method for preparing the compounds represented by the above formulae or a tautomer, a stereoisomer, or a pharmaceutically acceptable salt thereof.

The compounds can be prepared, for example, using the methods shown in the following schemes.

Method 1:

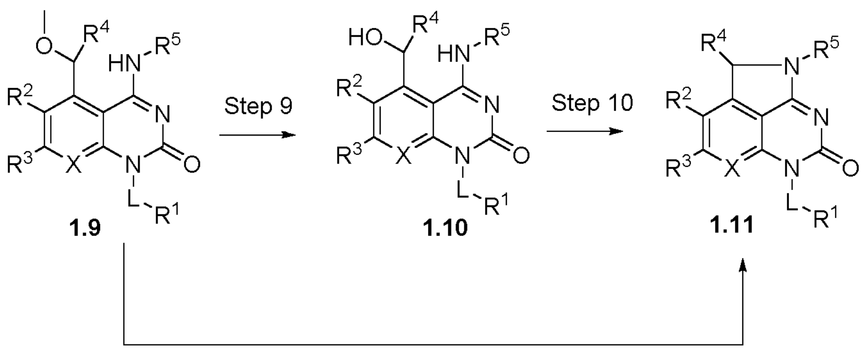


1.9 1.10 1.11

The methyl group of the methoxy group in Compound 1.9 is removed to obtain intermediate 1.10, which finally undergoes a ring-closing reaction to obtain the target compound 1.11. Alternatively, the methyl protecting group in intermediate 1.9 was removed and simultaneously an in-situ ring-closing reaction occurs to obtain the target compound 1.11.

In a further preferred embodiment, compound 1.9 can be prepared by the following steps:

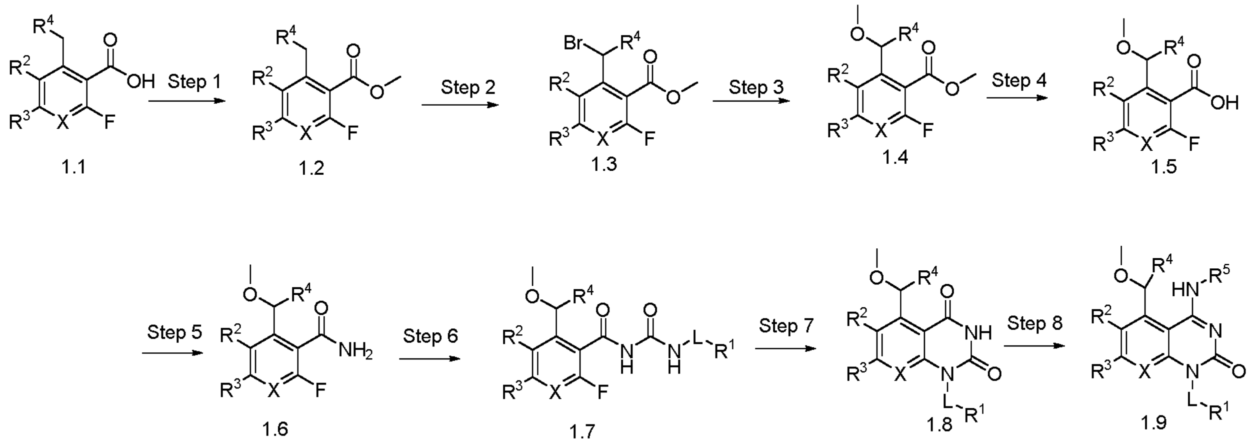


1.1 1.2 1.3 1.4 1.5 1.6 1.7 1.8 1.9

Fluorinated aryl-carboxylic acid (1.1) is used as a starting material, which is esterified with methanol to obtain intermediate 1.2, and then a bromination reaction is carried out at the benzylic position of the aromatic ring of intermediate 1.2 to obtain intermediate 1.3. Then, the bromine atom of intermediate 1.3 is replaced with a methoxy group to obtain compound 1.4, which undergoes esterolysis reaction under a basic condition to obtain intermediate 1.5 containing a carboxyl group, which is converted to amide to obtain intermediate 1.6. Intermediate 1.6 reacts with $R^1$-L-$NH_2$ under the action of oxalyl chloride to obtain intermediate 1.7, followed by a ring-closing reaction under a basic condition to obtain intermediate 1.8, which reacts with phosphorus oxychloride, and then reacts with $R^5NH_2$ to obtain intermediate 1.9, wherein, $R^1$, $R^2$, $R^3$, $R^4$, $R^5$, L and X are as defined in the compound of formula (III-a).

Method 2:

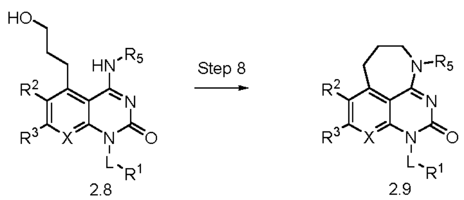


2.8 2.9

Compound 2.8 undergoes a ring-closing reaction under the action of methanesulfonyl chloride to obtain the target compound 2.9.

In a further preferred embodiment, compound 2.8 can be prepared by the following steps:

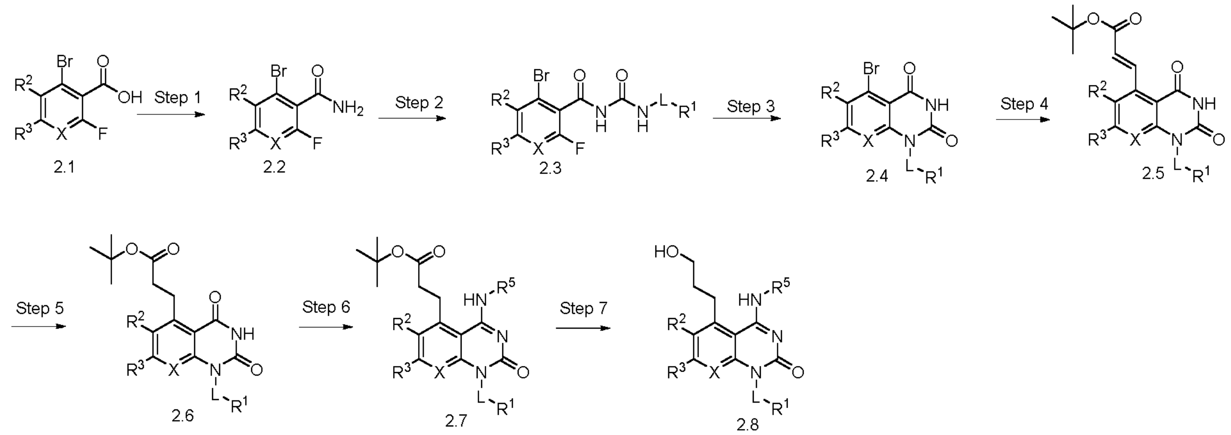


2.1 2.2 2.3 2.4 2.5 2.6 2.7 2.8

Fluorinated and brominated aryl-carboxylic acid (2.1) is used as a starting material, and carboxylic acid is converted to amide intermediate 2.2, which reacts with $R^1$-L-$NH_2$ under the action of oxalyl chloride to obtain intermediate 2.3, followed by a ring-closing reaction under a basic condition to obtain intermediate 2.4, which undergoes a Heck reaction with isobutyl acrylate to obtain compound 2.5. Then, the double bond of compound 2.5 is reduced with sodium

borohydride to obtain intermediate 2.6, which reacts with phosphorus oxychloride and then reacts with $R_5NH_2$ to obtain intermediate 2.7, followed by reduction with lithium aluminum hydride to obtain intermediate 2.8 containing hydroxyl group, wherein, $R^1$, $R^2$, $R^3$, $R^5$, L and X are as defined in the compound of formula (III-b).

Method 3:

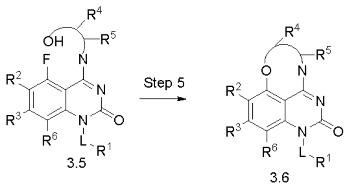


3.5 3.6

Compound 3.5 undergoes a substitution reaction under a basic condition to obtain the target compound 3.6.

In a further preferred embodiment, compound 3.5 can be prepared by the following steps:

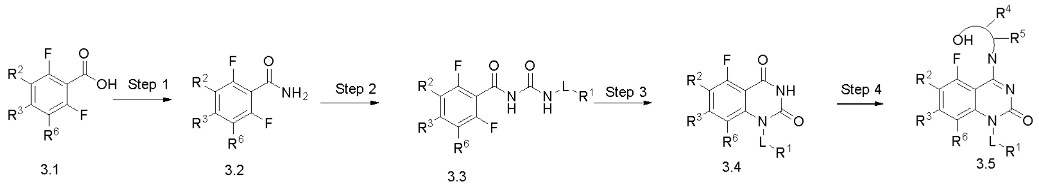


3.1 3.2 3.3 3.4 3.5

Substituted 2,6-difluorobenzoic acid (3.1) is used as a starting material, and the carboxylic acid thereof is converted to amide intermediate 3.2, which reacts with $R^1$-L-$NH_2$ under the action of oxalyl chloride to obtain intermediate 3.3, followed by a ring-closing reaction under a basic condition to obtain intermediate 3.4, which reacts with phosphorus oxychloride and then reacts with the added amine compound substituted with hydroxyl group at the end to obtain intermediate 3.5,

wherein $R^1$, $R^2$, $R^3$, $R^4$, $R^5$, $R^6$ and L are as defined in the compound of formula (III-c).

Method 4:

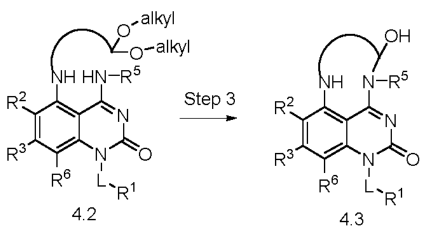


4.2 4.3

Compound 4.2 is subjected to removal of alkyl group and a ring-closing reaction under an acidic condition to obtain the target compound 4.3.

In a further preferred embodiment, compound 4.2 can be prepared by the following steps:

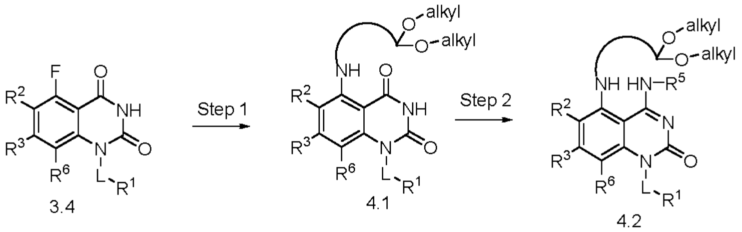


3.4 4.1 4.2 ,

wherein alkyl represents $C_{1-6}$ alkyl.

Compound 3.4 as a starting material undergoes a substitution reaction with an acetal substituted with a terminal amino group to obtain intermediate 4.1, which reacts with phosphorus oxychloride and then the added amine compound to obtain intermediate 4.2,
wherein $R^1$, $R^2$, $R^3$, $R^4$, $R^5$, $R^6$ and L are as defined in the compound of formula (III-d).

Method 5:

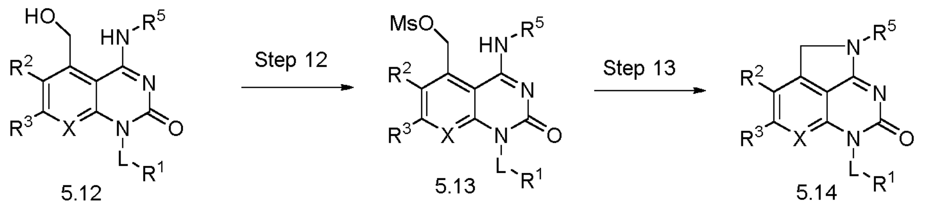


5.12 5.13 5.14

The hydroxy group of compound 5.12 is converted to a MsO fragment with a strong leaving ability, and finally an intramolecular ring-closing reaction occurs under a basic condition to obtain the target compound 5.14.

In a further preferred embodiment, compound 5.12 can be prepared by the following steps:

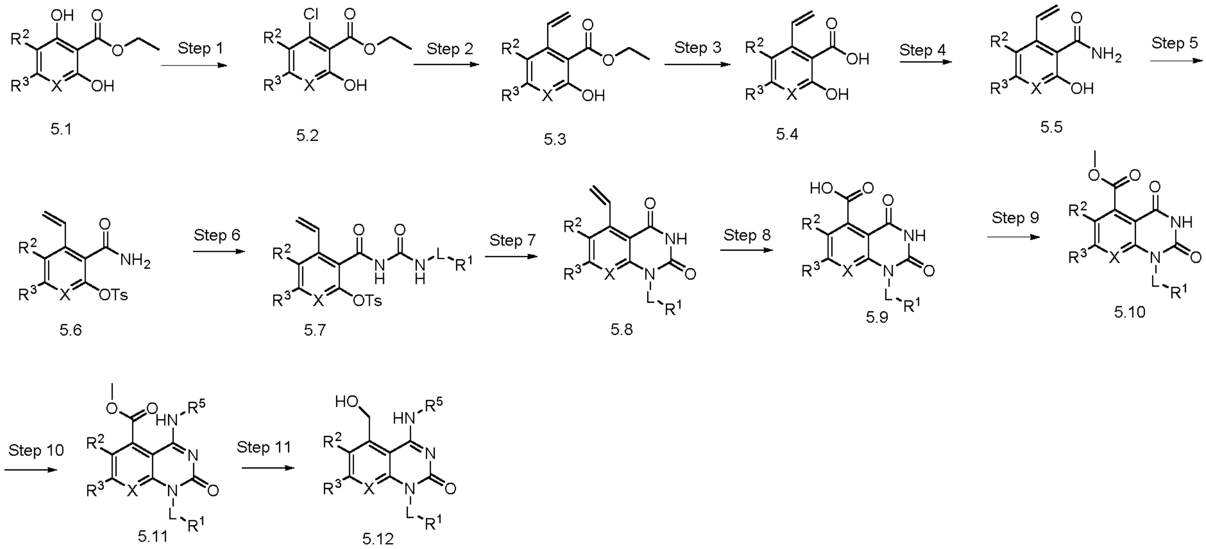


5.1 5.2 5.3 5.4 5.5 5.6 5.7 5.8 5.9 5.10 5.11 5.12

Compound 5.1 as a starting material reacts with phosphorus oxychloride to obtain intermediate 5.2, which reacts with potassium vinylboron trifluoride to obtain intermediate 5.3, followed by a hydrolysis reaction under a basic condition to obtain compound 5.4 containing a carboxyl fragment. Then, the carboxyl group is converted to amide, and then reacts with p-toluenesulfonyl chloride to obtain intermediate 5.6, which reacts with $R^1NH_2$ under the action of oxalyl chloride to obtain intermediate 5.7, followed by an intramolecular ring-closing reaction under a basic condition to obtain intermediate 5.8. The vinyl group of intermediate 5.8 is oxidized to a carboxylic acid moiety, followed by the conversion of the carboxyl group of compound 5.9 to a methyl ester, which then reacts with $R^5NH_2$ under the action of phosphorus oxychloride to obtain intermediate 5.11, followed by the reduction of the methyl ester moiety to obtain intermediate 5.12,
wherein $R^1$, $R^2$, $R^3$, $R^5$, L and X are as defined in the compound of formula (III-a).

Method 6:

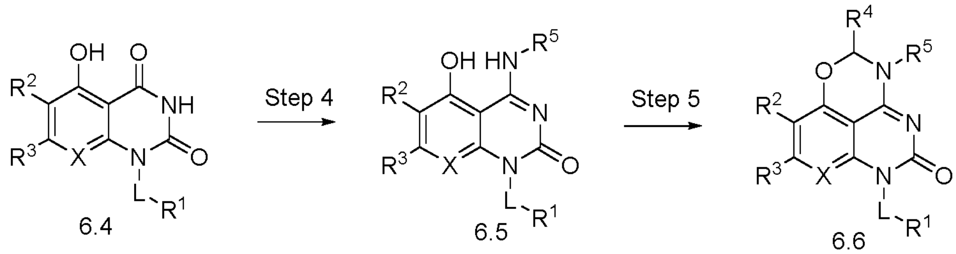


6.4 6.5 6.6

Compound 6.4 reacts with phosphorus oxychloride, and then reacts with $R^5NH_2$ to obtain intermediate 6.5, which finally reacts with the corresponding aldehyde $R^4CHO$ to obtain the target compound 6.6.

In a further preferred embodiment, compound 6.4 can be prepared by the following steps:

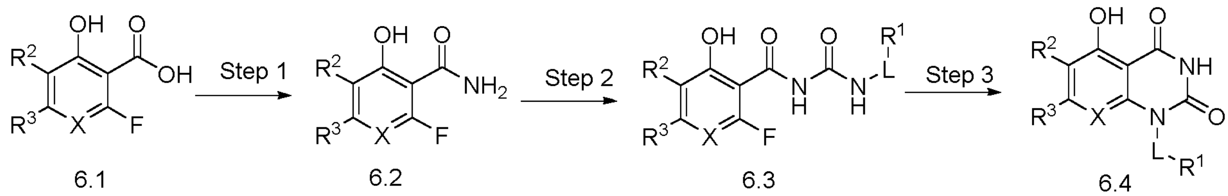


6.1 6.2 6.3 6.4

Compound 6.1 is used as a starting material, and the carboxyl group thereof is converted to amide to obtain intermediate 6.2, which then reacts with $R^1$-L-$NH_2$ under the action of oxalyl chloride to obtain intermediate 6.3, followed by a ring-closing reaction under a basic condition to obtain intermediate 6.4,
wherein $R^1$, $R^2$, $R^3$, $R^5$, $R^6$, L are as defined in the compound of formula (III-c).

Method 7:

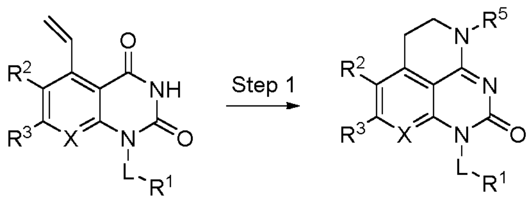


5.8 7.1

Compound 5.8 as a starting material reacts with $R^5NH_2$ under the action of PyBop and DBU to obtain the target compound 7.1,
wherein $R^1$, $R^2$, $R^3$, $R^5$, L and X are as defined in the compound of formula (III-e).

Note: Reaction steps for protection and deprotection involved in this step have been omitted. The substituents are as defined in the foregoing formulae.

The object of the present application also includes provision of the following intermediates for preparing the compounds represented by the general formulae:

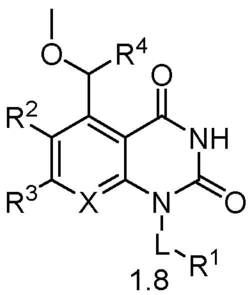
1.8 or

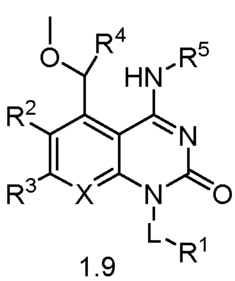
1.9 or

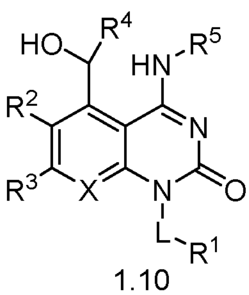
1.10

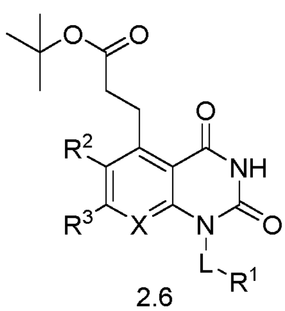
2.6

or

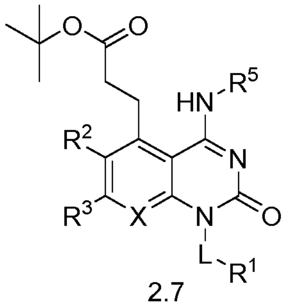
2.7 or

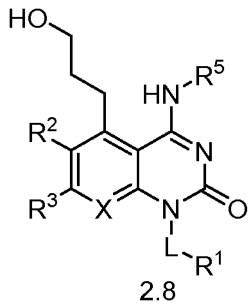
2.8

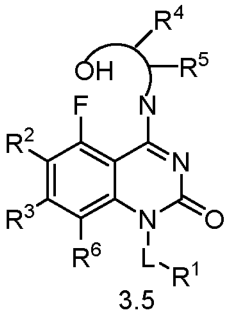
3.5 or

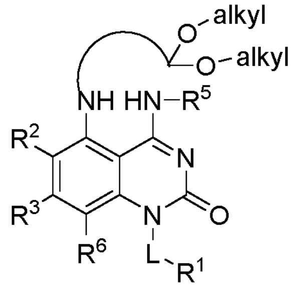
4.2

or

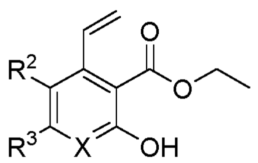
5.3 or

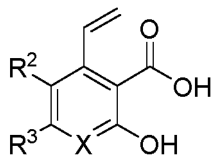
5.4 or

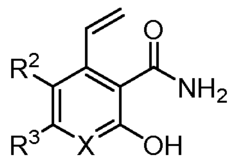
5.5 or

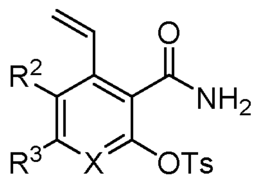
5.6

or

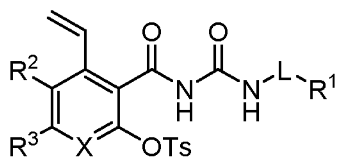
5.7 or

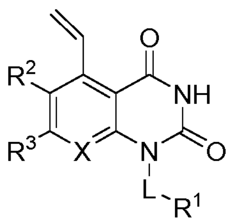
5.8 or

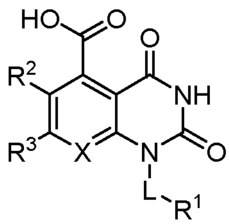
5.9 or

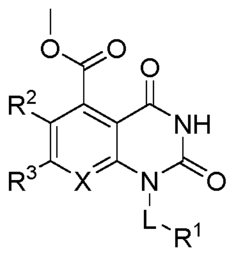
5.10

or

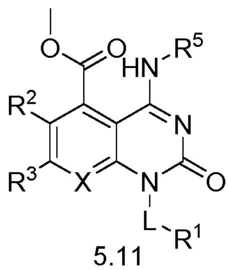
5.11 or

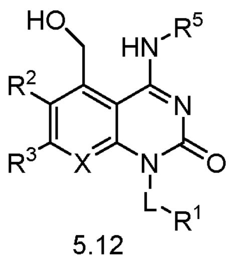
5.12 or

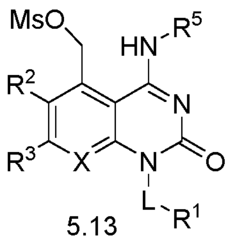
5.13 or

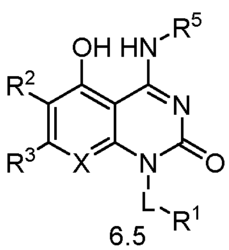
6.5 ;

wherein $R^1$, $R^2$, $R^3$, $R^4$, $R^5$, $R^6$, L and X are as defined for the compound of formula (I) according to the present application, wherein alkyl represents $C_{1-6}$ alkyl.

In another aspect, the present application further provides a pharmaceutical composition comprising the compound or the tautomer, the stereoisomer, or the pharmaceutically acceptable salt thereof of the present application.

Furthermore, the pharmaceutical composition of the present application comprises the compound or the tautomer, the stereoisomer, or the pharmaceutically acceptable salt thereof of the present application, and a pharmaceutically acceptable excipient.

The compound or the tautomer, the stereoisomer, or the pharmaceutically acceptable salt thereof of the present application may be administered in its pure form or in the form of a suitable pharmaceutical composition via any acceptable administration routes for a medicament providing a similar use. The pharmaceutical composition of the present application may be prepared by combining the compound of the present application with a suitable pharmaceutically acceptable

excipient.

In another aspect, the present application further provides a use of the compound, or the tautomer, the stereoisomer, or the pharmaceutically acceptable salt thereof of the present application and the pharmaceutical composition of the present application in the preparation of a medicament for preventing and/or treating a disease, disorder and condition mediated by MAT2A.

Furthermore, the present application further provides the above use, wherein the disease, disorder and condition is a tumor with MTAP deletion.

In yet another aspect, the present application further provides a use of the compound, or the tautomer, the stereoisomer, or the pharmaceutically acceptable salt thereof of the present application, or the pharmaceutical composition of the present application in the preparation of a medicament for treating and/or preventing a tumor.

Furthermore, the present application further provides the above use, wherein the tumor includes a solid tumor and a hematological malignancy; and preferably, the solid tumor includes a gastrointestinal cancer, further preferably a colorectal cancer. In certain contexts in this field, the cancer may also be referred to as a malignant tumor.

In yet another aspect, the present application provides a method for preventing and/or treating a disease, disorder and condition mediated by MAT2A, comprising administering to a subject in need thereof the compound, or the tautomer, the stereoisomer, or the pharmaceutically acceptable salt thereof of the present application or the pharmaceutical composition of the present application; preferably, the disease, disorder, and condition is a tumor; preferably, the disease, disorder and condition is a tumor with MTAP deletion; and more preferably, the tumor includes a solid tumor and a hematological malignancy.

In still another aspect, the present application provides the compound, or the tautomer, the stereoisomer, or the pharmaceutically acceptable salt thereof of the present application, or the pharmaceutical composition of the present application, for use in prevention and/or treatment of a disease, disorder, and condition mediated by MAT2A; preferably, the disease, disorder, and condition is a tumor with MTAP deletion; preferably, the disease, disorder, and condition is a tumor; and more preferably, the tumor includes a solid tumor and a hematological tumor. Furthermore, the present application provides the above use or method, wherein the compound, or the tautomer, the stereoisomer, or the pharmaceutically acceptable salt thereof of the present application, or the pharmaceutical composition of the present application is used in combination with one, two, or more drugs with tumor-inhibitory activity.

The present application also provides a pharmaceutical composition comprising the compound, or the tautomer, the stereoisomer, or the pharmaceutically acceptable salt thereof of the present application, and additional one, two or more drugs with tumor-inhibitory activity.

### Definitions

The term "optional" or "optionally" means that the subsequently described event or circumstance may but does not certainly occur, and that the description includes instances where said event or circumstance occurs and instances where said event or circumstance does not occur.

Unless otherwise specified, the term "optionally substituted" means that a substituent is independently selected from one or more of hydroxyl, halogen, hydroxyl, amino, nitro, sulfhydryl, cyano, azido, carboxyl, -C(O)$C_{1-6}$ alkyl, -C(O)O-$C_{1-6}$ alkyl, -OC(O)-$C_{1-6}$ alkyl, -NH($C_{1-6}$ alkyl), - N($C_{1-6}$ alkyl)($C_{1-6}$ alkyl), -C(O)NH-$C_{1-6}$ alkyl, -NHC(O)-$C_{1-6}$ alkyl, $C_{2-6}$ alkenyl, $C_{2-6}$ alkynyl, $C_{1-6}$ alkyl, $C_{1-6}$ alkoxy, $C_{3-10}$ cycloalkyl, $C_{3-10}$ cycloalkylsulfonyl, 3-10 membered heterocycloalkyl, $C_{6-14}$ aryl, and 5-12 membered heteroaryl; wherein the $C_{2-6}$ alkenyl, $C_{2-6}$ alkynyl, $C_{1-6}$ alkyl, $C_{1-6}$ alkoxy, $C_{3-10}$ cycloalkyl, $C_{3-10}$ cycloalkylsulfonyl, 3-10 membered heterocycloalkyl, $C_{6-14}$ aryl, or 5-12 membered heteroaryl may be optionally substituted with one or more groups selected from the group consisting of halogen, hydroxyl, amino, cyano, $C_{1-6}$ alkyl, and $C_{1-6}$ alkoxy.

The term "oxo" means that two hydrogen atoms at the same substitution site are replaced by the same oxygen atom to form a double bond.

Unless otherwise specified, the term "carbocyclyl" refers to a saturated or partially unsaturated cyclic carbon-containing group, such as a 4-6 membered (e.g., 5-6 membered) saturated carbocycle and a 5-6 membered partially unsaturated carbocycle. In one embodiment, carbocyclyl is 3- to 4-membered monocyclic, 3- to 5-membered monocyclic, 3- to 6-membered monocyclic, 3- to 8-membered monocyclic, 3- to 10-membered monocyclic, 5- to 8-membered monocyclic, 5- to 6-membered monocyclic, 4- to 12-membered bicyclic, or 10- to 15-membered tricyclic ring system. Carbocycle includes bridged ring or spiro ring. Non-limiting examples of carbocyclyl group include cyclopropyl, cyclobutyl, cyclopentyl, cyclohexyl, cyclohexenyl, cycloheptyl, cyclopentenyl, cyclohexadienyl, cycloheptatrienyl, benzocyclopentyl, bicyclo[3.2.1]octanyl, bicyclo[5.2.0]nonanyl , tricyclo[5.3.1.1]dodecyl, adamantyl or spiro[3.3]heptanyl, and the like. Carbocyclyl group can be optionally substituted. When carbocyclyl group is substituted, the number of the substituent is preferably 1 to 5, and the substituent is independently selected from the group consisting of F, Cl, Br, I, =O, alkyl, alkenyl, alkynyl, alkoxy, hydroxy, nitro, cyano and amino.

Unless otherwise specified, the term "alkyl" refers to a monovalent saturated aliphatic hydrocarbon group, i.e., a

linear or branched group comprising 1 to 20 carbon atoms, preferably 1 to 10 carbon atoms (i.e., $C_{1-10}$ alkyl), further preferably 1 to 8 carbon atoms ($C_{1-8}$ alkyl), more preferably 1 to 6 carbon atoms (i.e., $C_{1-6}$ alkyl). For example, "$C_{1-6}$ alkyl" refers to an alkyl group with the number of carbon atoms between 1 and 6 (specifically, 1, 2, 3, 4, 5, or 6) in the carbon chain. Examples include, but are not limited to, methyl, ethyl, n-propyl, isopropyl, n-butyl, isobutyl, *tert*-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, and the like.

Unless otherwise specified, the term "alkenyl" refers to a linear or branched unsaturated aliphatic hydrocarbon group consisting of carbon atoms and hydrogen atoms and having at least one double bond. Alkenyl can comprise 2 to 20 carbon atoms, preferably 2 to 10 carbon atoms (i.e., $C_{2-10}$ alkenyl), further preferably 2 to 8 carbon atoms ($C_{2-8}$ alkenyl), more preferably 2 to 6 carbon atoms (i.e., $C_{2-6}$ alkenyl), 2 to 5 carbon atoms (i.e., $C_{2-5}$ alkenyl), 2 to 4 carbon atoms (i.e., $C_{2-4}$ alkenyl), 2 to 3 carbon atoms (i.e., $C_{2-3}$ alkenyl), 2 carbon atoms (i.e., $C_2$ alkenyl). For example, "$C_{2-6}$ alkenyl" refers to an alkenyl group with the number of carbon atoms between 2 and 6 (specifically, 1, 2, 3, 4, 5, or 6) in the carbon chain. Non-limiting examples of alkenyl include, but are not limited to, ethenyl, 1-propenyl, 2-propenyl, 1-butenyl, isobutenyl, 1,3-butadienyl, and the like.

Unless otherwise specified, the term "alkynyl" refers to a linear or branched unsaturated aliphatic hydrocarbon group consisting of carbon atoms and hydrogen atoms and having at least one triple bond. Alkynyl can comprise 2-20 carbon atoms, preferably 2-10 carbon atoms (i.e., $C_{2-10}$ alkynyl), further preferably 2-8 carbon atoms ($C_{2-8}$ alkynyl), more preferably 2-6 carbon atoms (i.e., $C_{2-6}$ alkynyl), 2-5 carbon atoms (i.e., $C_{2-5}$ alkynyl), 2-4 carbon atoms (i.e., $C_{2-4}$ alkynyl), 2-3 carbon atoms (i.e., $C_{2-3}$ alkynyl), 2 carbon atoms (i.e., $C_2$ alkenyl). For example, "$C_{2-6}$ alkynyl" refers to an alkynyl group with the number of carbon atoms between 2 and 6 (specifically, 1, 2, 3, 4, 5, or 6) in the carbon chain. Non-limiting examples of alkynyl include, but are not limited to, ethynyl, 1-propynyl, 2-propynyl, 1-butynyl, and the like.

Unless otherwise specified, the term "cycloalkyl" refers to a monocyclic saturated aliphatic hydrocarbon group with a specific number of carbon atoms, preferably comprising 3-12 carbon atoms (i.e. $C_{3-12}$ cycloalkyl), more preferably 3-10 carbon atoms ($C_{3-10}$ cycloalkyl), further preferably 3-7 carbon atoms ($C_{3-7}$ cycloalkyl), 4-6 carbon atoms ($C_{4-6}$ cycloalkyl), and 5-6 carbon atoms ($C_{5-6}$ cycloalkyl). Examples include, but are not limited to, cyclopropyl, cyclobutyl, cyclopentyl, cyclohexyl, methylcyclopropyl, 2-ethyl-cyclopentyl, dimethylcyclobutyl, and the like.

Unless otherwise specified, the term "alkoxy" refers to -O-alkyl, wherein alkyl is as defined above, i.e., containing 1-20 carbon atoms, preferably 1-10 carbon atoms, further preferably 1-8 carbon atoms, and more preferably 1-6 carbon atoms (specifically 1, 2, 3, 4, 5, or 6). Representative examples include, but are not limited to, methoxy, ethoxy, propoxy, isopropoxy, butoxy, 1-methylpropoxy, 2-methylpropoxy, *tert*-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, and the like.

Unless otherwise specified, the term "alkylthio" refers to -S-alkyl, wherein alkyl is as defined above, i.e., containing 1-20 carbon atoms, preferably 1-10 carbon atoms, further preferably 1-8 carbon atoms, and more preferably 1-6 carbon atoms (specifically 1, 2, 3, 4, 5, or 6). Representative examples include, but are not limited to, methylthio, ethylthio, propylthio, isopropylthio, butylthio, 1-methylpropylthio, 2-methylpropylthio, tert-butylthio, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, and the like.

Unless otherwise specified, the term "halogen" or "halo" refers to F, Cl, Br, and I. The term "haloalkyl" refers to an alkyl group as defined above, wherein one, two or more hydrogen atoms or all hydrogen atoms are replaced by a halogen. Representative examples of haloalkyl include $CCl_3$, $CF_3$, $CHCl_2$, $CH_2Cl$, $CH_2Br$, $CH_2I$, $CH_2CF_3$, $CF_2CF_3$, and the like.

Unless otherwise specified, the term "heterocyclyl" or "heterocycle" refers to a saturated or partially unsaturated monocyclic or polycyclic non-aromatic substituent having ring carbon atom(s) and 1 to 4 ring heteroatoms, and containing 3-20 ring atoms, wherein 1, 2, 3 or more ring atoms are selected from the group consisting of N, O, and S, and the remaining ring atoms are C. Preferably, heterocyclyl contains 3-12 ring atoms (3-12 membered heterocyclyl), more preferably 3-10 ring atoms (3-10 membered heterocyclyl), or 3-8 ring atoms (3-8 membered heterocyclyl), or 3-6 ring atoms (3-6 membered heterocyclyl), or 4-6 ring atoms (4-6 membered heterocyclyl), or 5-6 ring atoms (5-6 membered heterocyclyl). The number of the heteroatoms is preferably 1-4, more preferably 1-3 (i.e.1, 2 or 3). Examples of monocyclic heterocyclyl include pyrrolidinyl, imidazolidinyl, tetrahydrofuranyl, dihydropyrrolyl, piperidinyl, piperazinyl, pyranyl, and the like. Polycyclic heterocyclyl include spiro, fused, and bridged heterocyclyl. "Heterocyclyl" can be a monocyclic ("monocyclic heterocyclyl") or a fused ("fused-heterocyclyl" or "hetero-fused cyclyl"), bridged ("hetero-bridged cyclyl" or "bridged-heterocyclyl") or spiro-fused ("hetero-spiro cyclyl" or "spiro-heterocyclyl") ring system, such as a bicyclic ring system ("bicyclic heterocyclyl"), and may be saturated or partially unsaturated. The bicyclic heterocyclyl ring system may include one or more heteroatoms in one or two rings. "Heterocyclyl" also includes such a ring system in which the ring of the heterocyclyl as defined above is fused with one or more carbocyclyl groups, wherein the attachment point is on either the ring of the carbocyclyl or the ring of the heterocyclyl, or "heterocyclyl" also includes such a ring system in which the ring of the heterocyclyl as defined above is fused with one or more aryl or heteroaryl groups, or such a ring system in which the ring of the cycloalkyl as defined above is fused with one or more heteroaryl groups, wherein the attachment point is on either the ring of the heterocyclyl or the ring of the cycloalkyl, and in such cases, the number of members of the heterocyclyl ring

system is the number of ring atoms of the resulting ring system after fusion. In certain examples, each example of heterocyclyl is independently optionally substituted, e.g., unsubstituted (an "unsubstituted heterocyclyl") or substituted with one or more substituents (a "substituted heterocyclyl"). Exemplary 3-membered heterocyclyl groups containing 1 heteroatom include, but are not limited to, aziridinyl, oxiranyl, and thiiranyl. Exemplary 4-membered heterocyclyl groups containing 1 heteroatom include, but are not limited to, azetidinyl, oxetanyl, and thietanyl. Exemplary 5-membered heterocyclyl groups containing 1 heteroatom include, but are not limited to, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing 2 heteroatoms include, but are not limited to, dioxolanyl, oxathiolanyl, dithiolanyl, and oxazolidin-2-one. Exemplary 5-membered heterocyclyl groups containing 3 heteroatoms include, but are not limited to, triazolinyl, oxadiazolinyl, and thiadiazolinyl. Exemplary 6-membered heterocyclyl groups containing 1 heteroatom include, but are not limited to, piperidinyl, tetrahydropyranyl, dihydropyridyl, and thianyl. Exemplary 6-membered heterocyclyl groups containing 2 heteroatoms include, but are not limited to, piperazinyl, morpholinyl, dithianyl, and dioxanyl. Exemplary 6-membered heterocyclyl groups containing 3 heteroatoms include, but are not limited to, triazinanyl, oxadiazinanyl, thiadiazinanyl, oxathiazinanyl, and dioxazinanyl. Exemplary 7-membered heterocyclyl groups containing 1 heteroatom include, but are not limited to, azepanyl, oxepanyl, and thiepanyl. Exemplary 8-membered heterocyclyl groups containing 1 heteroatom include, but are not limited to, azocanyl, oxocanyl, and thiocanyl. An exemplary 5-membered heterocyclyl group fused with one $C_6$ aryl ring (also referred to herein as a 5,6-bicyclic heterocycle) includes, but is not limited to, indolinyl, isoindolinyl, dihydrobenzofuranyl, dihydrobenzothiophenyl, benzoxazolinonyl, and the like. An exemplary 6-membered heterocyclyl group fused with one aryl ring (also referred to herein as a 6,6-bicyclic heterocycle) includes, but is not limited to, tetrahydroquinolinyl, tetrahydroisoquinolinyl, and the like.

Unless otherwise specified, "heterocycloalkyl" refers to a monocyclic, saturated "heterocyclyl" or "heterocycle" as defined above, wherein the ring atoms are defined as above, i.e., containing 3-20 ring atoms ("3 to 20 membered heterocycloalkyl") and having 1-4 (1, 2, 3 or 4), preferably 1-3 (1, 2 or 3) heteroatoms, wherein the heteroatom is each independently selected from the group consisting of N, O, and S. Preferably, heterocycloalkyl contains 3-12 ring atoms ("3-12 membered heterocycloalkyl"), further preferably 3-10 ring atoms ("3-10 membered heterocycloalkyl"), more further preferably 3-8 ring atoms ("3-8 membered heterocycloalkyl"), more further preferably 4-7 ring atoms ("4-7 membered heterocycloalkyl"), more further preferably 5-10 ring atoms ("5-10 membered heterocycloalkyl"), and more further preferably 5-6 ring atoms ("5-6 membered heterocycloalkyl"). In certain examples, each example of heterocycloalkyl is independently optionally substituted, e.g., unsubstituted (an "unsubstituted heterocycloalkyl") or substituted with one or more substituents (a "substituted heterocycloalkyl"). A part of exemplary "heterocycloalkyl" groups have been given in the above description for "heterocyclyl" or "heterocycle", and "heterocycloalkyl" group also includes, but is not limited to, aziridinyl, oxiranyl, thiiranyl, azetidinyl, oxetanyl, thietanyl, tetrahydrofuranyl, oxanyl, piperidinyl, piperazinyl, morpholinyl, thiomorpholinyl, oxathianyl, oxazolidinyl, dioxanyl, dithianyl, thiazolidinyl, pyrrolidinyl, pyrazolidinyl, imidazolidine, and the like.

Unless otherwise specified, the term "aryl" or "aromatic ring group" refers to monocyclic, bicyclic and tricyclic aromatic carbocyclic ring systems containing 6-16 carbon atoms, or 6-14 carbon atoms, or 6-12 carbon atoms, or 6-10 carbon atoms, preferably 6-10 carbon atoms. The term "aryl" can be used interchangeably with the term "aromatic ring group". Examples of aryl groups may include, but are not limited to, phenyl, naphthyl, anthracenyl, phenanthrenyl, pyrenyl, and the like.

Unless otherwise specified, the term "heteroaryl" or "heteroaromatic ring group" refers to an aromatic monocyclic or polycyclic ring system containing a 5-14 membered structure, or preferably a 5-10 membered structure, or preferably a 5-8 membered structure, more preferably a 5-6 membered structure, wherein 1, 2, 3 or more ring atoms are heteroatoms and the remaining ring atom(s) is/are carbon, the heteroatom(s) is/are independently selected from the group consisting of O, N, and S, and the number of heteroatom is preferably 1, 2 or 3. Examples of heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridyl, benzopyrimidinyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridyl, and the like.

Unless otherwise specified, the term "pharmaceutically acceptable salt" or "pharmaceutical salt" refers to those salts that are, within the scope of sound medical judgment, suitable for use in contact with mammalian tissues, particularly human tissues, without excessive toxicity, irritation, allergic responses, and the like, and are commensurate with a reasonable benefit/risk ratio. For example, medically acceptable salts of amines, carboxylic acids, and other types of compounds are well-known in the art. The salts can be prepared in situ during the final isolation and purification of the compounds according to the present application, or separately by reacting the free base or free acid with an appropriate reagent.

The compounds according to the present application also include an "isotopic derivative" thereof. Unless

otherwise specified, the term "isotopic derivative" means that the compounds according to the present application can exist in isotopically labelled or enriched form, and contain one or more atoms having an atomic mass or mass number different from the atomic mass or mass number of the atom most abundantly found in the nature. Isotopes may be radioactive or nonradioactive isotopes. Isotopes commonly used as isotopic labels are: hydrogen isotopes, $^{2}H$ and $^{3}H$; carbon isotopes: $^{13}C$ and $^{14}C$; chlorine isotopes: $^{35}Cl$ and $^{31}Cl$; fluorine isotope: $^{18}F$; iodine isotopes: $^{123}I$ and $^{125}I$; nitrogen isotopes: $^{13}N$ and $^{15}N$; oxygen isotopes: $^{15}O$, $^{17}O$ and $^{18}O$; and sulfur isotope: $^{35}S$. These isotopically labeled compounds can be used to study the distribution of pharmaceutical molecules in tissues. In particular, $^{3}H$ and $^{13}C$ are more widely used due to their ease of labeling and ease of detection. The substitution with certain heavy isotopes, such as heavy hydrogen ($^{2}H$), can enhance metabolic stability, and prolong half-life, thereby achieving the purpose of reducing dosage and providing therapeutic advantages. The isotopically labeled compounds are generally synthesized starting from labeled starting materials in the same way as non-isotopically labeled compounds using known synthetic techniques.

The compounds according to the present application also include a "solvate" thereof. Unless otherwise specified, the term "solvate" means a physical association of a compound of the present application with one or more solvent molecules, whether organic or inorganic. This physical association includes hydrogen bond. In certain instances, the solvate will be capable of isolation, for example, when one or more solvent molecules are incorporated into the crystal lattice of a crystalline solid. The solvent molecules in the solvate may be present in a regular arrangement and/or disordered arrangement. The solvate may comprise either a stoichiometric or non-stoichiometric amount of solvent molecule(s). "Solvate" encompasses both solution phase and isolable solvates. Exemplary solvates include, but are not limited to, hydrates, ethanolates, methanolates, and isopropanolates. Methods of solvation are well known in the art.

Unless otherwise specified, the term "stereoisomer" refers to a compound that has the identical chemical constitution, but differs in the arrangement of atom(s) or group(s) in space. Stereoisomers include enantiomers, diastereomers, conformers (rotamers), geometric isomers (cis/trans), atropisomers, and the like. A mixture of the resulting any stereoisomers can be separated into pure or substantially pure geometric isomers, enantiomers, and diastereomers, for example, by chromatography and/or fractional crystallization, on the basis of a difference in a physicochemical property of the components.

The term "atropisomer" refers to a conformational stereoisomer that occurs when rotation around a single bond in a molecule is prevented or greatly slowed down, due to steric interactions with other parts of the molecule. The compounds of the present application include all atropisomers as a single atropisomer, or as an unspecified mixture of each. If the rotational barrier around a single bond is high enough, and the interconversion between conformations is slow enough, then separation and isolation of isomeric species as distinct compounds may be allowed. For example, a group such as, but not limited to, the following R1 group

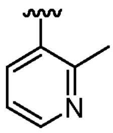
,
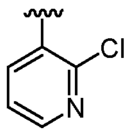

or

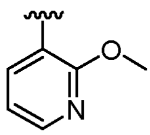
,

may exhibit restricted rotation.

Unless otherwise specified, the term "tautomer" refers to structural isomers with different energies which are interconvertible via a low energy barrier. If tautomerism is possible (such as, in solution), a chemical equilibrium of tautomers can be achieved. For example, proton tautomers (also known as prototropic tautomers) include interconversions via migration of a proton, such as keto-enol and imine-enamine isomerizations. Valence tautomers include interconversions by reorganization of some of the bonding electrons.

Unless otherwise specified, the structural formulae depicted herein include all isomeric (e.g., enantiomeric, diastereomeric, and geometric (or conformational)) forms: e.g., R and S configurations containing an asymmetric center, (Z) and (E) isomers of double bond, and (Z) and (E) conformational isomers. Therefore, individual stereochemical isomers of the compounds of the present application or mixtures of enantiomers, diastereomers, or geometrical isomers (or conformational isomers) thereof are within the scope of the present disclosure.

The compounds of the present application also include a "prodrug" thereof. Unless otherwise specified, the term "prodrug" refers to a drug that is converted to its parent drug in vivo. Prodrugs are usually useful, which may improve some

of the identified and undesirable physical or biological properties. Physical properties are generally related solubility (excessive or insufficient solubility in lipid or water) or stability, while problematic biological properties include too rapid metabolism or poor bioavailability, which may be related to physical and chemical properties. For example, a prodrug may be bioavailable by oral administration, while its parent drug is not. The solubility of a prodrug in a pharmaceutical composition is also improved compared to a parent drug thereof. One example of a prodrug may be, but is not limited to, any one of the compounds of the present application, which is administered as an ester ("prodrug") to facilitate delivery through cell membranes, where the water solubility thereof is detrimental to migration, but beneficial once the prodrug enters a cell, and the prodrug is subsequently metabolized and hydrolyzed to a carboxylic acid, i.e., the active entity. Another example of a prodrug may be a short peptide (polyamino acid) bound to an acid group, in which the peptide is metabolized to release an active moiety.

The abbreviations used in the Preparation Examples, Examples and elsewhere herein are:

| | |
|---|---|
| DMF | N,N-dimethylformamide |
| DBU | 1,8-diazabicyclo[5.4.0]undecyl-7-ene |
| DIEA | N, N-diisopropylethylamine |
| SPhos | 2-bicyclohexylphosphino-2',6'-dimethoxybiphenyl |
| PyBop | 1H-benzotriazol-1-yloxytripyrrolidinyl hexafluorophosphate |

**Beneficial effects of the present application**

The present application designs a class of structurally novel compounds that provide a new direction for the treatment of diseases, such as tumors. Enzymatic assays showed that the compounds of the present application have strong inhibitory effects on MAT2A. The compounds exhibit stronger cell proliferation activity in MTAP-knockout type HCT116 cells, while exhibiting weaker cell proliferation activity in MTAP wild-type HCT116 cells, thereby showing better selectivity. The compounds have good therapeutic effects in vivo. Moreover, the human UGT1A1 enzyme activity assay showed that the compounds of the present application had a low risk of inhibiting UGT1A1. Pharmacokinetic experiments showed that the compounds of the present application have high oral bioavailability and good dose-exposure correlation. The hERG experiment showed that the compounds of the present application had little cardiotoxicity. In addition, the present application has studied a specific synthesis method, which is simple in process, convenient in operation, and conducive to large-scale industrial production and application.

**EXAMPLE**

The present application will be further elaborated below in conjunction with specific examples. It should be understood that these examples are only used to illustrate the present application, and are not intended to limit the scope of the present application. For an experimental method without specifying specific conditions in the following examples, it is usually carried out following conventional conditions or conditions recommended by the manufacturer. Unless otherwise defined, all professional and scientific terms used herein generally have the same meaning as commonly understood by one of ordinary skill in the art. In addition, any methods and materials similar or equivalent to those described herein can be applied to the methods of the present application. The preferred implementation methods and materials shown herein are illustrative only.

The following Examples are Preparation Examples of the exemplary compounds of the present application.

**Example 1: 7-chloro-1-(2-methylpyridin-3-yl)-4,5-dihydropyrrolo[2,3,4-*de*]quinazolin-2(1*H*)-one (A1)**

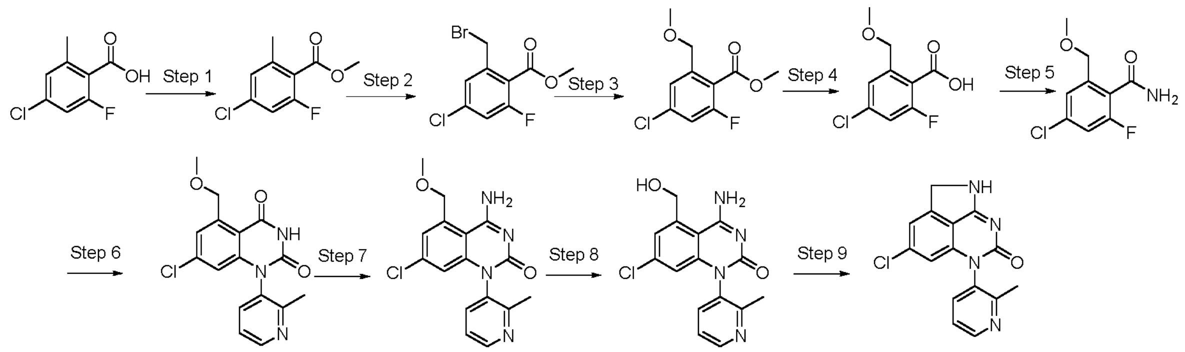


A1

**Step 1: synthesis of methyl 4-chloro-2-fluoro-6-methylbenzoate**

To a 250 mL round-bottomed flask were added 4-chloro-2-fluoro-6-methylbenzoic acid (8.44 g, 44.70 mmol) and dichloromethane (100 mL), followed by the addition of oxalyl chloride (8.51 g, 67.10 mmol) and DMF (0.2 mL). The reaction was stirred at room temperature. After completion of the reaction monitored by TLC, the solvent was removed under reduced pressure. Methanol (200 mL) and 5 M sodium methoxide (13.41 mL, 67.05 mmol) were added to the resulting residue, and reacted for 0.5 h. After completion of the reaction, the solvent was removed under reduced pressure, and 100 mL water was added, and then the resulting mixture was extracted with 80 mL ethyl acetate twice. The organic phases were combined. The combined organic phases were washed successively with water and saturated sodium chloride solution, and concentrated to afford the title product, methyl 4-chloro-2-fluoro-6-methylbenzoate (8.43 g, 93% yield).

**Step 2: synthesis of methyl 2-(bromomethyl)-4-chloro-6-fluorobenzoate**

To a 250 mL round-bottomed flask were added the product obtained in the above step, methyl 4-chloro-2-fluoro-6-methylbenzoate (8.43 g, 41.70 mmol), NBS (9.66 g, 54.27 mmol), azodiisobutyronitrile (2.05 g, 12.50 mmol) and carbon tetrachloride (160 mL). The resulting mixture was heated to 85°C and reacted for 8 h. After completion of the reaction, the reaction mixture was concentrated and used as is for the next step.

**Step 3: synthesis of methyl 4-chloro-2-fluoro-6-(methoxymethyl)benzoate**

To a 250 mL round-bottomed flask were added the concentrated product obtained in the above step, methyl 2-(bromomethyl)-4-chloro-6-fluorobenzoate, 5M sodium methoxide (12.51 mL, 62.55 mmol) and methanol (50 mL). The resulting mixture was stirred at room temperature and reacted for 1 h. After completion of the reaction, the solvent was removed under reduced pressure, and 100 mL water was added, and then the resulting mixture was extracted with 80 mL ethyl acetate twice. The organic phases were combined. The combined organic phases were washed successively with water and saturated sodium chloride solution, concentrated, and purified by column chromatography to afford methyl 4-chloro-2-fluoro-6-(methoxymethyl)benzoate (5.1 g, 53% yield over two steps).

**Step 4: synthesis of 4-chloro-2-fluoro-6-(methoxymethyl)benzoic acid**

To a 100 mL round-bottomed flask were added the product obtained in the above step, methyl 4-chloro-2-fluoro-6-(methoxymethyl)benzoate (4.60 g, 19.70 mmol), sodium hydroxide (3.10 g, 79.09 mmol), methanol (40 mL) and water (10 mL). The resulting mixture was reacted with stirring at room temperature for 2 h. After completion of the reaction, 100 mL water was added, and the resulting mixture was adjusted to pH 4~5 with the concentrated hydrochloric acid, and then extracted with 80 mL ethyl acetate twice. The organic phases were combined. The combined organic phases were washed successively with water and saturated sodium chloride solution, and concentrated to afford the title product, 4-chloro-2-fluoro-6-(methoxymethyl)benzoic acid (4.24 g, 98.1% yield). ESI-MS (m/z): 217.0$[M-H]^-$.

**Step 5: synthesis of 4-chloro-2-fluoro-6-(methoxymethyl)benzamide**

To a 100 mL round-bottomed flask were added the product obtained in the above step, 4-chloro-2-fluoro-6-(methoxymethyl)benzoic acid (4.24 g, 19.39 mmol), ammonium chloride (1.35 g, 25.21 mmol), 2-(7-azabenzotriazolyl)-N,N,N',N'-tetramethyluronium hexafluorophosphate (11.06 g, 29.09 mmol) and DMF (35 mL), followed by the addition of N,N-diisopropylethylamine (5.0 g, 38.78 mmol). The reaction mixture was reacted at room temperature overnight. After completion of the reaction, 200 mL water was added, and the resulting mixture was extracted with 100 mL

ethyl acetate twice. The organic phases were combined. The combined organic phases were washed successively with water and saturated sodium chloride solution, concentrated under reduced pressure, and purified by column chromatography to afford the title compound, 4-chloro-2-fluoro-6-(methoxymethyl)benzamide (2.50 g, 59% yield). ESI-MS (m/z): 218.0[M+H]$^+$.

**Step 6: synthesis of 7-chloro-5-(methoxymethyl)-1-(2-methylpyridin-3-yl)quinazoline-2,4(1*H*,3*H*)-dione**

To a 50 mL round-bottomed flask were added the product obtained in the above step, 4-chloro-2-fluoro-6-(methoxymethyl)benzamide (300 mg, 1.37 mmol), oxalyl chloride (174 μL, 2.06 mmol) and anhydrous tetrahydrofuran (10 mL). The resulting mixture was heated to 80°C and reacted. After completion of the reaction, the reaction solution was cooled to room temperature, and then 2-methylpyridin-3-amine (149 mg, 1.37 mmol) was added to the reaction solution, and reacted at room temperature for 3 h. After completion of the reaction, 1 M potassium bis(trimethylsilyl)amide (5.48 mL, 5.48 mmol) was added to the reaction solution, and then the reaction was stirred at room temperature for 4 h. After completion of the reaction, 100 mL water was added, and the resulting mixture was adjusted to pH 6~7 with acetic acid, and extracted with 100 mL ethyl acetate twice. The organic phases were combined. The combined organic phases were washed successively with water and saturated sodium chloride solution, concentrated, and purified by column chromatography, to afford the title product, 7-chloro-5-(methoxymethyl)-1-(2-methylpyridin-3-yl)quinazoline-2,4(1*H*,3*H*)-dione (310 mg, 68% yield), ESI-MS (m/z): 332.1[M+H]$^+$.

**Step 7: synthesis of 4-amino-7-chloro-5-(methoxymethyl)-1-(2-methylpyridin-3-yl)quinazolin-2(1*H*)-one**

To a 50 mL round-bottomed flask were added the product obtained in the above step, 7-chloro-5-(methoxymethyl)-1-(2-methylpyridin-3-yl)quinazoline-2,4(1*H*,3*H*)-dione (286 mg, 0.86 mmol), phosphorus oxychloride (262 mg, 2.58 mmol), N,N-diisopropylethylamine (749 μL, 4.31 mmol) and anhydrous dioxane (10 mL). The resulting mixture was heated to 100°C and reacted with stirring. After completion of the reaction, the reaction solution was cooled to room temperature. Ammonia water (5 mL) was added to the reaction solution, and reacted at room temperature for 0.5 h. After completion of the reaction, 100 mL water was added to the reaction solution. The resulting mixture was extracted with 100 mL ethyl acetate twice. The organic phases were combined. The combined organic phases were washed successively with water and saturated sodium chloride solution, concentrated, and purified by column chromatography, to afford the title product, 4-amino-7-chloro-5-(methoxymethyl)-1-(2-methylpyridin-3-yl)quinazolin-2(1*H*)-one (170 mg, 60% yield), ESI-MS (m/z): 331.1[M+H]$^+$.

**Step 8: synthesis of 4-amino-7-chloro-5-(hydroxymethyl)-1-(2-methylpyridin-3-yl)quinazolin-2(1*H*)-one**

To a 25 mL round-bottomed flask were added the product obtained in the above step, 4-amino-7-chloro-5-(methoxymethyl)-1-(2-methylpyridin-3-yl)quinazolin-2(1*H*)-one (150 mg, 0.45 mmol), boron tribromide (750 μL) and dichloromethane (7.5 mL). The resulting mixture was reacted at room temperature overnight. After completion of the reaction, 100 mL water was added to the reaction solution. The resulting mixture was adjusted to pH of about 7 with sodium hydroxide, and then extracted with 100 mL dichloromethane three times. The organic phases were combined. The combined organic phases were washed successively with water and saturated sodium chloride solution, concentrated, and purified directly by column chromatography, to afford the title product, 4-amino-7-chloro-5-(hydroxymethyl)-1-(2-methylpyridin-3-yl)quinazolin-2(1*H*)-one (100 mg, 70% yield), ESI-MS (m/z): 317.1[M+H]$^+$.

**Step 9: synthesis of 7-chloro-1-(2-methylpyridin-3-yl)-4,5-dihydropyrrolo[2,3,4-*de*]quinazolin-2(1*H*)-one**

To a 25 mL round-bottomed flask were added the product obtained in the above step, 4-amino-7-chloro-5-(hydroxymethyl)-1-(2-methylpyridin-3-yl)quinazolin-2(1*H*)-one (100 mg, 0.32 mmol), N-bromosuccinimide (64 mg, 0.38 mmol), triphenylphosphine (124 mg, 0.47 mmol), triethylamine (96 mg, 0.95 mmol) and dichloromethane (5 mL). Methanesulfonyl chloride (43 mg, 0.38 mmol) was added, and the resulting mixture was reacted at room temperature overnight. After completion of the reaction, 100 mL water was added to the reaction solution, and then the resulting mixture was extracted with 100 mL ethyl acetate twice. The organic phases were combined. The combined organic phases were washed successively with water and saturated sodium chloride solution, concentrated, and purified directly by column chromatography, to afford the title product, 7-chloro-1-(2-methylpyridin-3-yl)-4,5-dihydropyrrolo[2,3,4-*de*]quinazolin-2(1*H*)-one (40 mg, 42% yield), ESI-MS (m/z): 299.1[M+H]$^+$; $^1$H NMR (600 MHz, DMSO) δ 9.39 (s, 1H), 8.57 (d, *J* = 3.9 Hz, 1H), 7.73 (d, *J* = 7.6 Hz, 1H), 7.47 - 7.40 (m, 1H), 7.30 (s, 1H), 6.17 (s, 1H), 4.85 (s, 2H), 2.21 (s, 3H).

Two separate atropisomers A1-P1 and A1-P2 were obtained following the resolution method of A16. A1-P1, ESI-MS (m/z): 299.1[M+H]$^+$; $^1$H NMR (600 MHz, DMSO) δ 9.39 (s, 1H), 8.57 (d, *J* = 3.9 Hz, 1H), 7.73 (d, *J* = 7.6 Hz, 1H), 7.47 - 7.40 (m, 1H), 7.30 (s, 1H), 6.17 (s, 1H), 4.85 (s, 2H), 2.21 (s, 3H). A1-P2, ESI-MS (m/z): 299.1[M+H]$^+$; $^1$H NMR (600 MHz,

DMSO) δ 9.39 (s, 1H), 8.57 (d, *J* = 3.9 Hz, 1H), 7.73 (d, *J* = 7.6 Hz, 1H), 7.47 - 7.40 (m, 1H), 7.30 (s, 1H), 6.17 (s, 1H), 4.85 (s, 2H), 2.21 (s, 3H).

**Example 2: 7-chloro-4-methyl-1-(2-methylpyridin-3-yl)-4,5-dihydropyrrolo[2,3,4-*de*]quinazolin-2(1*H*)-one** (A2)

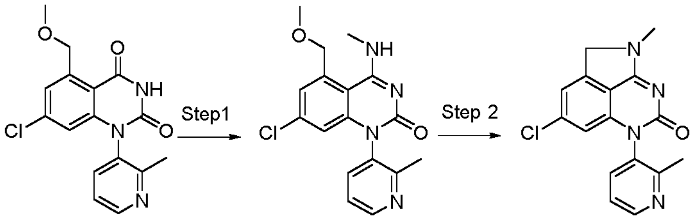


A2

**Step 1: synthesis of 7-chloro-5-(methoxymethyl)-4-(methylamino)-1-(2-methylpyridin-3-yl)quinazolin-2(1*H*)-one**

To a 50 mL round-bottomed flask were added 7-chloro-5-(methoxymethyl)-1-(2-methylpyridin-3-yl)quinazoline-2,4(1*H*,3*H*)-dione (100 mg, 0.30 mmol), phosphorus oxychloride (137 mg, 0.90 mmol), N,N-diisopropylethylamine (194 mg, 1.51 mmol) and anhydrous dioxane (5 mL). The resulting mixture was heated to 100°C and reacted with stirring. After completion of the reaction, the reaction solution was cooled to room temperature, and then an aqueous solution of methylamine (5 mL) was added to the reaction solution, and then reacted at room temperature for 0.5 h. After completion of the reaction, 100 mL water was added to the reaction solution, and then the mixture was extracted with 100 mL ethyl acetate twice. The organic phases were combined. The combined organic phases were washed successively with water and saturated sodium chloride solution, and concentrated, to afford the title product, 7-chloro-5-(methoxymethyl)-4-(methylamino)-1-(2-methylpyridin-3-yl)quinazolin-2(1*H*)-one (104 mg, 100% yield), ESI-MS (m/z): 345.1 $[M+H]^+$.

**Step 2: synthesis of 7-chloro-4-methyl-1-(2-methylpyridin-3-yl)-4,5-dihydropyrrolo[2,3,4-*de*]quinazolin-2(1*H*)-one**

To a 25 mL round-bottomed flask were added the product obtained in the above step, 7-chloro-5-(methoxymethyl)-4-(methylamino)-1-(2-methylpyridin-3-yl)quinazolin-2(1*H*)-one (104 mg, 0.30 mmol), boron tribromide (500 μL) and dichloromethane (5 mL). The resulting mixture was reacted at room temperature overnight. After completion of the reaction, 100 mL water was added to the reaction solution. The resulting solution was adjusted to the pH of about 7 with sodium hydroxide, and then extracted with 100 mL ethyl acetate twice. The organic phases were combined. The combined organic phases were washed successively with water and saturated sodium chloride solution, concentrated and purified by column chromatography, to afford the title product, 7-chloro-4-methyl-1-(2-methylpyridin-3-yl)-4,5-dihydropyrrolo [2,3,4-de]quinazolin-2(1H)-one (35 mg, 37% yield), ESI-MS (m/z): 313.1 $[M+H]^+$. $^1$H NMR (600 MHz, DMSO) δ 8.61 - 8.54 (m, 1H), 7.73 - 7.70 (m, 1H), 7.43 (dd, *J* = 7.8, 4.8 Hz, 1H), 7.31 (s, 1H), 6.18 (s, 1*H*), 4.92(s, 2H), 3.23 (s, 3H), 2.21 (s, 3H).

Two separate atropisomers A2-P1 and A2-P2 were obtained following the resolution method of A16. A2-P1, ESI-MS (m/z): 313.1 $[M+H]^+$. $^1$H NMR (600 MHz, DMSO) δ 8.61 - 8.54 (m, 1H), 7.73 - 7.70 (m, 1H), 7.43 (dd, *J*= 7.8, 4.8 Hz, 1H), 7.31 (s, 1H), 6.18 (s, 1H), 4.92(s, 2H), 3.23 (s, 3H), 2.21 (s, 3H). A2-P1, ESI-MS (m/z): 313.1 $[M+H]^+$. $^1$H NMR (600 MHz, DMSO) δ 8.61 - 8.54 (m, 1H), 7.73 - 7.70 (m, 1H), 7.43 (dd, *J* = 7.8, 4.8 Hz, 1H), 7.31 (s, 1H), 6.18 (s, 1H), 4.92(s, 2H), 3.23 (s, 3H), 2.21 (s, 3H).

**Example 3: 7-chloro-4-phenyl-1-(2-methylpyridin-3-yl)-4,5-dihydropyrrolo[2,3,4-*de*]quinazolin-2(1*H*)-one (A3)**

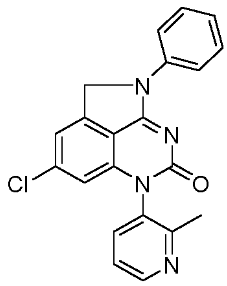

According to the synthetic method of Compound **A2** with aniline as the starting material, 7-chloro-4-phenyl-1-(2-methylpyridin-3-yl)-4,5-dihydropyrrolo[2,3,4-de]quinazolin-2(1H)-one was obtained (15 mg, 56% yield), ESI-MS (m/z): 375.1[M+H]$^+$. $^1$H NMR (600 MHz, DMSO) δ 8.61 (dd, *J* = 4.8, 1.3 Hz, 1H), 8.08 (d, *J* = 8.0 Hz, 2H), 7.80 (dd, *J* = 7.8, 1.2 Hz, 1H), 7.51 (t, *J* = 8.0 Hz, 2H), 7.47 (dd, *J* = 7.8, 4.9 Hz, 1H), 7.41 (s, 1H), 7.25 (t, *J* = 7.4 Hz, 1H), 6.29 (s, 1H), 5.51 (s, 2H), 2.25 (s, 3H).

Two separate atropisomers A3-P1 and A3-P2 were obtained following the resolution method of A16. A3-P1, ESI-MS (m/z): 375.1[M+H]$^+$. $^1$H NMR (600 MHz, DMSO) δ 8.61 (dd, *J* = 4.8, 1.3 Hz, 1H), 8.08 (d, *J* = 8.0 Hz, 2H), 7.80 (dd, *J* = 7.8, 1.2 Hz, 1H), 7.51 (t, *J* = 8.0 Hz, 2H), 7.47 (dd, *J* = 7.8, 4.9 Hz, 1H), 7.41 (s, 1H), 7.25 (t, *J* = 7.4 Hz, 1H), 6.29 (s, 1H), 5.51 (s, 2H), 2.25 (s, 3H). A3-P2, ESI-MS (m/z): 375.1[M+H]$^+$。 $^1$H NMR (600 MHz, DMSO) δ 8.61 (dd, *J* = 4.8, 1.3 Hz, 1H), 8.08 (d, *J* = 8.0 Hz, 2H), 7.80 (dd, *J* = 7.8, 1.2 Hz, 1H), 7.51 (t, *J* = 8.0 Hz, 2H), 7.47 (dd, *J* = 7.8, 4.9 Hz, 1H), 7.41 (s, 1H), 7.25 (t, *J* = 7.4 Hz, 1H), 6.29 (s, 1H), 5.51 (s, 2H), 2.25 (s, 3H).

**Example 4: 7-chloro-4-benzoyl-1-(2-methylpyridin-3-yl)-4,5-dihydropyrrolo[2,3,4-*de*]quinazolin-2(1*H*)-one (A4)**

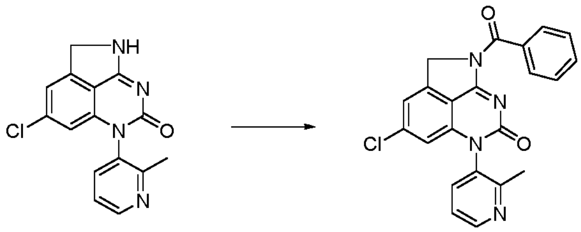

To a 25 mL round-bottomed flask were added Compound A1 (27 mg, 0.09 mmol) and dichloromethane (5 mL), followed by the addition of triethylamine (27 mg, 0.27 mmol) and benzoyl chloride (19 mg, 0.14 mmol). The resulting mixture was reacted at room temperature for 2 h. After completion of the reaction, the reaction mixture was purified directly by column chromatography, to afford the title product, 7-chloro-4-benzoyl-1-(2-methylpyridin-3-yl)-4,5-dihydropyrrolo [2,3,4-*de*]quinazolin-2(1*H*)-one (25 mg, 64% yield), ESI-MS (m/z): 403.1[M+H]+. $^1$H NMR (600 MHz, DMSO) δ 8.60 (d, *J* = 3.7 Hz, 1H), 7.79 (d, *J* = 7.3 Hz, 2H), 7.75 (d, *J* = 7.3 Hz, 1H), 7.62(t, *J* = 7.4 Hz, 1H), 7.51 (t, *J* = 7.7 Hz, 2H), 7.46-7.45 (m, *J* = 7.9, 5.0 Hz, 2H), 6.36 (s, 1H), 5.43 (d, *J* = 5.0 Hz, 2H), 2.19 (s, 3H).

**Example 5: 7-chloro-4-methyl-1-(2-chloropyridin-3-yl)-4,5-dihydropyrrolo[2,3,4-*de*]quinazolin-2(1*H*)-one (A16)**

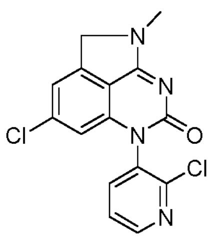

According to the synthetic method of Compound A2 with methylamine as the starting material, 7-chloro-4-methyl-1-(2-chloropyridin-3-yl)-4,5-dihydropyrrolo[2,3,4-de]quinazolin-2(1H)-one was obtained (205 mg, 63% yield), ESI-MS (m/z): 333.0[M+H]$^+$ ; $^1$H NMR (600 MHz, DMSO) δ 8.56 (dd, *J* = 4.8 Hz, 1.7 Hz, 1H), 8.04 (dd, *J* = 7.7 Hz,

1.7 Hz, 1H), 7.65 (dd, *J* = 7.7 Hz, 4.8 Hz, 1H), 7.34 (s, 1H), 6.38 (s, 1H), 4.93 (s, 2H), 3.23 (s, 3H).

Compound A16 is an axially chiral compound containing two atropisomers, as shown in the following structure formulae:

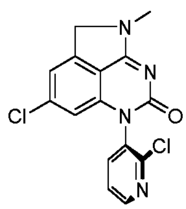
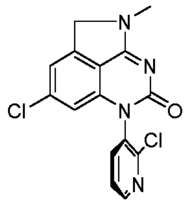

Compound A16 was separated by supercritical fluid chromatography (SFC) (instrument: SFC 150, column: Daicel CHIRALCEL AD, 250 mm*30 mm, 10 μM, mobile phase: $CO_2$ : EtOH=70:30, flow rate: 80g/min, wavelength: 214nm, temperature: 35°C) to afford compound A16-P1 (the first eluting isomer) and A16-P2 (the second eluting isomer), successively. Then, the two compounds were analyzed by high performance liquid chromatography (instrument: Waters e2695-2998, column: IG-3 (150 mm*4.6 mm, 3 μm), mobile phase: 0.1% diethylamine in n-hexane: Ethanol = 30:70, flow rate: 1.0 mL/min, wavelength: 239nm, column temperature: 30°C), with the retention time of compound A16-P1 of 4.89 min and the retention time of compound A16-P2 of 7.73min.

Compound A16-P1, ESI-MS (m/z): 333.0[M+H]$^+$ ; $^1$H NMR (600 MHz, DMSO-$d_6$) δ 8.56 (dd, *J* = 4.8, 1.8 Hz, 1H), 8.05 (dd, *J* = 7.8, 1.8 Hz, 1H), 7.65 (dd, *J* = 7.7, 4.8 Hz, 1H), 7.34 (d, *J* = 1.3 Hz, 1H), 6.38 (d, *J* = 1.2 Hz, 1H), 4.93 (s, 2H), 3.23 (s, 3H).

Compound A16-P2, ESI-MS (m/z): 333.0[M+H]$^+$ ; $^1$H NMR (600 MHz, DMSO-$d_6$) δ 8.56 (dd, *J* = 4.8, 1.8 Hz, 1H), 8.05 (dd, *J* = 7.7, 1.8 Hz, 1H), 7.65 (dd, *J* = 7.7, 4.8 Hz, 1H), 7.34 (d, *J* = 1.2 Hz, 1H), 6.38 (d, *J* = 1.2 Hz, 1H), 4.93 (s, 2H), 3.23 (s, 3H).

**Example 6: 9-chloro-1-(2-methylpyridin-3-yl)-4,5,6,7-tetrahydroazepino[2,3,4-de]quinazolin-2(1H)-one (B1)**

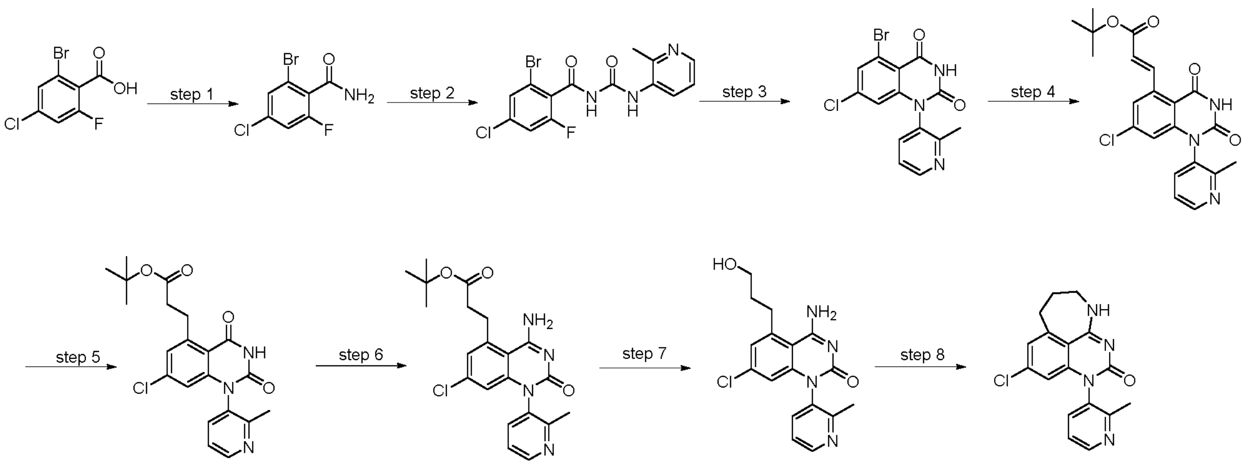


**Step 1: 2-bromo-4-chloro-6-fluorobenzamide**

To dichloromethane (100 mL) was added 2-bromo-4-chloro-6-fluorobenzoic acid (2.5 g, 10 mmol), followed by the addition of oxalyl chloride (3.2 g, 25 mmol) and two drops of DMF in an ice bath. The reaction was stirred at room temperature for 1 hour. After completion of the reaction, the reaction system was concentrated. Dioxane (100 mL) was added, followed by the addition of ammonia water (2.8 g, 50 mmol) dropwise in an ice bath, and then the reaction mixture was warmed to room temperature and reacted with stirring. After completion of the reaction, the reaction solution was poured into water (300 mL), and extracted with ethyl acetate three times. The organic phases were combined. The combined organic phases were washed with saturated sodium chloride solution, dried over anhydrous sodium sulfate, filtered and concentrated, to afford 2-bromo-4-chloro-6-fluorobenzamide (2 g, 80% yield). ESI-MS (m/z): 251.9[M+H]$^+$.

**Step 2: 2-bromo-4-chloro-6-fluoro-N-((2-methylpyridin-3-yl)carbamoyl)benzamide**

To tetrahydrofuran (10 mL) was added 2-bromo-4-chloro-6-fluorobenzamide (990 mg, 3.9 mmol), followed by the addition of oxalyl chloride (541 mg, 4.3 mmol). The resulting mixture was heated to 70°C and stirred for 1 hour. After cooling, the mixture was added to a solution of 2-methylpyridin-3-amine (426 mg, 3.9 mmol) in tetrahydrofuran (10 mL) dropwise, and then the reaction was stirred at room temperature. After completion of the reaction, the reaction solution was poured into water (300 mL), and extracted with dichloromethane five times. The organic phases were combined. The combined organic phases were washed with saturated sodium chloride solution, dried over anhydrous sodium sulfate, filtered and concentrated, to afford 2-bromo-4-chloro-6-fluoro-N-((2-methylpyridin-3-yl)carbamoyl)benzamide (1.4 g, 93% yield). ESI-MS (m/z): 386.0[M+H]$^+$.

**Step 3: 5-bromo-7-chloro-1-(2-methylpyridin-3-yl)quinazoline-2,4(1H,3H)-dione**

To tetrahydrofuran (30 mL) was added 2-bromo-4-chloro-6-fluoro-N-((2-methylpyridin-3-yl)carbamoyl)benzamide (560 mg, 1.45 mmol), followed by the addition of 1 M potassium bis(trimethylsilyl)amide (2.9 mL, 2.9 mmol) dropwise in an ice bath, and then the reaction was stirred in an ice bath. After completion of the reaction, to the reaction solution was added water (200 mL), and the pH of the reaction solution was adjusted to about 5 with acetic acid. After stirring for several minutes, the reaction solution was extracted with ethyl acetate three times. The organic phases were combined. The combined organic phases were washed with saturated sodium chloride solution, dried over anhydrous sodium sulfate, filtered and concentrated, to afford the crude product, which was purified with ethyl acetate, and dried to afford 5-bromo-7-chloro-1-(2-methylpyridin-3-yl)quinazoline-2,4(1H,3H)-dione (400 mg, 75% yield). ESI-MS (m/z): 366.0 [M+H]$^+$; $^1$H NMR (600 MHz, DMSO-$d_6$) $\delta$ 11.93 (s, 1H), 8.65 (dd, *J* = 4.8 Hz, *J* = 1.8 Hz, 1H), 7.85 (dd, *J* = 8.4 Hz, *J* = 1.8 Hz, 1H), 7.68 (d, *J* = 1.8 Hz, 1H), 7.49 (dd, *J* = 7.8 Hz, *J* = 4.8 Hz, 1H), 6.30 (d, *J* = 1.8 Hz, 1H), 2.28 (s, 3H).

**Step 4: *tert*-butyl 3-(7-chloro-1-(2-methylpyridin-3-yl)-2,4-dicarbonyl-1,2,3,4-tetrahydroquinazolin-5-yl)acrylate**

To DMF (5 mL) was added 5-bromo-7-chloro-1-(2-methylpyridin-3-yl)quinazoline-2,4(1H,3H)-dione (150 mg, 0.41 mmol), followed by the addition of tert-butyl acrylate (263 mg, 2.05 mmol), palladium acetate (18 mg, 0.082 mmol), tri(*o*-tolyl)phosphine (50 mg, 0.164 mmol) and potassium carbonate (113 mg, 0.8 mmol). The reaction system was stirred and reacted at 140°C under argon atmosphere. After completion of the reaction, the reaction solution was cooled, and poured into water (100 mL), and extracted with ethyl acetate three times. The organic phases were combined. The combined organic phases were washed with saturated sodium chloride solution, dried over anhydrous sodium sulfate, filtered and concentrated, to afford the crude product, which was separated and purified by thin layer chromatography (developing solvent: dichloromethane : methanol = 20:1) to afford *tert*-butyl 3-(7-chloro-1-(2-methylpyridin-3-yl)-2,4-dicarbonyl-1,2,3,4-tetrahydroquinazolin-5-yl)acrylate (120 mg, 71% yield). ESI-MS (m/z): 414.1[M+H]$^+$.

**Step 5: *tert-butyl* 3-(7-chloro-1-(2-methylpyridin-3-yl)-2,4-dicarbonyl-1,2,3,4-tetrahydroquinazolin-5-yl)propionate**

To tetrahydrofuran (5 mL) was added *tert*-butyl 3-(7-chloro-1-(2-methylpyridin-3-yl)-2,4-dicarbonyl-1,2,3,4-tetrahydroquinazolin-5-yl)acrylate (120 mg, 0.29 mmol), followed by the addition of methanol (1 mL). Sodium borohydride (55 mg, 1.45 mmol) was added in an ice bath, and the reaction was stirred at room temperature. After completion of the reaction, water (50 mL) was added to the reaction solution, and the resulting mixture was extracted with ethyl acetate (3x50 mL). The organic phases were combined. The combined organic phases were washed with saturated sodium chloride solution, dried over anhydrous sodium sulfate, filtered and concentrated, to afford the crude product, which was purified by thin layer chromatography (developing solvent: dichloromethane : methanol = 20:1) to afford *tert*-butyl 3-(7-chloro-1-(2-methylpyridin-3-yl)-2,4-dicarbonyl-1,2,3,4-tetrahydroquinazolin-5-yl)propionate (100 mg, 83% yield). ESI-MS (m/z): 416.1[M+H]$^+$.

**Step 6: *tert-butyl* 3-(4-amino-7-chloro-1-(2-methylpyridin-3-yl)-2-carbonyl-1,2-dihydroquinazolin-5-yl)propionate**

To dioxane (10 mL) was added *tert*-butyl 3-(7-chloro-1-(2-methylpyridin-3-yl)-2,4-dicarbonyl-1,2,3,4-tetrahydroquinazolin-5-yl)propionate (100 mg, 0.24 mmol), followed by the addition of phosphorus oxychloride (110 mg, 0.72 mmol) and DIEA (155 mg, 1.2 mmol). The resulting mixture was heated to 100°C and stirred for 1 hour. After cooling, ammonia water (0.2 mL) was added dropwise, and the reaction was stirred at room temperature. After completion of the reaction, water (100 mL) was added to the reaction solution, and then the solution was extracted with ethyl acetate (3x50 mL). The organic phases were combined. The combined organic phases were washed with saturated sodium chloride

solution, dried over anhydrous sodium sulfate, filtered and concentrated. The crude product was separated and purified by thin layer chromatography (developing solvent: dichloromethane: methanol=15: 1) to afford *tert*-butyl 3-(4-amino-7-chloro-1-(2-methylpyridin-3-yl)-2-carbonyl-1,2-dihydroquinazolin-5-yl)propionate (50 mg, 50% yield). ESI-MS (m/z): 415.1[M+H]$^+$ .

**Step 7: 4-amino-7-chloro-5-(3-hydroxypropyl)-1-(2-methylpyridin-3-yl)quinazolin-2(1H)-one**

To tetrahydrofuran (5 mL) was added *tert*-butyl 3-(4-amino-7-chloro-1-(2-methylpyridin-3-yl)-2-carbonyl-1,2-dihydroquinazolin-5-yl)propionate (50 mg, 0.12 mmol), followed by the addition of lithium aluminum hydride (13 mg, 0.36 mmol) in an ice bath, and the reaction was stirred in an ice bath. After completion of the reaction, 1 mL methanol was added to the reaction solution. The resulting mixture was concentrated, and then separated and purified by thin layer chromatography (developing solvent: dichloromethane: methanol=10: 1) to afford 4-amino-7-chloro-5-(3-hydroxypropyl)-1-(2-methylpyridin-3-yl)quinazolin-2(1H)-one (30 mg, 73% yield). ESI-MS (m/z): 345.1[M+H]$^+$.

**Step 8: 9-chloro-1-(2-methylpyridin-3-yl)-4,5,6,7-tetrahydroazepino[2,3,4-de]quinazolin-2(1H)-one**

To dichloromethane (10 mL) was added 4-amino-7-chloro-5-(3-hydroxypropyl)-1-(2-methylpyridin-3-yl)quinazolin-2(1H)-one (30 mg, 0.087 mmol), followed by the addition of methanesulfonyl chloride (9 mg, 0.087 mmol), triethylamine (18 mg, 0.18 mmol) and triphenylphosphine (33 mg, 0.132 mmol). The resulting mixture was stirred at room temperature for 1 hour, and then methanesulfonyl chloride (45 mg) and triethylamine (36 mg) were added in portions. The reaction solution was stirred at room temperature overnight. After completion of the reaction, a small amount of methanol was added to quench the reaction. After concentration, the residue was separated and purified by thin layer chromatography with a developing solvent of dichloromethane : ethyl acetate : methanol = 5:5:1. The product layers were collected and combined. The combined product layers were rinsed and concentrated to afford 9-chloro-1-(2-methylpyridin-3-yl)-4,5,6,7-tetrahydroazepino[2,3,4-de]quinazolin-2(1H)-one (3.5 mg, 12% yield), ESI-MS (m/z): 327.1[M+H]$^+$; $^1$H NMR (600 MHz, $CDCl_3$) δ 9.77 (br, 1H), 8.66 (d, *J* = 4.8 Hz, 1H), 7.55 (d, *J* = 7.8 Hz, 1H), 7.37 (dd, $J_1$ = 7.8 Hz, $J_2$ = 4.8 Hz, 1H), 7.00 (s, 1H), 6.24 (s, 1H), 3.65-3.62(m, 2H), 3.05 (t, *J* = 7.2 Hz, 2H), 2.36 (s, 3H), 2.26-2.23 (m, 2H).

Two separate atropisomers B1-P1 and B1-P2 were obtained following the resolution method of A16. B1-P1, ESI-MS (m/z): 327.1[M+H]$^+$; $^1$H NMR (600 MHz, $CDCl_3$) δ 9.77 (br, 1H), 8.66 (d, *J* = 4.8 Hz, 1H), 7.55 (d, *J* = 7.8 Hz, 1H), 7.37 (dd, $J_1$ = 7.8 Hz, $J_2$ = 4.8 Hz, 1H), 7.00 (s, 1H), 6.24 (s, 1H), 3.65-3.62(m, 2H), 3.05 (t, *J* = 7.2 Hz, 2H), 2.36 (s, 3H), 2.26-2.23 (m, 2H). B2-P1, ESI-MS (m/z): 327.1[M+H]$^+$; $^1$H NMR (600 MHz, $CDCl_3$) δ 9.77 (br, 1H), 8.66 (d, *J* = 4.8 Hz, 1H), 7.55 (d, *J* = 7.8 Hz, 1H), 7.37 (dd, $J_1$ = 7.8 Hz, $J_2$ = 4.8 Hz, 1H), 7.00 (s, 1H), 6.24 (s, 1H), 3.65-3.62(m, 2H), 3.05 (t, *J* = 7.2 Hz, 2H), 2.36 (s, 3H), 2.26-2.23 (m, 2H).

**Example 7: 9-chloro-1-(2-methylpyridin-3-yl)-5,6-dihydro-1*H*-[1,4]oxazepino[5,6,7-de]quinazolin-2(4*H*)-one (B2)**

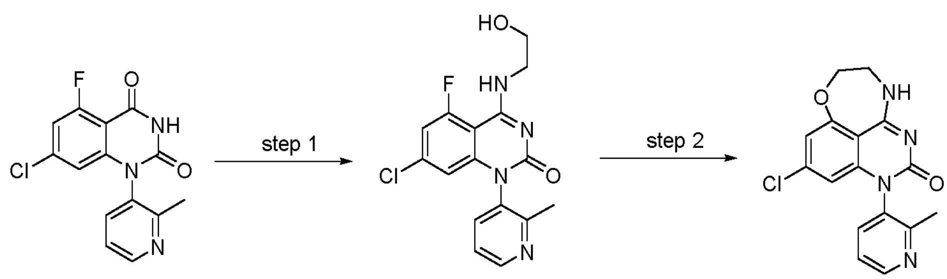


According to the synthesis method of 5-bromo-7-chloro-1-(2-methylpyridin-3-yl)quinazoline-2,4(1*H*,3*H*)-dione with 4-chloro-2,6-difluorobenzoic acid and 2-methylpyridin-3-amine as starting materials, 7-chloro-5-fluoro-1-(2-methylpyridin-3-yl)quinazoline-2,4(1*H*,3*H*)-dione was obtained, ESI-MS (m/z): 306.1[M+H]$^+$.

**Step 1: 7-chloro-5-fluoro-4-((2-hydroxyethyl)amino)-1-(2-methylpyridin-3-yl)quinazolin-2(1*H*)-one**

To a 100 mL round-bottomed flask were added 7-chloro-5-fluoro-1-(2-methylpyridin-3-yl)quinazoline-2,4(1*H*,3*H*)-dione (200 mg, 0.64 mmol) and dioxane (10 mL), followed by the addition of phosphorus oxychloride (292 mg, 1.92 mmol) and diisopropylethylamine (420 mg, 3.27 mmol). The reaction was stirred at 100°C for 1 hour. After cooling, aminoethanol (200 mg, 3.27 mmol) was added, and the reaction was stirred at room temperature. After completion

of the reaction, 100 mL water was added, and the resulting mixture was extracted with 300 mL ethyl acetate three times. The organic phases were combined. The combined organic phases were dried over anhydrous sodium sulfate, filtered and concentrated, and purified by column chromatography to afford 7-chloro-5-fluoro-4-((2-hydroxyethyl)amino)-1-(2-methylpyridin-3-yl)quinazolin-2(1*H*)-one (100 mg, 45% yield), ESI-MS (m/z): 349.1[M+H]+;

**Step 2: 9-chloro-1-(2-methylpyridin-3-yl)-5,6-dihydro-1*H*-[1,4]oxazepino[5,6,7-de]quinazolin-2(4*H*)-one**

To a 100 mL round-bottomed flask were added the product obtained in the above step, 7-chloro-5-fluoro-4-((2-hydroxyethyl)amino)-1-(2-methylpyridin-3-yl)quinazolin-2(1H)-one (100 mg, 0.29 mmol) and N,N-dimethylformamide (5 mL), followed by the addition of sodium hydride (23 mg, 0.57 mmol) with stirring in an ice bath. The reaction was stirred at room temperature. After completion of the reaction, 100 mL water was added, and the resulting mixture was extracted with 200 mL ethyl acetate twice. The organic phases were combined. The combined organic phases were washed with 100 mL water twice, then dried over anhydrous sodium sulfate, filtered and concentrated, and purified by thin layer chromatography to afford 9-chloro-1-(2-methylpyridin-3-yl)-5,6-dihydro-1*H*-[1,4]oxazepino[5,6,7-*de*]quinazolin-2(4*H*)-one (13 mg, 13% yield), ESI-MS (m/z): 329.1$[M+H]^+$.

**Example 8: 9-chloro-5-hydroxy-4-methyl-1-(2-methylpyridin-3-yl)-4,5,6,7-tetrahydro-[1,4]diazepino[5,6,7-*de*] quinazolin-2(1*H*)-one (B3)**

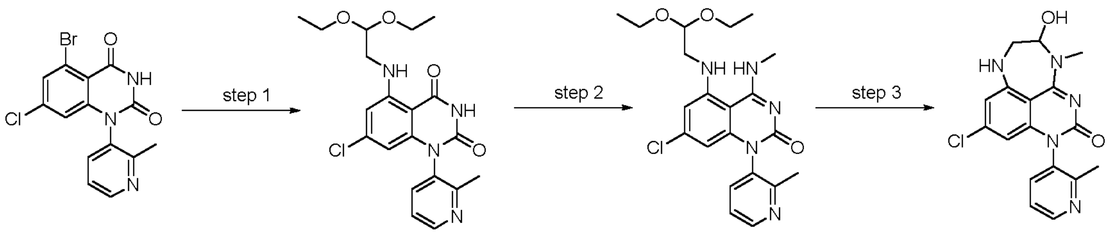


**Step 1: 7-chloro-5-((2,2-diethoxyethyl)amino)-1-(2-methylpyridin-3-yl)quinazoline-2,4(1*H*,3*H*)-dione**

To a 100 mL round-bottomed flask were added 5-bromo-7-chloro-1-(2-methylpyridin-3-yl)quinazoline-2,4(1*H*,3*H*)-dione (500 mg, 1.37 mmol), aminoacetaldehyde diethyl acetal (364 mg, 2.74 mmol), tris(dibenzylideneacetone)dipalladium (128 mg, 0.14 mmol), 1,1'-binaphthyl-2,2'-bis(diphenylphosphino) (174 mg, 0.28 mmol), sodium *tert*-butoxide (395 mg, 4.1 mmol) and toluene (10 mL). After purging with nitrogen gas, the reaction was stirred at 100°C. After completion of the reaction, the reaction was cooled, and 100 mL water was added. The resulting mixture was extracted with 200 mL ethyl acetate twice. The organic phases were combined. The combined organic phases were dried over anhydrous sodium sulfate, filtered and concentrated, and purified by column chromatography to afford 7-chloro-5-((2,2-diethoxyethyl)amino)-1-(2-methylpyridin-3-yl)quinazoline-2,4(1*H*,3*H*)-dione (500 mg, 87% yield), ESI-MS (m/z): 419. 1$[M+H]^+$.

**Step 2: 7-chloro-5-((2,2-diethoxyethyl)amino)-4-(methylamino)-1-(2-methylpyridin-3-yl)quinazolin-2(1*H*)-one**

To a 100 mL round-bottomed flask were added the product obtained in the above step 7-chloro-5-((2,2-diethoxyethyl)amino)-1-(2-methylpyridin-3-yl)quinazoline-2,4(1*H*,3*H*)-dione (200 mg, 0.48 mmol) and dioxane (10 mL), followed by the addition of phosphorus oxychloride (218 mg, 1.44 mmol) and diisopropylethylamine (310 mg, 2.4 mmol). The reaction was stirred at 100°C for 1 hour. After cooling, an aqueous solution of methylamine was added, and the reaction was stirred at room temperature. After completion of the reaction, 100 mL water was added. The resulting mixture was extracted with 300 mL ethyl acetate three times. The organic phases were combined. The combined organic phases were dried over anhydrous sodium sulfate, filtered and concentrated, and separated and purified by column chromatography to afford 7-chloro-5-((2,2-diethoxyethyl)amino)-4-(methylamino)-1-(2-methylpyridin-3-yl)quinazolin-2(1*H*)-one (100 mg, 48% yield), ESI-MS (m/z): 432.2$[M+H]^+$.

**Step 3: 9-chloro-5-hydroxy-4-methyl-1-(2-methylpyridin-3-yl)-4,5,6,7-tetrahydro-[1,4]diazepino[5,6,7-*de*]quinazolin-2(1*H*)-one**

To a 100 mL round-bottomed flask were added the product obtained in the above step, 7-chloro-5-((2,2-diethoxyethyl)amino)-4-(methylamino)-1-(2-methylpyridin-3-yl)quinazolin-2(1*H*)-one (100 mg, 0.23 mmol) and acetonitrile (2 mL), followed by the addition of the concentrated hydrochloric acid (0.1 mL). The reaction was stirred at 80°C under the sealed condition. After completion of the reaction, the reaction mixture was concentrated, followed by the addition of 100 mL ethyl acetate. The resulting mixture was washed with 100 mL an aqueous solution of sodium bicarbonate twice. The organic phase was concentrated and purified by thin layer chromatography to afford 9-chloro-5-hydroxy-4-methyl-1-(2-methylpyridin-3-yl)-4,5,6,7-tetrahydro-[1,4]diazepino[5,6,7-de]quinazolin-2(1*H*)-one, ESI-MS (m/z): 358.1 $[M+H]^+$; $^1$H NMR (600 MHz, DMSO-$d_6$) $\delta$ 8.56 (d, *J* = 4.2 Hz, 1H), 7.69-7.68 (m, 1H), 7.65 (t, *J* = 7.2 Hz, 1H), 7.419 (t, *J*= 6.0 Hz, 1H), 6.35 (d, *J* = 1.2 Hz, 1H), 5.25-5.23 (m, 2H), 4.89 (br, 1H), 3.64-3.59 (m, 1H), 3.57-3.52(m, 1H), 3.16 (s, 3H), 2.18 (d, *J* = 30 Hz, 3H).

Two separate atropisomers B3-P1 and B3-P2 were obtained following the resolution method of A16. B3-P1, ESI-MS (m/z): 358.1$[M+H]^+$; $^1$H NMR (600 MHz, DMSO-$d_6$) $\delta$ 8.56 (d, *J* = 4.2 Hz, 1H), 7.69-7.68 (m, 1H), 7.65 (t, *J* = 7.2 Hz, 1H), 7.419 (t, *J* = 6.0 Hz, 1H), 6.35 (d, *J* = 1.2 Hz, 1H), 5.25-5.23 (m, 2H), 4.89 (br, 1H), 3.64-3.59 (m, 1H), 3.57-3.52(m, 1H), 3.16 (s, 3H), 2.18 (d, *J* = 30 Hz, 3H). B3-P2, ESI-MS (m/z): 358.1$[M+H]^+$; $^1$H NMR (600 MHz, DMSO-$d_6$) $\delta$ 8.56 (d, *J* = 4.2 Hz, 1H), 7.69-7.68 (m, 1H), 7.65 (t, *J* = 7.2 Hz, 1H), 7.419 (t, *J* = 6.0 Hz, 1H), 6.35 (d, *J* = 1.2 Hz, 1H), 5.25-5.23 (m, 2H), 4.89 (br, 1H), 3.64-3.59 (m, 1H), 3.57-3.52(m, 1H), 3.16 (s, 3H), 2.18 (d, *J* = 30 Hz, 3H).

Compounds A5-A15, A17-A18, A20-A28, and A30-A37 were prepared according to the similar synthetic methods with the corresponding intermediates as the starting materials.

| Example No. | General Method | Main Starting Material(s) | Compound Structure and Compound No. | Characterization Data |
|---|---|---|---|---|
| 9 | Method One | 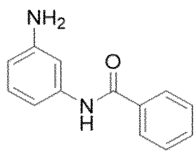 Ammonia water | 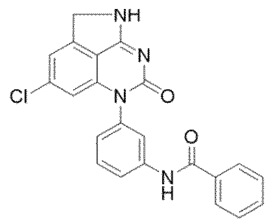 A5 | ESI-MS (m/z) : 403.1$[M+H]^+$ |
| 10 | Method One | 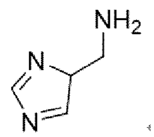 Ammonia water | 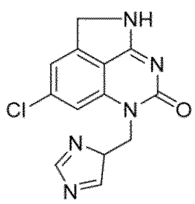 A6 | ESI-MS (m/z) : 288.1$[M+H]^+$ |
| 11 | Method One | 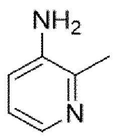 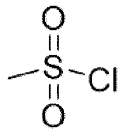 | 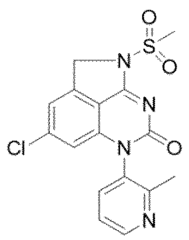 A7 | ESI-MS (m/z) : 377.1$[M+H]^+$ |
| 12 | Method One | 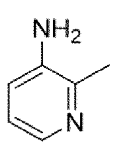 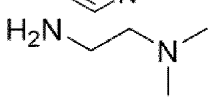 | 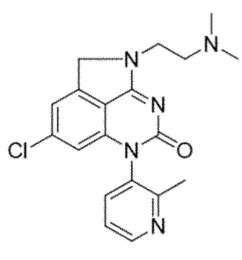 A8 | ESI-MS (m/z) : 370.1$[M+H]^+$ |

(continued)

| Example No. | General Method | Main Starting Material(s) | Compound Structure and Compound No. | Characterization Data |
|---|---|---|---|---|
| 13 | Method One | 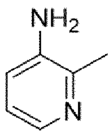 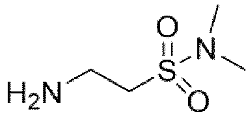 | 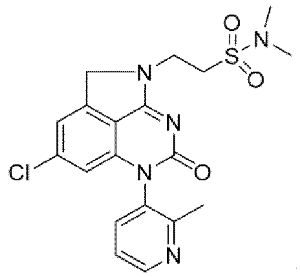 A9 | ESI-MS (m/z) : 434.1[M+H]$^+$ |
| 14 | Method One | 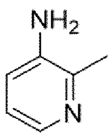 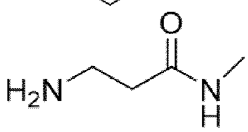 | 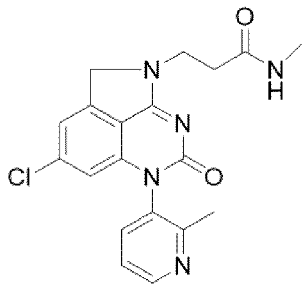 A10 | ESI-MS (m/z) : 384.1[M+H]$^+$ |
| 15 | Method One | 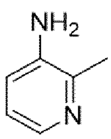 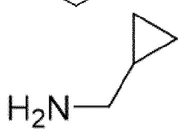 | 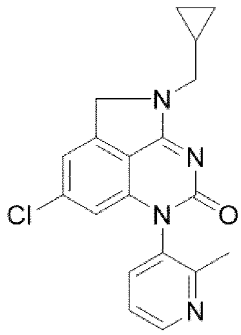 A11 | ESI-MS (m/z) : 353.1[M+H]$^+$ |
| 16 | Method One | 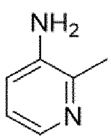 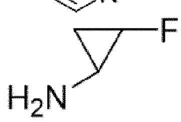 | 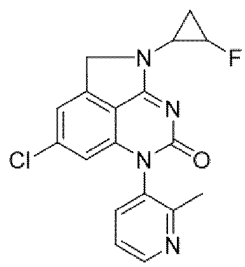 A12 | ESI-MS (m/z) : 357.1[M+H]$^+$ |

(continued)

| Example No. | General Method | Main Starting Material(s) | Compound Structure and Compound No. | Characterization Data |
|---|---|---|---|---|
| 17 | Method One | 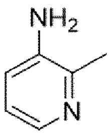 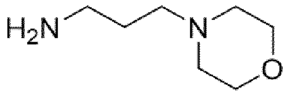 | 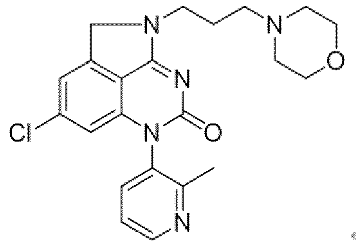 A13 | ESI-MS (m/z) : 426.1[M+H]+ |
| 18 | Method One | 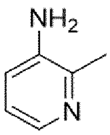 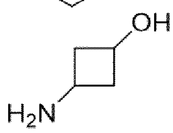 | 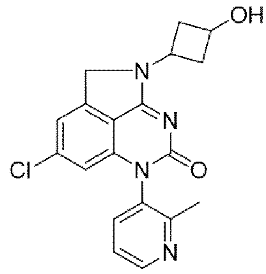 A14 | ESI-MS (m/z) : 369.1[M+H]+ |
| 19 | Method Six | 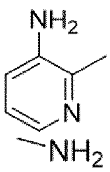 HCHO | 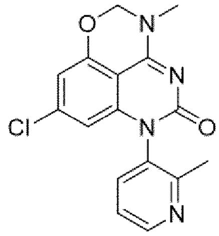 A15 | ESI-MS (m/z) : 329.1[M+H]+ |
| 20 | Method One | 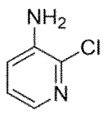 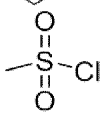 | 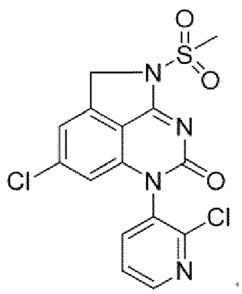 A17 | ESI-MS (m/z) : 397.1[M+H]+ |
| 21 | Method One | 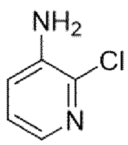 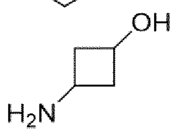 | 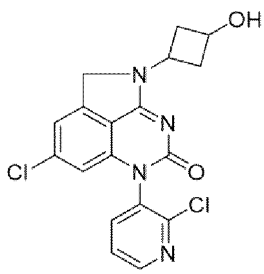 A18 | ESI-MS (m/z) : 389.1[M+H]+ |

(continued)

| Example No. | General Method | Main Starting Material(s) | Compound Structure and Compound No. | Characterization Data |
|---|---|---|---|---|
| 22 | Method Five | 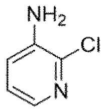 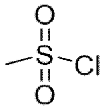 | 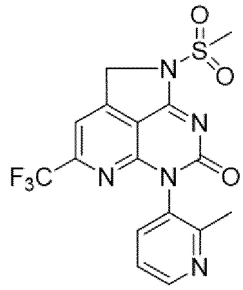 A20 | ESI-MS (m/z) : 412.1[M+H]$^+$ |
| 23 | Method Five | 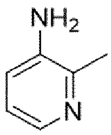 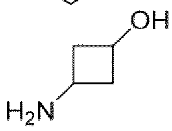 | 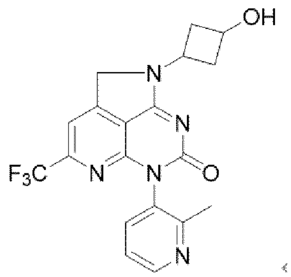 A21 | ESI-MS (m/z) : 404.1[M+H]$^+$ |
| 24 | Method Five | 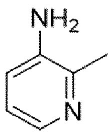 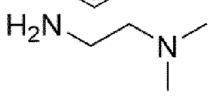 | 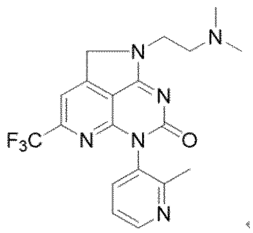 A22 | ESI-MS (m/z) : 405.1[M+H]$^+$ |
| 25 | Method Five | 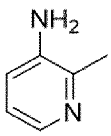 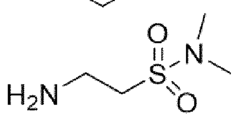 | 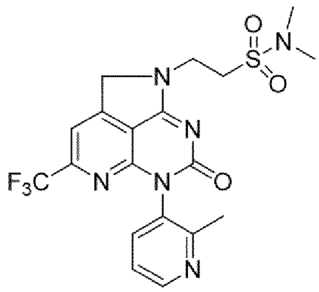 A23 | ESI-MS (m/z) : 469.1[M+H]$^+$ |

(continued)

| Example No. | General Method | Main Starting Material(s) | Compound Structure and Compound No. | Characterization Data |
|---|---|---|---|---|
| 26 | Method Five | 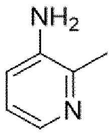 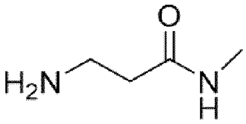 | 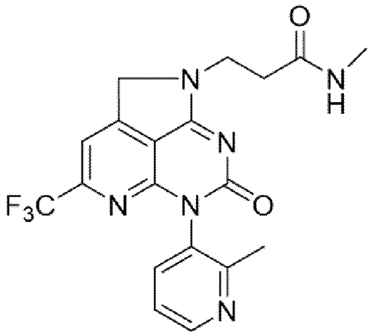 A24 | ESI-MS (m/z) : 419.1$[M+H]^+$ |
| 27 | Method Five | 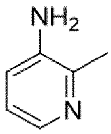 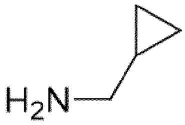 | 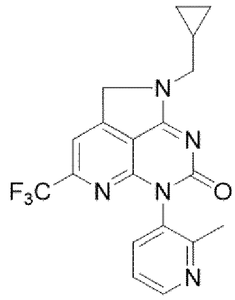 A25 | ESI-MS (m/z) : 388.1$[M+H]^+$ |
| 28 | Method Five | 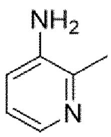 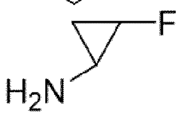 | 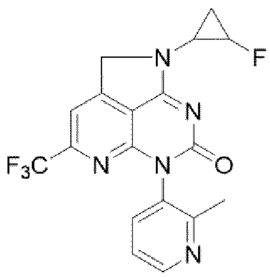 A26 | ESI-MS (m/z) : 392.1$[M+H]^+$ |
| 29 | Method Five | 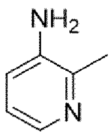 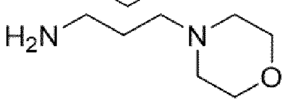 | 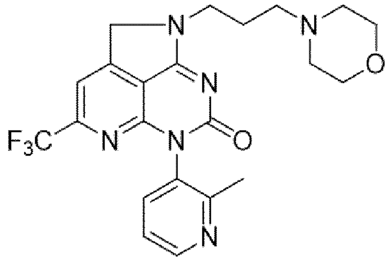 A27 | ESI-MS (m/z) : 461.1$[M+H]^+$ |

(continued)

| Example No. | General Method | Main Starting Material(s) | Compound Structure and Compound No. | Characterization Data |
|---|---|---|---|---|
| 30 | Method Five | 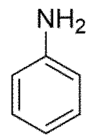 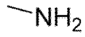 | 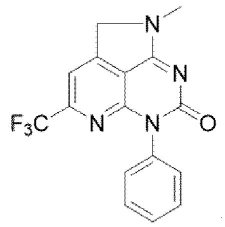 A28 | ESI-MS (m/z) : 333.1[M+H]+ |
| 31 | Method Five | 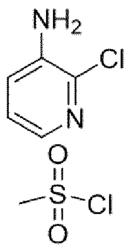 | 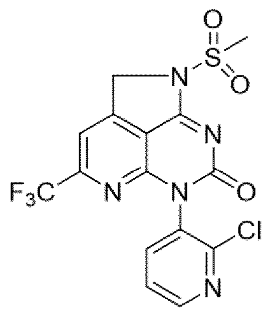 A30 | ESI-MS (m/z) : 432.1[M+H]+ |
| 32 | Method Five | 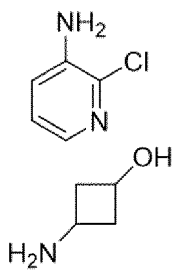 | 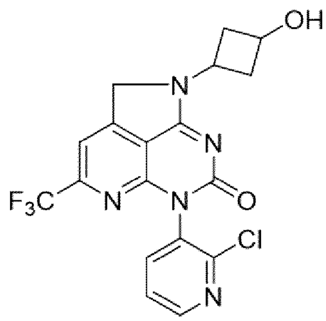 A31 | ESI-MS (m/z) : 424.1[M+H]+ |
| 33 | Method Five | 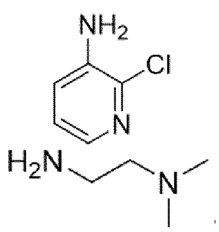 | 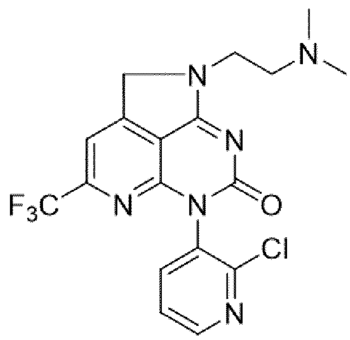 A32 | ESI-MS (m/z) : 425.1[M+H]+ |

(continued)

| **Example No.** | **General Method** | **Main Starting Material(s)** | **Compound Structure and Compound No.** | **Characterization Data** |
|---|---|---|---|---|
| 34 | Method Five | 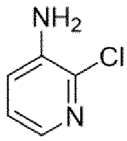 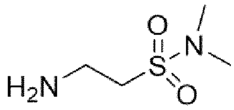 | 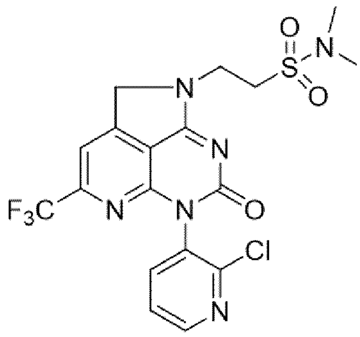 A33 | ESI-MS (m/z) : 489.1[M+H]$^+$ |
| 35 | Method Five | 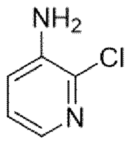 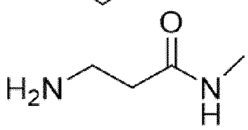 | 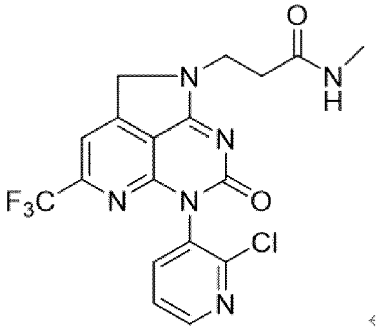 A34 | ESI-MS (m/z) : 439.1[M+H]$^+$ |
| 36 | Method Five | 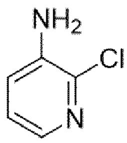 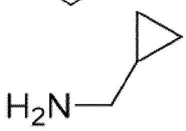 | 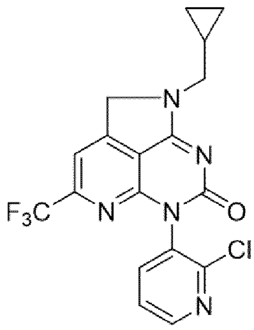 A35 | ESI-MS (m/z) : 408.1[M+H]$^+$ |
| 37 | Method Five | 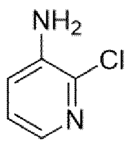 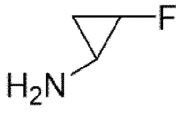 | 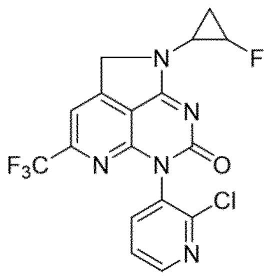 A36 | ESI-MS (m/z) : 412.1[M+H]$^+$ |

(continued)

| Example No. | General Method | Main Starting Material(s) | Compound Structure and Compound No. | Characterization Data |
|---|---|---|---|---|
| 38 | Method Five | 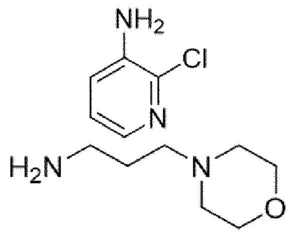 | 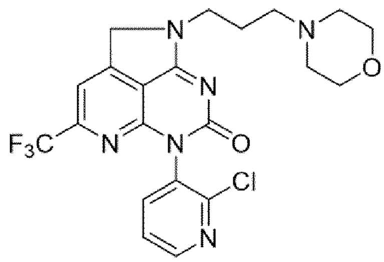 A37 | ESI-MS (m/z) : 481.1[M+H]+ |

**Example 39: 2-methyl-5-(2-methylpyridin-3-yl)-7-(trifluoromethyl)-1,2-dihydro-2,3,5,6-tetraazanaphthalen-4(5H)-one (A19)**

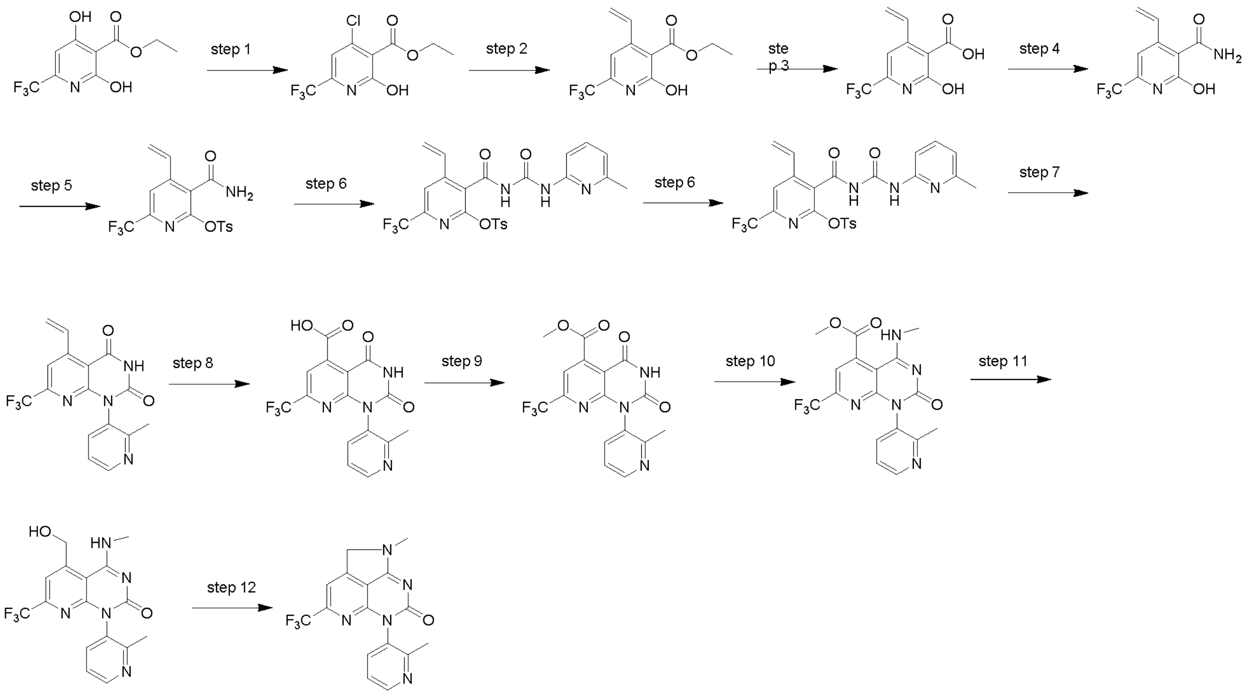

A19

**Step 1: synthesis of ethyl 4-chloro-2-hydroxy-6-(trifluoromethyl)nicotinate**

To DMF (62 mL) was added ethyl 2,4-dihydroxy-6-(trifluoromethyl)nicotinate (synthesized according to Reference "Org. Process Res. Dev., 2011, 15, 788-796" synthesis) (50 g, 199.1 mmol) at room temperature, and thoroughly stirred and dissolved. Then, the reaction flask was placed in ice water, and thereto was slowly added $POCl_3$ (122.10 g, 796 mmol) dropwise at 0°C. After completion of the dropwise addition, the reaction flask was placed in an oil bath at 90°C, and reacted for 1 h. After LCMS showed complete reaction, a saturated solution of $K_2HPO_4$ (2.5 L) was prepared, and then the reaction solution was poured into this saturated solution slowly, and the reaction was quenched. The resulting mixture was extracted with dichloromethane, washed with saturated sodium chloride solution (500 mL), dried over anhydrous sodium sulfate and concentrated under reduced pressure to afford the crude product, which was purified by a silica gel column ($SiO_2$, DCM: MeOH = 1:0) to afford ethyl 4-chloro-2-hydroxy-6-(trifluoromethyl)nicotinate (35 g).

**Step 2: synthesis of ethyl 2-hydroxy-6-(trifluoromethyl)-4-vinylnicotinate**

To a 100 mL PTFE sealed container were added the product obtained in the above step (5 g) and potassium vinyltrifluoroborate (4.28 g), followed by the addition of $H_2O$ (10 mL) and dioxane (40 mL). SPhos (761 mg) and $K_3PO_4$ (11.81 g) were added with stirring at room temperature, and $Pd(OAc)_2$(416 mg) was added under nitrogen atmosphere. The resulting mixture was heated to 90°C and reacted for 48 h. After LCMS showed complete reaction, the reaction mixture was concentrated directly under reduced pressure to remove the solvent, and then purified by a silica gel column (($SiO_2$, DCM: MeOH = 1:0 to 10:1)) to afford ethyl 2-hydroxy-6- (trifluoromethyl)-4-vinylnicotinate (2.57 g).

**Step 3: synthesis of 2-hydroxy-6-(trifluoromethyl)-4-vinylnicotinic acid**

To a mixed solvent of MeOH (110 mL), THF (110 mL) and $H_2O$ (55mL) was added the product obtained in the above step (18 g, 1 *eq*.) at room temperature, followed by the addition of NaOH (6.43 g, 3 *eq*.). The reaction solution was heated to 60°C, and reacted for 16 h. LCMS showed complete reaction. The reaction mixture was concentrated under reduced pressure to remove the solvent, and then purified to afford 2-hydroxy-6-(trifluoromethyl)-4-vinylnicotinic acid (7 g).

**Step 4: synthesis of 2-hydroxy-6-(trifluoromethyl)-4-vinylnicotinamide**

The product obtained in the above step (7 g, 1 eq.) was dissolved in DCM (100 mL), followed by the slow addition of oxalyl chloride (9.46 g) and DMF (0.4 g). The resulting mixture was reacted at room temperature for 1 hour. After sampling and adding methanol, TLC confirmed complete reaction. The reaction system was dried by rotary evaporation. A 500 mL $NH_3$. dioxane solution was added, and reacted at room temperature for 1 hour. After LCMS showed complete reaction, the reaction solution was concentrated directly under reduced pressure to remove the solvent, and purified by a silica gel column ($SiO_2$, DCM: MeOH = 1:0 to 5:1) to afford 2-hydroxy-6-(trifluoromethyl)-4-vinylnicotinamide (7 g).

**Step 5: 3-aminocarbonyl-6-(trifluoromethyl)-4-vinylpyridin-2-methyl *p*-toluenesulfonate**

To DCM (50 mL) was added the product obtained in the above step (7 g), followed by the addition of *p*-toluenesulfonyl chloride (TsCl, 8.6 g), triethylamine (TEA, 7.6 g) and DMAP (375 mg). The resulting mixture was stirred at room temperature for 2 hours. After LCMS showed complete reaction, the reaction mixture was extracted with water (100 mL) and ethyl acetate. The organic phases were combined. The combined organic phases were concentrated under reduced pressure to afford a crude product, which was purified by a silica gel column ($SiO_2$, PE: EA = 10:1 to pure DCM) to afford the product, 3-aminocarbonyl-6-(trifluoromethyl)-4-vinylpyridin-2-methyl *p*-toluenesulfonate (8.5 g).

**Step 6: 3-(((2-methylpyridin-3-yl)aminocarbonyl)aminocarbonyl)-6-(trifluoromethyl)-4-vinylpyridin-2-methyl *p*-toluenesulfonate**

To THF (40 mL) was added the product obtained in the above step (1.0 g), followed by the addition of oxalyl chloride (0.50 mL), and then the resulting mixture was heated to 80°C and stirred for 1 h. A sample was taken and quenched with methanol to confirm the formation of the intermediate state of the reaction. After cooling to room temperature, 3-amino-2-methylpyridine (400 mg) was added, and stirred at room temperature for one hour. After LCMS showed complete reaction, the pH of the reaction mixture was adjusted to acidic with an aqueous solution of acetic acid. The reaction mixture was extracted with $H_2O$ (100 mL) and ethyl acetate. The organic phases were combined. The combined organic phases were concentrated under reduced pressure to afford crude product, 3-(((2-methylpyridin-3-yl) aminocarbonyl)aminocarbonyl)-6-(trifluoromethyl)-4-vinylpyridin-2-methyl *p*-toluenesulfonate(4.5 g).

**Step 7: 1-(2-methylpyridin-3-yl)-7-(trifluoromethyl)-5-vinylpyrido[2,3-d]pyrimidine-2,4-(1*H*,3*H*)-dione**

To acetonitrile (ACN, 50 mL) was added the product obtained in the above step (4.5 g), followed by the addition of DBU (3.9 ml). The resulting mixture was stirred at room temperature for 1 h. After LCMS showed complete reaction, the pH of the reaction mixture was adjusted to acidic with an aqueous solution of acetic acid. The resulting mixture was concentrated under reduced pressure to afford the crude product, which was purificated by prep-TLC (ethyl acetate: dichloromethane = 1:4) to afford the product, 1-(2-methylpyridin-3-yl)-7-(trifluoromethyl)-5-vinylpyrido[2,3-d]pyrimidine-2,4-(1H,3H)-dione (280 mg).

**Step 8: synthesis of 1-(2-methylpyridin-3-yl)-2,4-dioxo-7-(trifluoromethyl)-1,2,3,4-tetrahydropyrido[2,3-d]pyrimidine-5-carboxylic acid**

To a solution of 1,4-dioxane (20 mL) and water (10 mL) was added 1-(2-methylpyridin-3-yl)-7-(trifluoromethyl)-5-vinylpyrido[2,3-*d*]pyrimidine-2,4(1*H*,3*H*)-dione (472 mg, 1.356 mmol.) at room temperature. After thoroughly stirring and dissolving, 10 drops of 1N sulfuric acid solution were added, followed by the addition of potassium permanganate (1.07 g, 6.781 mmol.). The resulting mixture was placed in an oil bath at 50°C and reacted for 1 h. After LCMS showed complete reaction, the reaction mixture was cooled to room temperature. 20 mL methanol was added, and the resulting mixture was stirred for 0.5 h, and filtered through Celite. The filter cake was then washed with methanol, and the filtrate was concentrated to afford the product which was used directly in the next step, ESI-MS (m/z): 367.0[M+H]+.

**Step 9: synthesis of methyl 1-(2-methylpyridin-3-yl)-2,4-dioxo-7-(trifluoromethyl)-1,2,3,4-tetrahydropyrido[2,3-*d*]pyrimidine-5-carboxylate**

The product obtained in the above step was dissolved in dichloromethane (100 mL), followed by the addition of oxalyl chloride (256 mg, 2.034 mmol.) and then DMF (1 drop). The resulting mixture was stirred at room temperature for 2 h. After TLC showed complete reaction, methanol (20 mL) was added and stirring was continued for 0.5 h. After TLC showed complete reaction, the reaction mixture was concentrated directly under reduced pressure to remove the solvent, and then purified by a silica gel column (($SiO_2$, DCM: MeOH = 1:0 to 50:1)) to afford methyl 1-(2-methylpyridin-3-yl)-2,4-dioxo-7-(trifluoromethyl)-1,2,3,4-tetrahydropyrido[2,3-*d*]pyrimidine-5-carboxylate (428 mg, 83% two-step yield), ESI-MS (m/z): 381.1[M+H]+.

**Step 10:** synthesis **of methyl 1-(2-methylpyridin-3-yl)-4-(methylamino)-2-oxo-7-(trifluoromethyl)-1,2-dihydropyrido[2,3-d]pyrimidine-5-carboxylate**

Methyl 1-(2-methylpyridin-3-yl)-2,4-dioxo-7-(trifluoromethyl)-1,2,3,4-tetrahydropyrido[2,3-*d*]pyrimidine-5-carboxylate (209 mg, 0.549 mmol) was dissolved in 1,4-dioxane (10 mL), followed by the addition of N,N-diisopropylethylamine (708 mg, 5.490 mmol) and phosphorus oxychloride (501 mg, 3.294mmol). The reaction solution was heated to 100°C and reacted for 1 h. LCMS showed complete reaction. The reaction was cooled to room temperature, and N,N-diisopropylethylamine (708 mg, 105.490 mmol) and methylamine hydrochloride (185 mg, 2.745 mmol) were added. The reaction was continued at room temperature for 2 h. After LCMS showed complete reaction, the system proceeds directly to the next step without processing, ESI-MS (m/z): 394.1[M+H]+.

**Step 11: synthesis of 1-(2-methylpyridin-3-yl)-5-(hydroxymethyl)-4-(methylamino)-7-(trifluoromethyl)pyrido [2,3-d] pyrimidin-2(1H)-one**

To the system obtained in the above step was added methanol (10 mL). Sodium borohydride (208 mg, 5.490 mmol) was added in portions in an ice bath. The resulting mixture was reacted at room temperature for 0.5 hours. After LCMS showed complete reaction, 100 mL water was added. The resulting mixture was extracted with 80 mL ethyl acetate twice. The organic phases were combined. The combined organic phases were concentrated under reduced pressure to remove the solvent, and purified by a silica gel column (($SiO_2$, DCM: MeOH = 1:0 to 20:1)) to afford 1-(2-methylpyridin-3-yl)-5-(hydroxymethyl)-4-(methylamino)-7-(trifluoromethyl)pyrido[2,3-*d*]pyrimidin-2(1*H*)-one (28.4 mg, 14% yield over two steps), ESI-MS (m/z): 366.1[M+H]+.

**Step 12: synthesis of 2-methyl-5-(2-methylpyridin-3-yl)-7-(trifluoromethyl)-1,2-dihydro-2,3,5,6-tetraazanaphthalen-4(5H)-one**

To DCM (5 mL) was added 1-(2-methylpyridin-3-yl)-5-(hydroxymethyl)-4-(methylamino)-7-(trifluoromethyl)pyrido[2,3-d]pyrimidin-2(1H)-one (28 mg, 0.078 mmol), followed by the addition of triethylamine (24 mg, 0.233 mmol) and methanesulfonyl chloride (18 mg, 0.155 mmol.). The resulting mixture was stirred at room temperature for 0.5 h. After LCMS showed complete reaction, the reaction mixture was concentrated directly under reduced pressure to afford the crude product, which was purified by a silica gel column ($SiO_2$, DCM: MeOH = 20:1) to afford the product, 5-(2-methylpyridin-3-yl)-2-methyl-7-(trifluoromethyl)-1,2-dihydro-2,3,5,6-tetraazanaphthalen-4(5H)-one (11 mg, 42% yield). ESI-MS (m/z): 348.1[M+H]+. $^1$H NMR (600 MHz, DMSO) δ 8.53 (d, *J* = 4.9 Hz, 1H), 7.81 (s, 1H), 7.69 (d, *J* = 7.7 Hz, 1H), 7.41-7.38 (m, 1H), 5.10 (d, *J* = 4.5 Hz, 2H), 3.29 (s, 3H), 2.19 (s, 3H).

Two separate atropisomers A19-P1 and A19-P2 were obtained following the resolution method of A16. A19-P1, ESI-MS (m/z): 348.1[M+H]+. $^1$H NMR (600 MHz, DMSO) δ 8.53 (d, *J* = 4.9 Hz, 1H), 7.81 (s, 1H), 7.69 (d, *J* = 7.7 Hz, 1H), 7.41-7.38 (m, 1H), 5.10 (d, *J* = 4.5 Hz, 2H), 3.29 (s, 3H), 2.19 (s, 3H). A19-P2, ESI-MS (m/z): 348.1[M+H]+. $^1$H NMR (600

MHz, DMSO) δ 8.53 (d, *J* = 4.9 Hz, 1H), 7.81 (s, 1H), 7.69 (d, *J* = 7.7 Hz, 1H), 7.41-7.38 (m, 1H), 5.10 (d, *J* = 4.5 Hz, 2H), 3.29 (s, 3H), 2.19 (s, 3H).

**Example 40: 5-(2-chloropyridin-3-yl)-2-methyl-7-(trifluoromethyl)-1,2-dihydro-2,3,5,6-tetraazanaphthalen-4(5H)-one (A29)**

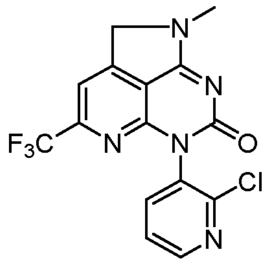

According to the synthetic method of Compound **A19** with 3-amino-2-chloropyridine and methylamine as the starting materials, the product 5-(2-chloropyridin-3-yl)-2-methyl-7-(trifluoromethyl)-1,2-dihydro-2,3,5,6-tetraazanaphthalen-4(5H)-one was obtained (10 mg, 36% yield), ESI-MS (m/z): 368.1[M+H]$^+$, $^1$H NMR (600 MHz, $CDCl_3$) δ 8.50 (d, *J* = 5.04, 1H), 7.76 (d, *J* = 5.16, 1H), 7.51 (s, 1H), 7.44 (t, *J* = 5.16, 1H), 4.93 (s, 2H), 3.44 (s, 3H).

Two separate atropisomers A29-P1 and A29-P2 were obtained following the resolution method of A16. A29-P1, ESI-MS (m/z): 368.1[M+H]$^+$, $^1$H NMR (600 MHz, $CDCl_3$) δ 8.50 (d, *J* = 5.04, 1H), 7.76 (d, *J* = 5.16, 1H), 7.51 (s, 1H), 7.44 (t, *J* = 5.16, 1H), 4.93 (s, 2H), 3.44 (s, 3H). A29-P2, ESI-MS (m/z): 368.1[M+H]$^+$, $^1$H NMR (600 MHz, $CDCl_3$) δ 8.50 (d, *J* = 5.04, 1H), 7.76 (d, *J* = 5.16, 1H), 7.51 (s, 1H), 7.44 (t, *J* = 5.16, 1H), 4.93 (s, 2H), 3.44 (s, 3H).

**Example 41: 7-chloro-4-(2-methyl-2*H*-indazol-5-yl)-1-(2-methylpyridin-3-yl)-4,5-dihydropyrrolo[2,3,4-*de*]quinazolin-2(1*H*)-one (A38)**

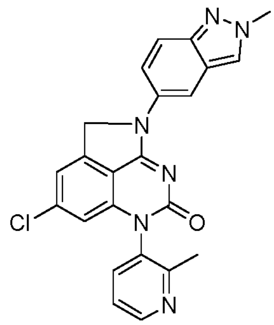

According to the synthetic method of Compound A2 with 2-methyl-2*H*-indazol-5-amine and 3-amino-2-methylpyridine as the starting materials, 7-chloro-4-(2-methyl-2*H*-indazol-5-yl)-1-(2-methylpyridin-3-yl)-4,5-dihydropyrrolo[2,3,4-*de*]quinazolin-2(1*H*)-one was obtained (5 mg, 60% yield), ESI-MS (m/z): 429.1[M+H]$^+$. $^1$H NMR (600 MHz, DMSO) δ 8.61 (d, *J* = 4.8 Hz, 1H), 8.44 (s, 1H), 8.28 (d, *J* = 1.8 Hz, 1H), 8.02(dd, *J* = 9.3, 2.1 Hz, 1H), 7.79 (d, *J* = 6.6 Hz, 1H), 7.74 (d, *J* = 9.4 Hz, 1H), 7.47 (dd, *J* = 7.8, 4.9 Hz, 1H), 7.41 (s, 1H), 6.28 (s, 1H), 5.55 (s, 2H), 4.20 (s, 3H), 2.26 (s, 3H).

Two separate atropisomers A38-P1 and A38-P2 were obtained following the resolution method of A16. A38-P1, ESI-MS (m/z): 429.1[M+H]$^+$. $^1$H NMR (600 MHz, DMSO) δ 8.61 (d, *J* = 4.8 Hz, 1H), 8.44 (s, 1H), 8.28 (d, *J* = 1.8 Hz, 1H), 8.02(dd, *J* = 9.3, 2.1 Hz, 1H), 7.79 (d, *J* = 6.6 Hz, 1H), 7.74 (d, *J* = 9.4 Hz, 1H), 7.47 (dd, *J* = 7.8, 4.9 Hz, 1H), 7.41 (s, 1H), 6.28 (s, 1H), 5.55 (s, 2H), 4.20 (s, 3H), 2.26 (s, 3H). A38-P2, ESI-MS (m/z): 429.1[M+H]$^+$. $^1$H NMR (600 MHz, DMSO) δ 8.61 (d, *J* = 4.8 Hz, 1H), 8.44 (s, 1H), 8.28 (d, *J* = 1.8 Hz, 1H), 8.02(dd, *J* = 9.3, 2.1 Hz, 1H), 7.79 (d, *J* = 6.6 Hz, 1H), 7.74 (d, *J* = 9.4 Hz, 1H), 7.47 (dd, *J* = 7.8, 4.9 Hz, 1H), 7.41 (s, 1H), 6.28 (s, 1H), 5.55 (s, 2H), 4.20 (s, 3H), 2.26 (s, 3H).

**Example 42: 7-chloro-4-(4-chlorophenyl)-1-(2-methylpyridin-3-yl)-4,5-dihydropyrrolo[2,3,4-*de*]quinazolin-2(1*H*)-one (A39)**

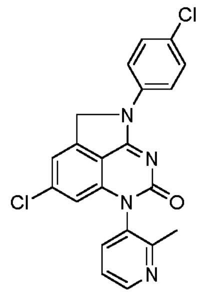

According to the synthetic method of Compound A2 with *p*-chloroaniline and 3-amino-2-methylpyridine as the starting materials, 7-chloro-4-(4-chlorophenyl)-1-(2-methylpyridin-3-yl)-4,5-dihydropyrrolo[2,3,4-*de*]quinazolin-2(1*H*)-one was obtained (15 mg, 50% yield), ESI-MS (m/z): 409.1[M+H]$^+$. $^1$H NMR (600 MHz, DMSO) δ 8.61 (dd, *J* = 4.8, 1.3 Hz, 1H), 8.12(d, *J* = 9.0 Hz, 2H), 7.82 - 7.77 (m, 1H), 7.58 (d, *J* = 9.0 Hz, 2H), 7.47 (dd, *J* = 7.8, 4.8 Hz, 1H), 7.41 (s, 1H), 6.31 (s, 1H), 5.50 (s, 2H), 2.25 (s, 3H).

Two separate atropisomers A39-P1 and A39-P2 were obtained following the resolution method of A16. A39-P1, ESI-MS (m/z): 409.1[M+H]$^+$. $^1$H NMR (600 MHz, DMSO) δ 8.61 (dd, *J* = 4.8, 1.3 Hz, 1H), 8.12(d, *J* = 9.0 Hz, 2H), 7.82 - 7.77 (m, 1H), 7.58 (d, *J* = 9.0 Hz, 2H), 7.47 (dd, *J* = 7.8, 4.8 Hz, 1H), 7.41 (s, 1H), 6.31 (s, 1H), 5.50 (s, 2H), 2.25 (s, 3H). A39-P2, ESI-MS (m/z): 409.1[M+H]$^+$. $^1$H NMR (600 MHz, DMSO) δ 8.61 (dd, *J* = 4.8, 1.3 Hz, 1H), 8.12(d, *J* = 9.0 Hz, 2H), 7.82 - 7.77 (m, 1H), 7.58 (d, *J* = 9.0 Hz, 2H), 7.47 (dd, *J* = 7.8, 4.8 Hz, 1H), 7.41 (s, 1H), 6.31 (s, 1H), 5.50 (s, 2H), 2.25 (s, 3H).

**Example 43: 7-chloro-1-(2-methylpyridin-3-yl)-4,5-dihydropyrrolo[2,3,4-de]quinazolin-2(1*H*)-one (A40)**

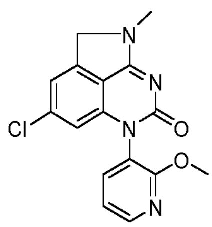

**A40**

According to the synthetic method of Compound A1 with 2-methoxypyridin-3-amine and methylamine as the starting materials, 7-chloro-1-(2-methoxypyridin-3-yl)-4-methyl-4,5-dihydropyrrolo[2,3,4-*de*]quinazolin-2(1*H*)-one was obtained (17 mg, 50% yield), ESI-MS (m/z): 329.1[M+H]$^+$. $^1$H NMR (600 MHz, DMSO) δ 8.30 (d, *J* = 3.8 Hz, 1H), 7.77 (d, *J* = 7.1 Hz, 1H), 7.28 (s, 1H), 7.19-7.17 (m, 1H), 6.25 (s, 1H), 4.90 (s, 2H), 3.80 (s, 3H), 3.21 (s, 3H).

Two separate atropisomers A40-P1 and A40-P2 were obtained following the resolution method of A16. A40-P1, ESI-MS (m/z): 329.1[M+H]$^+$. $^1$H NMR (600 MHz, DMSO) δ 8.30 (d, *J* = 3.8 Hz, 1H), 7.77 (d, *J* = 7.1 Hz, 1H), 7.28 (s, 1H), 7.19-7.17 (m, 1H), 6.25 (s, 1H), 4.90 (s, 2H), 3.80 (s, 3H), 3.21 (s, 3H). A40-P2, ESI-MS (m/z): 329.1[M+H]$^+$. $^1$H NMR (600 MHz, DMSO) δ 8.30 (d, *J* = 3.8 Hz, 1H), 7.77 (d, *J* = 7.1 Hz, 1H), 7.28 (s, 1H), 7.19-7.17 (m, 1H), 6.25 (s, 1H), 4.90 (s, 2H), 3.80 (s, 3H), 3.21 (s, 3H).

**Example 44: 4-methyl-1-(2-methylpyridin-3-yl)-8-(trifluoromethyl)-5,6-dihydro-1H-pyrimidino[4,5,6-ij][2,7]naphthyridin-2(4H)-one (C1)**

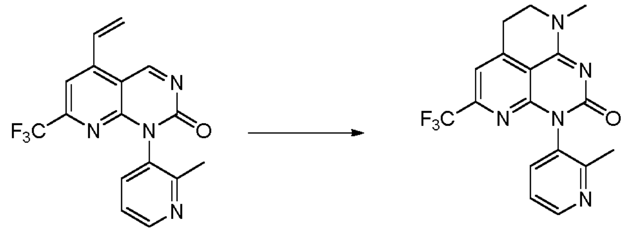

C1

To DMF (3 mL) was added 1-(2-methylpyridin-3-yl)-7-(trifluoromethyl)-5-vinylpyrido[2,3-d]pyrimidine-2,4-(1*H*,3*H*)-dione (100 mg, 0.287 mmol), followed by the addition of methylamine hydrochloride (58.16mg, 0.861mmol), PyBop (448.26 mg, 0.861mmol) and DBU (219 mg, 1.44 mmol) successively. The reaction solution was stirred at 20°C for 16 hours. LC-MS showed that the main peak was the product peak. The reaction solution was purified by prepliquid phase chromatography to afford the product (11.4 mg,11% yield). ESI-MS (m/z): 362.1[M+H]$^+$. $^1$H NMR (400 MHz, DMSO-$d_6$) δ = 8.52 - 8.47 (m, 1H), 7.65 - 7.55 (m, 2H), 7.40 - 7.33 (m, 1H), 3.85 - 3.76 (m, 2H), 3.28 - 3.19 (m, 5H), 2.14 (s, 3H).

Two separate atropisomers C1-P1 and C1-P2 were obtained following the resolution method of A16. C1-P1, ESI-MS (m/z): 362.1[M+H]$^+$. $^1$H NMR (400 MHz, DMSO-$d_6$) δ = 8.52 - 8.47 (m, 1H), 7.65 - 7.55 (m, 2H), 7.40 - 7.33 (m, 1H), 3.85 - 3.76 (m, 2H), 3.28 - 3.19 (m, 5H), 2.14 (s, 3H). C1-P2, ESI-MS (m/z): 362.1[M+H]$^+$. $^1$H NMR (400 MHz, DMSO-$d_6$) δ = 8.52 - 8.47 (m, 1H), 7.65 - 7.55 (m, 2H), 7.40 - 7.33 (m, 1H), 3.85 - 3.76 (m, 2H), 3.28 - 3.19 (m, 5H), 2.14 (s, 3H).

Compounds B4-B72 and C2-C23 were prepared according to the similar synthetic methods and using the corresponding intermediates as the starting materials.

| Example No. | General Method | Main Starting Material(s) | Compound Structure and Compound No. | Characterization Data |
|---|---|---|---|---|
| 45 | Method Two | 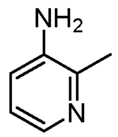 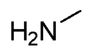 | 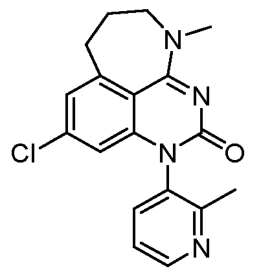 B4 | ESI-MS (m/z) : 341.1[M+H]$^+$ |
| 46 | Method Two | 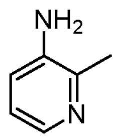 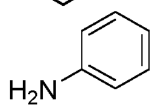 | 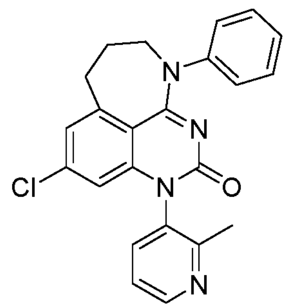 B5 | ESI-MS (m/z) : 403.1[M+H]$^+$ |

(continued)

| Example No. | General Method | Main Starting Material(s) | Compound Structure and Compound No. | Characterization Data |
|---|---|---|---|---|
| 47 | Method Two | 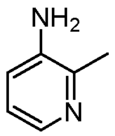 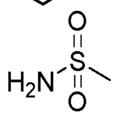 | 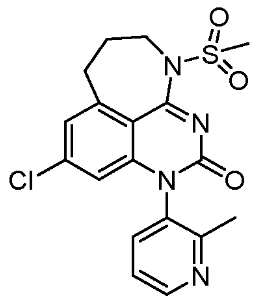 B6 | ESI-MS (m/z) : 405.1[M+H]+ |
| 48 | Method Two | 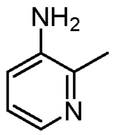 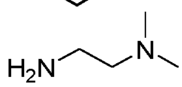 | 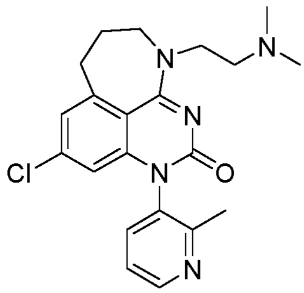 B7 | ESI-MS (m/z) : 398.2[M+H]+ |
| 49 | Method Two | 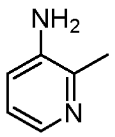 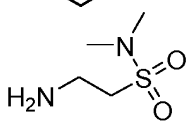 | 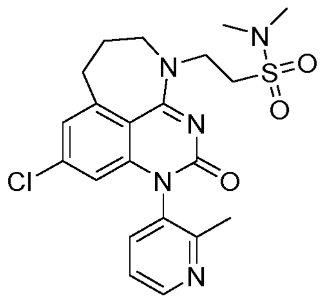 B8 | ESI-MS (m/z) : 462.1[M+H]+ |
| 50 | Method Two | 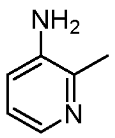 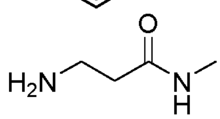 | 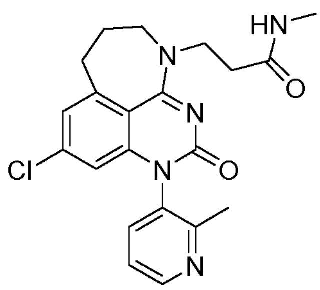 B9 | ESI-MS (m/z) : 412.1[M+H]+ |

(continued)

| Example No. | General Method | Main Starting Material(s) | Compound Structure and Compound No. | Characterization Data |
|---|---|---|---|---|
| 51 | Method Two | 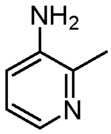 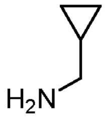 | 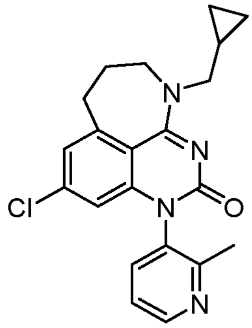 **B10** | ESI-MS (m/z) : 381.1[M+H]+ |
| 52 | Method Two | 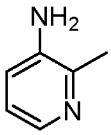 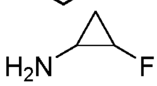 | 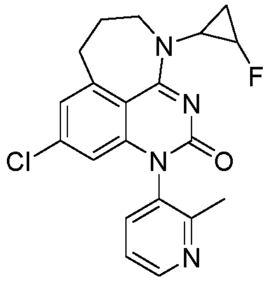 **B11** | ESI-MS (m/z) : 385.1[M+H]+ |
| 53 | Method Two | 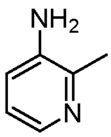 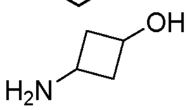 | 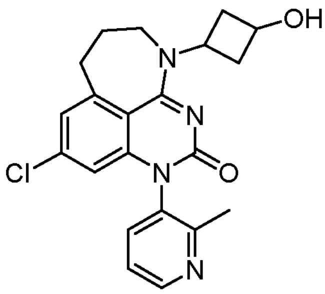 **B12** | ESI-MS (m/z) : 383.1[M+H]+ |
| 54 | Method Two | 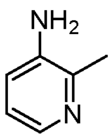 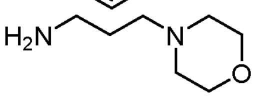 | 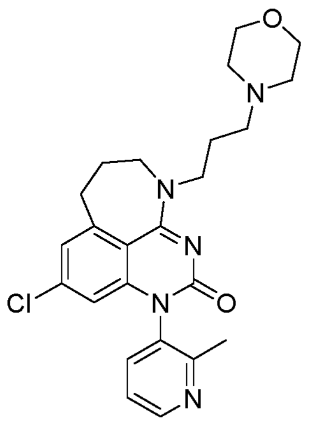 **B13** | ESI-MS (m/z) : 454.2[M+H]+ |

(continued)

| Example No. | General Method | Main Starting Material(s) | Compound Structure and Compound No. | Characterization Data |
|---|---|---|---|---|
| 55 | Method Two | 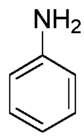 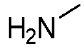 | 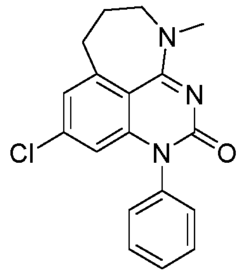 **B14** | ESI-MS (m/z) : 326.1[M+H]$^+$ |
| 56 | Method Two | 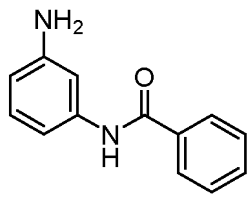 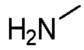 | 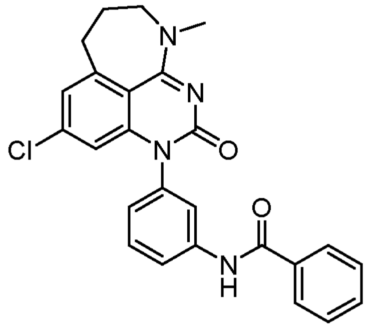 **B15** | ESI-MS (m/z) : 445.1[M+H]$^+$ |
| 57 | Method Two | 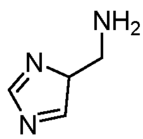 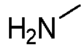 | 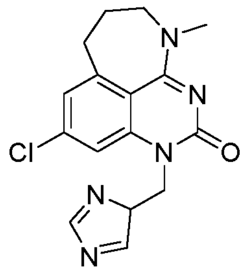 **B16** | ESI-MS (m/z) : 330.1[M+H]$^+$ |
| 58 | Method Two | 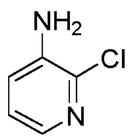 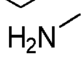 | 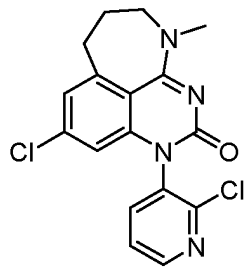 **B17** | ESI-MS (m/z) : 361.1[M+H]$^+$ |

(continued)

| Example No. | General Method | Main Starting Material(s) | Compound Structure and Compound No. | Characterization Data |
|---|---|---|---|---|
| 59 | Method Two | 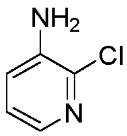 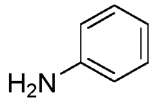 | 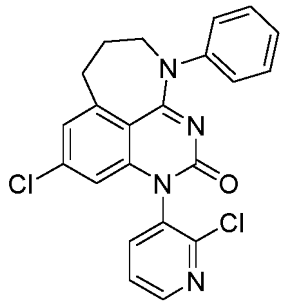 **B18** | ESI-MS (m/z) : 423.1[M+H]+ |
| 60 | Method Two | 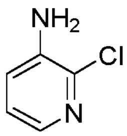 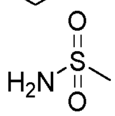 | 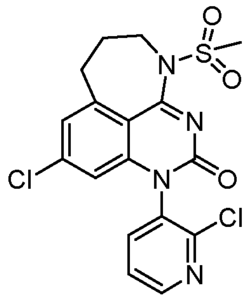 **B19** | ESI-MS (m/z) : 425.1[M+H]+ |
| 61 | Method Two | 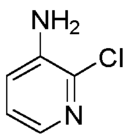 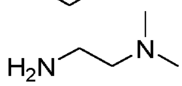 | 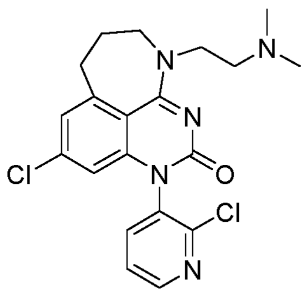 **B20** | ESI-MS (m/z) : 418.1[M+H]+ |
| 62 | Method Two | 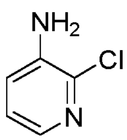 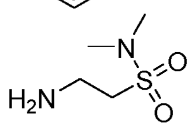 | 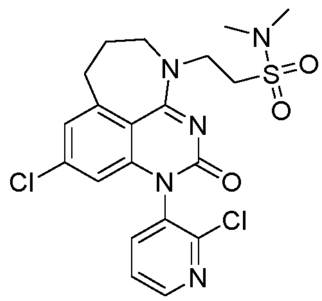 **B21** | ESI-MS (m/z) : 482.1[M+H]+ |

(continued)

| Example No. | General Method | Main Starting Material(s) | Compound Structure and Compound No. | Characterization Data |
|---|---|---|---|---|
| 63 | Method Two | 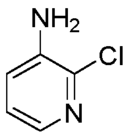 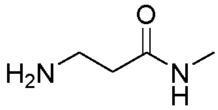 | 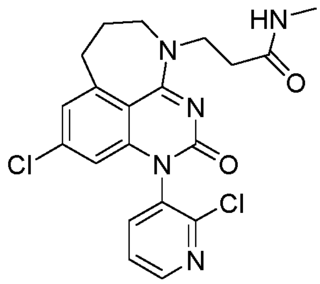 B22 | ESI-MS (m/z) : 432.1[M+H]$^+$ |
| 64 | Method Two | 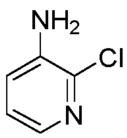 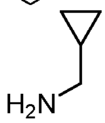 | 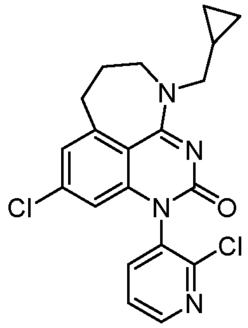 B23 | ESI-MS (m/z) : 401.1[M+H]$^+$ |
| 65 | Method Two | 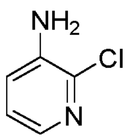 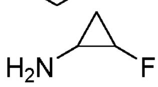 | 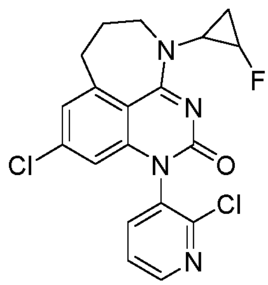 B24 | ESI-MS (m/z) : 405.1[M+H]$^+$ |
| 66 | Method Two | 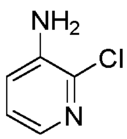 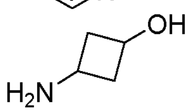 | 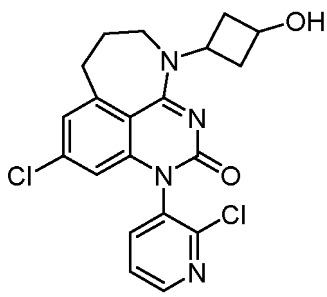 B25 | ESI-MS (m/z) : 417.1[M+H]$^+$ |

(continued)

| Example No. | General Method | Main Starting Material(s) | Compound Structure and Compound No. | Characterization Data |
|---|---|---|---|---|
| 67 | Method Two | 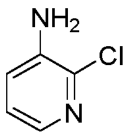 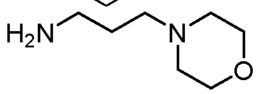 | 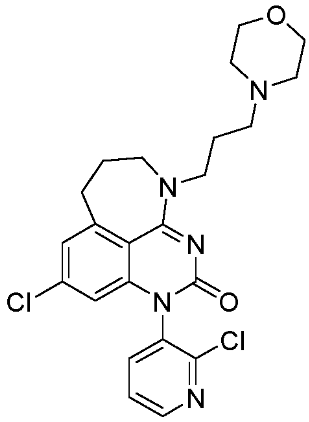 B26 | ESI-MS (m/z) : 474.1[M+H]+ |
| 68 | Method Two | 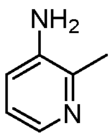 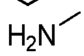 | 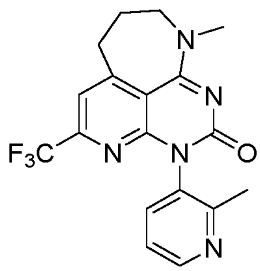 B27 | ESI-MS (m/z) : 376.1[M+H]+ |
| 69 | Method Two | 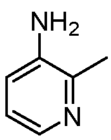 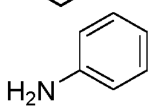 | 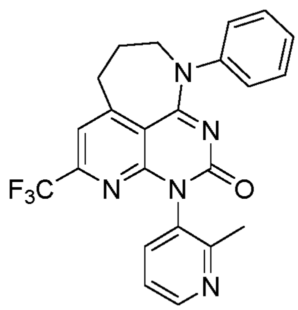 B28 | ESI-MS (m/z) : 438.1[M+H]+ |
| 70 | Method Two | 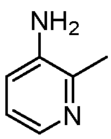 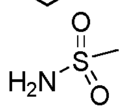 | 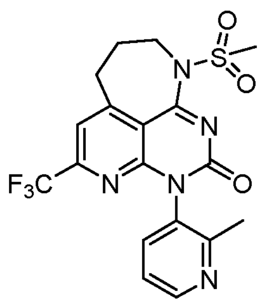 B29 | ESI-MS (m/z) : 440.1[M+H]+ |

(continued)

| Example No. | General Method | Main Starting Material(s) | Compound Structure and Compound No. | Characterization Data |
|---|---|---|---|---|
| 71 | Method Two | 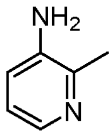 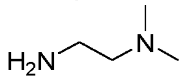 | 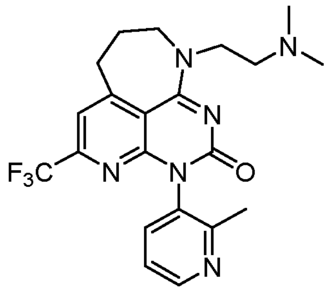 B30 | ESI-MS (m/z) : 433.2[M+H]+ |
| 72 | Method Two | 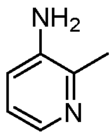 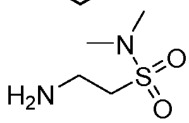 | 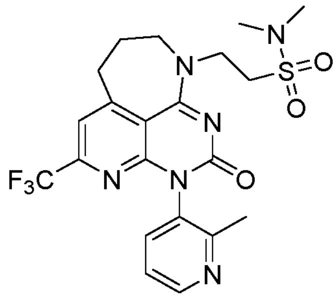 B31 | ESI-MS (m/z) : 497.2[M+H]+ |
| 73 | Method Two | 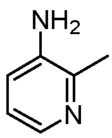 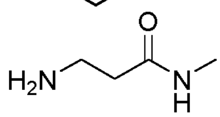 | 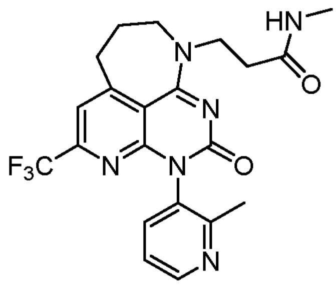 B32 | ESI-MS (m/z) : 447.2[M+H]+ |
| 74 | Method Two | 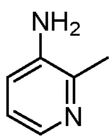 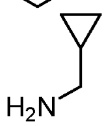 | 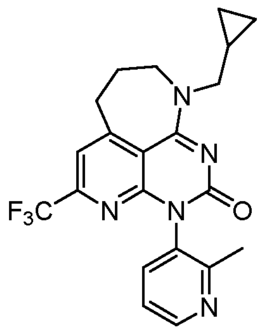 B33 | ESI-MS (m/z) : 416.2[M+H]+ |

(continued)

| Example No. | General Method | Main Starting Material(s) | Compound Structure and Compound No. | Characterization Data |
|---|---|---|---|---|
| 75 | Method Two | 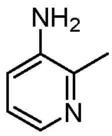 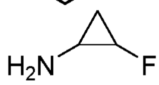 | 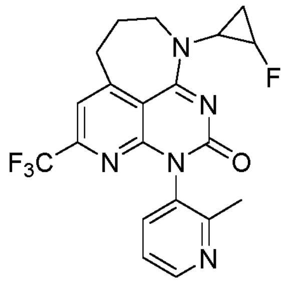 B34 | ESI-MS (m/z) : 420.1[M+H]$^+$ |
| 76 | Method Two | 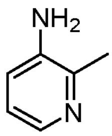 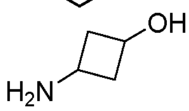 | 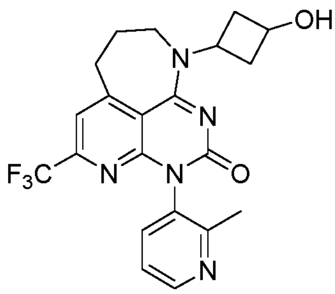 B35 | ESI-MS (m/z) : 432.1[M+H]$^+$ |
| 77 | Method Two | 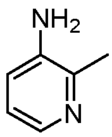 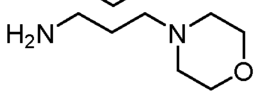 | 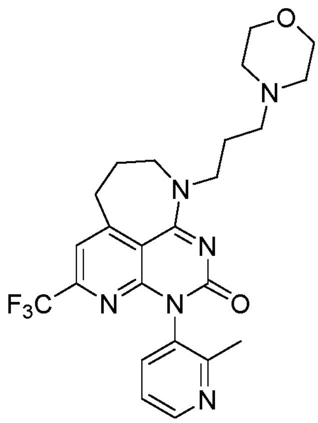 B36 | ESI-MS (m/z) : 489.2[M+H]$^+$ |
| 78 | Method Two | 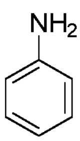 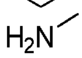 | 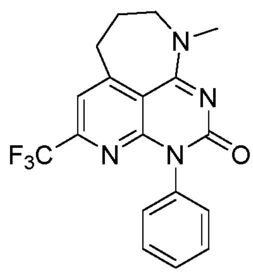 B37 | ESI-MS (m/z) : 361.1[M+H]$^+$ |

(continued)

| Example No. | General Method | Main Starting Material(s) | Compound Structure and Compound No. | Characterization Data |
|---|---|---|---|---|
| 79 | Method Two | 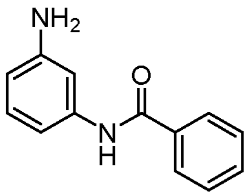 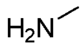 | 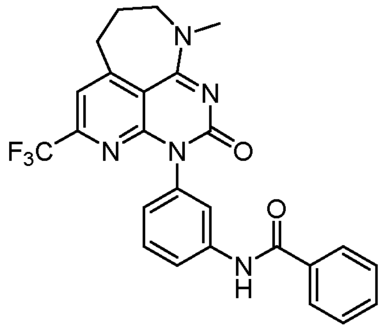 **B38** | ESI-MS (m/z) : $480.1[M+H]^+$ |
| 80 | Method Two | 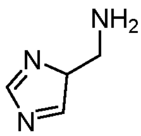 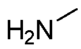 | 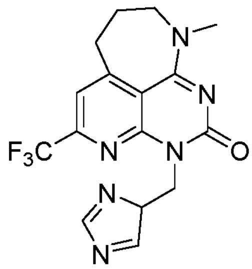 **B39** | ESI-MS (m/z) : $365.1[M+H]^+$ |
| 81 | Method Two | 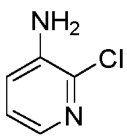 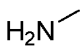 | 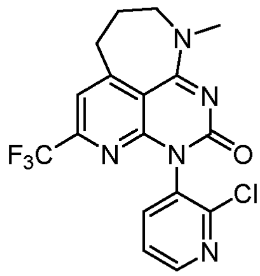 **B40** | ESI-MS (m/z) : $396.1[M+H]^+$ |
| 82 | Method Two | 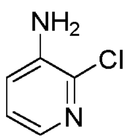 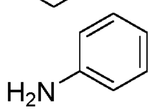 | 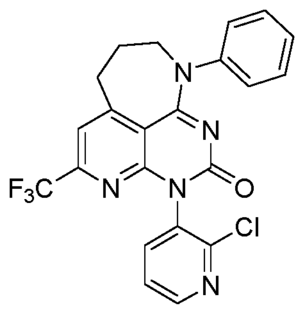 **B41** | ESI-MS (m/z) : $458.1[M+H]^+$ |

(continued)

| Example No. | General Method | Main Starting Material(s) | Compound Structure and Compound No. | Characterization Data |
|---|---|---|---|---|
| 83 | Method Two | 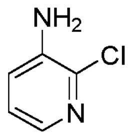 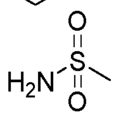 | 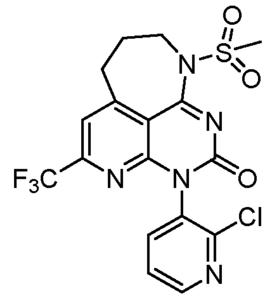 B42 | ESI-MS (m/z) : 460.1[M+H]+ |
| 84 | Method Two | 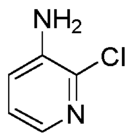 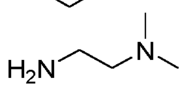 | 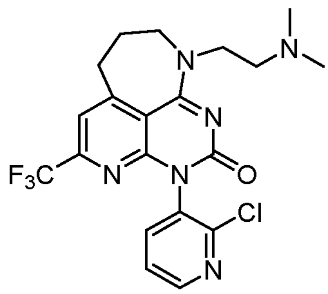 B43 | ESI-MS (m/z) : 453.1[M+H]+ |
| 85 | Method Two | 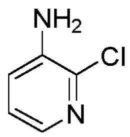 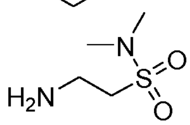 | 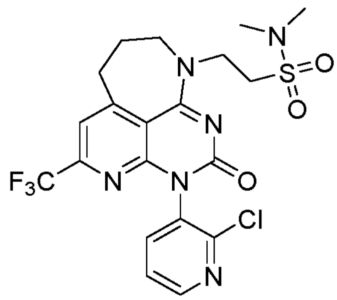 B44 | ESI-MS (m/z) : 517.1[M+H]+ |
| 86 | Method Two | 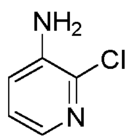 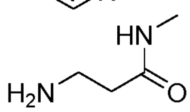 | 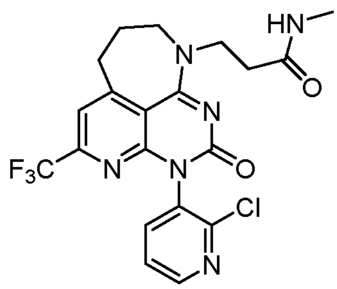 B45 | ESI-MS (m/z) : 467.1[M+H]+ |

(continued)

| Example No. | General Method | Main Starting Material(s) | Compound Structure and Compound No. | Characterization Data |
|---|---|---|---|---|
| 87 | Method Two | 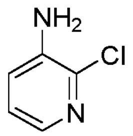 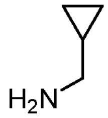 | 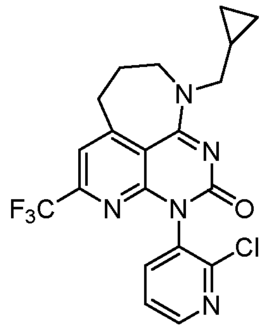 B46 | ESI-MS (m/z) : 436.1[M+H]$^+$ |
| 88 | Method Two | 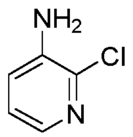 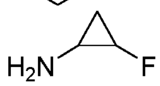 | 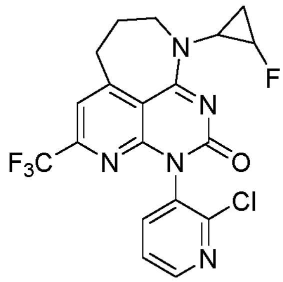 B47 | ESI-MS (m/z) : 440.1[M+H]$^+$ |
| 89 | Method Two | 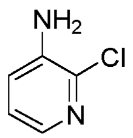 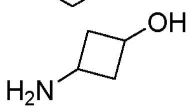 | 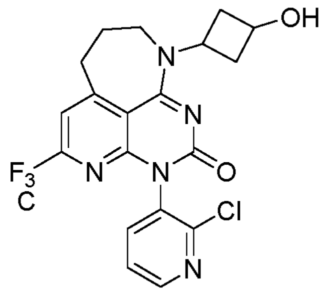 B48 | ESI-MS (m/z) : 452.1[M+H]$^+$ |
| 90 | Method Two | 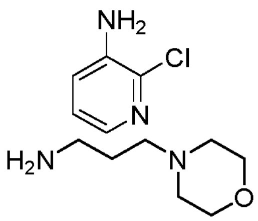 | 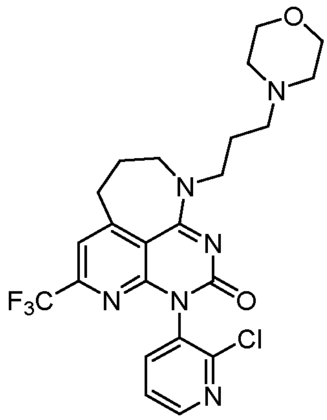 B49 | ESI-MS (m/z) : 509.1[M+H]$^+$ |

(continued)

| Example No. | General Method | Main Starting Material(s) | Compound Structure and Compound No. | Characterization Data |
|---|---|---|---|---|
| 91 | Method Three | 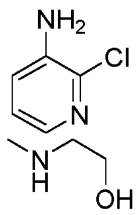 | 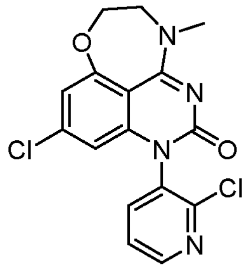 B50 | ESI-MS (m/z) : 363.1[M+H]+ |
| 92 | Method Three | 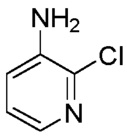 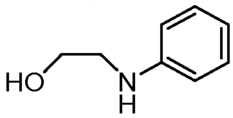 | 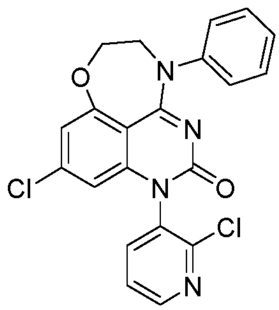 B51 | ESI-MS (m/z) : 425.1[M+H]+ |
| 93 | Method Three | 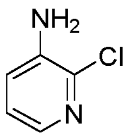 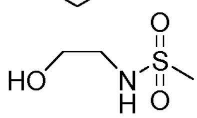 | 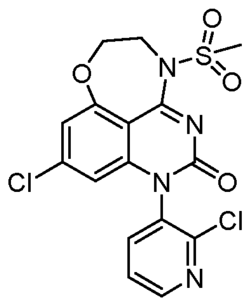 B52 | ESI-MS (m/z) : 427.1[M+H]+ |
| 94 | Method Three | 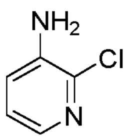 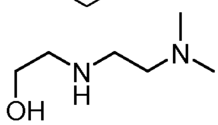 | 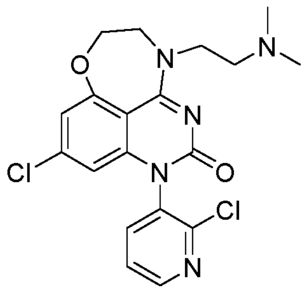 B53 | ESI-MS (m/z) : 420.1[M+H]+ |

(continued)

| Example No. | General Method | Main Starting Material(s) | Compound Structure and Compound No. | Characterization Data |
| --- | --- | --- | --- | --- |
| 95 | Method Three | 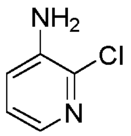 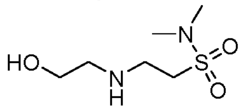 | 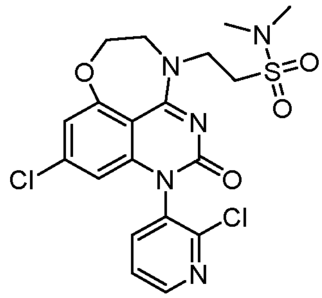 B54 | ESI-MS (m/z) : 484.1[M+H]$^+$ |
| 96 | Method Three | 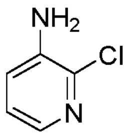 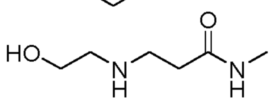 | 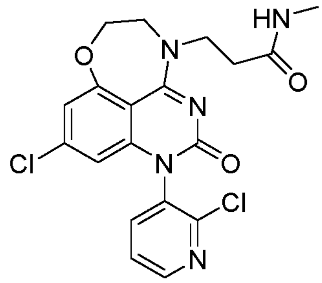 B55 | ESI-MS (m/z) : 434.1[M+H]$^+$ |
| 97 | Method Three | 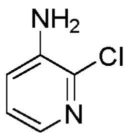 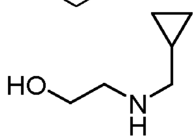 | 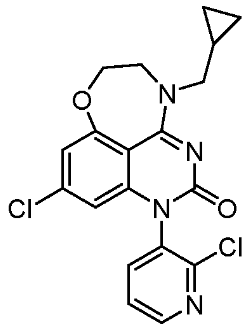 B56 | ESI-MS (m/z) : 403.1[M+H]$^+$ |
| 98 | Method Three | 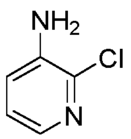 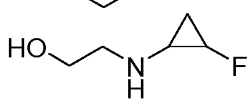 | 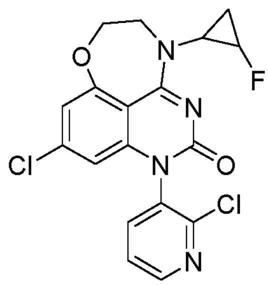 B57 | ESI-MS (m/z) : 407.1[M+H]$^+$ |

(continued)

| Example No. | General Method | Main Starting Material(s) | Compound Structure and Compound No. | Characterization Data |
|---|---|---|---|---|
| 99 | Method Three | 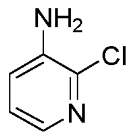 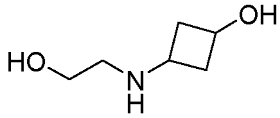 | 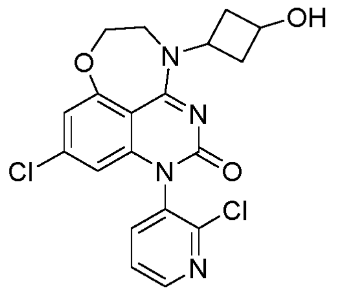 B58 | ESI-MS (m/z) : 419.1[M+H]+ |
| 100 | Method Three | 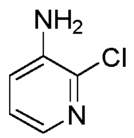 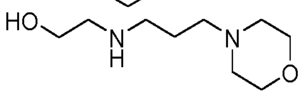 | 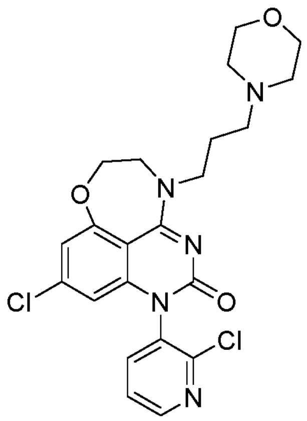 B59 | ESI-MS (m/z) : 476.1[M+H]+ |
| 101 | Method Three | 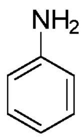 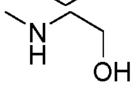 | 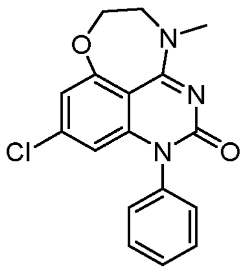 B60 | ESI-MS (m/z) : 328.1[M+H]+ |
| 102 | Method Three | 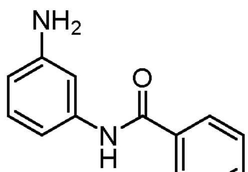 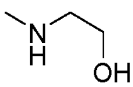 | 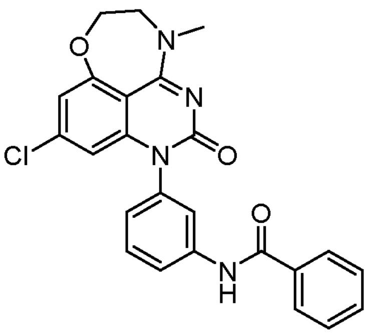 B61 | ESI-MS (m/z) : 447.0[M+H]+ |

(continued)

| Example No. | General Method | Main Starting Material(s) | Compound Structure and Compound No. | Characterization Data |
|---|---|---|---|---|
| 103 | Method Three | 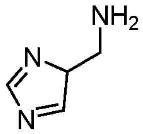 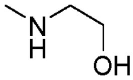 | 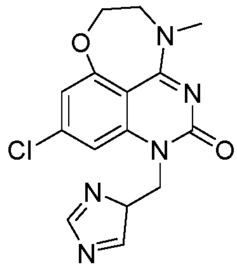 B62 | ESI-MS (m/z) : 332.1[M+H]+ |
| 104 | Method Three | 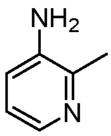 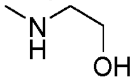 | 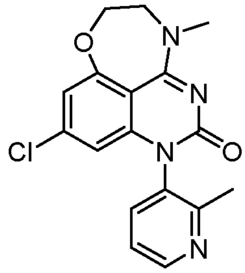 B63 | ESI-MS (m/z) : 343.1[M+H]+ |
| 105 | Method Three | 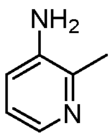 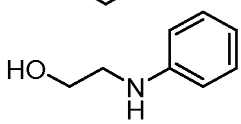 | 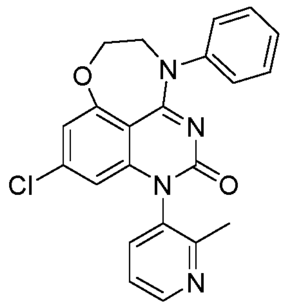 B64 | ESI-MS (m/z) : 405.1[M+H]+ |
| 106 | Method Three | 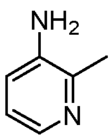 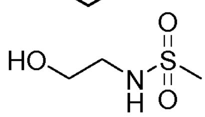 | 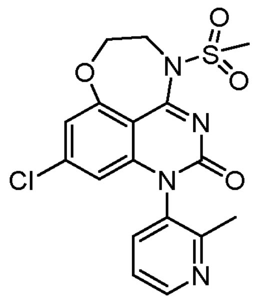 B65 | ESI-MS (m/z) : 407.1[M+H]+ |

(continued)

| Example No. | General Method | Main Starting Material(s) | Compound Structure and Compound No. | Characterization Data |
|---|---|---|---|---|
| 107 | Method Three | 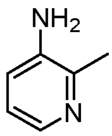 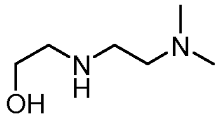 | 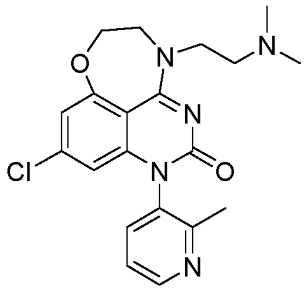 B66 | ESI-MS (m/z) : 400.1[M+H]+ |
| 108 | Method Three | 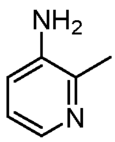 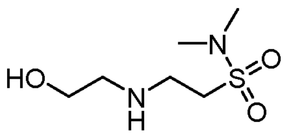 | 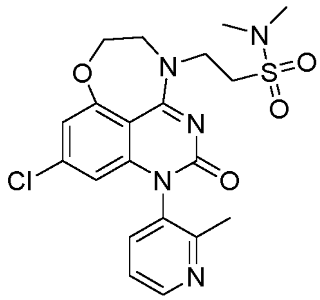 B67 | ESI-MS (m/z) : 464.1[M+H]+ |
| 109 | Method Three | 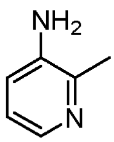 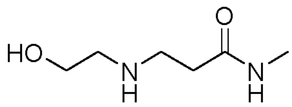 | 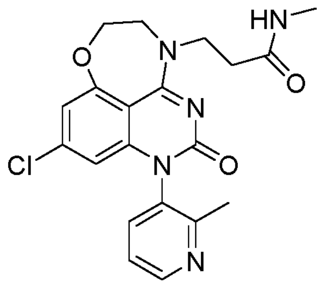 B68 | ESI-MS (m/z) : 414.1[M+H]+ |
| 110 | Method Three | 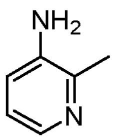 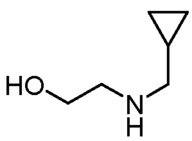 | 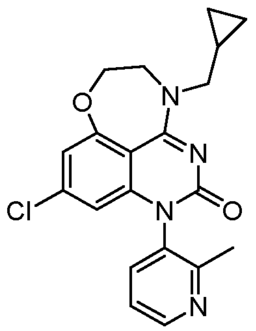 B69 | ESI-MS (m/z) : 383.1[M+H]+ |

(continued)

| Example No. | General Method | Main Starting Material(s) | Compound Structure and Compound No. | Characterization Data |
|---|---|---|---|---|
| 111 | Method Three | 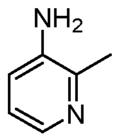 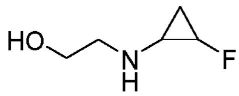 | 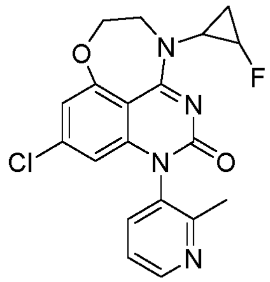 B70 | ESI-MS (m/z) : 387.1[M+H]+ |
| 112 | Method Three | 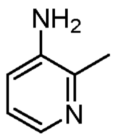 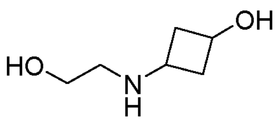 | 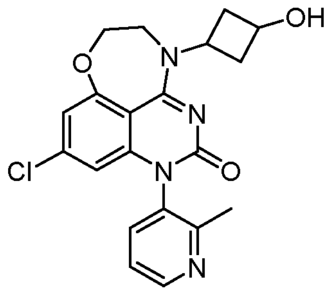 B71 | ESI-MS (m/z) : 399.1[M+H]+ |
| 113 | Method Three | 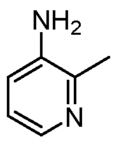 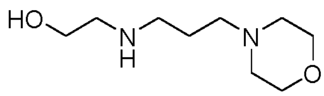 | 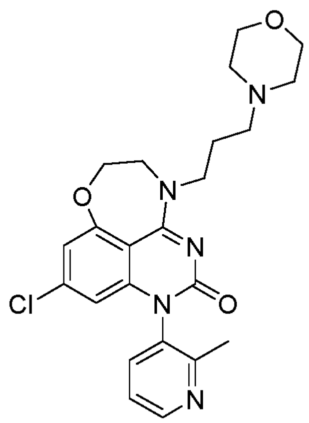 B72 | ESI-MS (m/z) : 456.2[M+H]+ |
| 114 | Method Seven | 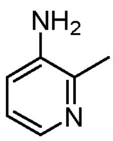 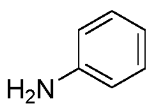 | 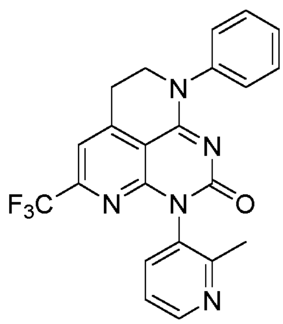 C2 | ESI-MS (m/z) : 424.1[M+H]+ |

(continued)

| Example No. | General Method | Main Starting Material(s) | Compound Structure and Compound No. | Characterization Data |
|---|---|---|---|---|
| 115 | Method Seven | 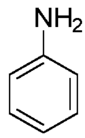 $NH_2$ | 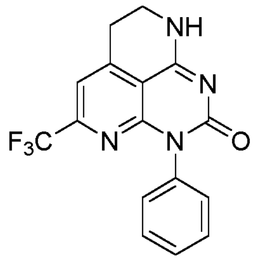 C3 | ESI-MS (m/z) : 333.1[M+H]$^+$ |
| 116 | Method Seven | 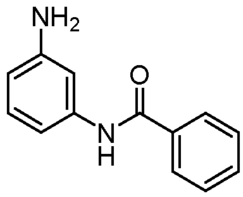 $NH_2$ | 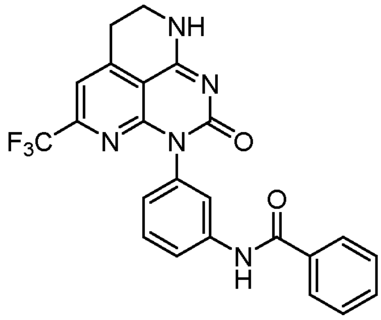 C4 | ESI-MS (m/z) : 452.4[M+H]$^+$ |
| 117 | Method Seven | 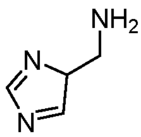 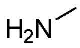 | 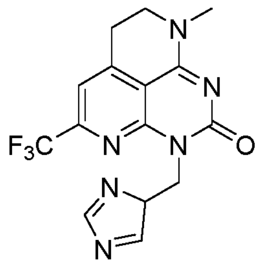 C5 | ESI-MS (m/z) : 351.1[M+H]$^+$ |
| 118 | Method Seven | 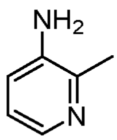 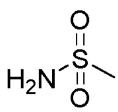 | 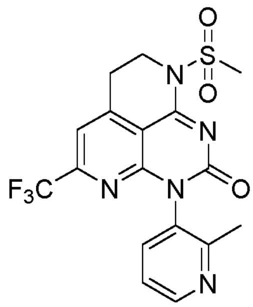 C6 | ESI-MS (m/z) : 426.1[M+H]$^+$ |

(continued)

| Example No. | General Method | Main Starting Material(s) | Compound Structure and Compound No. | Characterization Data |
|---|---|---|---|---|
| 119 | Method Seven | 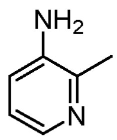 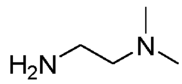 | 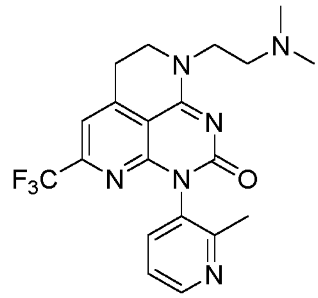 C7 | ESI-MS (m/z) : 419.2[M+H]+ |
| 120 | Method Seven | 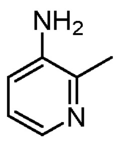 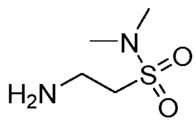 | 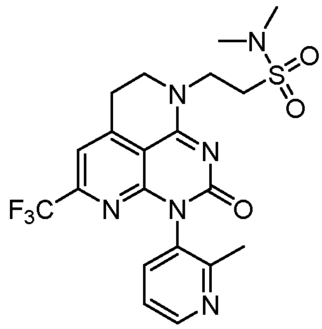 C8 | ESI-MS (m/z) : 483.1[M+H]+ |
| 121 | Method Seven | 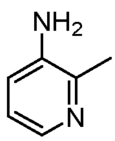 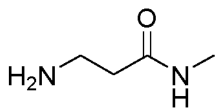 | 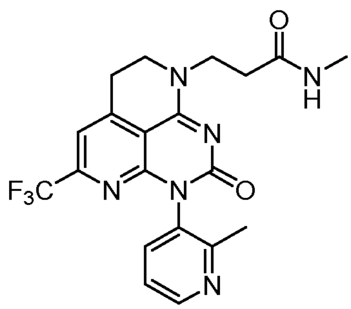 C9 | ESI-MS (m/z) : 433.2[M+H]+ |
| 122 | Method Seven | 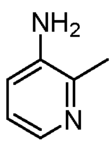 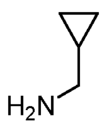 | 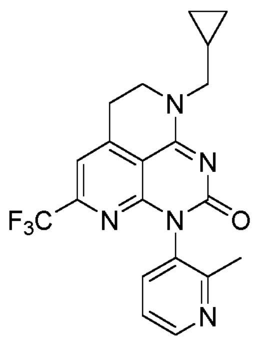 C10 | ESI-MS (m/z) : 402.2[M+H]+ |

(continued)

| Example No. | General Method | Main Starting Material(s) | Compound Structure and Compound No. | Characterization Data |
|---|---|---|---|---|
| 123 | Method Seven | 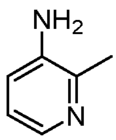 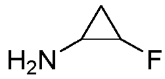 | 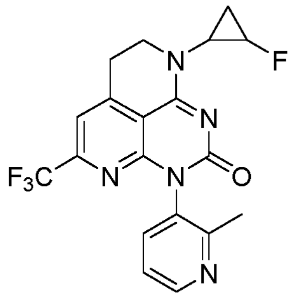 C11 | ESI-MS (m/z) : 406.1[M+H]+ |
| 124 | Method Seven | 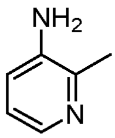 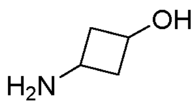 | 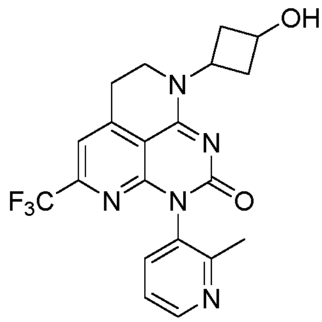 C12 | ESI-MS (m/z) : 418.1[M+H]+ |
| 125 | Method Seven | 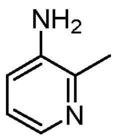 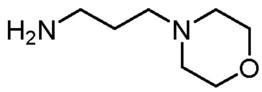 | 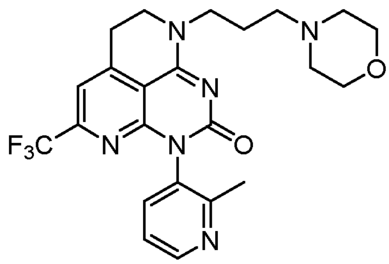 C13 | ESI-MS (m/z) : 475.2[M+H]+ |
| 126 | Method Seven | 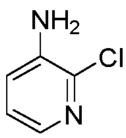 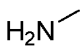 | 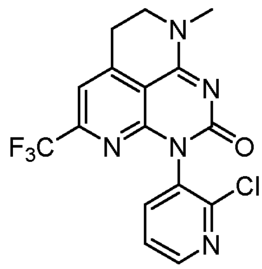 C14 | ESI-MS (m/z) : 382.1[M+H]+ |

(continued)

| Example No. | General Method | Main Starting Material(s) | Compound Structure and Compound No. | Characterization Data |
|---|---|---|---|---|
| 127 | Method Seven | 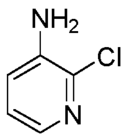 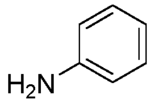 | 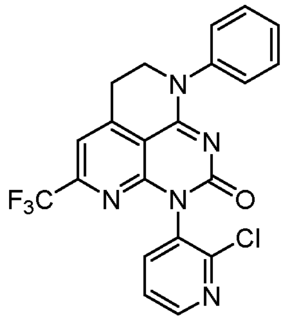 C15 | ESI-MS (m/z) : 444.1[M+H]+ |
| 128 | Method Seven | 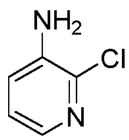 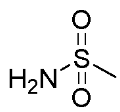 | 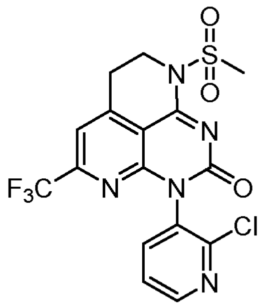 C16 | ESI-MS (m/z) : 446.1[M+H]+ |
| 129 | Method Seven | 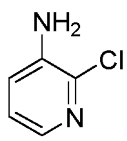 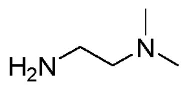 | 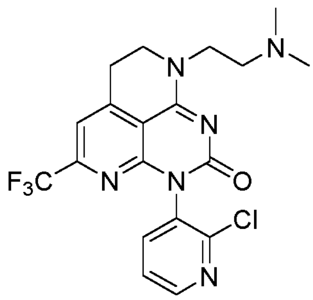 C17 | ESI-MS (m/z) : 439.1[M+H]+ |
| 130 | Method Seven | 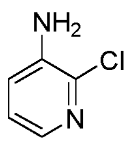 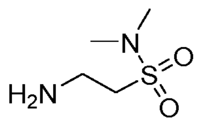 | 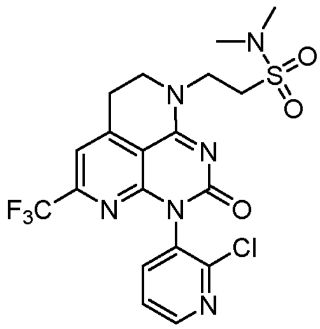 C18 | ESI-MS (m/z) : 503.1[M+H]+ |

(continued)

| Example No. | General Method | Main Starting Material(s) | Compound Structure and Compound No. | Characterization Data |
|---|---|---|---|---|
| 131 | Method Seven | 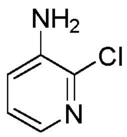 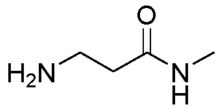 | 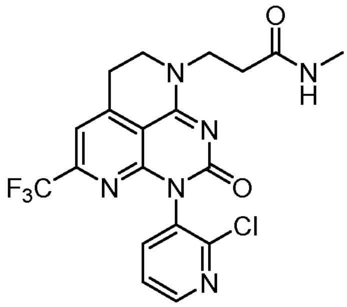 C19 | ESI-MS (m/z) : 453.1[M+H]+ |
| 132 | Method Seven | 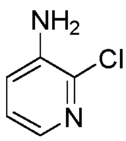 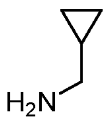 | 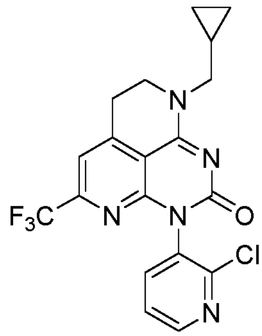 C20 | ESI-MS (m/z) : 422.1[M+H]+ |
| 133 | Method Seven | 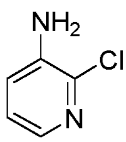 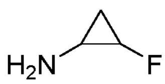 | 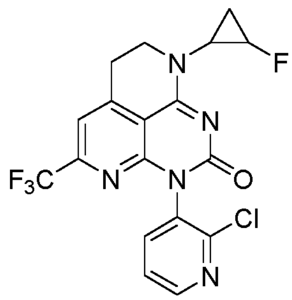 C21 | ESI-MS (m/z) : 426.1[M+H]+ |
| 134 | Method Seven | 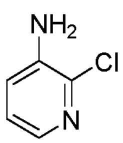 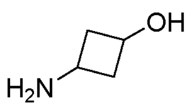 | 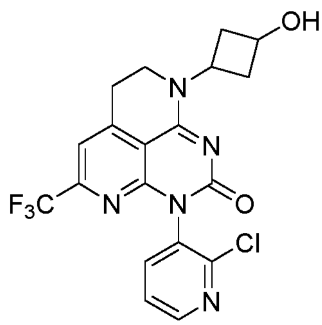 C22 | ESI-MS (m/z) : 438.1[M+H]+ |

(continued)

| Example No. | General Method | Main Starting Material(s) | Compound Structure and Compound No. | Characterization Data |
|---|---|---|---|---|
| 135 | Method Seven | 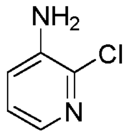 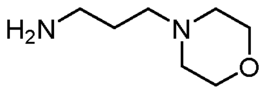 | 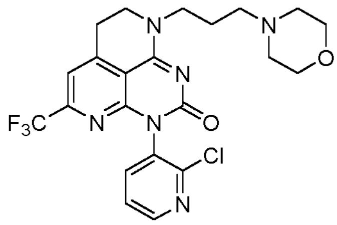 C23 | ESI-MS (m/z) : 495.1[M+H]+ |

The efficacy tests and data of the compounds of the present application are shown below.

**Test Example 1: Enzymatic Assay**

The inhibitory effect of the test compounds on the enzymatic activity of MAT2A was determined using MAT2A Inhibitor Enzymatic Screening Kit (BPS Bioscience).

1) Test compounds were dissolved in DMSO and mixed well until the test compounds were completely dissolved. All compounds were diluted with DMSO to a starting concentration of 2 mM, and diluted at a 3-fold ratio to a total of 10 concentration gradients. Duplicate wells were set. The compounds were added to the reaction system at a ratio of 1: 100 (20 μM of the maximum final concentration) during detection. 100× positive control (1 mM AGI-24512) and 100× negative control (100% DMSO) were prepared.
2) 200 nL of each of the diluted compounds were transferred to each well of the reaction plate (Coming3702) using an Echo 550 pipetting system, and the reaction plate was sealed with a sealing film, and centrifuged at 1000 g for 1 minute. The final concentration of DMSO was 1%.
3) Preparation of 1× MAT2A enzyme reaction buffer: 1× volume of 5× MAT2A enzyme reaction buffer was mixed well with 4× volumes of water.
4) 2×MAT2A enzyme solution was prepared with 1×MAT2A enzyme reaction buffer.
5) A 10 μL of 2×MAT2A enzyme solution was added to each well of the 384- reaction plate (Coming3702), and the plate was sealed with a sealing film.
6) The plate was centrifuged at 1000g for 60 seconds and incubated at room temperature for 30 minutes.
7) A mixture of 2× L-Methionine and ATP was prepared with 1×MAT2A enzyme reaction buffer.
8) 10 μL of the mixture of 2× L-Methionine and ATP was added to each well of the 384-reaction plate (Coming3702), and the plate was sealed with a sealing film. The total reaction system was 20 μL.
9) The plate was centrifuged at 1000g for 60 seconds and incubated at room temperature for 60 minutes.
10) The assay buffer Colorimetric Detection Rebgent was prepared.
11) 20 μL of the assay buffer was added to each well. The plate was centrifuged at 1000g for 30 seconds and reacted at room temperature for 15 minutes.
12) Fluorescence signal at 630 nm was detected by a multifunctional microplate reader (PerkinElmer, Nivo).

Experimental results showed that the compounds of the present application had a strong MAT2A inhibitory effect. Exemplary compounds were shown in the following table:

| Compound No. | Inhibitory activity against Enzyme ($IC_{50}$: nM) | Compound No. | Inhibitory activity against Enzyme ($IC_{50}$: nM) |
|---|---|---|---|
| A1 | A | A2 | A |
| B3 | B | A3 | A |
| A16 | A | A38 | A |
| A39 | A | A40 | B |
| C1 | A | A19 | C |
| A16-P2 | A | B1 | C |

(continued)

| Compound No. | Inhibitory activity against Enzyme ($IC_{50}$: nM) | Compound No. | Inhibitory activity against Enzyme ($IC_{50}$: nM) |
|---|---|---|---|
| B2 | C | A29 | C |

A represented $IC_{50} \leq 50$ nM, B represented 50 nM < $IC_{50} \leq 100$ nM, and C represented $IC_{50}$>100 nM.

**Test Example 2. Test for Inhibition on Cell Proliferation**

The specific operation procedures were as follows. Tumor cells were treated with test compounds for 5 days, and the effect of the test compounds on the proliferation of tumor cells was evaluated. HCT116-$MTAP^{-/-}$ and wild-type control HCT116-WT (colorectal cancer cells) cells were seeded in a 384-well plate at a density of 600 cells/well, and at the same time, different concentrations of the test compounds (starting from 20 μM, 10 concentration gradients) were added. The cells were incubated at 37°C, 5% $CO_2$, and saturated humidity for 5 days.

Cell proliferation was detected using an ATP-based Cell Proliferation Assay Kit (Cell Titer Glo, Promega Corporation). Cells were equilibrated at room temperature for 30 minutes and then treated with Cell Titer Glo reagent. The plate was then covered with aluminum foil and shaken for 15 minutes to allow mixing well and lysis. Chemiluminescence detection was performed using a multifunctional microplate reader (Envision 2105, PerkinElmer). A blank well (blank, cell-free) and a DMSO control well were set.

The inhibition rate (IR) of the test compounds was calculated using the following formula:

IR(%)=[1-(RLU compound -RLU blank control)/(RLU vehicle control-RLU blank control)] ×100%

GraphPad Prism was used for graphing, data analysis and $IC_{50}$ calculations.

The compounds of the present application were tested in the above assay and their $IC_{50}$ values for inhibiting cell proliferation were determined, as shown in the following table.

| Compound | HCT116 $MTAP^{-/-}$ cell proliferation inhibitory activity $IC_{50}$: nM | HCT116 $MTAP^{+/+}$ cell proliferation inhibitory activity $IC_{50}$: nM |
|---|---|---|
| **A1** | 199 | >20 000 |
| **A2** | 45 | >20 000 |
| **A16** | 112 | >20 000 |
| **A38** | 20 | >20 000 |
| **A39** | 308 | 1351 |

The results showed that the test compounds exhibited strong cell proliferation inhibitory activity on HCT116 MTAP-knockout cells and weak cell proliferation inhibitory activity on MTAP wild-type HCT116 cells. That is, the test compounds exhibited good selectivity.

Note: when the maximum inhibition rate was <50%, the result was expressed as $IC_{50}$ > the maximum initial concentration.

**Test Example 3. Human UGT1A1 Enzymatic Activity Assay**

The enzymatic inhibitory activity of the compounds against UGT1A1 was determined, and the specific operation procedures were as follows.

1) 1 μL of 2 mM control compound (atazanavir), 1 μL of 440 μM of each test compound, or 1 μL of dimethyl sulfoxide (solvent control) was added to a culture plate, and a master reaction solution containing a final concentration of 0.01 mg/mL UGT1A1 (Corning, 456411), 0.5 μM substrate bilirubin, and Tris buffer was added. The plate was pre-incubated in a 37°C water bath for 10 minutes. The final concentration of the control compound was 10 μM, and the final concentration of the test compound was 2.2 μM.
2) The reaction was initiated by the addition of 20 μL of UDPGA solution at a final concentration of 2 mM, carried out at 37°C for 5 minutes, and then stopped by the addition of 400 μL of cold acetonitrile containing the internal standard.

3) The sample was vortexed for 5 minutes, and centrifuged at 3220 g for 40 minutes at 4°C. 100 μL of the supernatant was then transferred to a new 96-well plate, and analyzed for metabolite formation by LC-MS/MS. The % inhibition rate was calculated by comparing the reduction amount (which was quantified through peak area) in metabolite formation between the test compound and the control.

UGT1A1 is involved in the metabolism of bilirubin in the body. When UGT1A1 is inhibited, bilirubin metabolism is blocked, eventually resulting in an increase in bilirubin in the body. The $IC_{50}$ values of the above compounds of the present application against UGT1A1 were greater than 50 μM, indicating that the compounds of the present application had a low risk of inhibiting UGT1A1.

**Text Example 4. Pharmacokinetic Study in Mice**

Laboratory animals:CD-1 mice (male, 22~25 g) were purchased from Beijing Vital River Laboratory Animal Technology Co., Ltd.

Test procedures:CD-1 mice (male, 22-25 g) were administered with the test compounds intravenously or intragastrically. The plasma samples of mice were collected at 5 min, 15 min, 0.5 h, 1 h, 2 h, 4 h, 8 h, and 24 h after administration. The concentrations of the compounds were detected by LC-MS/MS. The pharmacokinetic parameters, such as plasma clearance Cl, elimination half-life $T_{1/2}$, peak time $T_{max}$, peak concentration $C_{max}$, area under the concentrationtime curve AUC, apparent distribution volume Vss, and absolute bioavailability F, were investigated.

Determination method:A series of the desired working solution concentrations were obtained using acetonitrile/-water (1:1) as a stock solution to dilute the analyte. 10 μL of working solution (1 ng/mL, 2 ng/mL, 5 ng/mL, 10 ng/mL, 50 ng/mL, 100 ng/mL, 500 ng/mL, 1,000 ng/mL, 5,000 ng/mL and 10,000 ng/mL) was added to 10 μL of blank CD1 mouse plasma to achieve 1-10,000 ng/mL (1 ng/mL, 2 ng/mL, 5 ng/mL, 10 ng/mL, 50 ng/mL, 100 ng/mL, 500 ng/mL, 1,000 ng/mL, 5,000 ng/mL and 10,000 ng/mL) calibration standards with a total volume of 20 μL. Five quality control (QC) samples (2 ng/mL, 5 ng/mL, 10 ng/mL, 800 ng/mL, and 8,000 ng/ml) were prepared in the same manner as the calibration standards on the day of analysis. 20 μL of standard, 20 μL of QC sample, and 20 μL of unknown sample (10 μL of unknown sample plasma and 10 μL of blank solution) were added to 200 μL of IS mixture containing acetonitrile to precipitate protein, respectively. The samples were then shaken and vortexed for 3 minutes. After centrifugation at 4700 rpm for 15 minutes at 4°C, the supernatant was diluted with ultrapure water at a ratio of 1: 2 (V/V), and then 10 μL of the diluted supernatant was injected into LC/MS/MS system for quantitative analysis.

Pharmacokinetic results showed that the compounds of the present application had a high oral absolute bioavailability (more than 70% for the preferred compounds), a moderate clearance rate, and a good dose-exposure correlation.

**Test Example 5: hERG Test**

The potential inhibitory effect of the test compounds on the hERG channel was evaluated by an automatic patch clamp system. In this study, a CHO cell line stably expressing hERG gene was used and Cisapride was used as a positive control.

A CHO-hERG-DUO cell line stably expressing the hERG channel was purchased from B'SYS GmbH. Cells were cultured in a medium containing F12 (HAM) medium, 10%FBS, 100 U/mL penicillin-streptomycin, 100 μg/mL hygromycin, and 100 μg/mL G418. Cells were passaged three times a week and maintained at about 80% confluence.

The test compounds were dissolved in DMSO as a stock solution with a final concentration of 10 mM. Then, the stock solution (3.33 mM, 1.11 mM and 0.37 mM) was serially diluted with DMSO at a ratio of 1:3. The final concentrations of the compounds were 30 μM, 10 μM, 3.33 μM, 1.11 μM and 0.37 μM.

A baseline was established using the blank solvent. After the hERG current was stabilized for at least 5 minutes, the compound working solution was perfused. The hERG current was recorded for no less than 5 minutes to reach a steady state, and then 5 scans were captured. If the steady state was not reached within 10 minutes, then the average peak current of the last 5 scans would be used instead of the steady state value. The experiment was carried out twice (n = 2) and the hERG current inhibition of five concentrations of each compound was measured to determine the $IC_{50}$ value.

The current inhibition percentage was calculated using the following equation.

$$\text{Peak current inhibition} = \left(1 - \frac{\text{Peak tail current}_{\text{compound}} - \text{Peak tail current}_{\text{postive control}}}{\text{Peak tail current}_{\text{Blank vehicle}} - \text{Peak tail current}_{\text{postive control}}}\right) \times 100$$

The $IC_{50}$ value was calculated using Graphpad Prism 8.0.

The test results showed that the above-described preferred compounds of the present application had no

significant inhibitory effect on the hERG channel within the detection concentration range of this test, with $IC_{50}$ values greater than 30 μM, demonstrating that the compounds of the present application had a low risk of cardiotoxicity.

The embodiments of the present application have been described above. However, the present application is not limited to the above-described embodiments. Any modifications, equivalent substitutions, improvements or the like as made within the spirit and principle of the application should be included in the protection scope of the present application.

## Claims

1. A compound represented by formula (I), or a tautomer, a stereoisomer, or a pharmaceutically acceptable salt thereof, which has the following structures:

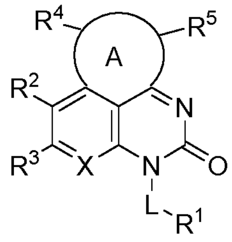

(I),

wherein,

ring A is selected from the group consisting of $C_{5-10}$ carbocyclyl, $C_{6-14}$ aryl, 5-12 membered heteroaryl, and 5-12 membered heterocyclyl;

$R^1$ is selected from the group consisting of optionally substituted $C_{3-12}$ carbocyclyl, optionally substituted $C_{6-14}$ aryl, optionally substituted 5-14 membered heteroaryl, and optionally substituted 5-14 membered heterocyclyl; wherein each of the carbocyclyl, the aryl, the heteroaryl, and the heterocyclyl is optionally substituted by one or more $R^a$;

L is selected from the group consisting of bond, -O-, -S-, -$C_{1-4}$ alkylene-, -$OC_{1-4}$ alkylene-, -C(O)-, -C(O)O-, -OC(O)-, -N($R^{a1}$)C(O)-, -C(O)N($R^{a1}$)- and -N($R^{a1}$)-; wherein the alkylene is optionally substituted by one or more $R^{a1}$;

$R^a$ is, at each occurrence, independently selected from the group consisting of deuterium, halogen, oxo, -CN, -OH, -SH, -$NO_2$, -$NH_2$, -W-$OR^{a1}$, -W-$SR^{a1}$, -W-C(O)$R^{a4}$, -W-C(O)$OR^{a1}$, -W-OC(O)$R^{a1}$, -W-OC(O)$OR^{a1}$, -W-C(O)$NR^{a2}R^{a3}$, -W-C(O)$NR^{a2}OR^{a1}$, -W-OC(O)$NR^{a2}R^{a3}$, -W-$NR^{a2}R^{a3}$, -W-$NR^{a2}$C(O)$R^{a4}$, -W-$NR^{a2}$C(O)$OR^{a1}$, -W-$NR^{a2}$C(O)$NR^{a2}R^{a3}$, -W-S(O)$R^{a4}$, -W-$S(O)_2R^{a4}$, -W-$SO_2NR^{a2}R^{a3}$, -W-$NR^{a2}S(O)_2R^{a4}$, -W-$OS(O)_2R^{a4}$, -W-$NR^{a2}S(O)_2NR^{a2}R^{a3}$, -W-$OS(O)_2NR^{a2}R^{a3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, $C_{6-14}$ aryl, 3-12 membered heterocyclyl, and 5-16 membered heteroaryl; wherein each of the alkyl, the alkenyl, the alkynyl, the alkoxy, the alkylthiol, the cycloalkyl, the cycloalkenyl, the aryl, the heterocyclyl, and the heteroaryl is optionally substituted by one or more substituents selected from the group consisting of deuterium, halogen, oxo, oxime, -CN, -OH, - $NO_2$, -$NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

$R^{a1}$ is, at each occurrence, independently hydrogen, deuterium, $C_{1-6}$ alkyl, $C_{1-6}$ alkoxy, $C_{3-10}$ cycloalkyl, $C_{6-14}$ aryl, 5-12 membered heteroaryl, or 3-20 membered heterocyclyl, wherein each of the alkyl, the alkoxy, the cycloalkyl, the aryl, the heteroaryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^{a2}$ and $R^{a3}$ are, at each occurrence, independently hydrogen, deuterium, $C_{1-6}$ alkyl, or $C_{1-6}$ alkoxy, wherein each of the alkyl and the alkoxy is optionally substituted by one or more substituents selected from the group consisting of halogen, deuterium, cyano, hydroxyl, amino, carboxyl, $C_{1-6}$ alkyl, $C_{1-6}$ alkoxy, $C_{1-6}$ alkyl, $C_{3-8}$ cycloalkyl, phenyl, $C_{1-6}$ haloalkyl, $C_{1-6}$ haloalkoxy, and halophenyl; or when $R^{a2}$ and $R^{a3}$ are attached to the same nitrogen atom, $R^{a2}$ and $R^{a3}$, together with the nitrogen atom to which they are attached, form 3-10 membered heterocycloalkyl or 5-12 membered heteroaryl, each of which is optionally substituted by one or more of halogen, hydroxyl, amino, nitro, sulfhydryl, cyano, oxo, $C_{1-6}$ alkyl, $C_{1-6}$ alkoxy, $C_{3-10}$ cycloalkyl, 3-10 membered heterocycloalkyl, $C_{6-14}$ aryl, and 5-12 membered heteroaryl;

$R^{a4}$ is, at each occurrence, independently hydrogen, deuterium, $C_{1-6}$ alkyl, $C_{1-6}$ alkoxy, $C_{3-8}$ cycloalkyl, $C_{6-14}$ aryl,

5-12 membered heteroaryl, or 3-20 membered heterocyclyl, wherein each of the alkyl, the alkoxy, the cycloalkyl, the aryl, the heteroaryl, and the heterocyclyl is optionally substituted by one or more substituents selected from the group consisting of halogen, deuterium, cyano, hydroxyl, amino, $C_{1-6}$ alkyl, $C_{1-6}$ alkoxy, phenyl, $C_{1-6}$ haloalkyl, $C_{1-6}$ haloalkoxy, and halophenyl;

X is N or $CR^6$;

$R^2$, $R^3$ and $R^6$ are each independently selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-OC(O)NR^{b2}R^{b3}$, $-W-NR^{b2}R^{b3}$, $-W-NR^{b2}C(O)R^{b4}$, $-W-NR^{b2}C(O)OR^{b1}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $-W-NR^{b2}S(O)_2NR^{b2}R^{b3}$, $-W-OS(O)_2NR^{b2}R^{b3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, $C_{6-14}$ aryl, 3-12 membered heterocyclyl, and 5-16 membered heteroaryl; wherein each of the alkyl, the alkenyl, the alkynyl, the alkoxy, the alkylthiol, the cycloalkyl, the cycloalkenyl, the aryl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

$R^{b1}$ is, at each occurrence, independently hydrogen, deuterium, $C_{1-6}$ alkyl, $C_{1-6}$ alkoxy, $C_{3-10}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^{b2}$ and $R^{b3}$ are, at each occurrence, independently hydrogen, deuterium, $C_{1-6}$ alkyl, or $C_{1-6}$ alkoxy, wherein each of the alkyl and the alkoxy is optionally substituted by one or more substituents selected from the group consisting of halogen, deuterium, cyano, hydroxyl, amino, carboxyl, $C_{1-6}$ alkyl, $C_{1-6}$ alkoxy, $C_{1-6}$ alkyl, $C_{3-8}$ cycloalkyl, phenyl, $C_{1-6}$ haloalkyl, $C_{1-6}$ haloalkoxy, and halophenyl; or when $R^{b2}$ and $R^{b3}$ are attached to the same nitrogen atom, $R^{b2}$ and $R^{b3}$, together with the nitrogen atom to which they are attached, form 3-10 membered heterocycloalkyl or 5-12 membered heteroaryl, each of which is optionally substituted by one or more of halogen, hydroxyl, amino, nitro, sulfhydryl, cyano, oxo, $C_{1-6}$ alkyl, $C_{1-6}$ alkoxy, $C_{3-10}$ cycloalkyl, 3-10 membered heterocycloalkyl, $C_{6-14}$ aryl, and 5-12 membered heteroaryl;

$R^{b4}$ is, at each occurrence, independently hydrogen, deuterium, $C_{1-6}$ alkyl, $C_{1-6}$ alkoxy, $C_{3-8}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^4$ and $R^5$ are each independently selected from the group consisting of hydrogen, deuterium, halogen, oxime, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, $C_{6-14}$ aryl, 3-12 membered heterocyclyl, and 5-16 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, the aryl, and the heteroaryl is optionally substituted by one or more $R^c$; or

$R^4$ and $R^5$ are attached to the same ring atom, to the adjacent ring atoms, or to the ring atoms separated by one atom, and $R^4$ and $R^5$, together with the carbon atom(s) and/or nitrogen atom(s) to which they are attached, form optionally substituted $C_{3-10}$ cycloalkyl, optionally substituted $C_{6-14}$ aryl, optionally substituted 5-12 membered heteroaryl, or optionally substituted 5-12 membered heterocyclyl; and the term "optionally substituted" means that the hydrogen on the substituted group is not substituted or one or more substitutable sites of the substituted group are independently substituted by substituents selected from $R^c$;

$R^c$ is independently selected from the group consisting of deuterium, halogen, oxime, - CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, $C_{3-10}$ cycloalkenyl, 3-12 membered heterocyclyl, and 5-16 membered heteroaryl; wherein each of the alkyl, the alkenyl, the alkynyl, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

$R^{c1}$ is, at each occurrence, independently hydrogen, deuterium, $C_{1-6}$ alkyl, $C_{1-6}$ alkoxy, $C_{3-10}$ cycloalkyl, $C_{6-14}$

aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

$R^{c2}$ and $R^{c3}$ are, at each occurrence, independently hydrogen, deuterium, $C_{1-6}$ alkyl, or $C_{1-6}$ alkoxy, wherein each of the alkyl and the alkoxy is optionally substituted by one or more substituents selected from the group consisting of halogen, deuterium, cyano, hydroxyl, amino, carboxyl, $C_{1-6}$ alkyl, $C_{1-6}$ alkoxy, $C_{1-6}$ alkyl, $C_{3-8}$ cycloalkyl, phenyl, $C_{1-6}$ haloalkyl, $C_{1-6}$ haloalkoxy, and halophenyl; or

when $R^{c2}$ and $R^{c3}$ are attached to the same nitrogen atom, $R^{c2}$ and $R^{c3}$, together with the nitrogen atom to which they are attached, form 3-10 membered heterocycloalkyl or 5-12 membered heteroaryl, each of which is optionally substituted by one or more of halogen, hydroxyl, amino, nitro, sulfhydryl, cyano, oxo, $C_{1-6}$ alkyl, $C_{1-6}$ alkoxy, $C_{3-10}$ cycloalkyl, 3-10 membered heterocycloalkyl, $C_{6-14}$ aryl, and 5-12 membered heteroaryl;

$R^{c4}$ is, at each occurrence, independently hydrogen, deuterium, $C_{1-6}$ alkyl, $C_{1-6}$ alkoxy, $C_{3-8}$ cycloalkyl, $C_{6-14}$ aryl, or 3-20 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{6-14}$ aryl, $C_{1-6}$ haloalkyl, $C_{1-6}$ haloalkoxy, and halophenyl;

W is selected from the group consisting of bond, $C_{1-3}$ alkylene, $-OC_{1-3}$ alkylene, and - $SC_{1-3}$ alkylene, and the alkylene is optionally substituted by one or more substituents selected from the group consisting of deuterium, halogen, oxo, -CN, -OH, and $-NH_2$;

unless otherwise stated, the heteroatom(s) in the heterocyclyl and the heteroaryl is(are) independently selected from the group consisting of O, N and S, and the number of the heteroatoms is 1, 2 or 3.

**2.** The compound, or the tautomer, the stereoisomer, or the pharmaceutically acceptable salt thereof according to claim 1, **characterized in that**: ring A is selected from the group consisting of $C_{5-10}$ cycloalkyl, $C_{6-10}$ aryl, 5-10 membered heteroaryl, and 5-10 membered heterocyclyl; preferably, ring A is selected from the group consisting of $C_{5-10}$ cycloalkyl, $C_6$ aryl, 5-8 membered heteroaryl, and 5-10 membered heterocyclyl; preferably, ring A is selected from the group consisting of $C_{5-10}$ cycloalkyl, and 5-10 membered heterocyclyl; preferably, ring A is selected from the group consisting of 5-10 membered heterocyclyl; preferably, ring A is selected from the group consisting of 5-8 membered heterocyclyl; preferably, ring A is selected from the group consisting of 5-7 membered heterocyclyl, and the heteroatom(s) is(are) independently selected from the group consisting of O and N, and the number of the heteroatoms is 1 or 2; preferably, ring A is selected from the group consisting of

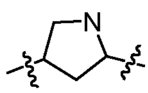 , 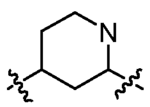 , 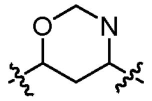 , 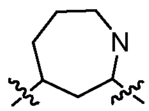 ,

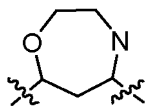 and 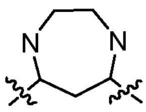 ;

preferably, ring A is selected from the group consisting of

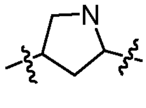 ,

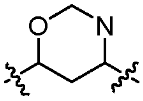 , 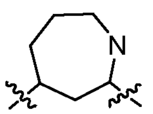 and 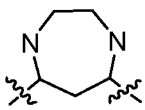 ;

and preferably, ring A is selected from the group consisting of

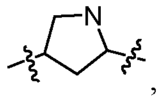, 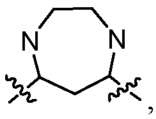, and 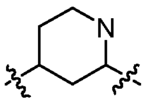.

3. The compound, or the tautomer, the stereoisomer, or the pharmaceutically acceptable salt thereof according to claim 1 or 2, **characterized in that**: X is selected from the group consisting of N and CH; preferably, X is selected from the group consisting of N; and preferably, X is selected from the group consisting of CH.

4. The compound, or the tautomer, the stereoisomer, or the pharmaceutically acceptable salt thereof according to any one of claims 1-3, **characterized in that**: $R^1$ is selected from the group consisting of the following optionally substituted groups: $C_{3-10}$ cycloalkyl, $C_{5-10}$ bridged cycloalkyl, $C_{5-10}$ fused cycloalkyl, $C_{5-10}$ spirocycloalkyl, $C_{6-10}$ aryl, 5-10 membered heteroaryl, 5-10 membered monocyclic heterocyclyl, 5-10 membered bridged heterocyclyl, 5-10 membered fused heterocyclyl, and 5-10 membered spirocyclic heterocyclyl; wherein each of the cycloalkyl, the bridged cycloalkyl, the fused cycloalkyl, the spirocycloalkyl, the aryl, the heteroaryl, the monocyclic heterocyclyl, the bridged heterocyclyl, the fused heterocyclyl and the spirocyclic heterocyclyl is optionally substituted by one or more $R^a$;

preferably, $R^1$ is selected from the group consisting of the following optionally substituted groups: $C_{3-6}$ cycloalkyl, $C_{5-10}$ fused cycloalkyl, $C_{5-10}$ spirocycloalkyl, $C_{6-10}$ aryl, 5-10 membered heteroaryl, 5-10 membered monocyclic heterocyclyl, 5-10 membered bridged heterocyclyl, 5-10 membered fused heterocyclyl, and 5-10 membered spirocyclic heterocyclyl; wherein each of the cycloalkyl, the fused cycloalkyl, the spirocycloalkyl, the aryl, the heteroaryl, the monocyclic heterocyclyl, the bridged heterocyclyl, the fused heterocyclyl and the spirocyclic heterocyclyl is optionally substituted by one or more $R^a$;
preferably, $R^1$ is selected from the group consisting of the following optionally substituted groups: $C_{6-10}$ aryl, 5-10 membered heteroaryl, 5-10 membered monocyclic heterocyclyl, 5-10 membered bridged heterocyclyl, 5-10 membered fused heterocyclyl, and 5-10 membered spirocyclic heterocyclyl; wherein each of the aryl, the heteroaryl, the monocyclic heterocyclyl, the bridged heterocyclyl, the fused heterocyclyl and the spirocyclic heterocyclyl is optionally substituted by one or more $R^a$;
preferably, $R^1$ is selected from the group consisting of the following optionally substituted groups: $C_{6-10}$ aryl, 5-8 membered heteroaryl, 5-8 membered monocyclic heterocyclyl, 5-8 membered bridged heterocyclyl, 5-8 membered fused heterocyclyl, and 5-8 membered spirocyclic heterocyclyl; wherein each of the aryl, the heteroaryl, the monocyclic heterocyclyl, the bridged heterocyclyl, the fused heterocyclyl and the spirocyclic heterocyclyl is optionally substituted by one or more $R^a$;
preferably, $R^1$ is selected from the group consisting of the following optionally substituted groups: $C_6$ aryl, 5-8 membered heteroaryl, and 5-8 membered monocyclic heterocyclyl; wherein each of the aryl, the heteroaryl, and the monocyclic heterocyclyl is optionally substituted by one or more $R^a$;
preferably, $R^1$ is selected from the group consisting of the following optionally substituted groups: $C_6$ aryl, 5-6 membered heteroaryl, and 5-6 membered monocyclic heterocyclyl; wherein each of the aryl, the heteroaryl, and the monocyclic heterocyclyl is optionally substituted by one or more $R^a$;
preferably, $R^1$ is selected from the group consisting of the following optionally substituted groups: $C_6$ aryl and 5-6 membered heteroaryl; wherein each of the aryl and the heteroaryl is optionally substituted by one or more $R^a$;
preferably, $R^1$ is selected from the group consisting of phenyl, pyridinyl and imidazolyl, each of which is optionally substituted by one or more $R^a$;
preferably, $R^1$ is selected from the group consisting of pyridinyl optionally substituted by one or more $R^a$;
preferably, $R^1$ is selected from the group consisting of

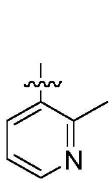, 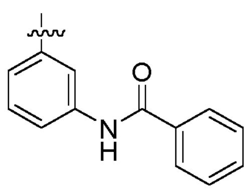,

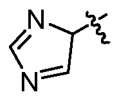, 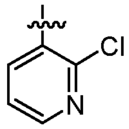, 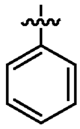 and 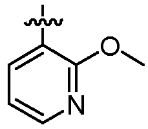 ;

and
preferably, $R^1$ is selected from the group consisting of

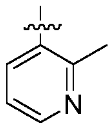, 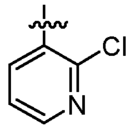

and

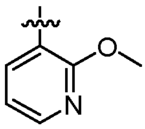.

**5.** The compound, or the tautomer, the stereoisomer, or the pharmaceutically acceptable salt thereof according to any one of claims 1-4, **characterized in that**: L is selected from the group consisting of bond, -O-, -S-, $-C_{1-4}$ alkylene- and $-OC_{1-4}$ alkylene-; wherein the alkylene is optionally substituted by one or more substituents selected from the group consisting of deuterium, halogen, oxo, -CN, -OH, and $-NH_2$;

preferably, L is selected from the group consisting of bond, -O-, -S-, $-C_{1-2}$ alkylene- and - $OC_{1-2}$ alkylene-; wherein the alkylene is optionally substituted by one or more substituents selected from the group consisting of deuterium, halogen, oxo, -CN, -OH, and $-NH_2$;
preferably, L is selected from the group consisting of bond, -O-, -S-, methylene and ethylene; wherein the methylene or the ethylene is optionally substituted by one or more substituents selected from the group consisting of deuterium, halogen, oxo, -CN, -OH, and $-NH_2$;
preferably, L is selected from the group consisting of bond, -O-, methylene and ethylene;
preferably, L is selected from the group consisting of bond and methylene; and
preferably, L is selected from the group consisting of bond.

**6.** The compound, or the tautomer, the stereoisomer, or the pharmaceutically acceptable salt thereof according to any one of claims 1-5, **characterized in that**: $R^a$ is, at each occurrence, independently selected from the group consisting of deuterium, halogen, -CN, -OH, -SH, $-NO_2$, - $NH_2$, $-W-OR^{a1}$, $-W-SR^{a1}$, $-W-C(O)R^{a4}$, $-W-C(O)OR^{a1}$, $-W-OC(O)R^{a1}$, $-W-C(O)NR^{a2}R^{a3}$, $-W-OC(O)NR^{a2}R^{a3}$, $-W-NR^{a2}R^{a3}$, $-W-NR^{a2}C(O)R^{a4}$, $-W-S(O)R^{a4}$, $-W-S(O)_2R^{a4}$, $-W-SO_2N-R^{a2}R^{a3}$, $-W-NR^{a2}S(O)_2R^{a4}$, $-W-OS(O)_2R^{a4}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, 3-12 membered heterocyclyl, and 5-16 membered heteroaryl; wherein each of the alkyl, the alkenyl, the alkynyl, the alkoxy, the alkylthiol, the cycloalkyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, phenyl, $C_{1-6}$ alkoxy, $C_{1-6}$ haloalkyl, $C_{1-6}$ haloalkoxy, and halophenyl;

preferably, $R^a$ is, at each occurrence, independently selected from the group consisting of deuterium, halogen, -CN, -OH, -SH, $-NO_2$, $-NH_2$, $-W-OR^{a1}$, $-W-SR^{a1}$, $-W-C(O)R^{a4}$, $-W-C(O)OR^{a1}$, $-W-OC(O)R^{a1}$, $-W-C(O)NR^{a2}R^{a3}$, $-W-NR^{a2}R^{a3}$, $-W-NR^{a2}C(O)R^{a4}$, $-W-S(O)R^{a4}$, $-W-S(O)_2R^{a4}$, $-W-SO_2NR^{a2}R^{a3}$, $-W-NR^{a2}S(O)_2R^{a4}$, $C_{1-6}$ alkyl, $C_{2-6}$ alkenyl, $C_{2-6}$ alkynyl, $C_{1-6}$ alkoxy, $C_{1-6}$ alkylthiol, $C_{3-10}$ cycloalkyl, 5-10 membered heterocyclyl, and 5-10 membered heteroaryl; wherein each of the alkyl, the alkenyl, the alkynyl, the alkoxy, the alkylthiol, the cycloalkyl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy;
preferably, $R^a$ is, at each occurrence, independently selected from the group consisting of deuterium, halogen, -CN, -OH, -SH, $-NO_2$, $-NH_2$, $-W-OR^{a1}$, $-W-SR^{a1}$, $-W-C(O)R^{a4}$, $-W-C(O)OR^{a1}$, $-W-OC(O)R^{a1}$, $-W-C(O)NR^{a2}R^{a3}$, $-W-NR^{a2}R^{a3}$, $-W-NR^{a2}C(O)R^{a4}$, $-W-S(O)_2R^{a4}$, $-W-SO_2NR^{a2}R^{a3}$, $-W-NR^{a2}S(O)_2R^{a4}$, $C_{1-6}$ alkyl, $C_{2-6}$ alkenyl,

$C_2$-$_6$ alkynyl, and $C_1$-$_6$ alkoxy; wherein each of the alkyl, the alkenyl, the alkynyl, and the alkoxy is optionally substituted by one or more substituents selected from the group consisting of deuterium, halogen, oxo, -CN, -OH, -$NO_2$, -$NH_2$, $C_1$-$_6$ alkyl, $C_1$-$_6$ alkoxy, $C_1$-$_6$ haloalkyl, and $C_1$-$_6$ haloalkoxy;

preferably, $R^a$ is, at each occurrence, independently selected from the group consisting of deuterium, halogen, -CN, -OH, -$NH_2$, -W-$OR^{a1}$, -W-$SR^{a1}$, -W-C(O)$R^{a4}$, -W-C(O)$OR^{a1}$, -W-OC(O)$R^{a1}$, -W-$NR^{a2}$C(O)$R^{a4}$, -$C_{1-4}$ alkyl, and $C_1$-$_4$ alkoxy; wherein the alkyl or the alkoxy is optionally substituted by one or more substituents selected from the group consisting of deuterium, halogen, oxo, -CN, -OH, -$NO_2$, -$NH_2$, $C_1$-$_4$ alkyl, and $C_1$-$_4$ alkoxy;

preferably, $R^a$ is, at each occurrence, independently selected from the group consisting of deuterium, halogen, -OH, -$NH_2$, -W-$NR^{a2}$C(O)$R^{a4}$, $C_1$-$_4$ alkyl, and $C_1$-$_4$ alkoxy; wherein the alkyl or the alkoxy is optionally substituted by one or more substituents selected from the group consisting of deuterium, halogen, oxo, -OH, -$NH_2$, $C_1$-$_4$ alkyl, $C_1$-$_4$ alkoxy, $C_1$-$_4$ haloalkyl, and $C_1$-$_4$ haloalkoxy;

preferably, $R^a$ is, at each occurrence, independently selected from the group consisting of deuterium, halogen, $C_1$-$_4$ alkyl, -W-$NR^{a2}$C(O)$R^{a4}$, and $C_1$-$_4$ haloalkyl; wherein the alkyl or the haloalkyl is optionally substituted by one or more deuterium;

preferably, $R^a$ is, at each occurrence, independently selected from the group consisting of deuterium, halogen, $C_1$-$_4$ alkyl, $C_1$-$_4$ alkoxy, and -W-$NR^{a2}$C(O)$R^{a4}$; wherein the alkyl or the haloalkyl is optionally substituted by one or more deuterium;

preferably, $R^a$ is, at each occurrence, independently selected from the group consisting of deuterium, halogen, $C_1$-$_4$ alkyl, $C_1$-$_4$ alkoxy, and -NHC(O)$R^{a4}$; wherein the alkyl is optionally substituted by one or more deuterium;

preferably, $R^a$ is, at each occurrence, independently selected from the group consisting of halogen, $C_1$-$_4$ alkyl, and $C_1$-$_4$ alkoxy;

preferably, $R^a$ is, at each occurrence, independently selected from the group consisting of deuterium, F, Cl, Br, methyl, ethyl, propyl, methoxy, ethoxy, trifluoromethyl, trifluoroethyl and - NHC(O)-phenyl;

preferably, $R^a$ is, at each occurrence, independently selected from the group consisting of Cl, methyl, methoxy, and -NHC(O)-phenyl.

**7.** The compound, or the tautomer, the stereoisomer, or the pharmaceutically acceptable salt thereof according to any one of claims 1-6, **characterized in that**: $R^{a1}$ is, at each occurrence, independently hydrogen, deuterium, $C_1$-$_6$ alkyl, $C_1$-$_6$ alkoxy, $C_{3-6}$ cycloalkyl, $C_{6-10}$ aryl, and 3-10 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_1$-$_6$ alkyl, $C_1$-$_6$ alkoxy, $C_1$-$_6$ haloalkyl, and $C_1$-$_6$ haloalkoxy; the heteroatom(s) is(are) independently selected from the group consisting of O, N and S, and the number of the heteroatoms is 1, 2 or 3;

preferably, $R^{a1}$ is, at each occurrence, independently hydrogen, deuterium, $C_1$-$_4$ alkyl, $C_1$-$_4$ alkoxy, $C_{3-6}$ cycloalkyl, $C_{6-10}$ aryl, and 3-8 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_1$-$_4$ alkyl, $C_1$-$_4$ alkoxy, $C_1$-$_4$ haloalkyl, and $C_1$-$_4$ haloalkoxy;

preferably, $R^{a1}$ is, at each occurrence, independently hydrogen, deuterium, $C_1$-$_4$ alkyl, $C_1$-$_4$ alkoxy, $C_{3-6}$ cycloalkyl, $C_6$ aryl, and 5-6 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_1$-$_4$ alkyl, $C_1$-$_4$ alkoxy, $C_1$-$_4$ haloalkyl, and $C_1$-$_4$ haloalkoxy;

preferably, $R^{a1}$ is, at each occurrence, independently hydrogen, deuterium, methyl, ethyl, propyl, methoxy, ethoxy or propoxy, wherein methyl, ethyl, propyl, methoxy, ethoxy or propoxy is optionally substituted by one or more substituents selected from the group consisting of halogen, deuterium, hydroxyl, amino, and $C_1$-$_4$ alkyl;

preferably, $R^{a1}$ is, at each occurrence, independently hydrogen, methyl, ethyl, or propyl, wherein methyl, ethyl, or propyl is optionally substituted by one or more substituents selected from the group consisting of halogen, deuterium, hydroxyl, amino, and $C_1$-$_4$ alkyl.

**8.** The compound, or the tautomer, the stereoisomer, or the pharmaceutically acceptable salt thereof according to any one of claims 1-7, **characterized in that**: $R^{a2}$ and $R^{a3}$ are, at each occurrence, independently hydrogen, deuterium, $C_1$-$_4$ alkyl, or $C_1$-$_4$ alkoxy, wherein the alkyl or the alkoxy is optionally substituted by one or more substituents selected from the group consisting of halogen, deuterium, hydroxyl, amino, $C_1$-$_4$ alkyl, $C_1$-$_4$ alkoxy, $C_1$-$_4$ haloalkyl, and $C_1$-$_4$ haloalkoxy;

preferably, $R^{a2}$ and $R^{a3}$ are, at each occurrence, independently hydrogen, deuterium, $C_1$-$_4$ alkyl, or $C_1$-$_4$ alkoxy, wherein the alkyl or the alkoxy is optionally substituted by one or more substituents selected from the group

consisting of halogen, deuterium, hydroxyl, $C_{1-4}$ alkyl, and $C_{1-4}$ alkoxy;

preferably, $R^{a2}$ and $R^{a3}$ are, at each occurrence, independently hydrogen, deuterium, methyl, ethyl, propyl, methoxy, ethoxy, or propoxy, wherein methyl, ethyl, propyl, methoxy, ethoxy, or propoxy is optionally substituted by one or more substituents selected from the group consisting of halogen, deuterium, hydroxyl, $C_{1-4}$ alkyl, and $C_{1-4}$ alkoxy;

preferably, $R^{a2}$ and $R^{a3}$ are, at each occurrence, independently hydrogen, methyl, ethyl, or propyl, wherein methyl, ethyl, or propyl is optionally substituted by one or more substituents selected from the group consisting of halogen, deuterium, hydroxyl, amino, and $C_{1-4}$ alkyl.

**9.** The compound, or the tautomer, the stereoisomer, or the pharmaceutically acceptable salt thereof according to any one of claims 1-8, **characterized in that**: $R^{a4}$ is, at each occurrence, independently hydrogen, deuterium, $C_{1-4}$ alkyl, $C_{1-4}$ alkoxy, $C_{3-8}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-6}$ alkyl, $C_{1-6}$ alkoxy, phenyl, $C_{1-6}$ haloalkyl, $C_{1-6}$ haloalkoxy, and halophenyl; the heteroatom(s) is(are) independently selected from the group consisting of O, N and S, and the number of the heteroatoms is 1, 2 or 3;

preferably, $R^{a4}$ is, at each occurrence, independently hydrogen, deuterium, $C_{1-4}$ alkyl, $C_{1-4}$ alkoxy, $C_{3-6}$ cycloalkyl, $C_6$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy;

preferably, $R^{a4}$ is, at each occurrence, independently hydrogen, deuterium, $C_{1-4}$ alkyl, $C_{1-4}$ alkoxy, $C_{3-6}$ cycloalkyl, or $C_6$ aryl, wherein each of the alkyl, the alkoxy, the cycloalkyl, and the aryl is optionally substituted by one or more substituents selected from the group consisting of halogen, deuterium, hydroxyl, amino, $C_{1-4}$ alkyl, and $C_{1-4}$ alkoxy;

preferably, $R^{a4}$ is, at each occurrence, independently hydrogen, deuterium, methyl, ethyl, propyl, methoxy, ethoxy, propoxy, cyclopropanyl, cyclobutanyl or phenyl; wherein methyl, ethyl, propyl, methoxy, ethoxy, propoxy, cyclopropanyl, cyclobutanyl or phenyl is optionally substituted by one or more substituents selected from the group consisting of halogen, deuterium, hydroxyl, amino, $C_{1-4}$ alkyl, and $C_{1-4}$ alkoxy; and

preferably, $R^{a4}$ is, at each occurrence, independently hydrogen, deuterium, methyl, ethyl, propyl, methoxy, ethoxy, propoxy or phenyl; wherein methyl, ethyl, propyl, methoxy, ethoxy, propoxy or phenyl is optionally substituted by one or more substituents selected from the group consisting of halogen, deuterium, hydroxyl, amino, $C_{1-4}$ alkyl, and $C_{1-4}$ alkoxy.

**10.** The compound, or the tautomer, the stereoisomer, or the pharmaceutically acceptable salt thereof according to any one of claims 1-9, **characterized in that**: $R^2$ is selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$,$-W-NR^{b2}R^{b3}$, $-W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-6}$ cycloalkyl, $C_{6-12}$ aryl, 3-6 membered heterocyclyl, and 5-12 membered heteroaryl; wherein each of the alkyl, the alkenyl, the alkynyl, the haloalkyl, the haloalkoxy, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy;

preferably, $R^2$ is selected from the group consisting of hydrogen, deuterium, halogen, - CN, -OH, -SH, $-NO_2$, $-NH_2$, $-W-OR^{b1}$, $-W-SR^{b1}$, $-W-C(O)R^{b4}$ , $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$, $-W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-6}$ alkyl, $C_{1-6}$ haloalkyl, $C_{1-6}$ haloalkoxy, $C_{1-6}$ alkoxy, $C_{1-6}$ alkylthiol, $C_{3-6}$ cycloalkyl, $C_{6-12}$ aryl, 5-6 membered heterocyclyl, and 5-6 membered heteroaryl; wherein each of the alkyl, the alkenyl, the alkynyl, the haloalkyl, the haloalkoxy, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy;

preferably, $R^2$ is selected from the group consisting of hydrogen, deuterium, halogen,-CN, -OH, -SH, $-NO_2$, $-NH_2$, $-W-OR^{b1}$, $-W-SO_2NR^{b2}R^{b3}$, $-W-NR^{b2}S(O)_2R^{b4}$, $C_{1-4}$ alkyl, $C_{1-4}$ alkoxy, $C_{3-6}$ cycloalkyl, and $C_6$ aryl; wherein each of the alkyl, the alkoxy, the cycloalkyl, and the aryl is optionally substituted by one or more substituents selected from the group consisting of deuterium, halogen, oxo, -CN, -OH, $-NO_2$, $-NH_2$, $C_{1-4}$ alkyl, $C_{1-4}$ alkoxy, $C_{1-4}$

haloalkyl, and $C_{1-4}$ haloalkoxy;

preferably, $R^2$ is selected from the group consisting of hydrogen, deuterium, halogen, - CN, -OH, -SH, $-NO_2$, $-NH_2$, methyl, ethyl, isopropyl, methoxy, ethoxy, propoxy, $-W-SO_2NR^{b2}R^{b3}$, and $-W-NR^{b2}S(O)_2R^{b4}$; wherein methyl, ethyl, isopropyl, methoxy, ethoxy, or propoxy is optionally substituted by one or more substituents selected from the group consisting of deuterium, halogen, oxo, -CN, -OH, $-NO_2$, $-NH_2$, $C_{1-4}$ alkyl, $C_{1-4}$ alkoxy, $C_{1-4}$ haloalkyl, and $C_{1-4}$ haloalkoxy;

preferably, $R^2$ is selected from the group consisting of hydrogen, deuterium, halogen, - CN, -OH, $-NH_2$, methyl, ethyl, isopropyl, $-SO_2NH_2$, and $-NHSO_2H$; and

preferably, $R^2$ is selected from the group consisting of hydrogen.

**11.** The compound, or the tautomer, the stereoisomer, or the pharmaceutically acceptable salt thereof according to any one of claims 1-10, **characterized in that**: $R^3$ is selected from the group consisting of hydrogen, deuterium, halogen, -CN, -OH, -SH, $-NO_2$, $-NH_2$, $-W-OR^{b1}$, $-W-SR^{b1}$, $-W-C(O)R^{b4}$, $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$, $-W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-6}$ cycloalkyl, $C_{6-12}$ aryl, 3-6 membered heterocyclyl, and 5-10 membered heteroaryl; wherein each of the alkyl, the alkenyl, the alkynyl, the haloalkyl, the haloalkoxy, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy;

preferably, $R^3$ is selected from the group consisting of hydrogen, deuterium, halogen, - CN, -OH, -SH, $-NO_2$, $-NH_2$, $-W-OR^{b1}$, $-W-SR^{b1}$, $-W-C(O)R^{b4}$ , $-W-C(O)OR^{b1}$, $-W-OC(O)R^{b1}$, $-W-OC(O)OR^{b1}$, $-W-C(O)NR^{b2}R^{b3}$, $-W-C(O)NR^{b2}OR^{b1}$, $-W-NR^{b2}R^{b3}$, $-W-NR^{b2}C(O)R^{b4}$, $-W-NR^{b2}C(O)NR^{b2}R^{b3}$, $-W-S(O)R^{b4}$, $-W-S(O)_2R^{b4}$, $-W-SO_2NR^{b2}R^{b3}$, $-W-NR^{b2}S(O)_2R^{b4}$, $-W-OS(O)_2R^{b4}$, $C_{1-4}$ alkyl, $C_{2-4}$ alkenyl, $C_{2-4}$ alkynyl, $C_{1-4}$ alkoxy, $C_{1-4}$ alkylthiol, $C_{3-6}$ cycloalkyl, $C_6$ aryl, 3-6 membered heterocyclyl, and 5-6 membered heteroaryl; wherein each of the alkyl, the alkenyl, the alkynyl, the alkoxy, the alkylthiol, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy;

preferably, $R^3$ is selected from the group consisting of hydrogen, deuterium, halogen, - CN, -OH, $-NH_2$, $-W-OR^{b1}$, $-W-SR^{b1}$, $-W-C(O)R^{b4}$ , $-W-C(O)OR^{b1}$, $-W-OC(O)R^{b1}$ , $-W-C(O)NR^{b2}R^{b3}$, $-W-NR^{b2}R^{b3}$, $-W-NR^{b2}C(O)R^{b4}$, $-W-NR^{b2}C(O)NR^{b2}R^{b3}$, $-W-S(O)R^{b4}$, $-W-S(O)_2R^{b4}$, $C_{1-4}$ alkyl, $C_{1-4}$ alkoxy, $C_{3-6}$ cycloalkyl, $C_6$ aryl, 3-6 membered heterocyclyl, and 5-6 membered heteroaryl; wherein each of the alkyl, the alkoxy, the cycloalkyl, the aryl, the heterocyclyl, and the heteroaryl is optionally substituted by one or more substituents selected from the group consisting of deuterium, halogen, oxo, -CN, -OH, $-NO_2$, $-NH_2$, $C_{1-6}$ alkyl, $C_{1-6}$ alkoxy, $C_{1-6}$ haloalkyl, and $C_{1-6}$ haloalkoxy;

preferably, $R^3$ is selected from the group consisting of hydrogen, deuterium, halogen, - OH, $-NH_2$, $C_{1-4}$ alkyl, $C_{1-4}$ alkoxy, $C_{3-6}$ cycloalkyl, and 5-6 membered heterocyclyl; wherein each of the alkyl, the alkoxy, the cycloalkyl, and the heterocyclyl is optionally substituted by one or more substituents selected from the group consisting of deuterium, halogen, oxo, -CN, -OH, $-NO_2$, $-NH_2$, $C_{1-4}$ alkyl, $C_{1-4}$ alkoxy, $C_{1-4}$ haloalkyl, and $C_{1-4}$ haloalkoxy;

preferably, $R^3$ is selected from the group consisting of F, Cl, Br, $-CF_3$, $-CHF_2-CH_2$, - $CH_2FCH_3$, $-OCF_3$ and cyclopropanyl;

preferably, $R^3$ is selected from the group consisting of Cl and $-CF_3$;

preferably, $R^3$ is selected from the group consisting of halogen, and $C_{1-4}$ alkyl optionally substituted by one or more halogen;

preferably, $R^3$ is selected from the group consisting of halogen; and

preferably, $R^3$ is selected from the group consisting of Cl.

**12.** The compound, or the tautomer, the stereoisomer, or the pharmaceutically acceptable salt thereof according to any one of claims 1-11, **characterized in that**: $R^{b1}$ is, at each occurrence, independently hydrogen, deuterium, $C_{1-6}$ alkyl, $C_{1-6}$ alkoxy, $C_{3-6}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{1-6}$ haloalkyl, and $C_{1-6}$ haloalkoxy;

preferably, $R^{b1}$ is, at each occurrence, independently hydrogen, deuterium, $C_{1-4}$ alkyl, $C_{1-4}$ alkoxy, $C_{3-6}$ cycloalkyl, $C_{6-10}$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-4}$ alkyl, $C_{1-4}$ alkoxy, $C_{1-4}$ haloalkyl, and $C_{1-4}$ haloalkoxy;

preferably, $R^{b1}$ is, at each occurrence, independently hydrogen, deuterium, $C_{1}$-$_{4}$ alkyl, $C_{1}$-$_{4}$ alkoxy, $C_{3}$-$_{6}$ cycloalkyl, $C_6$ aryl, or 5-6 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1}$-$_{4}$ alkyl, $C_{1}$-$_{4}$ alkoxy, $C_{1}$-$_{4}$ haloalkyl, and $C_{1}$-$_{4}$ haloalkoxy;
preferably, $R^{b1}$ is, at each occurrence, independently hydrogen, deuterium, methyl, ethyl, propyl, methoxy, ethoxy or propoxy, wherein methyl, ethyl, propyl, methoxy, ethoxy or propoxy is optionally substituted by one or more substituents selected from the group consisting of halogen, deuterium, hydroxyl, amino, and $C_{1}$-$_{4}$ alkyl;
preferably, $R^{b1}$ is, at each occurrence, independently hydrogen, methyl, ethyl, or propyl, wherein methyl, ethyl, or propyl is optionally substituted by one or more substituents selected from the group consisting of halogen, deuterium, hydroxyl, amino, and $C_{1}$-$_{4}$ alkyl.

**13.** The compound, or the tautomer, the stereoisomer, or the pharmaceutically acceptable salt thereof according to any one of claims 1-12, **characterized in that**: $R^{b2}$ and $R^{b3}$ are, at each occurrence, independently hydrogen, deuterium, $C_{1}$-$_{4}$ alkyl, or $C_{1}$-$_{4}$ alkoxy, wherein the alkyl or the alkoxy is optionally substituted by one or more substituents selected from the group consisting of halogen, deuterium, hydroxyl, amino, $C_{1}$-$_{4}$ alkyl, $C_{1}$-$_{4}$ alkoxy, $C_{1}$-$_{4}$ haloalkyl, and $C_{1}$-$_{4}$ haloalkoxy;

preferably, $R^{b2}$ and $R^{b3}$ are, at each occurrence, independently hydrogen, deuterium, $C_{1}$-$_{4}$ alkyl, or $C_{1}$-$_{4}$ alkoxy, wherein the alkyl or the alkoxy is optionally substituted by one or more substituents selected from the group consisting of halogen, deuterium, hydroxyl, $C_{1}$-$_{4}$ alkyl, and $C_{1}$-$_{4}$ alkoxy;
preferably, $R^{b2}$ and $R^{b3}$ are, at each occurrence, independently hydrogen, deuterium, methyl, ethyl, propyl, methoxy, ethoxy, or propoxy, wherein methyl, ethyl, propyl, methoxy, ethoxy, or propoxy is optionally substituted by one or more substituents selected from the group consisting of halogen, deuterium, hydroxyl, $C_{1}$-$_{4}$ alkyl, and $C_{1}$-$_{4}$ alkoxy;
preferably, $R^{b2}$ and $R^{b3}$ are, at each occurrence, independently hydrogen, methyl, ethyl, or propyl, wherein methyl, ethyl, or propyl is optionally substituted by one or more substituents selected from the group consisting of halogen, deuterium, hydroxyl, amino, and $C_{1}$-$_{4}$ alkyl.

**14.** The compound, or the tautomer, the stereoisomer, or the pharmaceutically acceptable salt thereof according to any one of claims 1-13, **characterized in that**: $R^{b4}$ is, at each occurrence, independently hydrogen, deuterium, $C_{1}$-$_{4}$ alkyl, $C_{1}$-$_{4}$ alkoxy, $C_{3}$-$_{8}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1}$-$_{6}$ alkyl, $C_{1}$-$_{6}$ alkoxy, phenyl, $C_{1}$-$_{6}$ haloalkyl, $C_{1}$-$_{6}$ haloalkoxy, and halophenyl;

preferably, $R^{b4}$ is, at each occurrence, independently hydrogen, deuterium, $C_{1}$-$_{4}$ alkyl, $C_{1}$-$_{4}$ alkoxy, $C_{3}$-$_{6}$ cycloalkyl, $C_6$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1}$-$_{4}$ alkyl, $C_{1}$-$_{4}$ alkoxy, $C_{1}$-$_{4}$ haloalkyl, and $C_{1}$-$_{4}$ haloalkoxy;
preferably, $R^{b4}$ is, at each occurrence, independently hydrogen, deuterium, $C_{1}$-$_{4}$ alkyl, $C_{1}$-$_{4}$ alkoxy, $C_{3}$-$_{6}$ cycloalkyl, or $C_6$ aryl, wherein each of the alkyl, the alkoxy, the cycloalkyl, and the aryl is optionally substituted by one or more substituents selected from the group consisting of halogen, deuterium, hydroxyl, amino, $C_{1}$-$_{4}$ alkyl, and $C_{1}$-$_{4}$ alkoxy;
preferably, $R^{b4}$ is, at each occurrence, independently hydrogen, deuterium, methyl, ethyl, propyl, methoxy, ethoxy, propoxy, cyclopropanyl, cyclobutanyl or phenyl; wherein methyl, ethyl, propyl, methoxy, ethoxy, propoxy, cyclopropanyl, cyclobutanyl or phenyl is optionally substituted by one or more substituents selected from the group consisting of halogen, deuterium, hydroxyl, amino, $C_{1}$-$_{4}$ alkyl, and $C_{1}$-$_{4}$ alkoxy;
preferably, $R^{b4}$ is, at each occurrence, independently hydrogen, deuterium, methyl, ethyl, propyl, methoxy, ethoxy or propoxy; wherein methyl, ethyl, propyl, methoxy, ethoxy, or propoxy is optionally substituted by one or more substituents selected from the group consisting of halogen, deuterium, hydroxyl, amino, $C_{1}$-$_{4}$ alkyl, and $C_{1}$-$_{4}$ alkoxy.

**15.** The compound, or the tautomer, the stereoisomer, or the pharmaceutically acceptable salt thereof according to any one of claims 1-14, **characterized in that**: $R^4$ and $R^5$ are each independently selected from the group consisting of hydrogen, deuterium, halogen, -CN, -OH, - SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}$ $S(O)_2R^{c4}$, $-W-OS(O)_2R^{c4}$, $C_{1}$-$_{6}$ alkyl, $C_{1}$-$_{6}$ alkoxy, $C_{1}$-$_{6}$ alkylthiol, $C_{3}$-$_{8}$ cycloalkyl, $C_{3}$-$_{8}$ cycloalkenyl, $C_{6-12}$ aryl, 4-12 membered heterocyclyl, and 5-12

membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; or

$R^4$ and $R^5$ are attached to the same ring atom, to the adjacent ring atoms, or to the ring atoms separated by one atom, and $R^4$ and $R^5$, together with the carbon atom(s) and/or nitrogen atom(s) to which they are attached, form optionally substituted $C_{3-10}$ cycloalkyl, optionally substituted $C_{6-12}$ aryl, optionally substituted 5-12 membered heteroaryl, or optionally substituted 5-12 membered heterocyclyl; and the term "optionally substituted" means that the hydrogen on the substituted group is not substituted or one or more substitutable sites of the substituted group are independently substituted by substituents selected from $R^c$;

preferably, $R^4$ and $R^5$ are each independently selected from the group consisting of hydrogen, deuterium, halogen, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)NR^{c2}R^{c3}$, $-W-S(O)R^{c4}$, $-W-SO)_2R^{c4}$, $-W-SO_2NR^{c2}R^{c3}$, $-W-NR^{c2}S(O)_2R^{c4}$, $-W-OS(O)_2R^{c4}$, $C_{1-4}$ alkyl, $C_{1-4}$ alkoxy, $C_{1-4}$ alkylthiol, $C_{3-6}$ cycloalkyl, $C_{4-6}$ cycloalkenyl, $C_{6-12}$ aryl, 4-10 membered heterocyclyl, and 5-12 membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; or

$R^4$ and $R^5$ are attached to the same ring atom, to the adjacent ring atoms, or to the ring atoms separated by one atom, and $R^4$ and $R^5$, together with the carbon atom(s) and/or nitrogen atom(s) to which they are attached, form optionally substituted $C_{3-10}$ cycloalkyl, optionally substituted $C_{6-12}$ aryl, optionally substituted 5-12 membered heteroaryl, or optionally substituted 5-12 membered heterocyclyl; and the term "optionally substituted" means that the hydrogen on the substituted group is not substituted or one or more substitutable sites of the substituted group are independently substituted by substituents selected from $R^c$;

preferably, $R^4$ and $R^5$ are each independently selected from the group consisting of hydrogen, deuterium, halogen, -CN, -OH, $-NH_2$, $-W-OR^{c1}$, $-W-SR^{c1}$, $-W-C(O)R^{c4}$, $-W-C(O)OR^{c1}$, $-W-OC(O)R^{c1}$, $-W-C(O)NR^{c2}R^{c3}$, $-W-OC(O)NR^{c2}R^{c3}$, $-W-NR^{c2}R^{c3}$, $-W-NR^{c2}C(O)R^{c4}$, $-W-S(O)R^{c4}$, $-W-S(O)2R^{c4}$, $-W-SO_2NR^{c2}R^{c3}$, $-W-NR^{c2}S(O)_2R^{c4}$, $-W-OS(O)_2R^{c4}$, C1-4 alkyl, $C_{1-4}$ alkoxy, $C_{1-4}$ alkylthiol, $C_{3-6}$ cycloalkyl, $C_{4-6}$ cycloalkenyl, $C_6$ aryl, 4-10 membered heterocyclyl, and 5-12 membered heteroaryl; wherein each of the alkyl, the alkoxy, the alkylthiol, the cycloalkyl, the cycloalkenyl, the aryl, the heterocyclyl, and the heteroaryl is optionally substituted by one or more $R^c$; the heteroatom(s) is(are) independently selected from the group consisting of O, N and S, and the number of the heteroatoms is 1, 2 or 3;

preferably, $R^4$ and $R^5$ are each independently selected from the group consisting of hydrogen, deuterium, halogen, -OH, $-W-C(O)NR^{c2}R^{c3}$, $-W-NR^{c2}R^{c3}$, $-W-SO_2NR^{c2}R^{c3}$, $-W-C(O)R^{c4}$, $-W-S(O)_2R^{c4}$, $C_{1-4}$ alkyl, $C_{3-6}$ cycloalkyl, 4-10 membered heterocyclyl, $C_{6-12}$ aryl and 5-10 membered heteroaryl; wherein each of the alkyl, the cycloalkyl, the heterocyclyl, phenyl and the heteroaryl is optionally substituted by one or more $R^c$; or

$R^4$ and $R^5$, together with the carbon atom(s) and/or nitrogen atom(s) to which they are attached, form optionally substituted $C_{3-10}$ cycloalkyl, and optionally substituted 5-12 membered heterocyclyl; and the term "optionally substituted" means that the hydrogen on the substituted group is not substituted or one or more substitutable sites of the substituted group are independently substituted by substituents selected from $R^c$;

preferably, $R^4$ is hydrogen, deuterium, halogen, -OH, $C_{1-4}$ alkyl, $C_{1-4}$ alkoxy, or 4-6 membered heterocycloalkyl; wherein each of the alkyl, the alkoxy, azetidinyl and pyrrolidinyl is optionally substituted by one or more $R^c$; and $R^5$ is selected from the group consisting of hydrogen, deuterium, halogen, -OH, $-W-C(O)NR^{c2}R^{c3}$, $-W-NR^{c2}R^{c3}$, $-W-SO_2NR^{c2}R^{c3}$, $-W-C(O)R^{c4}$, $-W-S(O)_2R^{c4}$, $C_{1-4}$ alkyl, $C_{3-6}$ cycloalkyl, 4-6 membered heterocycloalkyl, phenyl and 9-10 membered heteroaryl; wherein each of the alkyl, the cycloalkyl, the heterocycloalkyl, the phenyl and the heteroaryl is optionally substituted by one or more $R^c$;

preferably, $R^4$ is hydrogen, deuterium, halogen, -OH, methyl, ethyl, propyl, methoxy, ethoxy, propoxy, azetidinyl or pyrrolidinyl; wherein the methyl, ethyl, propyl, methoxy, ethoxy, propoxy, azetidinyl or pyrrolidinyl is optionally substituted by one or more $R^c$; and $R^5$ is selected from the group consisting of hydrogen, deuterium, halogen, -OH, $-W-C(O)NR^{c2}R^{c3}$, $-W-NR^{c2}R^{c3}$, $-W-SO_2NR^{c2}R^{c3}$, $-W-C(O)R^{c4}$, $-W-S(O)_2R^{c4}$, methyl, ethyl, propyl, methoxy, ethoxy, propoxy, cyclopropyl, cyclobutyl, cyclopentyl, cyclohexyl, azetidinyl, pyrrolidinyl, phenyl, imidazolyl, pyrazolyl, pyridinyl, pyrimidyl, and benzopyrazolyl; wherein methyl, ethyl, propyl, methoxy, ethoxy, propoxy, cyclopropyl, cyclobutyl, cyclopentyl, cyclohexyl, azetidinyl, pyrrolidinyl, phenyl, imidazolyl, pyrazolyl, pyridinyl, pyrimidyl, or benzopyrazolyl is optionally substituted by one or more $R^c$;

preferably, $R^4$ is hydrogen, halogen, -OH, methyl, ethyl, propyl, methoxy, ethoxy, azetidinyl or pyrrolidinyl; wherein the methyl, ethyl, propyl, methoxy, ethoxy, azetidinyl or pyrrolidinyl is optionally substituted by one or more $R^c$; and $R^5$ is selected from the group consisting of hydrogen, halogen, -OH, $-C_{1-3}$ alkylene-$C(O)NR^{c2}R^{c3}$, $-C_{1-3}$ alkylene-$NR^{c2}R^{c3}$, $-C_{1-3}$ alkylene-$SO_2NR^{c2}R^{c3}$, $-C(O)R^{c4}$, $-S(O)_2R^{c4}$, methyl, ethyl, phenyl, cyclopropyl, cyclobutyl, phenyl, pyridinyl, and benzopyrazolyl; wherein each of the methyl, ethyl, phenyl, cyclopropyl, cyclobutyl, phenyl, pyridinyl, and benzopyrazolyl is optionally substituted by one or more $R^c$;

preferably, $R^4$ is hydrogen, deuterium, halogen, -OH, methyl,

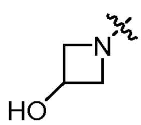, 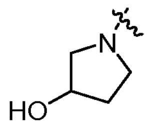

or

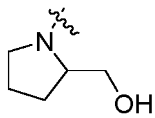;

and $R^5$ is selected from the group consisting of hydrogen, methyl, phenyl, benzoyl, - $SO_2CH_3$,

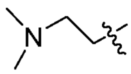, 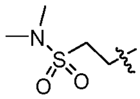, 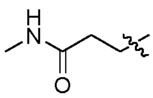, 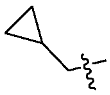, 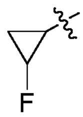, 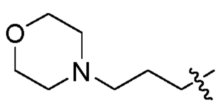, 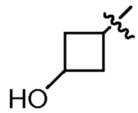,

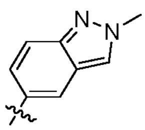 and 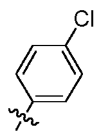;

preferably, $R^4$ is hydrogen, halogen, -OH or methyl; and $R^5$ is selected from the group consisting of hydrogen, methyl, phenyl, benzoyl, $-SO_2CH_3$,

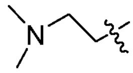, 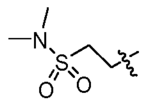, 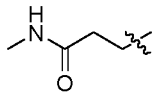,

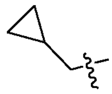, 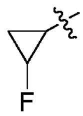, 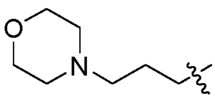, 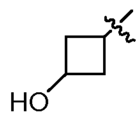, 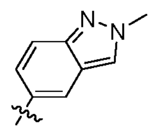 and 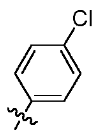;

preferably, $R^4$ is hydrogen; and $R^5$ is selected from the group consisting of hydrogen, $C_{1-6}$ alkyl, phenyl and 9-10 membered bicyclic heteroaryl, wherein the phenyl and the heteroaryl are optionally substituted by one or more $R^c$;
preferably, $R^4$ is hydrogen; and $R^5$ is selected from the group consisting of hydrogen, $C_{1-4}$ alkyl, phenyl and 9-10 membered bicyclic heteroaryl, wherein the phenyl and the heteroaryl are optionally substituted by one or more of halogen, -OH, and $C_{1-4}$ alkyl;
preferably, $R^4$ is hydrogen; and $R^5$ is selected from the group consisting of hydrogen, $C_{1-4}$ alkyl, phenyl and 9-10 membered bicyclic heteroaryl, wherein the phenyl and the heteroaryl are optionally substituted by one or more of -F, -Cl, -Br, -OH, methyl, ethyl, n-propyl, and isopropyl; wherein the heteroatom in the heteroaryl is selected from N, and the number of the heteroatom is 1, 2, or 3.

**16.** The compound, or the tautomer, the stereoisomer, or the pharmaceutically acceptable salt thereof according to any one of claims 1-15, **characterized in that**: $R^c$ is independently selected from the group consisting of deuterium, halogen, oxime, -CN, -OH, -SH, $-NO_2$, $-NH_2$, $-W-OR^{c1}$, - $W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-10}$ cycloalkyl, $C_{3-10}$ cycloalkenyl, $C_{6-12}$ aryl, 3-12 membered

heterocyclyl, and 5-16 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, - $NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

preferably, $R^c$ is independently selected from the group consisting of deuterium, halogen, oxime, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-10}$ cycloalkyl, $C_{3-10}$ cycloalkenyl, $C_{6-12}$ aryl, 3-12 membered heterocyclyl, and 5-16 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

preferably, $R^c$ is independently selected from the group consisting of deuterium, halogen, oxime, -CN, -OH, -SH, $-NO_2$, $-NH_2$, $-W-OR^{c1}$, $-W-SR^{c1}$, $-W-C(O)R^{c4}$, $-W-C(O)OR^{c1}$, $-W-OC(O)R^{c1}$, $-W-OC(O)OR^{c1}$, $-W-C(O)NR^{c2}R^{c3}$, $-W-C(O)NR^{c2}OR^{c1}$, $-W-OC(O)NR^{c2}R^{c3}$, $-W-NR^{c2}R^{c3}$, $-W-NR^{c2}C(O)R^{c4}$, $-W-NR^{c2}C(O)OR^{c1}$, $-W-NR^{c2}C(O)NR^{c2}R^{c3}$, $-W-S(O)R^{c4}$, $-W-S(O)_2R^{c4}$, $-W-SO_2NR^{c2}R^{c3}$, $-W-NR^{c2}S(O)_2R^{c4}$, $-W-OS(O)_2R^{c4}$, $-W-NR^{c2}S(O)_2NR^{c2}R^{c3}$, $-W-OS(O)_2NR^{c2}R^{c3}$, $C_{1-6}$ alkyl, $C_{2-6}$ alkenyl, $C_{2-6}$ alkynyl, $C_{1-6}$ haloalkyl, $C_{1-6}$ haloalkoxy, $C_{1-6}$ alkoxy, $C_{1-6}$ alkylthiol, $C_{3-10}$ cycloalkyl, $C_{3-10}$ cycloalkenyl, 3-12 membered heterocyclyl, $C_{6-12}$ aryl, and 5-12 membered heteroaryl; wherein each of the alkyl, the alkenyl, the alkynyl, the haloalkyl, the haloalkoxy, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-6}$ alkyl, $C_{6-14}$ aryl, $C_{1-6}$ alkoxy, $C_{1-6}$ haloalkyl, $C_{1-6}$ haloalkoxy, and halophenyl;

preferably, $R^c$ is independently selected from the group consisting of deuterium, halogen, -CN, -OH, -SH, $-NH_2$, $-W-OR^{c1}$, $-W-SR^{c1}$, $-W-C(O)R^{c4}$, $-W-C(O)OR^{c1}$ , $-W-OC(O)R^{c1}$, $-W-C(O)NR^{c2}R^{c3}$, $-W-NR^{c2}R^{c3}$, $-W-NR^{c2}C(O)R^{c4}$, $-W-S(O)_2R^{c4}$, $-W-SO_2NR^{c2}R^{c3}$, $-W-NR^{c2}S(O)_2R^{c4}$, $C_{1-4}$ alkyl, $C_{1-4}$ alkoxy, $C_{3-8}$ cycloalkyl, 4-8 membered heterocyclyl, $C_{6-12}$ aryl, and 5-8 membered heteroaryl; wherein each of the alkyl, the alkenyl, the alkynyl, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-4}$ alkyl, $C_{6-12}$ aryl, $C_{1-4}$ alkoxy, $C_{1-4}$ haloalkyl, and $C_{1-4}$ haloalkoxy;

preferably, $R^c$ is independently selected from the group consisting of deuterium, halogen, -CN, -OH, -SH, $-NH_2$, $-W-OR^{c1}$, $-W-SR^{c1}$, $-W-C(O)R^{c4}$, $-W-C(O)OR^{c1}$, $-W-OC(O)R^{c1}$, $-W-C(O)NR^{c2}R^{c3}$, $-W-NR^{c2}R^{c3}$, $-W-NR^{c2}C(O)R^{c4}$, $-W-S(O)_2R^{c4}$, $-W-SO_2NR^{c2}R^{c3}$, $-W-NR^{c2}S(O)_2R^{c4}$, $C_{1-4}$ alkyl, $C_{1-4}$ alkoxy, $C_{3-8}$ cycloalkyl, 4-8 membered heterocyclyl, $C_6$ aryl, and 5-6 membered heteroaryl; wherein each of the alkyl, the alkenyl, the alkynyl, the alkoxy, the alkylthiol, the cycloalkyl, the cycloalkenyl, the heterocyclyl, and the heteroaryl is optionally substituted by one or more substituents selected from the group consisting of deuterium, halogen, oxo, oxime, -CN, -OH, $-NO_2$, $-NH_2$, $C_{1-4}$ alkyl, $C_{6-12}$ aryl, $C_{1-4}$ alkoxy, $C_{1-4}$ haloalkyl, and $C_{1-4}$ haloalkoxy;

preferably, $R^c$ is independently selected from the group consisting of deuterium, halogen, -CN, -OH, $-W-OR^{c1}$, $-W-C(O)NR^{c2}R^{c3}$, $-W-NR^{c2}R^{c3}$, $-W-SO_2NR^{c2}R^{c3}$, $C_{1-4}$ alkyl, and 4-8 membered heterocyclyl; wherein the heterocyclyl is optionally substituted by one or more substituents selected from the group consisting of deuterium, halogen, -OH, $C_{1-4}$ alkyl$C_{1-4}$ haloalkyl, and $C_{1-4}$ haloalkoxy;

preferably, $R^c$ is independently selected from the group consisting of -F, -Cl, -Br, $-CH_2-OH$, -OH, $-C(O)NH-CH_3$, $-N(CH_3)_2$, $-SO_2CH_3$, $-SO_2N(CH_3)_2$, morpholinyl, methyl, ethyl, n-propyl, and isopropyl.

**17.** The compound, or the tautomer, the stereoisomer, or the pharmaceutically acceptable salt thereof according to any one of claims 1-16, **characterized in that**: $R^{c1}$ is, at each occurrence, independently hydrogen, deuterium, $C_{1-6}$ alkyl, $C_{1-6}$ alkoxy, $C_{3-6}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-6}$ alkyl, $C_{1-6}$ alkoxy, $C_{1-6}$ haloalkyl, and $C_{1-6}$ haloalkoxy; preferably, $R^{c1}$ is, at each occurrence, independently hydrogen, deuterium, $C_{1-4}$ alkyl, $C_{1-4}$ alkoxy, $C_{3-6}$ cycloalkyl, $C_{6-10}$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, cyano, hydroxyl, amino, $C_{1-4}$ alkyl, $C_{1-4}$ alkoxy, $C_{1-4}$ haloalkyl, and $C_{1-4}$ haloalkoxy; preferably, $R^{c1}$ is, at each occurrence, independently hydrogen, deuterium, $C_{1-4}$ alkyl, $C_{1-4}$ alkoxy, $C_{3-6}$ cycloalkyl, $C_6$ aryl, or 5-6 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino,

$C_{1-4}$ alkyl, $C_{1-4}$ alkoxy, $C_{1-4}$ haloalkyl, and $C_{1-4}$ haloalkoxy; preferably, $R^{c1}$ is, at each occurrence, independently hydrogen, deuterium, methyl, ethyl, propyl, methoxy, ethoxy or propoxy, wherein methyl, ethyl, propyl, methoxy, ethoxy or propoxy is optionally substituted by one or more substituents selected from the group consisting of halogen, deuterium, hydroxyl, amino, and $C_{1-4}$ alkyl; preferably, $R^{c1}$ is, at each occurrence, independently hydrogen, methyl, ethyl, or propyl, wherein methyl, ethyl, or propyl is optionally substituted by one or more substituents selected from the group consisting of halogen, deuterium, hydroxyl, amino, and $C_{1-4}$ alkyl.

**18.** The compound, or the tautomer, the stereoisomer, or the pharmaceutically acceptable salt thereof according to any one of claims 1-17, **characterized in that** $R^{c4}$ is, at each occurrence, independently hydrogen, deuterium, $C_{1-4}$ alkyl, $C_{1-4}$ alkoxy, $C_{3-8}$ cycloalkyl, $C_{6-10}$ aryl, or 3-10 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-6}$ alkyl, $C_{1-6}$ alkoxy, phenyl, $C_{1-6}$ haloalkyl, $C_{1-6}$ haloalkoxy, and halophenyl; preferably, $R^{c4}$ is, at each occurrence, independently hydrogen, deuterium, $C_{1-4}$ alkyl, $C_{1-4}$ alkoxy, $C_{3-6}$ cycloalkyl, $C_6$ aryl, or 3-8 membered heterocyclyl, wherein each of the alkyl, the alkoxy, the cycloalkyl, the aryl, and the heterocyclyl is optionally substituted by one or more substituents selected from the group consisting of halogen, deuterium, hydroxyl, amino, $C_{1-4}$ alkyl, $C_{1-4}$ alkoxy, $C_{1-4}$ haloalkyl, and $C_{1-4}$ haloalkoxy; preferably, $R^{c4}$ is, at each occurrence, independently hydrogen, deuterium, $C_{1-4}$ alkyl, $C_{1-4}$ alkoxy, $C_{3-6}$ cycloalkyl, or $C_6$ aryl, wherein each of the alkyl, the alkoxy, the cycloalkyl, and the aryl is optionally substituted by one or more substituents selected from the group consisting of halogen, deuterium, hydroxyl, amino, $C_{1-4}$ alkyl, and $C_{1-4}$ alkoxy; preferably, $R^{c4}$ is, at each occurrence, independently hydrogen, deuterium, methyl, ethyl, propyl, methoxy, ethoxy, propoxy, cyclopropanyl, cyclobutanyl or phenyl; wherein the methyl, ethyl, propyl, methoxy, ethoxy, propoxy, cyclopropanyl, cyclobutanyl or phenyl is optionally substituted by one or more substituents selected from the group consisting of halogen, deuterium, hydroxyl, amino, $C_{1-4}$ alkyl, and $C_{1-4}$ alkoxy; preferably, $R^{c4}$ is, at each occurrence, independently hydrogen, deuterium, methyl, ethyl, propyl, methoxy, ethoxy or propoxy; wherein the methyl, ethyl, propyl, methoxy, ethoxy, or propoxy is optionally substituted by one or more substituents selected from the group consisting of halogen, deuterium, hydroxyl, amino, $C_{1-4}$ alkyl, and $C_{1-4}$ alkoxy.

**19.** The compound, or the tautomer, the stereoisomer, or the pharmaceutically acceptable salt thereof according to any one of claims 1-18, **characterized in that**: W is selected from the group consisting of bond, and $C_{1-3}$ alkylene, wherein the alkylene is optionally substituted by one or more substituents selected from the group consisting of deuterium, halogen, oxo, -CN, -OH, and - $NH_2$; preferably, W is selected from the group consisting of bond, and $C_{1-3}$ alkylene, wherein the alkylene is optionally substituted by one or more substituents selected from the group consisting of deuterium, halogen, oxo, -CN, -OH, and -$NH_2$; preferably, W is selected from the group consisting of bond, methylene and ethylene, wherein the methylene or ethylene is optionally substituted by one or more substituents selected from the group consisting of deuterium, halogen, oxo, -CN, -OH, and -$NH_2$.

**20.** An atropisomer of a compound represented by formula (III-a), (III-b), (III-c), (III-d) or (III-e), or a pharmaceutically acceptable salt of the atropisomer thereof:

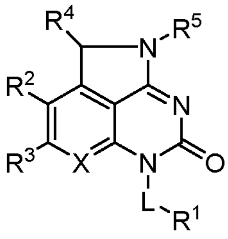
(III-a)

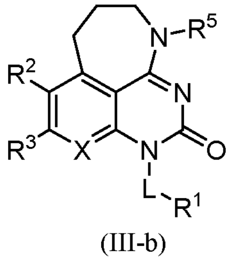
(III-b)

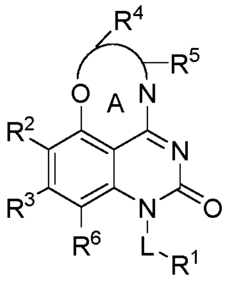
(III-c)

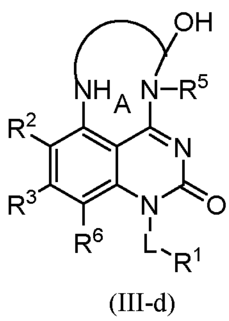
(III-d)

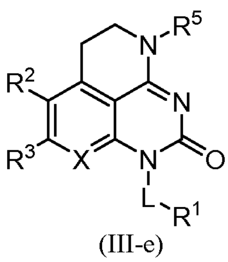
(III-e)

wherein $R^1$, $R^2$, $R^3$, $R^4$, $R^5$, $R^6$ and X are as defined in the compound represented by formula (I) in any one of claims 1-18, wherein L is bond, and $R^1$ is at least substituted by one $R^a$.

**21.** The atropisomer of the compound represented by formula (III-a), (III-b), (III-c), (III-d) or (III-e), or the pharmaceutically acceptable salt of the atropisomer thereof according to claim 20, preferably, $R^1$ is selected from pyridinyl optionally substituted by one or more $R^a$; preferably, $R^a$ is, at each occurrence, independently selected from the group consisting of Cl, methyl, and methoxy; further preferably, $R^1$ is selected from the group consisting of

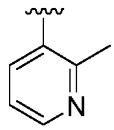 , 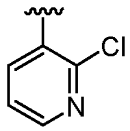

and

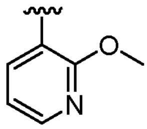 .

**22.** A compound, which is selected from the group consisting of the following compounds, and a tautomer, a stereoisomer, an atropisomer and a pharmaceutically acceptable salt thereof:

| Structural formula and number of compound | Structural formula and number of compound | Structural formula and number of compound |
|---|---|---|
| 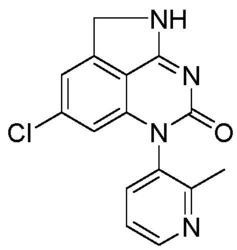 A1 | 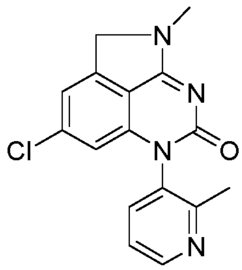 A2 | 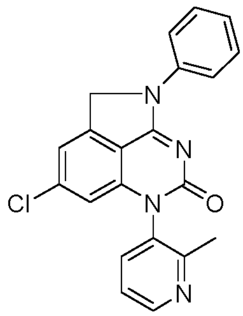 A3 |
| 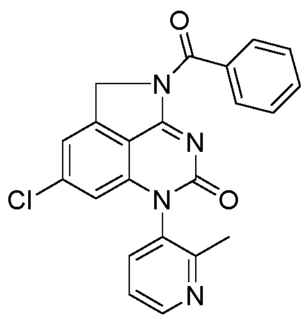 A4 | 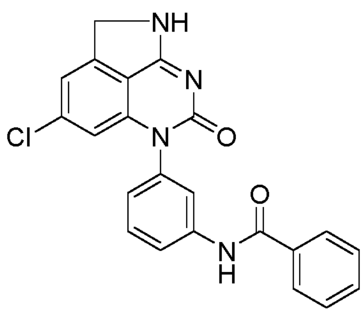 A5 | 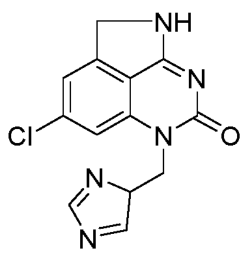 A6 |
| 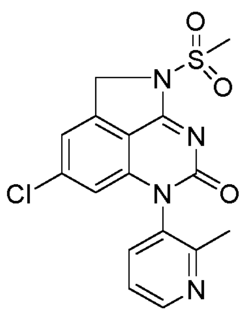 A7 | 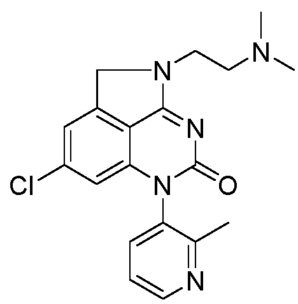 A8 | 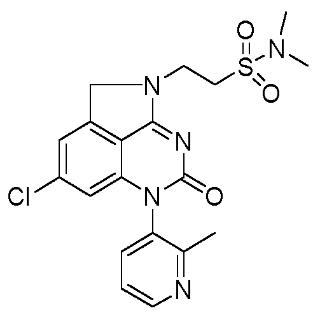 A9 |

(continued)

| Structural formula and number of compound | Structural formula and number of compound | Structural formula and number of compound |
|---|---|---|
| 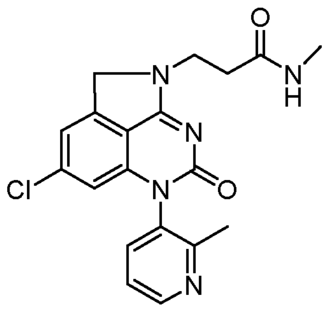 A10 | 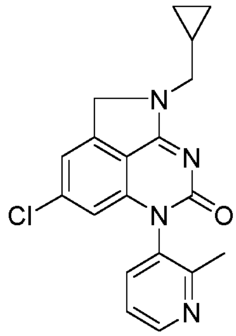 A11 | 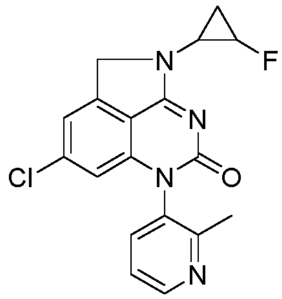 A12 |
| 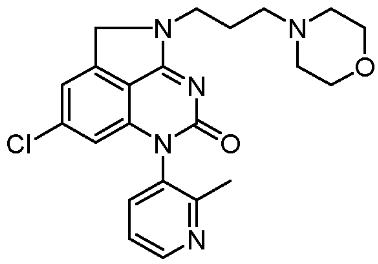 A13 | 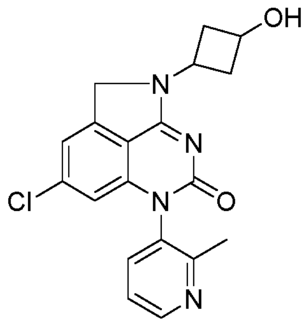 A14 | 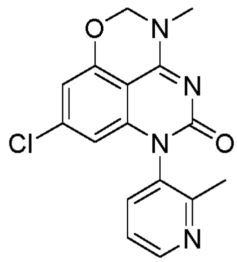 A15 |
| 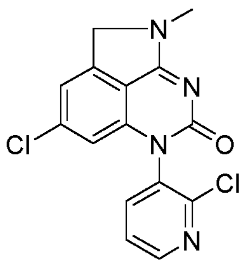 A16 | 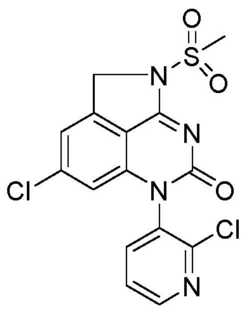 A17 | 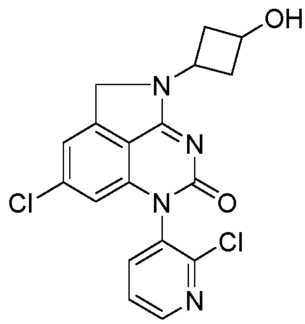 A18 |
| 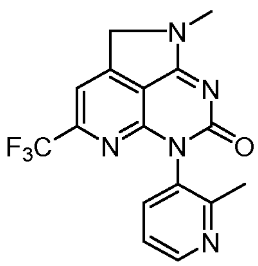 A19 | 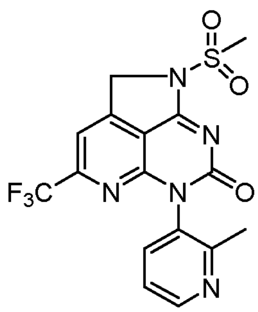 A20 | 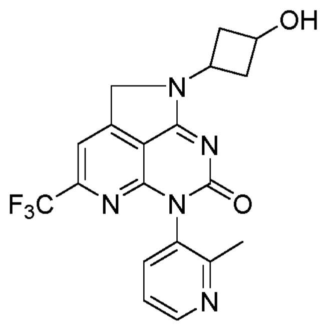 A21 |

(continued)

| Structural formula and number of compound | Structural formula and number of compound | Structural formula and number of compound |
|---|---|---|
| 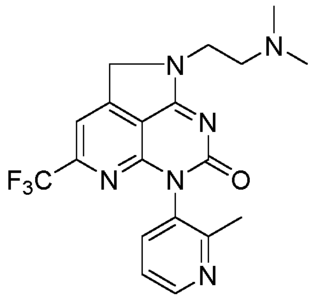 A22 | 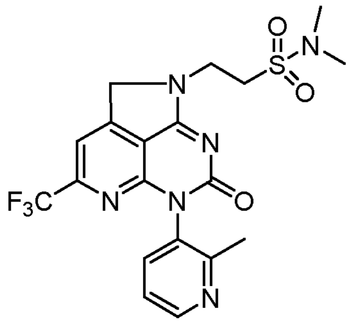 A23 | 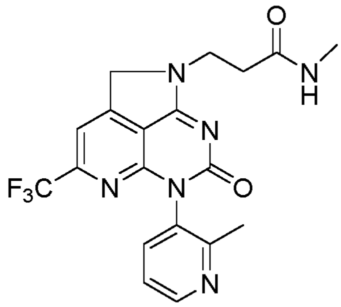 A24 |
| 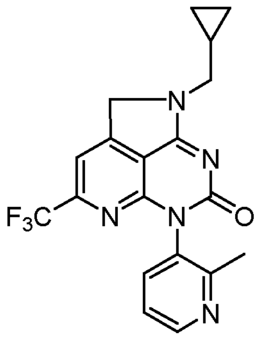 A25 | 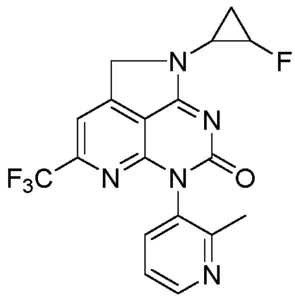 A26 | 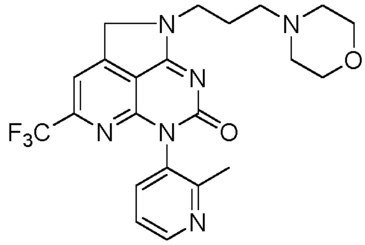 A27 |
| 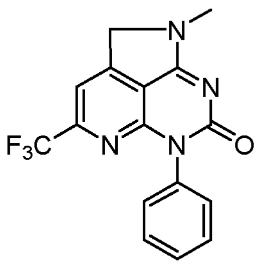 A28 | 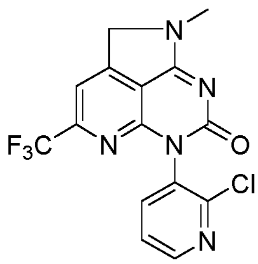 A29 | 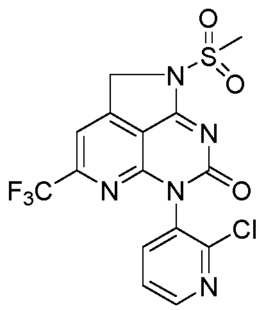 A30 |
| 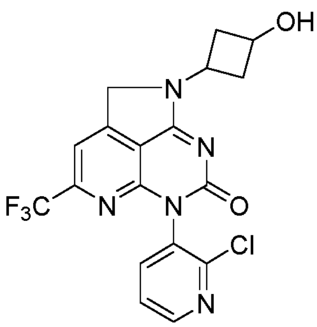 A31 | 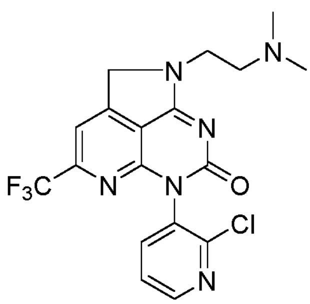 A32 | 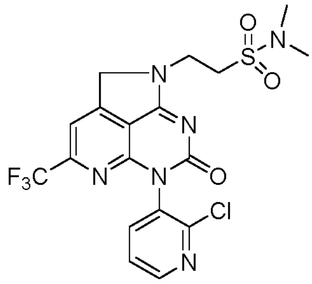 A33 |

(continued)

| Structural formula and number of compound | Structural formula and number of compound | Structural formula and number of compound |
|---|---|---|
| 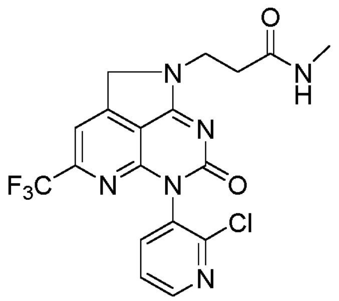 A34 | 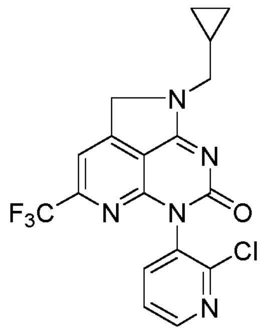 A35 | 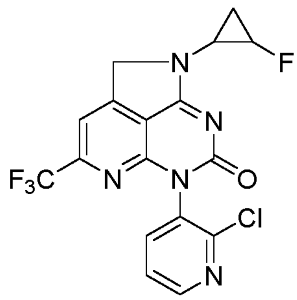 A36 |
| 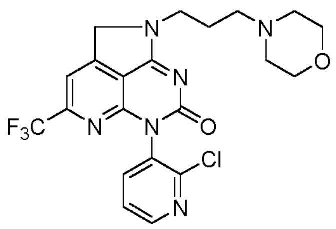 A37 | 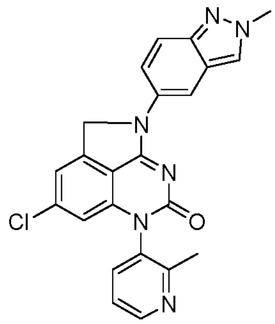 A38 | 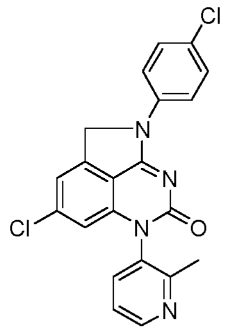 A39 |
| 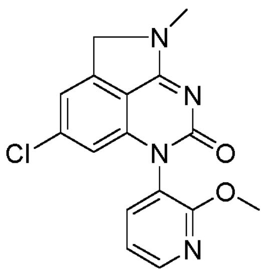 A40 | 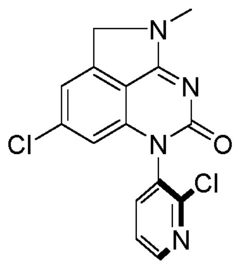 | 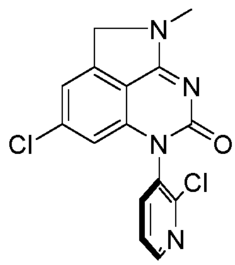 |
| 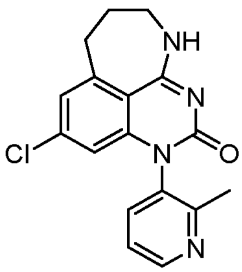 B1 | 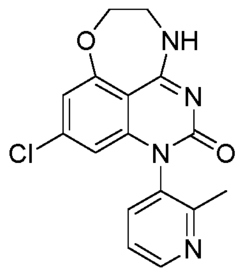 B2 | 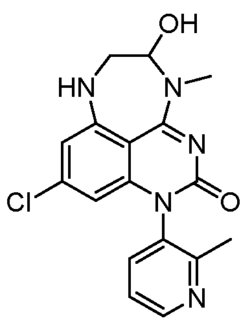 B3 |

(continued)

| Structural formula and number of compound | Structural formula and number of compound | Structural formula and number of compound |
|---|---|---|
| 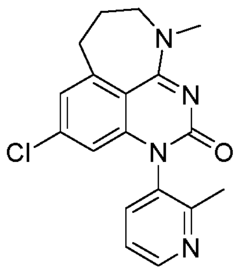 **B4** | 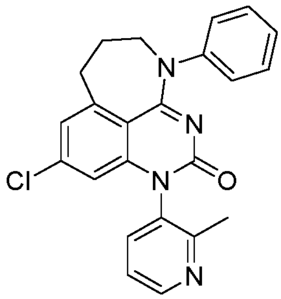 **B5** | 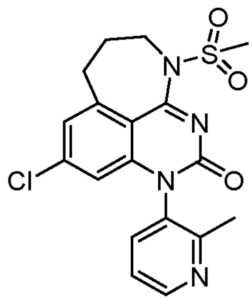 **B6** |
| 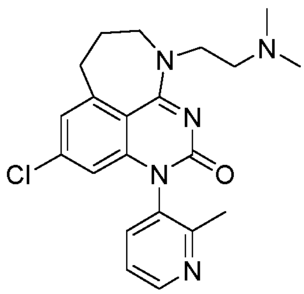 **B7** | 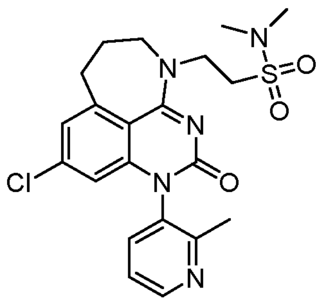 **B8** | 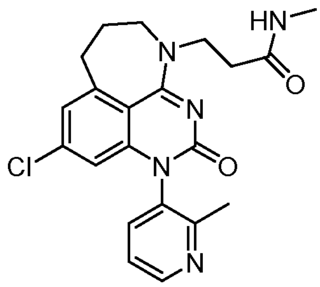 **B9** |
| 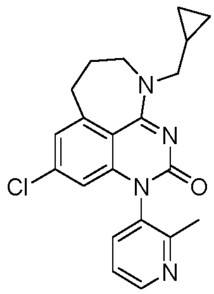 **B10** | 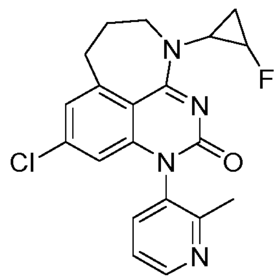 **B11** | 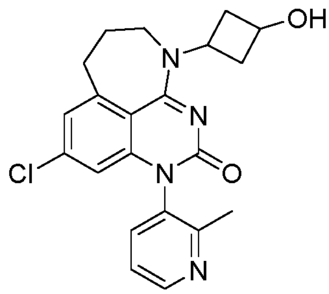 **B12** |
| 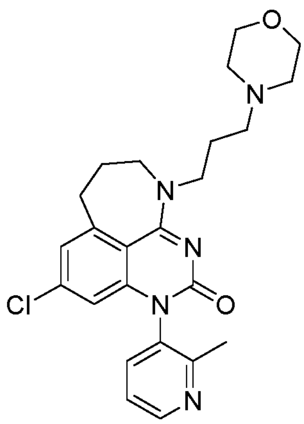 **B13** | 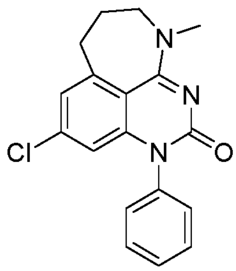 **B14** | 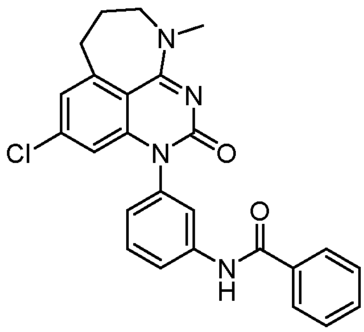 **B15** |

(continued)

| Structural formula and number of compound | Structural formula and number of compound | Structural formula and number of compound |
|---|---|---|
| 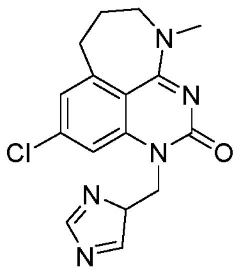 B16 | 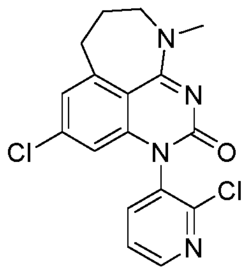 B17 | 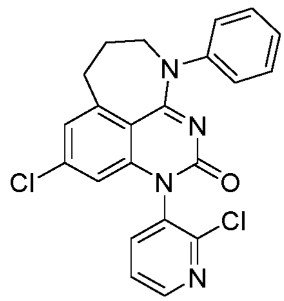 B18 |
| 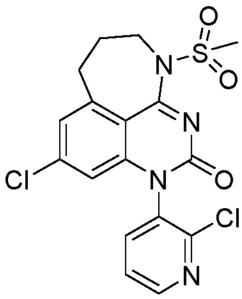 B19 | 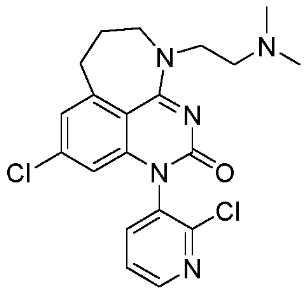 B20 | 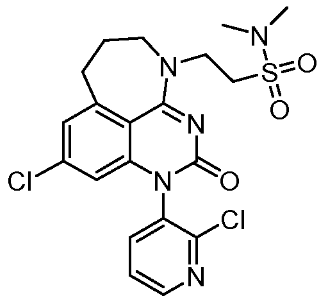 B21 |
| 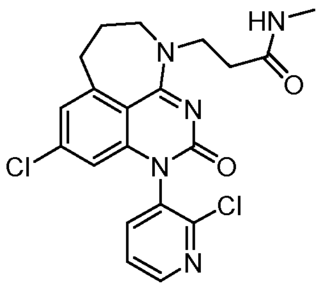 B22 | 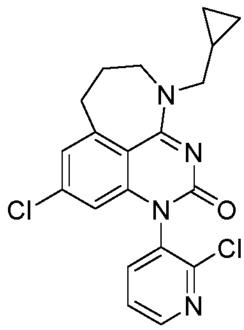 B23 | 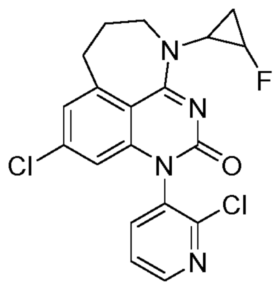 B24 |
| 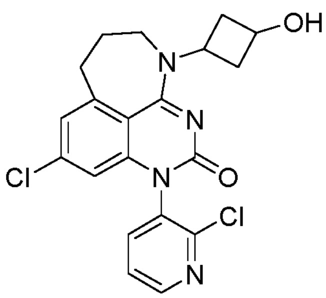 B25 | 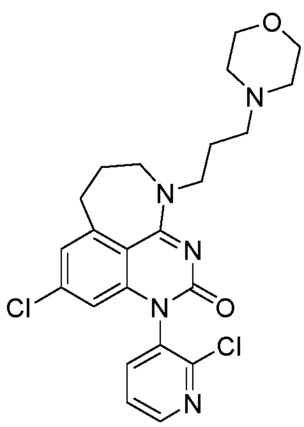 B26 | 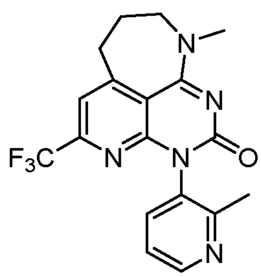 B27 |

(continued)

| Structural formula and number of compound | Structural formula and number of compound | Structural formula and number of compound |
|---|---|---|
| 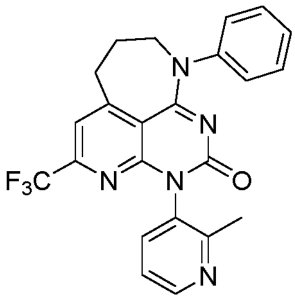 **B28** | 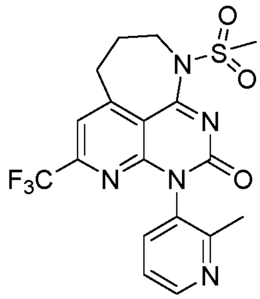 **B29** | 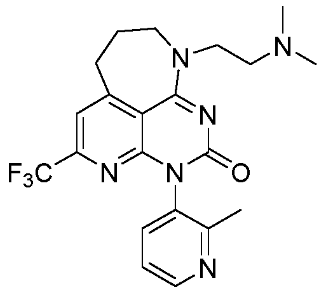 **B30** |
| 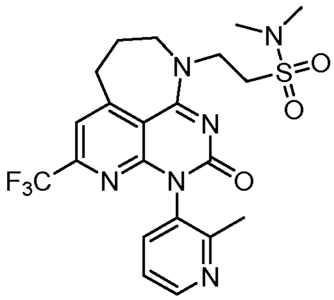 **B31** | 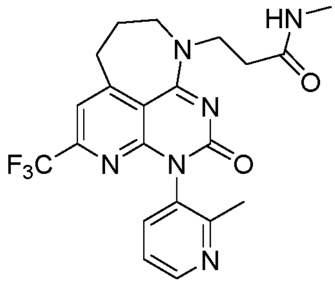 **B32** | 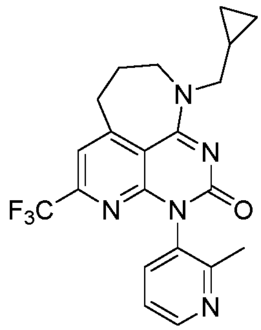 **B33** |
| 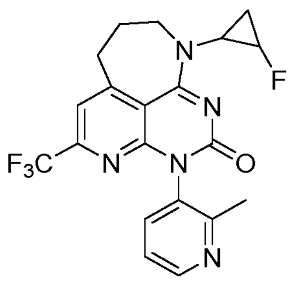 **B34** | 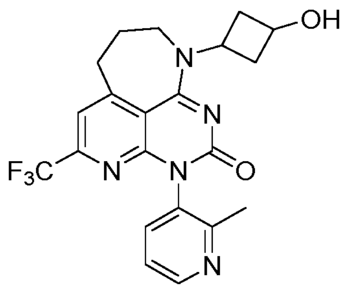 **B35** | 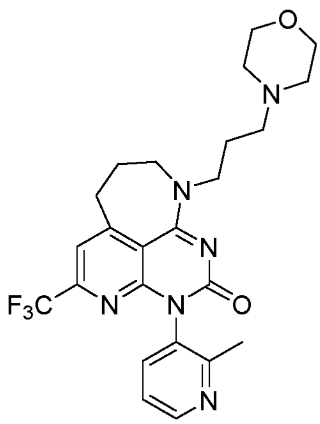 **B36** |
| 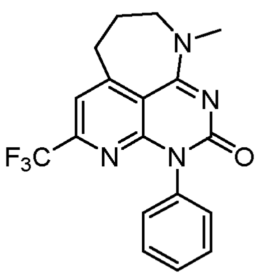 **B37** | 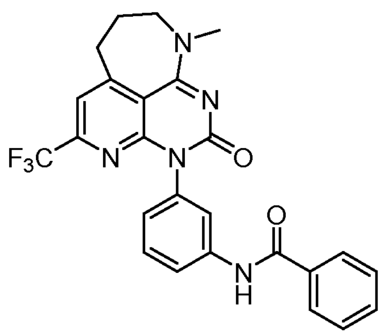 **B38** | 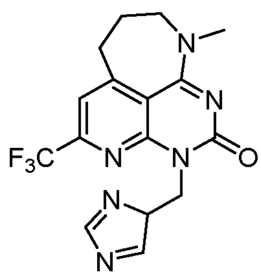 **B39** |

(continued)

| Structural formula and number of compound | Structural formula and number of compound | Structural formula and number of compound |
|---|---|---|
| 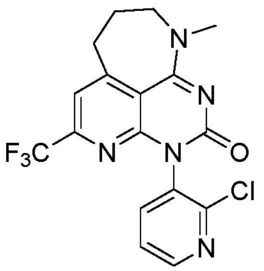 **B40** | 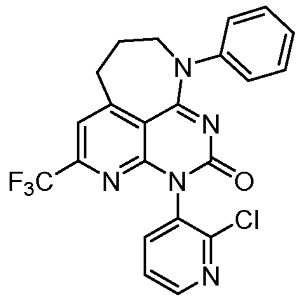 **B41** | 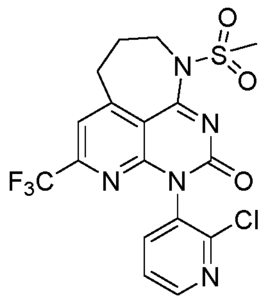 **B42** |
| 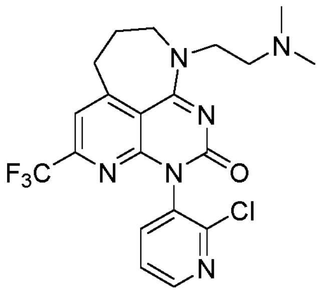 **B43** | 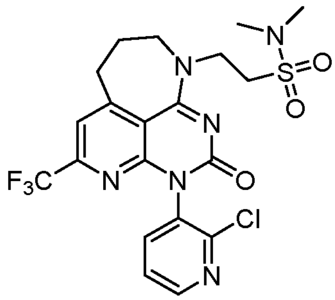 **B44** | 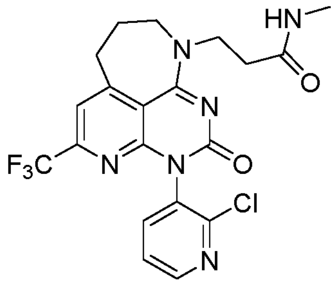 **B45** |
| 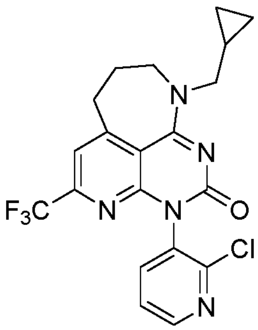 **B46** | 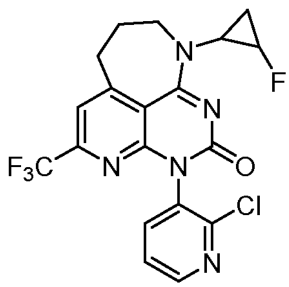 **B47** | 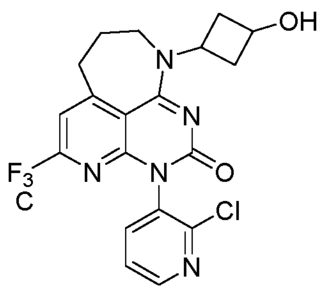 **B48** |
| 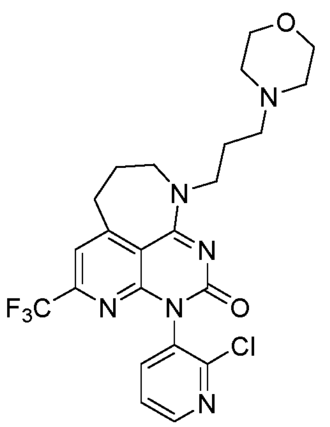 **B49** | 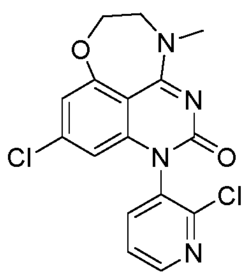 **B50** | 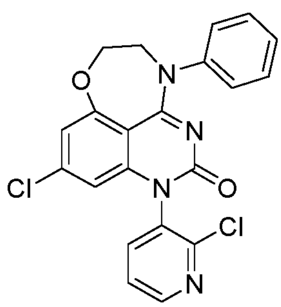 **B51** |

(continued)

| Structural formula and number of compound | Structural formula and number of compound | Structural formula and number of compound |
|---|---|---|
| 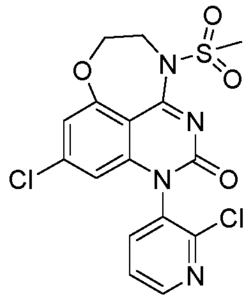 B52 | 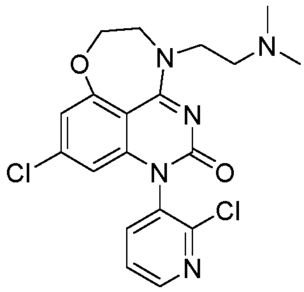 B53 | 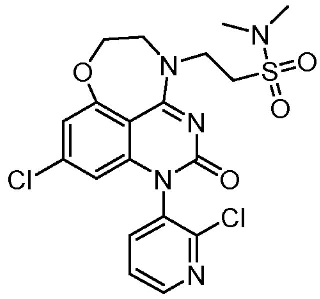 B54 |
| 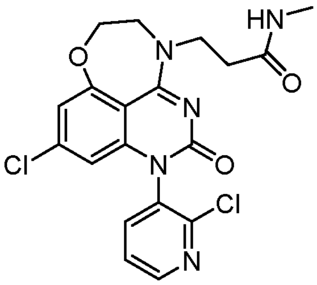 B55 | 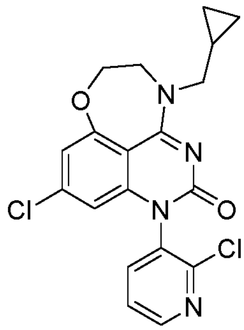 B56 | 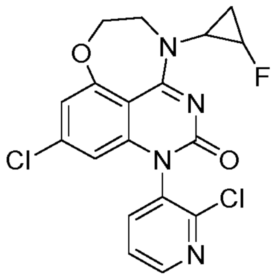 B57 |
| 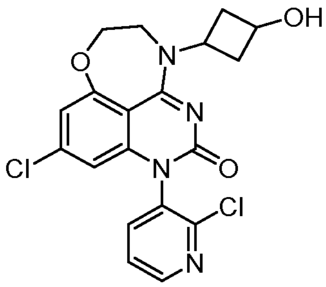 B58 | 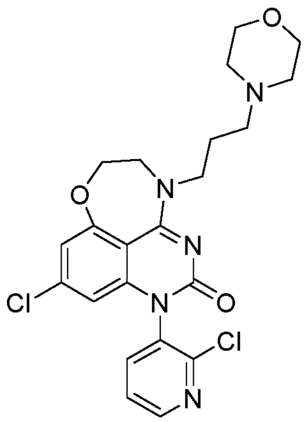 B59 | 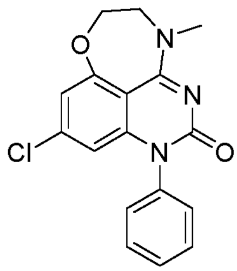 B60 |
| 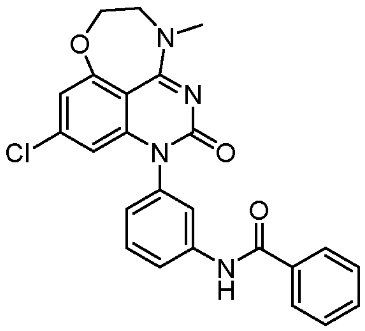 B61 | 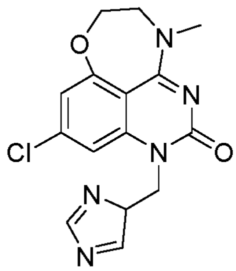 B62 | 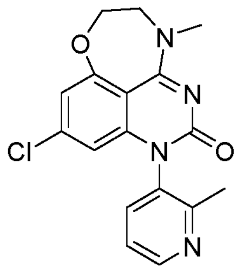 B63 |

(continued)

| Structural formula and number of compound | Structural formula and number of compound | Structural formula and number of compound |
|---|---|---|
| 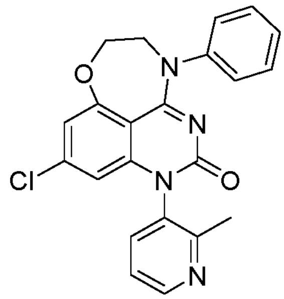 B64 | 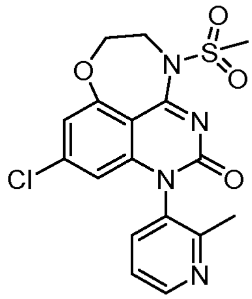 B65 | 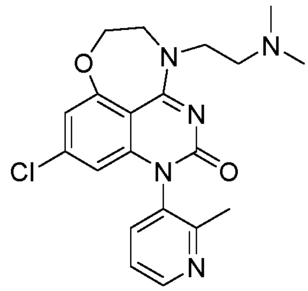 B66 |
| 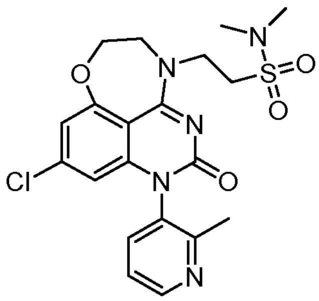 B67 | 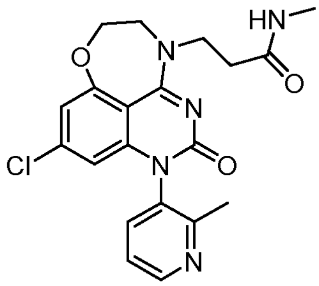 B68 | 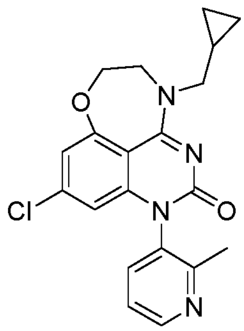 B69 |
| 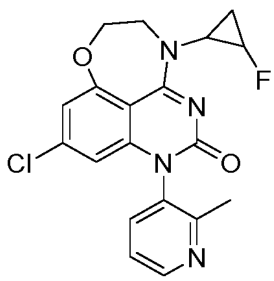 B70 | 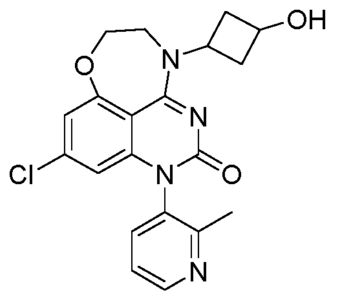 B71 | 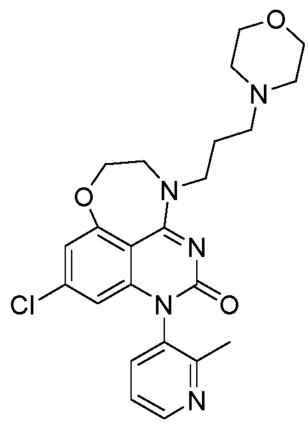 B72 |
| 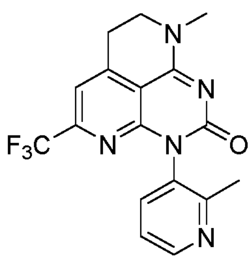 C1 | 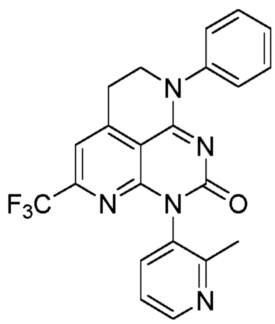 C2 | 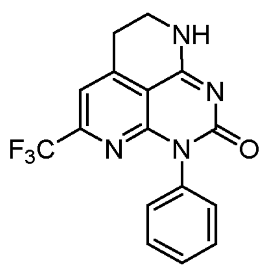 C3 |

(continued)

| Structural formula and number of compound | Structural formula and number of compound | Structural formula and number of compound |
|---|---|---|
| 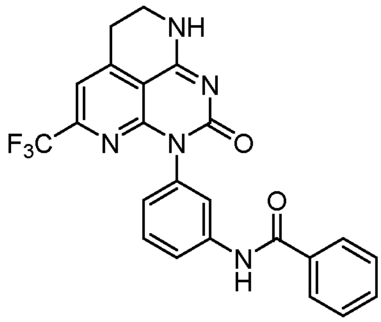 C4 | 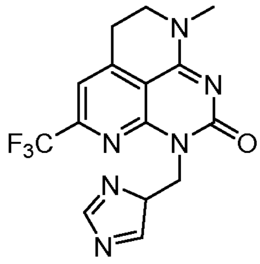 C5 | 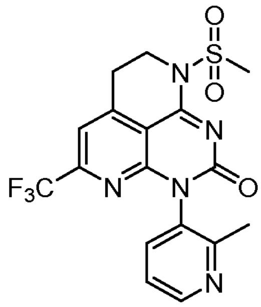 C6 |
| 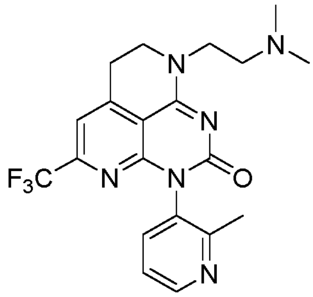 C7 | 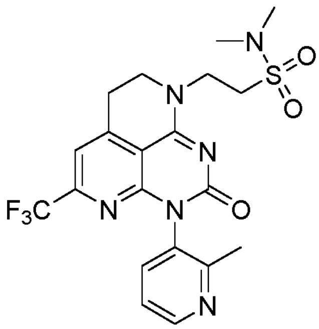 C8 | 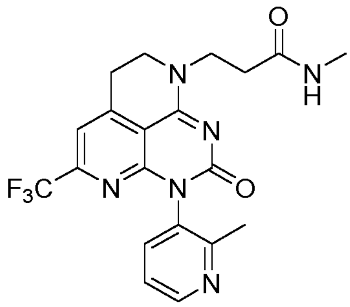 C9 |
| 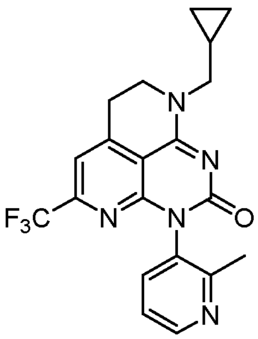 C10 | 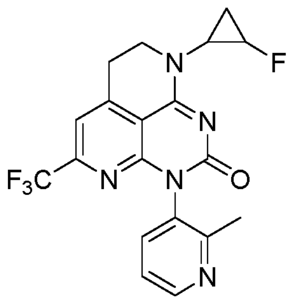 C11 | 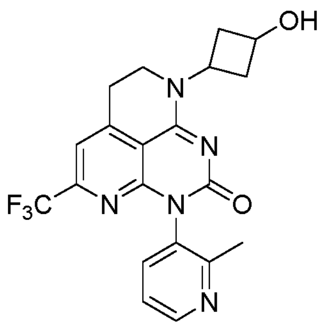 C12 |
| 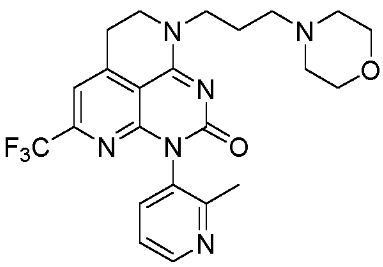 C13 | 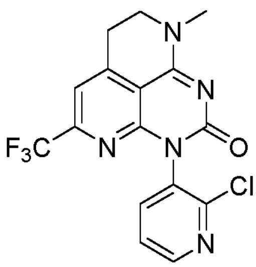 C14 | 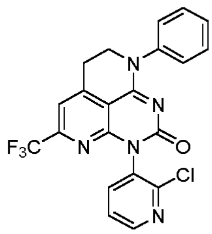 C15 |

(continued)

| Structural formula and number of compound | Structural formula and number of compound | Structural formula and number of compound |
|---|---|---|
| 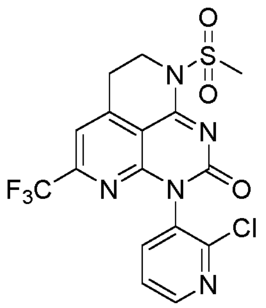 C16 | 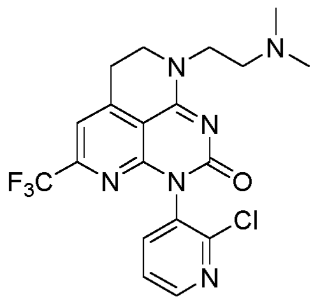 C17 | 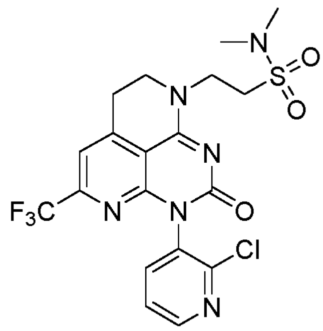 C18 |
| 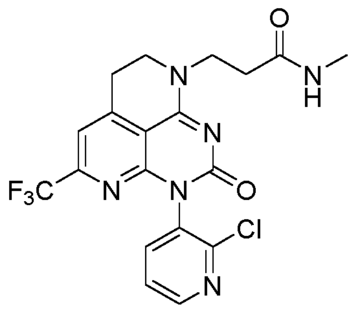 C19 | 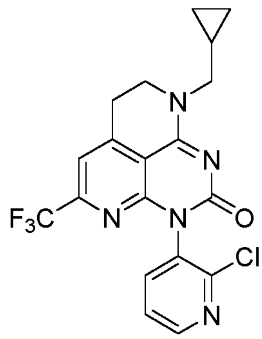 C20 | 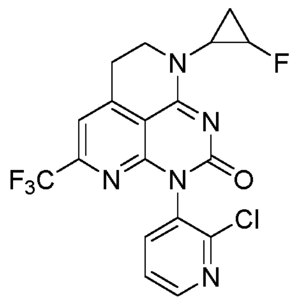 C21 |
| 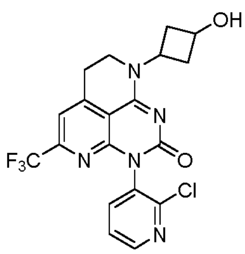 C22 | 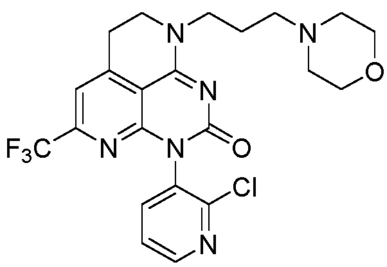 C23 | |

**23.** A pharmaceutical composition comprising the compound, or the tautomer, the stereoisomer, or the pharmaceutically acceptable salt thereof of any one of claims 1-22, and optionally a pharmaceutically acceptable excipient.

**24.** A use of the compound, or the tautomer, the stereoisomer, or the pharmaceutically acceptable salt thereof according to any one of claims 1-22 and the pharmaceutical composition according to claim 23 in the preparation of a medicament for treating and/or preventing a disease, disorder and condition mediated by MAT2A, and preferably, the disease, disorder and condition is a tumor with MTAP deletion, and further preferably, the tumor includes a solid tumor and a hematological malignancy; and the solid tumor includes a colorectal cancer.

**25.** A use of the compound, or the tautomer, the stereoisomer, or the pharmaceutically acceptable salt thereof according to any one of claims 1-22 and the pharmaceutical composition according to claim 23 in the preparation of a medicament for treating and/or preventing a tumor.

**26.** A pharmaceutical composition comprising the compound, or the tautomer, the stereoisomer, or the pharmaceutically acceptable salt thereof of any one of claims 1-22 or the pharmaceutical composition according to claim 23, and additional one, two or more drugs with tumor-inhibitory activity.

## INTERNATIONAL SEARCH REPORT

International application No.
**PCT/CN2023/102500**

**A. CLASSIFICATION OF SUBJECT MATTER**

C07D487/06(2006.01)i; A61K31/519(2006.01)i; A61P35/00(2006.01)i

According to International Patent Classification (IPC) or to both national classification and IPC

**B. FIELDS SEARCHED**

Minimum documentation searched (classification system followed by classification symbols)

C07D487/-; A61K31/-; A61P35/-

Documentation searched other than minimum documentation to the extent that such documents are included in the fields searched

Electronic data base consulted during the international search (name of data base and, where practicable, search terms used)

CNTXT; VEN; ENTXT; WOTXT; USTXT; EPTXT; GBTXT; JPTXT; CNMED; MEDNPL; CNKI; 万方, WANFANG; 百度学术, BAIDU SCHOLAR; 读秀, DUXIU; STN; Web of Science: 三环; 甲硫氨酸转移酶; 甲硫基腺苷; 腺苷甲硫氨酸; 肿瘤; 癌; 石药集团; 王矿磊; 郭见桥; 赵传武; 褚文浩; 张晓琳; 张雪娇; 郭文敏; 耿佳; 刘咏梅; MAT2A; MTAP; Methionine adenosyltransferase 2A; S-Adenosylmethionine Synthase Isoform Type; tumor; cancer; CSPC ZHONGQI PHARMACEUTICAL TECHNOLOGY; 式(I-III).

**C. DOCUMENTS CONSIDERED TO BE RELEVANT**

| Category* | Citation of document, with indication, where appropriate, of the relevant passages | Relevant to claim No. |
|---|---|---|
| X | WO 2020221239 A1 (GENFLEET THERAPEUTICS SHANGHAI INC. et al.) 05 November 2020 (2020-11-05)<br>claims 1-29, description, pages 20, 21, and 24, and page 33, lines 1-2 and 28-32, and embodiment 35 | 1-26 |
| X | CN 113980032 A (JIANGSU HENGRUI PHARMACEUTICALS CO., LTD. et al.) 28 January 2022 (2022-01-28)<br>claims 1-8, and description, paragraphs 65, 71, 106, and 143-145 | 1-26 |
| A | WO 2021259815 A1 (HOFFMANN LA ROCHE et al.) 30 December 2021 (2021-12-30)<br>entire document | 1-26 |
| A | CN 112300195 A (SHANGHAI JEMINCARE PHARMACEUTICALS CO., LTD. et al.) 02 February 2021 (2021-02-02)<br>entire document | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 September 2023** | **26 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/102500**

**C. DOCUMENTS CONSIDERED TO BE RELEVANT**

| Category* | Citation of document, with indication, where appropriate, of the relevant passages | Relevant to claim No. |
|---|---|---|
| T | WO 2023122140 A1 (SYNNOVATION THERAPEUTICS INC.) 29 June 2023 (2023-06-29) entire document | 1-26 |

Form PCT/ISA/210 (second sheet) (July 2022)

**INTERNATIONAL SEARCH REPORT**
**Information on patent family members**

International application No.
**PCT/CN2023/102500**

| Patent document cited in search report | Publication date (day/month/year) | Patent family member(s) | Publication date (day/month/year) |
|---|---|---|---|
| WO 2020221239 A1 | 05 November 2020 | JP 2022531196 A | 06 July 2022 |
| | | JP 7280975 B2 | 24 May 2023 |
| | | US 2022251109 A1 | 11 August 2022 |
| | | EP 3964516 A1 | 09 March 2022 |
| | | EP 3964516 A4 | 11 January 2023 |
| | | KR 20220012255 A | 03 February 2022 |
| CN 113980032 A | 28 January 2022 | None | |
| WO 2021259815 A1 | 30 December 2021 | IL 297879 A | 01 January 2023 |
| | | TW 202216707 A | 01 May 2022 |
| | | US 2023227449 A1 | 20 July 2023 |
| | | KR 20230027042 A | 27 February 2023 |
| | | CA 3181790 A1 | 30 December 2021 |
| | | CR 20220638 A | 31 January 2023 |
| | | CO 2023000056 A2 | 27 March 2023 |
| | | JP 2023531021 A | 20 July 2023 |
| | | EP 4168394 A1 | 26 April 2023 |
| | | AU 2021295413 A1 | 01 December 2022 |
| | | AR 122704 A1 | 28 September 2022 |
| | | BR 112022026080 A2 | 17 January 2023 |
| CN 112300195 A | 02 February 2021 | AU 2020325477 A1 | 17 March 2022 |
| | | EP 4011886 A1 | 15 June 2022 |
| | | EP 4011886 A4 | 26 July 2023 |
| | | JP 2022543767 A | 14 October 2022 |
| | | IL 290276 A | 01 April 2022 |
| | | WO 2021023154 A1 | 11 February 2021 |
| | | US 2022298174 A1 | 22 September 2022 |
| | | KR 20220079521 A | 13 June 2022 |
| | | CA 3149403 A1 | 11 February 2021 |
| WO 2023122140 A1 | 29 June 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210734328
- CN 202211120467
- CN 202310135765
- WO 2018039972 A
- WO 2019191470 A
- WO 2020139991 A
- WO 2020139992 A
- WO 2020243376 A
- WO 2020123395 A


### Non-patent literature cited in the description


- *Org. Process Res. Dev.*, 2011, vol. 15, 788-796